# EUROPEAN PATENT APPLICATION

(11) **EP 4 029 501 A1**
(43) Date of publication of application: **20.07.2022**
(21) Application number: 21305057.8
(22) Date of filing: 19.01.2021
(51) Int. Cl.: A61K 31/415, A61K 31/437, A61K 31/519, A61P 21/00

(54) **COMBINATION OF POLYAROMATIC UREA DERIVATIVES AND GLUCOCORTICOID OR HDAC INHIBITOR FOR THE TREATMENT OF DISEASES OR CONDITIONS ASSOCIATED WITH MUSCLE CELLS AND/OR SATELLITE CELLS**

(71) Applicant: Anagenesis Biotechnologies, 67400 Illkirch-Graffenstaden (FR)
(72) Inventor: Bonnefoy, Jean-Yves, 67600 Ebersmunster (FR); Riguet, Eric, 31150 Bruguières (FR)
(74) Representative: Cabinet Becker et Associés

(57) **Abstract**

The current invention provides compounds for treating, ameliorating, delaying, curing and/or preventing a disease or condition associated with muscle cells and/or satellite cells, such as Duchenne muscular dystrophy, Becker muscular dystrophy, cachexia or sarcopenia, in combination with a corticosteroid or a HDAC inhibitor.

## Description

### Field

The invention relates to the field of compounds for the treatment of diseases or conditions associated with muscle cells and/or satellite cells.

### Background of the invention

Diseases and conditions associated with muscle cells have a wide range of underlying causes and symptoms. The most common examples are inflammatory myopathies, muscular dystrophies, metabolic myopathies, myopathies associated with systemic disorders, drug-induced myopathies, cachexia and sarcopenia. In these diseases the muscle cells have a reduced biological functionality relative to muscle cells of healthy individuals, or have been degenerated.

As an example, Duchenne muscular dystrophy is a severe type of muscular dystrophy caused by a mutation in the dystrophin gene, resulting in muscle cells with a reduced biological functionality, leading to progressive muscle weakness and degeneration. As another example, sarcopenia is the loss of skeletal muscle mass due to aging.

Although diseases and conditions associated with muscle cells often have different underlying causes and biological mechanisms, a reduced number of functional satellite cells, relative to a healthy individual, have been associated with several of these diseases and conditions. Satellite cells are small multipotent cells, present in muscle tissue, characterized by their location under the basal lamina and by the expression of paired box 7 (Pax7) protein, which are precursors of skeletal muscle cells. Although these cells are quiescent under normal physiological conditions, they are activated in response to trauma and therefore play an important role in muscle repair and regeneration.

For a lot of diseases and conditions associated with muscle cells, including Duchenne muscular dystrophy and sarcopenia, there are no approved medicaments, and treatment is mostly supportive. Hence, there is a continuing need in the art for compounds which may be used in treating, ameliorating, delaying, curing and/or preventing a disease or condition associated with muscle cells and/or satellite cells.

Patent Publications WO2002032872, WO2003072569, WO2006043090, WO2006071940, WO2006081034, WO2006105844, WO2007059202, WO2007064872, WO2008046003, WO2008079972, WO2008131276, US20080113967, WO2009077766, US20090012091, WO2010067130, WO2010067131, WO2010112936, WO2011158042, WO2011070369, WO2011092469, WO2011121366, WO2011124923, WO2011124930, WO2011158039, WO2011158042, WO2011158044, WO2012019015, US20120046290, US20120129893, US20120225057, WO2013036232, WO2013050757, WO2014015056, WO2015075483, US20160015697, WO2018137610, WO2018215668, WO2019084499, WO2019232275 describe polyaromatic urea derivatives primarily as antitumor agents, anti-inflammatory agents, antiviral agents, respiratory system agents and for diabetes mellitus.

None of the specifically disclosed compounds from the above Patent Publications are included in the present invention.

### Summary

The present invention provides new compounds promoting muscle progenitor differentiation, in particular improved promoting capacities, but with the ability to not deplete the pool of satellite cells, e.g., to preserve or even increase it. More particularly, these new compounds show an increased activity and efficacy when combined with a corticosteroid or a HDAC inhibitor, even with a reduced toxicity of Pax7 positive cells.

Therefore, the present invention relates to one of these compounds or a pharmaceutical composition comprising it for use in treating, ameliorating, delaying, curing and/or preventing a disease or condition associated with muscle cells and/or satellite cells, in combination with a corticosteroid or a HDAC inhibitor, a pharmaceutical composition comprising one of these compounds and a corticosteroid or a HDAC inhibitor, and a pharmaceutical composition comprising one of these compounds and a corticosteroid or a HDAC inhibitor for use in treating, ameliorating, delaying, curing and/or preventing a disease or condition associated with muscle cells and/or satellite cells.

The present invention further relates to the use of a compound according to the present invention for the manufacture of a medicament for treating, ameliorating, delaying, curing and/or preventing a disease or condition associated with muscle cells and/or satellite cells in combination with a corticosteroid or a HDAC inhibitor; or to the use of a pharmaceutical composition comprising a compound according to the present invention and a corticosteroid or a HDAC inhibitor for the manufacture of a medicament for treating, ameliorating, delaying, curing and/or preventing a disease or condition associated with muscle cells and/or satellite cells. In addition, it relates to a method of treating, ameliorating, delaying, curing and/or preventing a disease or condition associated with muscle cells and/or satellite cells in a subject in need thereof, comprising administering a therapeutic amount of a compound according to the present invention and administering a therapeutic amount of a corticosteroid or a HDAC inhibitor. Alternatively, it relates to a method of treating, ameliorating, delaying, curing and/or preventing a disease or condition associated with muscle cells and/or satellite cells in a subject in need thereof, comprising administering a therapeutic amount of a pharmaceutical composition comprising a compound according to the present invention and a corticosteroid or a HDAC inhibitor.

More particularly, the disease or condition is selected from the group consisting of Duchenne muscular dystrophy, Becker muscular dystrophy, sarcopenia and cachexia, preferably selected from the group consisting of Duchenne muscular dystrophy, Becker muscular dystrophy, and sarcopenia. In a particular aspect, the disease or condition is a muscular dystrophy such as Duchenne muscular dystrophy or Becker muscular dystrophy.

The present invention relates to a compound for use in treating, ameliorating, delaying, curing and/or preventing a disease or condition associated with muscle cells and/or satellite cells, or a pharmaceutically acceptable salt thereof in combination with a corticosteroid or a histone deacetylase (HDAC) inhibitor, wherein said compound is represented by structure (I), 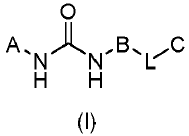 wherein
L is -O-
and
1)
   A is a ring system selected from the group consisting of 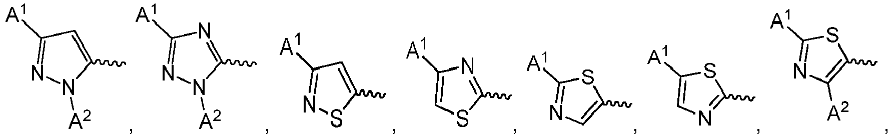 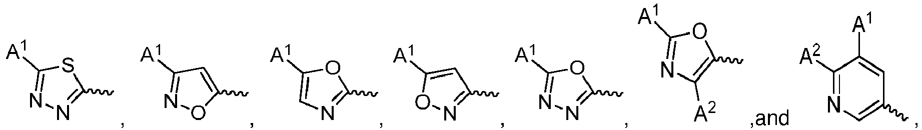 wherein A¹ is selected from the group consisting of C₁-C₄ alkyl, C₁-C₄ cycloalkyl, 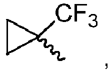 C3-C7 heterocycloalkyl, 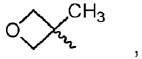 phenyl, benzyl and 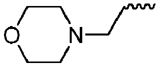 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 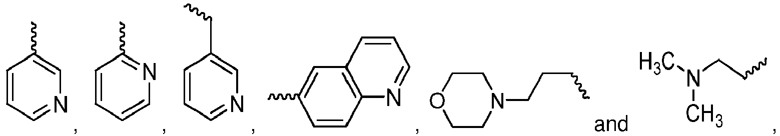 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 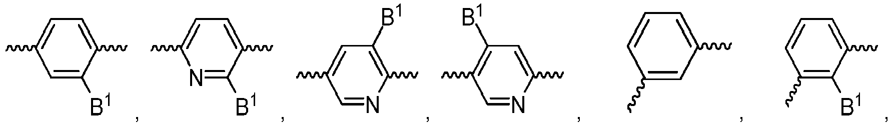 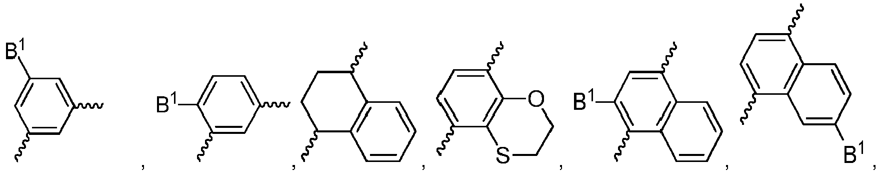 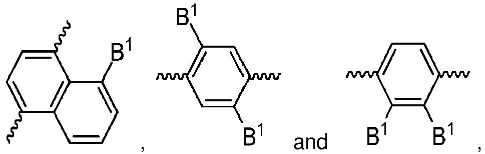 wherein B¹ is selected from the group consisting of -H, -F, -CI, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
   C is selected from the group consisting of 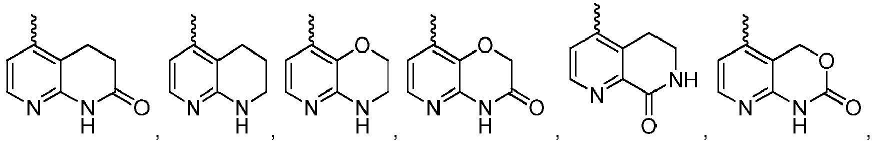 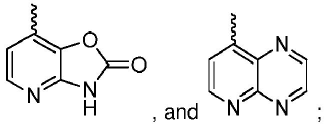 or
2)
   A is a ring system selected from the group consisting of 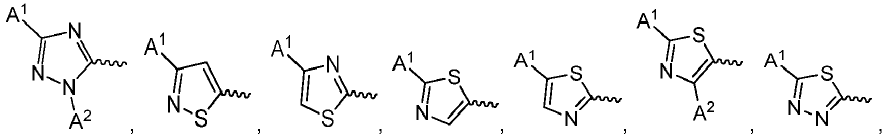 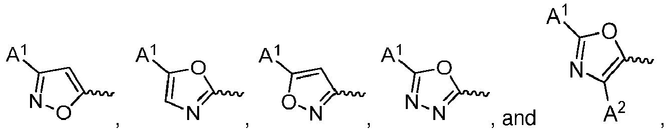 wherein A¹ is selected from the group consisting of C₁-C₄ alkyl, C₁-C₄ cycloalkyl, 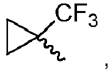 C3-C7 heterocycloalkyl, 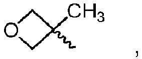 phenyl, benzyl and 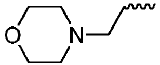 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 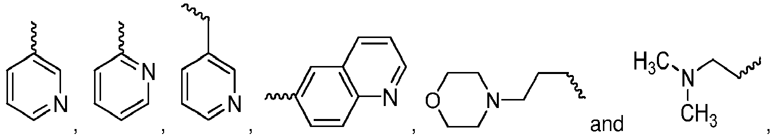 wherein A¹ and A² are optionally fluorinated, and A² is optionally substituted with 1, 2 or 3 substituents selected from the group consisting of C₁-C₄ alkyl, -CI, -F, -CN, -OR^{ABC}, and -SO₂ R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 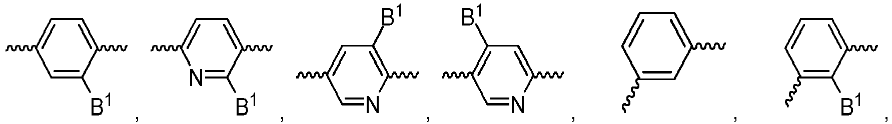 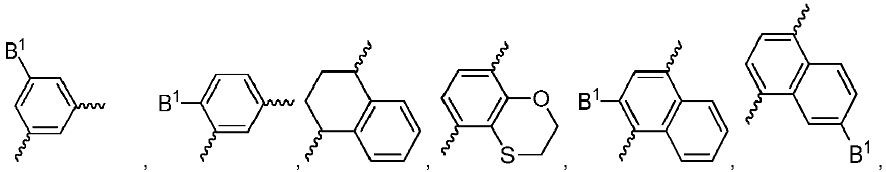 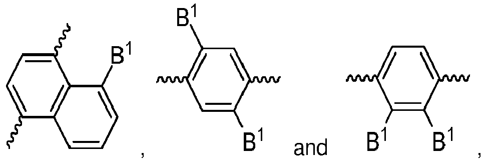 wherein B¹ is selected from the group consisting of -H, -F, -CI, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
   C is selected from the group consisting of 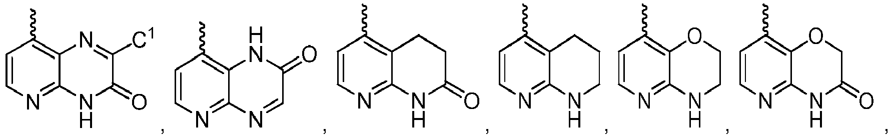 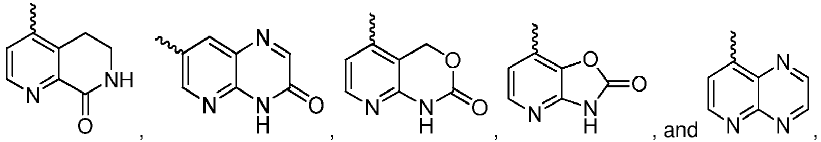 wherein C¹ is selected from the group consisting of -H, -CH₃, -OCH₃, -CN or 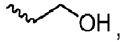 or C is selected from the group consisting of 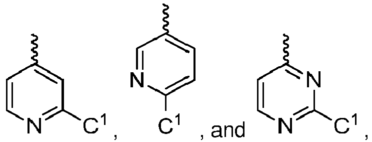 wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR'" or COOR"', wherein R', R" and R'" are independently a C₁-C₆ alkyl;
      or
3)
   A is a ring system selected from the group consisting of 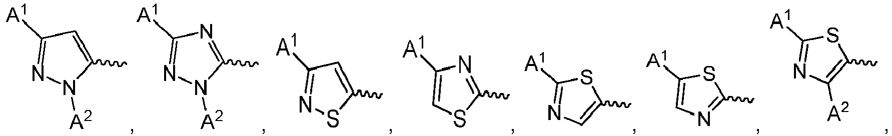 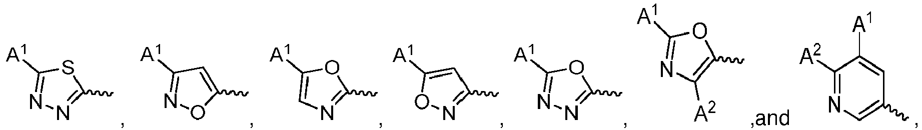 wherein A¹ is selected from the group consisting of C₁-C₄ alkyl, C₁-C₄ cycloalkyl, 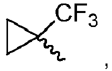 C3-C7 heterocycloalkyl, 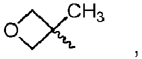 phenyl, benzyl and 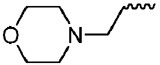 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 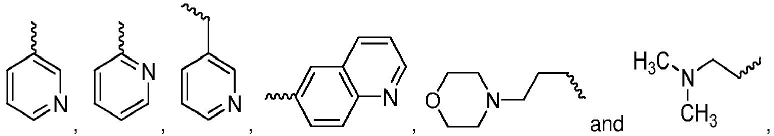 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 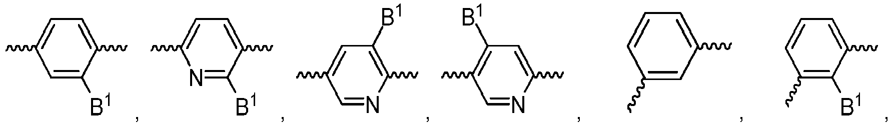 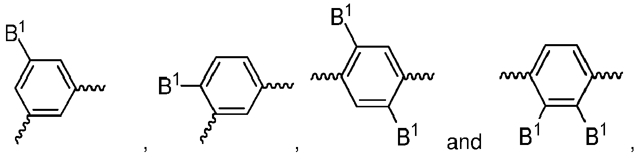 wherein B¹ is selected from the group consisting of -CF₃, -OCF₃, -OCH₃, and SCH₂CH₃, or B has two B¹ groups selected independently from the group consisting of -F, -CI, -SCH₃, -SCH₂CH₃, -CH₃, isopropyl, -CF₃, -OCH₃, -OCF₃,
   C is selected from the group consisting of 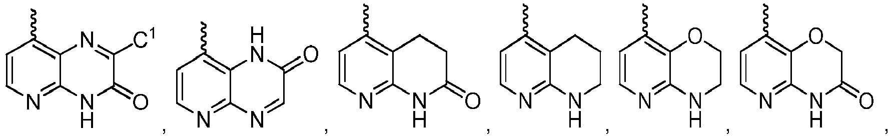 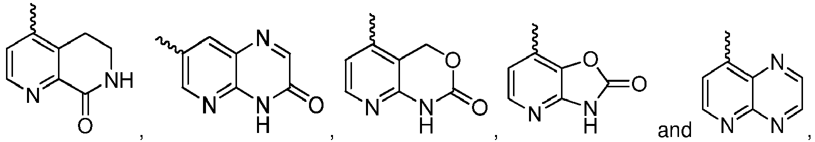 wherein C¹ is selected from the group consisting of -H, -CH₃, -OCH₃, -CN or 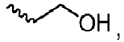 or C is selected from the group consisting of 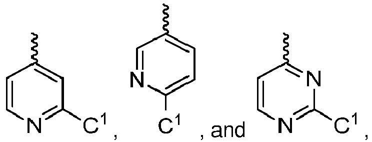 with C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR',-COOR', and -NR'R", each hydrogen in each of R' and R" may be independently replaced by -COR'" or COOR"', wherein R', R" and R'" are independently a C₁-C₆ alkyl, preferably with C¹ being selected from the group consisting of -H, -NH₂, 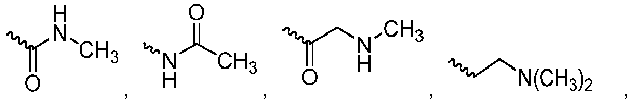 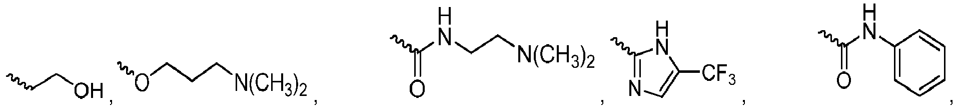 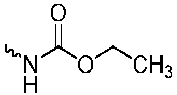 -NH-C(O)OtBu, -NH-C(O)N(CH3)-C(O)OtBu, -NH-C(O)-CH₂-N(CH₃)-C(O)OtBu,-NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH_{3;} more preferably from the group consisting of 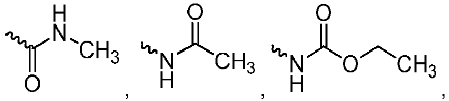 -NH-C(O)OtBu, -NH- C(O)N(CH₃)-C(O)OtBu, -NH-C(O)-CH₂-N(CH₃)-C(O)OtBu, -NH-C(O)-CH₂-NHCH₃,-NH-C(O)-Obenzyl, and -NH-C(O)-CH₂-OH;
      or
4)
   A is 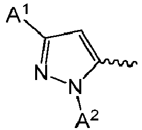 wherein A¹ is selected from the group consisting of C₁-C₄ alkyl, C₁-C₄ cycloalkyl, 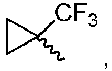 C3-C7 heterocycloalkyl, 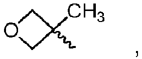 phenyl, benzyl and 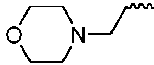 wherein A¹ is optionally fluorinated, and A² is selected from the group consisting of a phenyl substituted with 1, 2 or 3 substituents selected from the group consisting of -Cl, -F, -CN, C₁-C₃ alkyloxy optionally substituted by C₃-C₇ heterocycloalkyl or NRR, with R being a C₁-C₃ alkyl, and C₁-C₄ alkyl substituted by C₃-C₇ heterocycloalkyl or NRR, with R being a C₁-C₃ alkyl; a phenyl or pyridinyl substituted by 2 or 3 C₁-C₃ alkyl; and a pyridinyl substituted with 1, 2 or 3 substituents selected from the group consisting of -CI, -F, -CN, C₁-C₁₀ alkyloxy substituted by C₃-C₇ heterocycloalkyl or NRR, with R being a C₁-C₃ alkyl, and C₁-C₄ alkyl substituted by C₃-C₇ heterocycloalkyl or NRR, with R being a C₁-C₃ alkyl;
   B is a ring system selected from the group consisting of 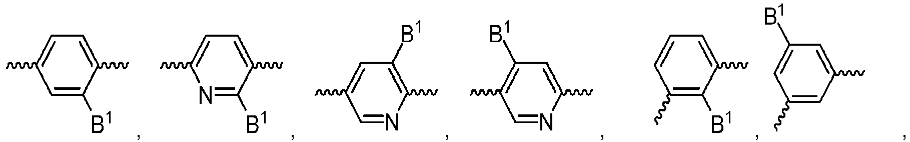 and 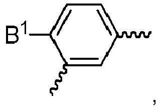 wherein B¹ is -SCH₃,
   C is selected from the group consisting of 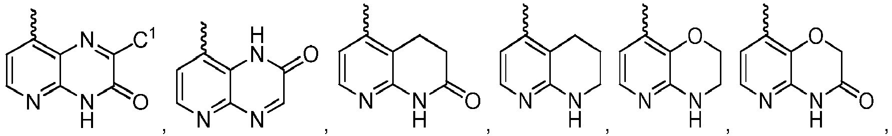 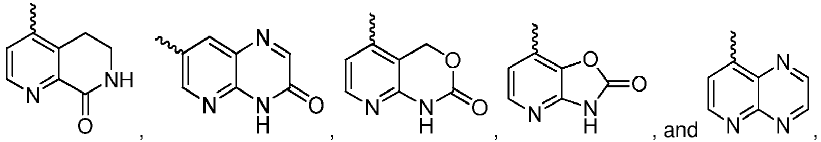 wherein C¹ is selected from the group consisting of -H, -CH₃, -OCH₃, -CN or 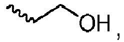 or
5)
   A is 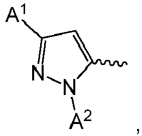 and
      A¹ is selected from the group consisting of C₁-C₄ alkyl, C₃-C₆ cycloalkyl, 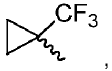 C3-C7 heterocycloalkyl, 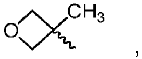 phenyl, benzyl and 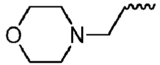 wherein A¹ is optionally fluorinated,
      A² is 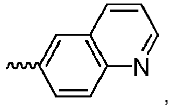
   B is a ring system selected from the group consisting of 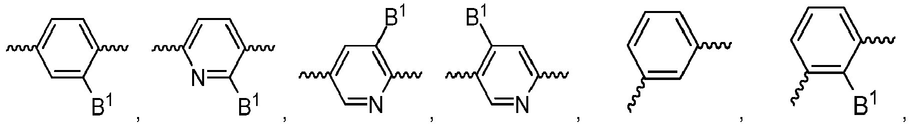 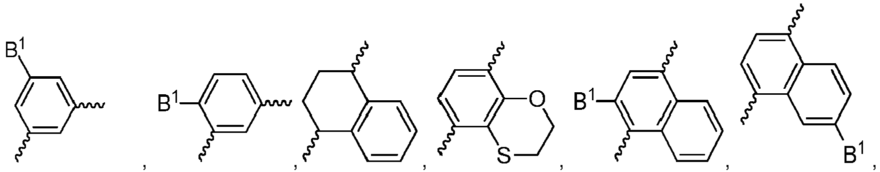 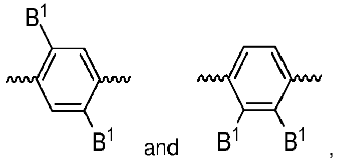 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
   C is selected from the group consisting of 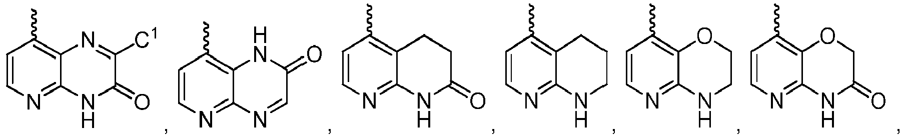 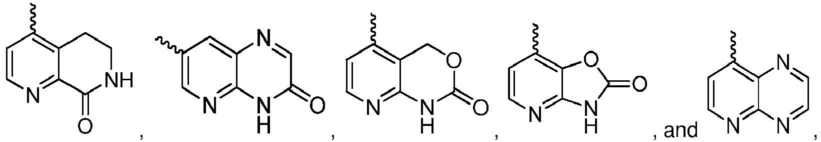 wherein C¹ is selected from the group consisting of -H, -CH₃, -OCH₃, -CN or 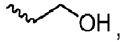 or
6)
   A is a ring system selected from the group consisting of 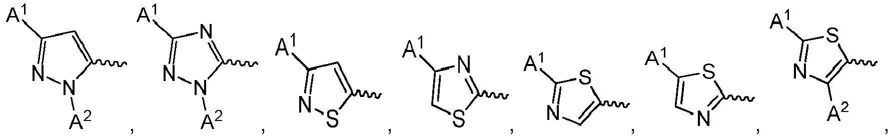 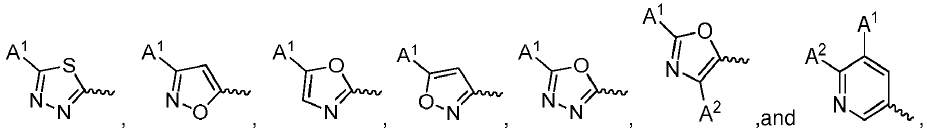 wherein A¹ is selected from the group consisting of C₁-C₄ alkyl, C₁-C₄ cycloalkyl, 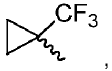 C3-C7 heterocycloalkyl, 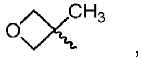 phenyl, benzyl and 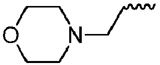 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 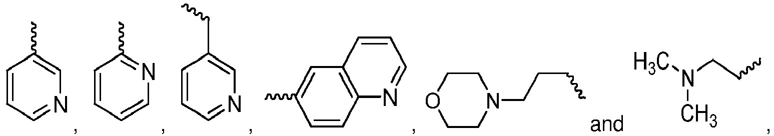 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 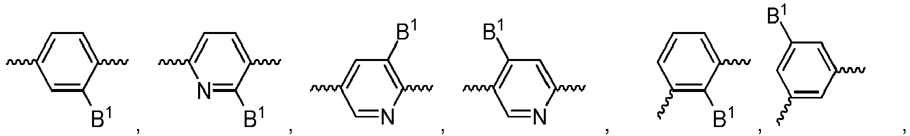 and 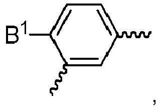 wherein B¹ is -SCH₃,
   C is selected from the group consisting of 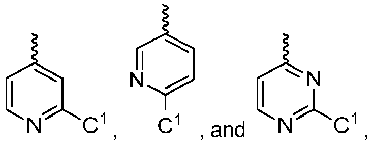 wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR'" or COOR"', wherein R', R" and R'" are independently a C₁-C₆ alkyl; or
7)
   A is a ring system selected from the group consisting of 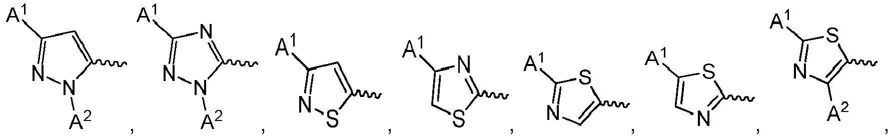 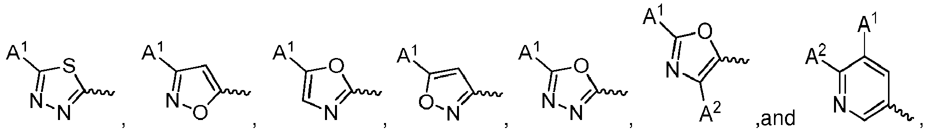 wherein A¹ is selected from the group consisting of C₁-C₄ alkyl, C₁-C₄ cycloalkyl, 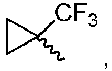 C3-C7 heterocycloalkyl, 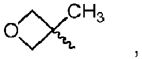 phenyl, benzyl and 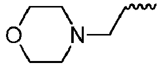 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 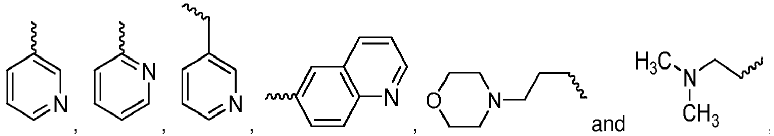 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 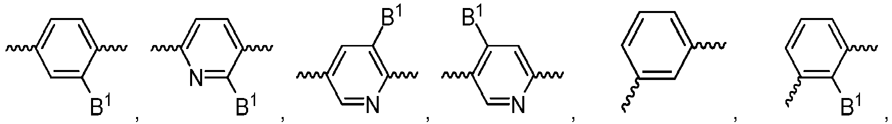 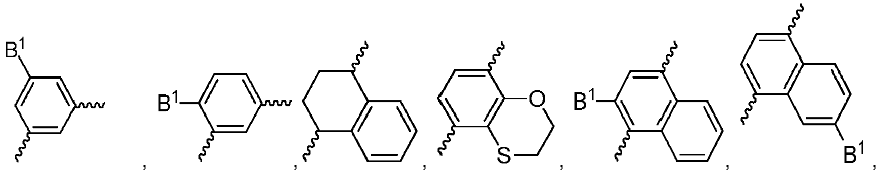 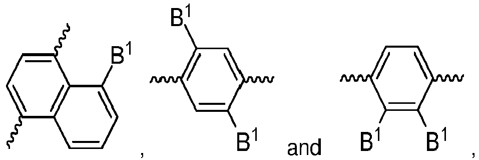 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
   C is selected from the group consisting of 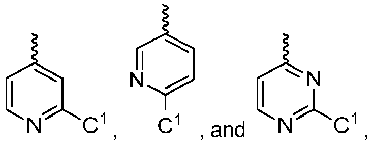 with C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR',-COOR', and -NR'R", each hydrogen in each of R' and R" may be independently replaced by -COR'" or COOR"', wherein R', R" and R'" are independently a C₁-C₆ alkyl, preferably with C¹ being selected from the group consisting of -H, -NH₂, 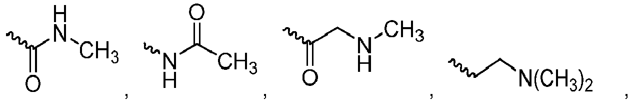 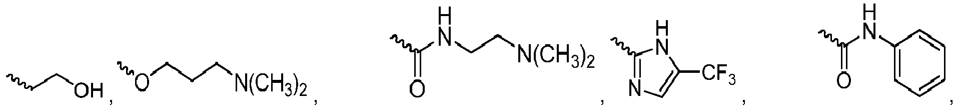 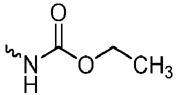 -NH-C(O)OtBu, -NH-C(O)N(CH3)-C(O)OtBu, -NH-C(O)-CH₂-N(CH₃)-C(O)OtBu,-NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH_{3;} more preferably from the group consisting of 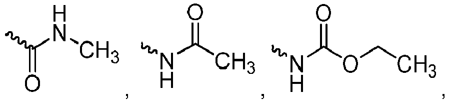 -NH-C(O)OtBu, -NH- C(O)N(CH₃)-C(O)OtBu, -NH-C(O)-CH₂-N(CH₃)-C(O)OtBu, -NH-C(O)-CH₂-NHCH₃,-NH-C(O)-Obenzyl, and -NH-C(O)-CH₂-OH.

The present invention further relates to a pharmaceutical composition comprising a compound as defined in the present disclosure and a corticosteroid or a HDAC inhibitor, preferably for use in treating, ameliorating, delaying, curing and/or preventing a disease or condition associated with muscle cells and/or satellite cells.

Preferably, the disease or condition associated with muscle cells and/or satellite cells is selected from the group consisting of Duchenne muscular dystrophy, Becker muscular dystrophy, cachexia and sarcopenia, more specifically selected from the group consisting of Duchenne muscular dystrophy, Becker muscular dystrophy and sarcopenia.

Optionally, the corticosteroid is selected from the group consisting of prednisone, prednisolone, methylprednisolone, oxandrolone, dexamethasone, deflazacort and valmorolone preferably selected from the group consisting of prednisolone, deflazacort and valmorolone.

Optionally, the HDAC inhibitor is selected from the group consisting of Abexinostat (PCI- 24781), Apicidin (OS 12040), AR-42, Belinostat (PXD101), BG45, BML-210, BML-281, BMN290, BRD0302, BRD2283, BRD3227, BRD3308, BRD3349, BRD3386, BRD3493, BRD4161, BRD4884, BRD6688, BRD8951, BRD9757, BRD9757, CBHA, Chromopeptide A, Citarinostat (ACY-214), CM-414, compound 25, CRA-026440, Crebinostat, CUDC-101, CUDC-907, Curcumin, Dacinostat (LAQ824), Depudecin, Domatinostat (4SC-202), Droxinostat, Entinostat (MS0275), EVX001688, FR901228, FRM-0334, Givinostat, HDACi-4b, HDACi-109, HPOB, 12, KD5170, LB-205, M344, Martinostat, Merck60 (BRD6929), Mocetinostat (MGCD0103), OBP-801, Oxamflatin, Panobinostat (LBH589), PCI-34051, PCI-48000, Pracinostat (SB939), Pyroxamide, Quisinostat (JNJ-26481585), Resminostat, RG2833 (RGFP109), RGFP963, RGFP966, RGFP968, Rocilinostat (ACY-1215), Romidepsin (FK228), Scriptaid, sodium phenylbutyrate, Splitomicin, T247, Tacedinaline (CI994), Trapoxin, Trichostatin A (TSA), Tucidinostat (chidamide), Valproic acid, vorinostat (SAHA), W2, MC1742, MC2625, A8B4, A14B3, A12B4, A14B4, A7B4, or any combination thereof, preferably is givinostat.

### Brief description of the Figures

**Figure 1** - the workflow of the myotube assay, aiming at monitoring myogenic activity of compounds and effect on satellite-like cells.
**Figure 2** - the myogenic activity of compound (i) in a dose-response assay, as shown by an increase of the total myotube surface/well upon increase of compound concentration.
**Figure 3** - the positive effect on satellite-like cells of compound (i) in a dose-response assay, as shown by the increase of Pax7-positive cell percentage upon increase of compound concentration.
**Figure 4** - representative images for the dose-response of the total myotube area readout for compound (i), corresponding to Figure 2.
**Figure 5** - representative images for the dose-response of the percentage of Pax7-positive cell readout for compound (i), corresponding to Figure 3.

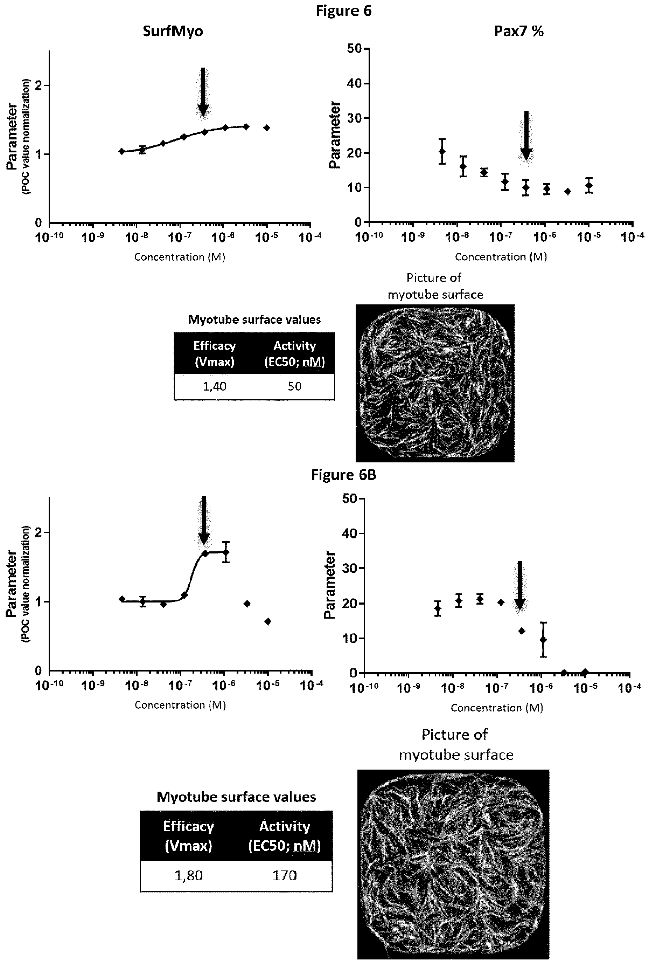

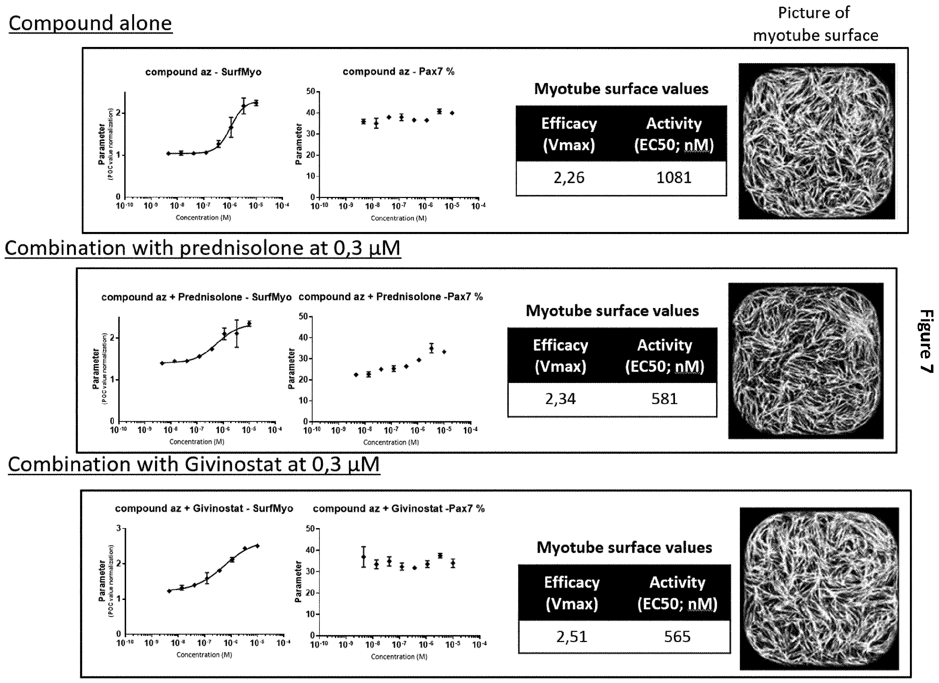

**Figure 6** - Dose-response curve on muscle progenitors derived from DMD hiPSC. Treatment of muscle progenitors derived from DMD hiPSC with Prednisolone or Givinostat alone inducing a beneficial effect on α-actinin expression (representative of the myotube surface) and a loss of nearly 50 % of Pax7 positive cells (representative of the population of satellite cells) in a dose-dependent manner. The highest concentration represented is 10 µM. The arrows show the point a 0.3 µM. This concentration was chosen for the combination treatment shown in figures 7 to 9. Efficacy values correspond to the highest score of normalized myotube surface (y-axis). Activity values correspond to the half maximal effective concentration (EC50; x-axis). The curve fits were calculated using a 4-parameter logistic regression model (Graphpad PRISM software). Error bars show standard deviation (SD). The picture on the right panel is representative of the highest myotube surface value of the dose-response curve.
**Figures 7-9** - Dose-response curve on muscle progenitors derived from DMD hiPSC. Treatment in a dose-dependent manner of muscle progenitors derived from DMD hiPSC with indicated compound alone or with combination with Prednisolone or Givinostat at 0.3 µM. The combination induced an improvement of myogenic capabilities seen with α-actinin expression (representative of the myotube surface) and harbor no worsening of the Pax7 positive cells (representative of the population of satellite cells) compared to Prednisolone or Givinostat alone. The highest concentration of DRC is 10 µM.

Efficacy values correspond to the highest score of normalized myotube surface (y-axis). Activity values correspond to the half maximal effective concentration (EC50; x-axis). The curve fits were calculated using a 4-parameter logistic regression model (Graphpad PRISM software). Error bars show standard deviation (SD). The picture on the right panel is representative of the highest myotube surface value of the dose-response curve.

In Figure 7, the compound is az.

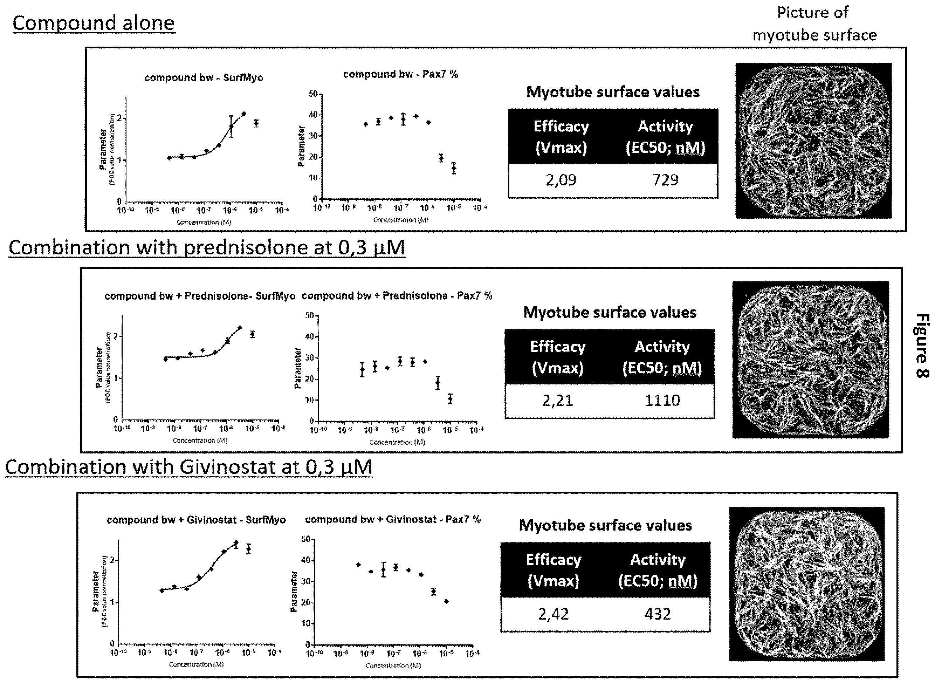

In Figure 8, the compound is bw.

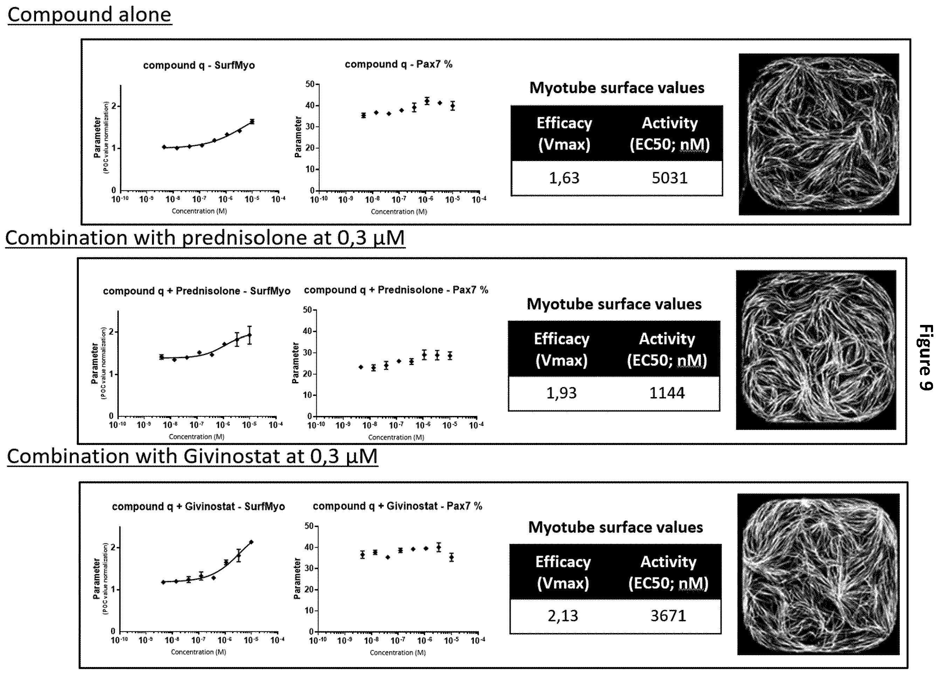

In Figure 9, the compound is q.

### Detailed description

The present invention relates to the combined use of a compound promoting muscle progenitor differentiation, in particular improved promoting capacities, but with the ability to not deplete the pool of satellite cells, e.g., to preserve or even increase it as defined in the present document with a corticosteroid or a HDAC inhibitor for use in treating, ameliorating, delaying, curing and/or preventing a disease or condition associated with muscle cells and/or satellite cells.

The present invention relates to
- a compound promoting muscle progenitor differentiation as defined herein for use in treating, ameliorating, delaying, curing and/or preventing a disease or condition associated with muscle cells and/or satellite cells in combination with a corticosteroid or a HDAC inhibitor;
- a pharmaceutical composition comprising a compound promoting muscle progenitor differentiation as defined herein for use in treating, ameliorating, delaying, curing and/or preventing a disease or condition associated with muscle cells and/or satellite cells in combination with a corticosteroid or a HDAC inhibitor;
- a pharmaceutical composition comprising a compound promoting muscle progenitor differentiation as defined herein and a corticosteroid or a HDAC inhibitor;
- a pharmaceutical composition comprising a compound promoting muscle progenitor differentiation as defined herein and a corticosteroid or a HDAC inhibitor for use in treating, ameliorating, delaying, curing and/or preventing a disease or condition associated with muscle cells and/or satellite cells;
- a kit (kit-of-parts) comprising a compound promoting muscle progenitor differentiation as defined herein and a corticosteroid or a HDAC inhibitor as a combined preparation for simultaneous, separate or sequential use, in particular for use in treating, ameliorating, delaying, curing and/or preventing a disease or condition associated with muscle cells and/or satellite cells;
- a compound promoting muscle progenitor differentiation as defined herein for the manufacture of a medicament for use in treating, ameliorating, delaying, curing and/or preventing a disease or condition associated with muscle cells and/or satellite cells in combination with a corticosteroid or a HDAC inhibitor;
- a pharmaceutical composition comprising a compound promoting muscle progenitor differentiation as defined herein and a corticosteroid or a HDAC inhibitor for the manufacture of a medicament for use in treating, ameliorating, delaying, curing and/or preventing a disease or condition associated with muscle cells and/or satellite cells in combination with a corticosteroid or a HDAC inhibitor;
- a method for treating, ameliorating, delaying, curing and/or preventing a disease or condition associated with muscle cells and/or satellite cells in a subject, comprising administering a therapeutic amount of a compound promoting muscle progenitor differentiation as defined herein and administering a therapeutic amount of a corticosteroid or a HDAC inhibitor;
- a method of treating, ameliorating, delaying, curing and/or preventing a disease or condition associated with muscle cells and/or satellite cells in a subject in need thereof, comprising administering a therapeutic amount of a pharmaceutical composition comprising a compound promoting muscle progenitor differentiation as defined herein and a corticosteroid or a HDAC inhibitor.

### Definitions

The terms mentioned herein with prefixes such as for example C₁-C₃, C₁-C₆ or C₂-C₆ can also be used with lower numbers of carbon atoms such as C₁-C₂, C₁-C₅, or C₂-C₅. If, for example, the term C₁-C₃ is used, it means that the corresponding hydrocarbon chain may comprise from 1 to 3 carbon atoms, especially 1, 2 or 3 carbon atoms. If, for example, the term C₁-C₆ is used, it means that the corresponding hydrocarbon chain may comprise from 1 to 6 carbon atoms, especially 1, 2, 3, 4, 5 or 6 carbon atoms. If, for example, the term C₂-C₆ is used, it means that the corresponding hydrocarbon chain may comprise from 2 to 6 carbon atoms, especially 2, 3, 4, 5 or 6 carbon atoms.

The term "alkyl" refers to a saturated, linear or branched aliphatic group. The term "(C₁-C₃)alkyl" more specifically means methyl, ethyl, propyl, or isopropyl. The term "(C₁-C₆)alkyl" more specifically means methyl, ethyl, propyl, isopropyl, butyl, isobutyl, *tert*-butyl, pentyl or hexyl. In a preferred embodiment, the "alkyl" is a methyl, an ethyl, a propyl, an isopropyl, or a tert-butyl, more preferably a methyl.

The term "alkenyl" refers to an unsaturated, linear or branched aliphatic group comprising at least one carbon-carbon double bound. The term "(C₂-C₆)alkenyl" more specifically means ethenyl, propenyl, isopropenyl, butenyl, isobutenyl, pentenyl, or hexenyl.

The term "alkoxy" or "alkyloxy" corresponds to the alkyl group as above defined bonded to the molecule by an -O- (ether) bond. (C₁-C₃)alkoxy includes methoxy, ethoxy, propyloxy, and isopropyloxy. (C₁-C₆)alkoxy includes methoxy, ethoxy, propyloxy, isopropyloxy, butyloxy, isobutyloxy, *tert*-butyloxy, pentyloxy and hexyloxy. In a preferred embodiment, the "alkoxy" or "alkyloxy" is a methoxy.

The term "cycloalkyl" corresponds to a saturated or unsaturated mono-, bi- or tri-cyclic alkyl group comprising between 3 and 20 atoms of carbons. It also includes fused, bridged, or spiro-connected cycloalkyl groups. The term "cycloalkyl" includes for instance cyclopropyl, cyclobutyl, cyclopentyl, and cyclohexyl. The term "cycloalkyl" may also refer to a 5-10 membered bridged carbocyclyl such as bicyclo[2,2,1]heptanyl, bicyclo[2,2,2]octanyl, bicyclo[1.1.1]pentanyl, or adamantyl, preferably bicyclo[2,2,1]heptanyl. In a preferred embodiment, the "cycloalkyl" is a cyclopropyl, cyclobutyl, cyclopentyl or a cyclohexyl.

The term "heterocycloalkyl" corresponds to a saturated or unsaturated cycloalkyl group as above defined further comprising at least one heteroatom such as nitrogen, oxygen, or sulphur atom. It also includes fused, bridged, or spiro-connected heterocycloalkyl groups. Representative heterocycloalkyl groups include, but are not limited to 3-dioxolane, benzo [1,3] dioxolyl, azetidinyl, oxetanyl, pyrazolinyl, pyranyl, thiomorpholinyl, pyrazolidinyl, piperidyl, piperazinyl, 1,4-dioxanyl, imidazolinyl, pyrrolinyl, pyrrolidinyl, piperidinyl, imidazolidinyl, morpholinyl, 1,4-dithianyl, pyrrolidinyl, oxozolinyl, oxazolidinyl, isoxazolinyl, isoxazolidinyl, thiazolinyl, thiazolidinyl, isothiazolinyl, isothiazolidinyl, dihydropyranyl, tetrahydropyranyl, tetrahydrofuranyl, and tetrahydrothiophenyl. The term "heterocycloalkyl" may also refer to a 5-10 membered bridged heterocyclyl such as 7-oxabicyclo[2,2,1]heptanyl, 6-oxa-3-azabicyclo[3,1,1]heptanyl, and 8-oxa-3-azabicyclo[3,1,1]octanyl. In a particular embodiment, it may also refer to spiro-connected heterocycloalkyl groups or spiroheterocycloalkyl groups such as for instance oxetanyl spiro-connected with azetidinyl or piperidinyl. In a preferred embodiment, the heterocycloalkyl group is azetidinyl, oxetanyl, pyranyl, tetrahydrofuranyl, tetrahydropyranyl, morpholinyl, piperidinyl, piperazinyl, and oxetanyl spiro-connected with azetidinyl or piperidinyl.

The term "aryl" corresponds to a mono- or bi-cyclic aromatic hydrocarbons having from 6 to 12 carbon atoms. For instance, the term "aryl" includes phenyl, biphenyl, or naphthyl. In a preferred embodiment, the aryl is a phenyl.

The term "heteroaryl" as used herein corresponds to an aromatic, mono- or poly-cyclic group comprising between 5 and 14 atoms and comprising at least one heteroatom such as nitrogen, oxygen or sulphur atom. Examples of such mono- and poly-cyclic heteroaryl group may be: pyridinyl, thiazolyl, thiophenyl, furanyl, pyrrolyl, pyrazolyl, imidazolyl, triazolyl, tetrazolyl, benzofuranyl, thianaphthalenyl, indolyl, indolinyl, quinolinyl, isoquinolinyl, benzimidazolyl, tetrahydroquinolinyl, tetrahydroisoquinolinyl, triazinyl, thianthrenyl, isobenzofuranyl, chromenyl, xanthenyl, phenoxanthinyl, isothiazolyl, isoxazolyl, pyrazinyl, pyridazinyl, indolizinyl, isoindolyl, indazolyl, purinyl, quinolizinyl, phtalazinyl, naphthyridinyl, quinoxalinyl, quinazolinyl, cinnolinyl, pteridinyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, indolinyl, isoindolinyl, oxazolidinyl, benzotriazolyl, benzoisoxazolyl, oxindolyl, benzoxazolinyl, benzothienyl, benzothiazolyl, isatinyl, dihydropyridyl, pyrimidinyl, s-triazinyl, oxazolyl, or thiofuranyl. In a preferred embodiment, the heteroaryl group is a pyridinyl, furanyl, pyrazolyl, pyrazinyl, pyrimidinyl, pyridazinyl, and isoxazolyl.

The term "halogen" corresponds to a fluorine, chlorine, bromine, or iodine atom, preferably a fluorine, chlorine or bromine.

The expression "substituted by at least" means that the radical is substituted by one or several groups of the list.

By the term "combination" as used herein is meant either, simultaneous administration of two Compounds A and B as defined in the present application or of a composition comprising such, or any manner of separate or sequential administration of a Compound A, or a composition comprising such, and Compound B or a composition comprising such. The combination can be administered by the same route or by different routes, e.g. one compound may be administered intravenously and the other compound may be administered orally.

The terms "kit", "product" or "combined preparation", as used herein, defines especially a "kit of parts" in the sense that the combination partners (a) and (b), as defined in the present application can be dosed independently or by use of different fixed combinations with distinguished amounts of the combination partners (a) and (b), i.e. simultaneously or at different time points. The parts of the kit of parts can then be administered simultaneously or chronologically staggered, that is at different time points for any part of the kit of parts. The ratio of the total amounts of the combination partner (a) to the combination partner (b) to be administered in the combined preparation can varied. The combination partners (a) and (b) can be administered by the same route or by different routes.

As used herein, the term "simultaneous" refers to a pharmaceutical composition, a kit, a product or a combined preparation according to the invention in which the active ingredients are used or administered simultaneously, i.e. at the same time. The active ingredients may or may not be part of the same composition.

As used herein, the term "sequential" refers to a pharmaceutical composition, a kit, a product or a combined preparation according to the invention in which the active ingredients are used or administered sequentially, i.e. one after the other. Preferably, when the administration is sequential, all the active ingredients are administered in less than about an hour, preferably less than about 10 minutes, even more preferably in less than about a minute.

As used herein, the term "separate" refers to a pharmaceutical composition, a kit, a product or a combined preparation according to the invention in which the active ingredients are used or administered at distinct time of the day. Preferably, when the administration is separate, the active ingredients are administered with an interval of about 1 hour to about 24 hours, preferably with an interval of about 1 hour and 15 hours, more preferably with an interval of about 1 hour and 8 hours, even more preferably with an interval of about 1 hour and 4 hours.

The "pharmaceutically salts" include inorganic as well as organic acids salts. Representative examples of suitable inorganic acids include hydrochloric, hydrobromic, hydroiodic, phosphoric, and the like. Representative examples of suitable organic acids include formic, acetic, trichloroacetic, trifluoroacetic, propionic, benzoic, cinnamic, citric, fumaric, maleic, methanesulfonic and the like. Further examples of pharmaceutically inorganic or organic acid addition salts include the pharmaceutically salts listed in J. Pharm. Sci. 1977, 66, 2, and in Handbook of Pharmaceutical Salts: Properties, Selection, and Use edited by P. Heinrich Stahl and Camille G. Wermuth 2002. In a preferred embodiment, the salt is selected from the group consisting of maleate, chlorhydrate, bromhydrate, and methanesulfonate. The "pharmaceutically salts" also include inorganic as well as organic base salts. Representative examples of suitable inorganic bases include sodium or potassium salt, an alkaline earth metal salt, such as a calcium or magnesium salt, or an ammonium salt. Representative examples of suitable salts with an organic base includes for instance a salt with methylamine, dimethylamine, trimethylamine, piperidine, morpholine or tris-(2-hydroxyethyl)amine. In a preferred embodiment, the salt is selected from the group consisting of sodium and potassium salt.

As used herein, the terms "treatment", "treat" or "treating" refer to any act intended to ameliorate the health status of patients such as treating, ameliorating, delaying, curing and/or preventing a disease or condition associated with muscle cells and/or satellite cells. In certain embodiments, such terms refer to the amelioration or eradication of the disease, or symptoms associated with it. In other embodiments, this term refers to minimizing the spread or worsening of the disease, resulting from the administration of one or more therapeutic agents to a subject with such a disease.

As used herein, the terms "subject", "individual" or "patient" are interchangeable and refer to an animal, preferably to a mammal, even more preferably to a human, including adult and child. However, the term "subject" can also refer to non-human animals, in particular mammals such as dogs, cats, horses, cows, pigs, sheep and non-human primates, among others.

The terms "quantity," "amount," and "dose" are used interchangeably herein and may refer to an absolute quantification of a molecule.

As used herein, the terms "active principle", "active ingredient" and "active pharmaceutical ingredient" are equivalent and refers to a component of a pharmaceutical composition having a therapeutic effect.

As used herein, the term "therapeutic effect" refers to an effect induced by an active ingredient, or a pharmaceutical composition according to the invention, capable to prevent or to delay the appearance or development of a disease or disorder, or to cure or to attenuate the effects of a disease or disorder.

As used herein, the term "effective amount" refers to a quantity of an active ingredient or of a pharmaceutical composition which prevents, removes or reduces the deleterious effects of the disease. It is obvious that the quantity to be administered can be adapted by the man skilled in the art according to the subject to be treated, to the nature of the disease, etc. In particular, doses and regimen of administration may be function of the nature, of the stage and of the severity of the disease to be treated, as well as of the weight, the age and the global health of the subject to be treated, as well as of the judgment of the doctor.

As used herein, the term" pharmaceutically acceptable excipient or carrier" refers to any ingredient except active ingredients that is present in a pharmaceutical composition. Its addition may be aimed to confer a particular consistency or other physical or gustative properties to the final product. An excipient or pharmaceutically acceptable carrier must be devoid of any interaction, in particular chemical, with the active ingredients.

### Compounds promoting muscle progenitor differentiation

The compound for use in treating, ameliorating, delaying, curing and/or preventing a disease or condition associated with muscle cells and/or satellite cells in combination with a corticosteroid or a HDAC inhibitor or the compound to be included in a pharmaceutical composition with a corticosteroid or a HDAC inhibitor, in particular for use in treating, ameliorating, delaying, curing and/or preventing a disease or condition associated with muscle cells and/or satellite cell, is represented by structure (I), 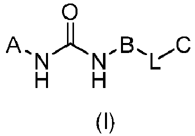 wherein
L is -O-
and
1)
   A is a ring system selected from the group consisting of 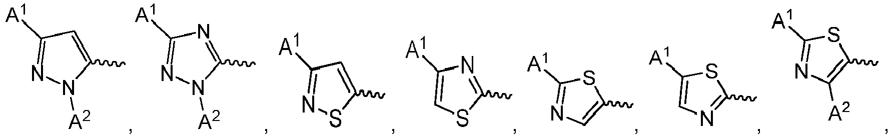 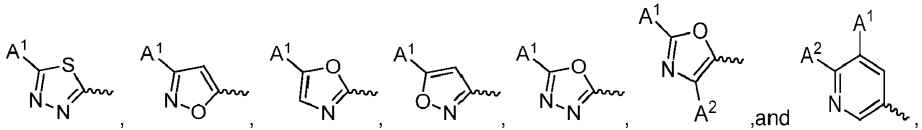 wherein A¹ is selected from the group consisting of C₁-C₄ alkyl, C₁-C₄ cycloalkyl, 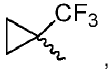 C3-C7 heterocycloalkyl, 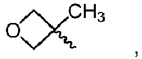 phenyl, benzyl and 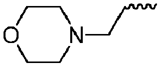 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 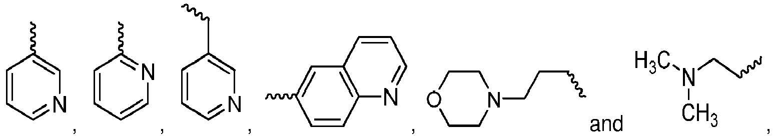 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 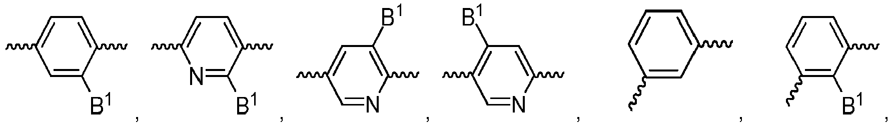 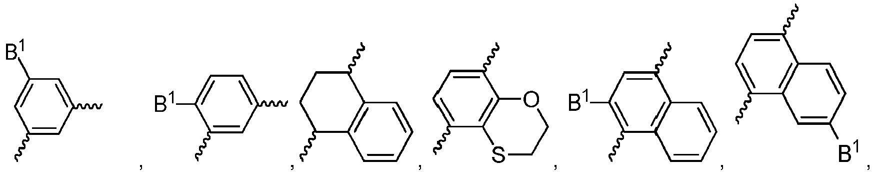 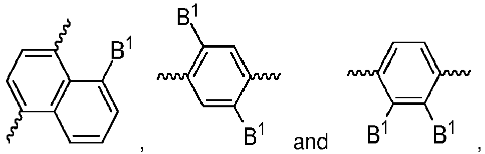 wherein B¹ is selected from the group consisting of -H, -F, -CI, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
   C is selected from the group consisting of 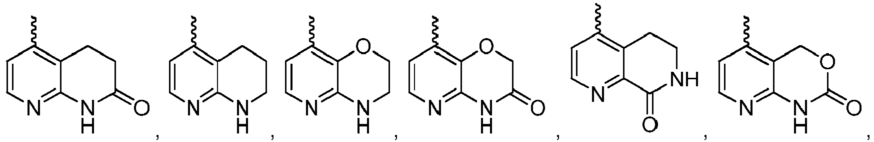 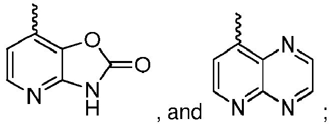 or
2)
   A is a ring system selected from the group consisting of 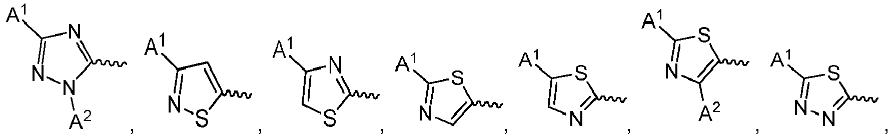 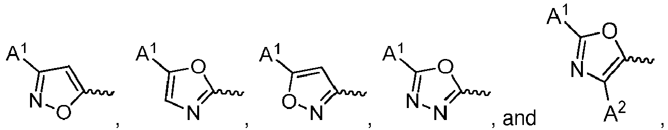 wherein A¹ is selected from the group consisting of C₁-C₄ alkyl, C₁-C₄ cycloalkyl, 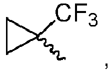 C3-C7 heterocycloalkyl, 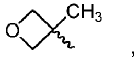 phenyl, benzyl and 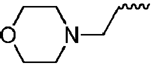 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 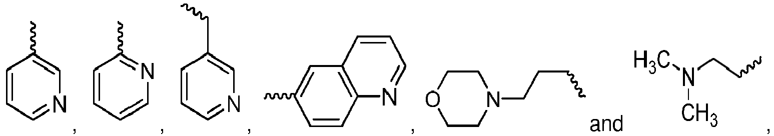 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂ R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 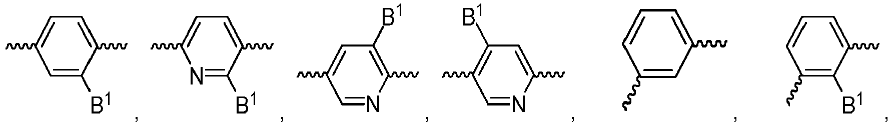 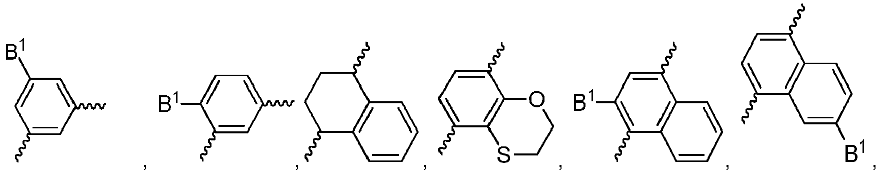 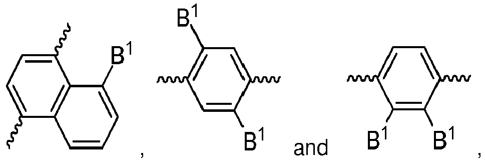 wherein B¹ is selected from the group consisting of -H, -F, -CI, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
   C is selected from the group consisting of 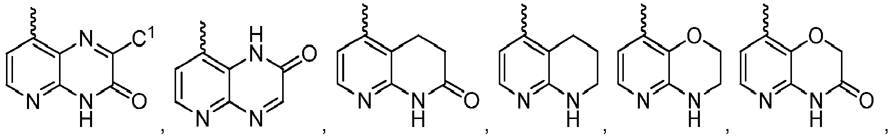 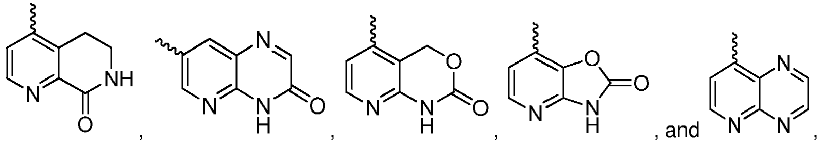 wherein C¹ is selected from the group consisting of -H, -CH₃, -OCH₃, -CN or 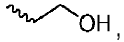 or C is selected from the group consisting of 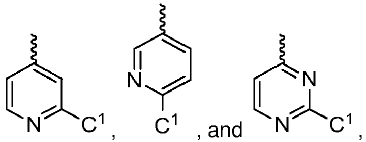 wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR'" or COOR"', wherein R', R" and R'" are independently a C₁-C₆ alkyl;
      or
3)
   A is a ring system selected from the group consisting of 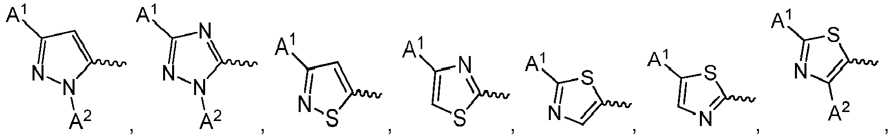 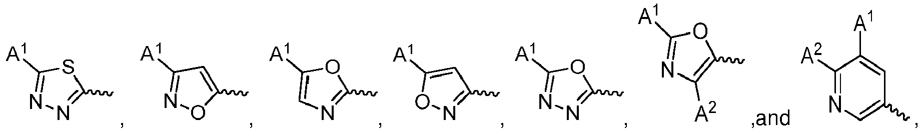 wherein A¹ is selected from the group consisting of C₁-C₄ alkyl, C₁-C₄ cycloalkyl, 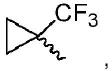 C3-C7 heterocycloalkyl, 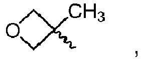 phenyl, benzyl and 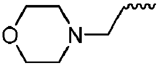 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 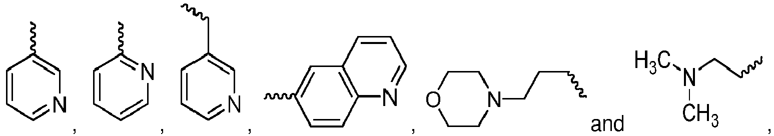 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 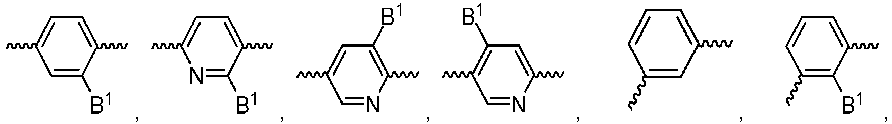 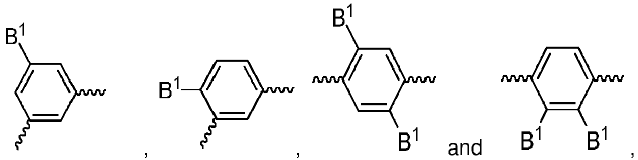 wherein B¹ is selected from the group consisting of -CF₃, -OCF₃, -OCH₃, and SCH₂CH₃, or B has two B¹ groups selected independently from the group consisting of -F, -Cl, -SCH₃, -SCH₂CH₃, -CH₃, isopropyl, -CF₃, -OCH₃, -OCF₃,
   C is selected from the group consisting of 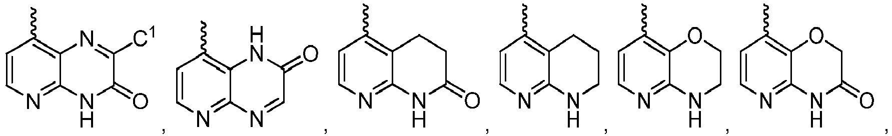 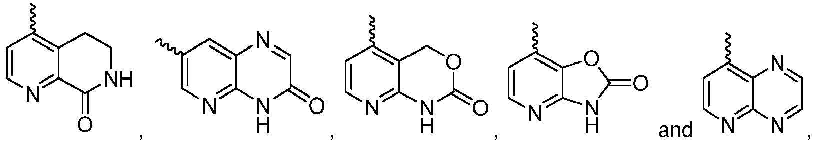 wherein C¹ is selected from the group consisting of -H, -CH₃, -OCH₃, -CN or 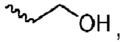 or C is selected from the group consisting of 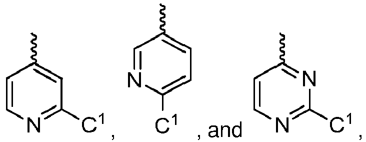 with C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', - COOR', and -NR'R", each hydrogen in each of R' and R" may be independently replaced by -COR'" or COOR"', wherein R', R" and R'" are independently a C₁-C₆ alkyl;
   or
4)
   A is 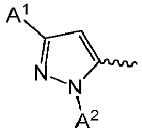 wherein A¹ is selected from the group consisting of C₁-C₄ alkyl, C₁-C₄ cycloalkyl, 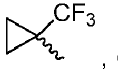 C₃-C₇ heterocycloalkyl, 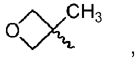 phenyl, benzyl and 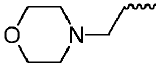 wherein A¹ is optionally fluorinated, and A² is selected from the group consisting of a phenyl substituted with 1, 2 or 3 substituents selected from the group consisting of -CI, -F, -CN, C₁-C₃ alkyloxy optionally substituted by C₃-C₇ heterocycloalkyl or NRR, with R being a C₁-C₃ alkyl, and C₁-C₄ alkyl substituted by C₃-C₇ heterocycloalkyl or NRR, with R being a C₁-C₃ alkyl; a phenyl or pyridinyl substituted by 2 or 3 C₁-C₃ alkyl; and a pyridinyl substituted with 1, 2 or 3 substituents selected from the group consisting of -Cl, -F, -CN, C₁-C₁₀ alkyloxy substituted by C₃-C₇ heterocycloalkyl or NRR, with R being a C₁-C₃ alkyl, and C₁-C₄ alkyl substituted by C₃-C₇ heterocycloalkyl or NRR, with R being a C₁-C₃ alkyl;
   B is a ring system selected from the group consisting of 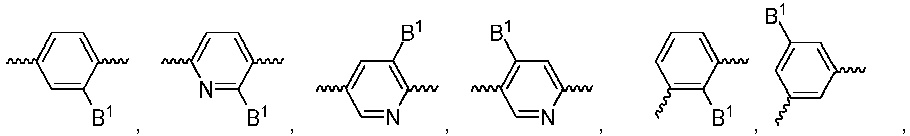 and 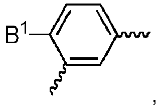 wherein B¹ is -SCH₃,
   C is selected from the group consisting of 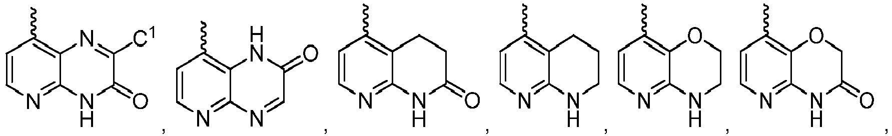 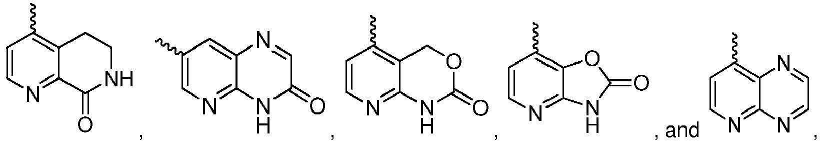 wherein C¹ is selected from the group consisting of -H, -CH₃, -OCH₃, -CN or 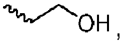 or
5)
   A is 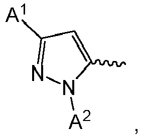 and A¹ is selected from the group consisting of C₁-C₄ alkyl, C₃-C₆ cycloalkyl, 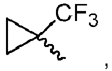 C3-C7 heterocycloalkyl, 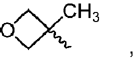 phenyl, benzyl and 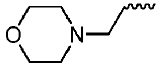 wherein A¹ is optionally fluorinated, A² is 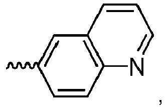
   B is a ring system selected from the group consisting of 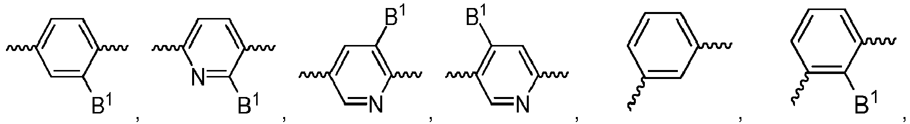 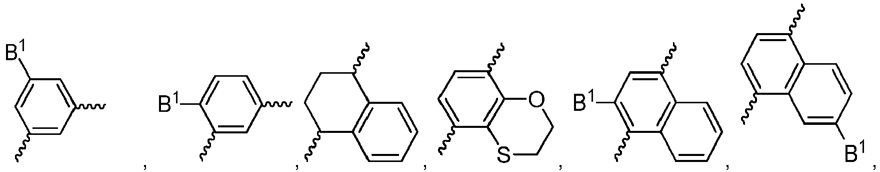 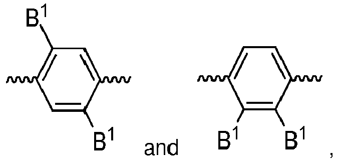 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
   C is selected from the group consisting of 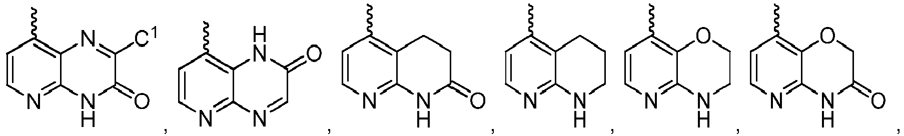 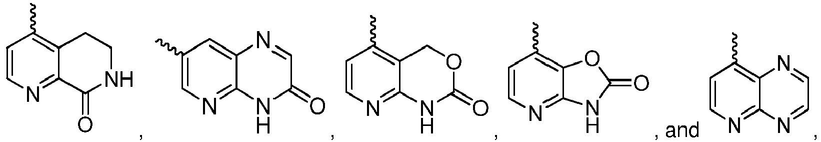 wherein C¹ is selected from the group consisting of -H, -CH₃, -OCH₃, -CN or 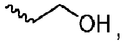 or
6)
   A is a ring system selected from the group consisting of 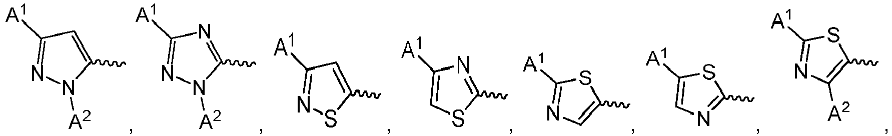 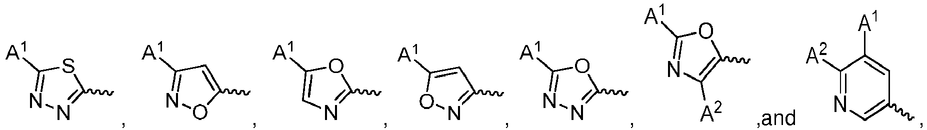 wherein A¹ is selected from the group consisting of C₁-C₄ alkyl, C₁-C₄ cycloalkyl, 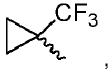 C3-C7 heterocycloalkyl, 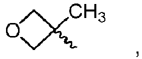 phenyl, benzyl and 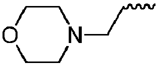 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 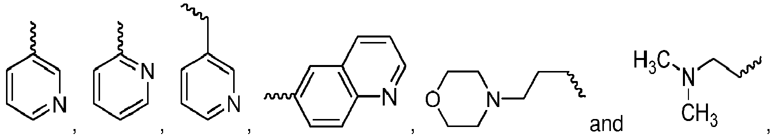 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 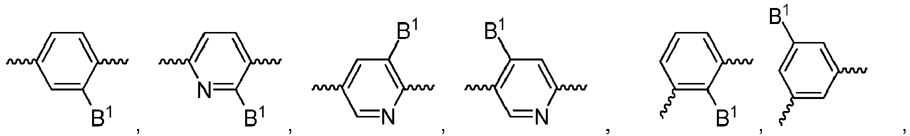 and 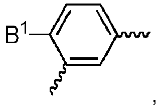 wherein B¹ is -SCH₃,
   C is selected from the group consisting of 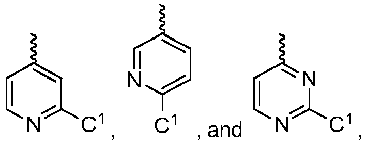 wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR'" or COOR"', wherein R', R" and R'" are independently a C₁-C₆ alkyl;
   or
7)
   A is a ring system selected from the group consisting of 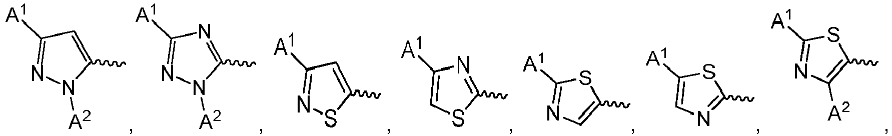 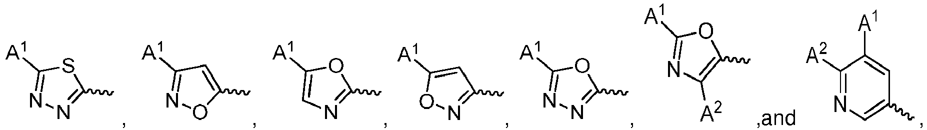 wherein A¹ is selected from the group consisting of C₁-C₄ alkyl, C₁-C₄ cycloalkyl, 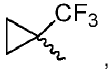 C3-C7 heterocycloalkyl, 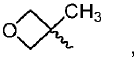 phenyl, benzyl and 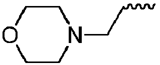 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 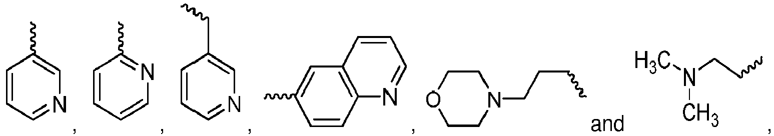 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
   B is a ring system selected from the group consisting of 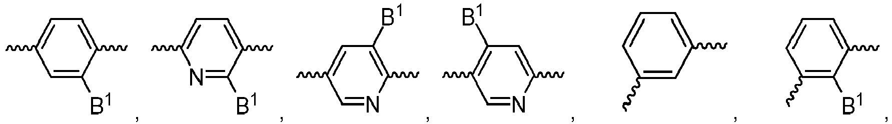 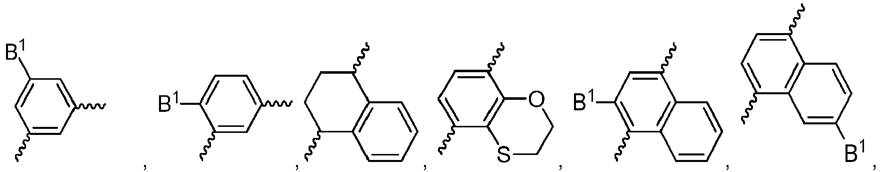 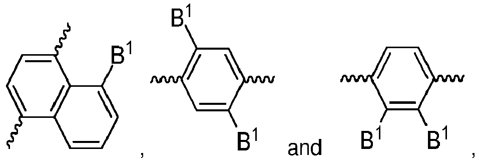 wherein B¹ is selected from the group consisting of -H, -F, -CI, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
   C is selected from the group consisting of 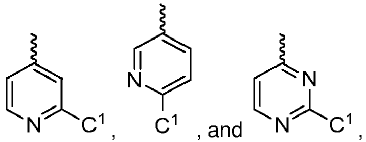 with C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR',-COOR', and -NR'R", each hydrogen in each of R' and R" may be independently replaced by -COR'" or COOR"', wherein R', R" and R'" are independently a C₁-C₆ alkyl.

It is clear that there are two orientations in which a divalent ring system B may be incorporated into a compound according to the invention. For example, if B is 2-fluoro-1,4-phenylene, two compounds (II) and (II') may be referred to. Unless explicitly stated otherwise, whenever the general notation of B, as shown in the table below, or a general name such as "2-fluoro-1,4-phenylene" is used in this application, reference is made to both (II) and (II'). Whenever a specific notation of B is used, reference is made to one of the compounds (II) and (II'), as shown in the table below.

| General notation of B | Specific notation of B | Compound |
|---|---|---|
| 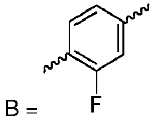 | 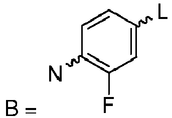 | 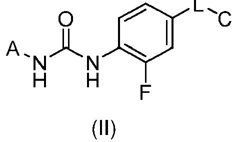 |
| | 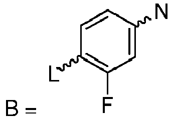 | 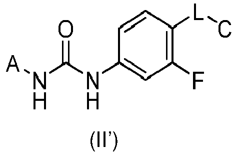 |

In the specific structures for B, it is clear that L and N are not comprised in B, but are merely present to indicate the orientation of B.

For all structures in this application representing a compound according to the invention, letters A and C refer to ring systems A and C if said letters correspond with monovalent groups, unless explicitly stated otherwise. For all structures in this application representing a compound according to the invention, letter B refers to ring system B if said letter corresponds with a divalent group, unless explicitly stated otherwise. Evidently, a letter C which corresponds with a tetravalent group should be interpreted as a carbon atom, whereas a letter B which corresponds with a trivalent group should be interpreted as a boron atom.

In a first aspect, the compound is represented by structure (I), 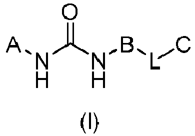
L is -O-
A is a ring system selected from the group consisting of 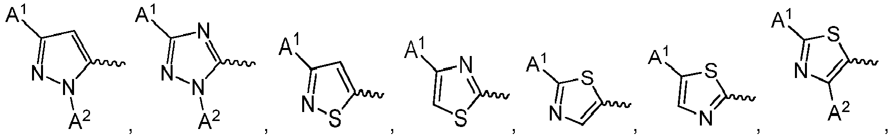 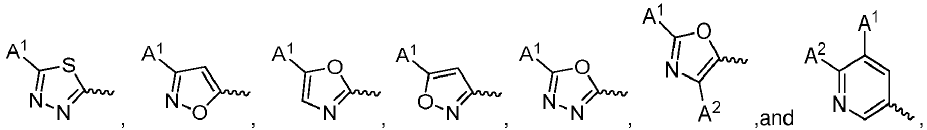 wherein A¹ is selected from the group consisting of C₁-C₄ alkyl, C₁-C₄ cycloalkyl, 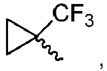 C3-C7 heterocycloalkyl, 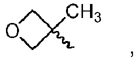 phenyl, benzyl and 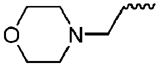 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 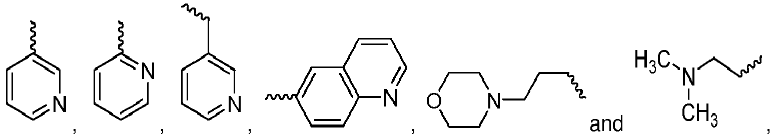 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 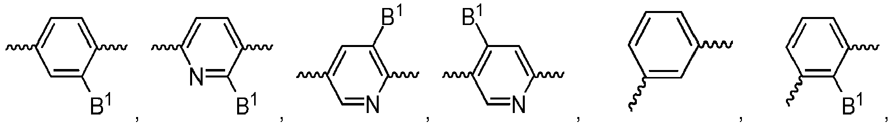 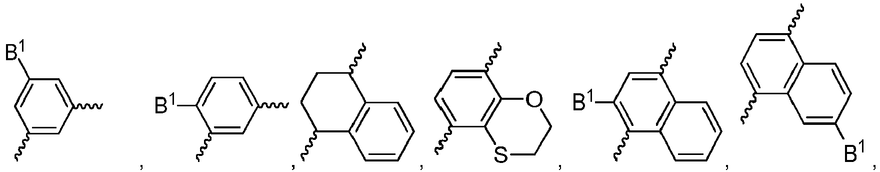 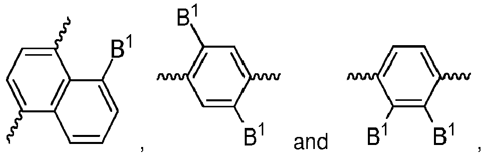 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
C is selected from the group consisting of

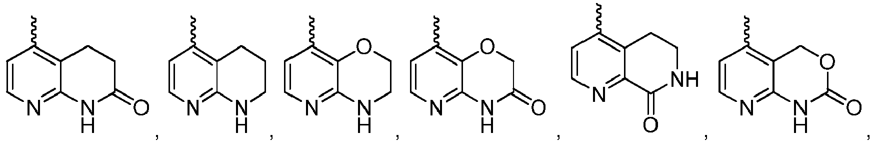

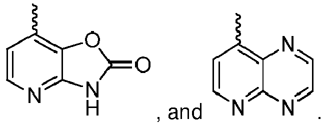

Preferably,
A is a ring system selected from the group consisting of 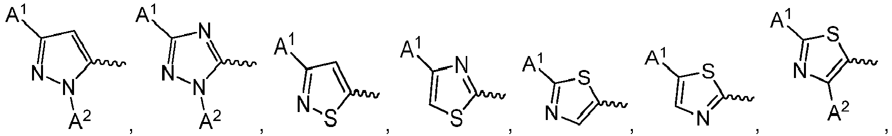 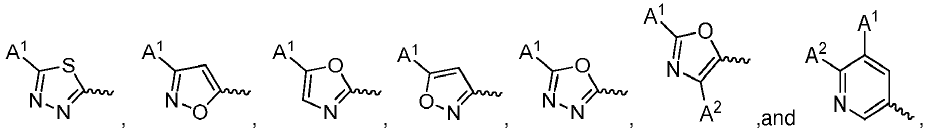 A¹ is selected from the group consisting of methyl, -CF₃, isopropyl, cyclopropyl, isobutyl, sec-butyl, tert-butyl, 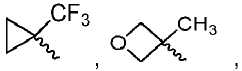 and A² is selected from the group consisting of H, methyl, iso-propyl, benzyl, phenyl, chlorine, 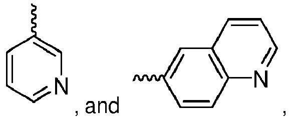 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 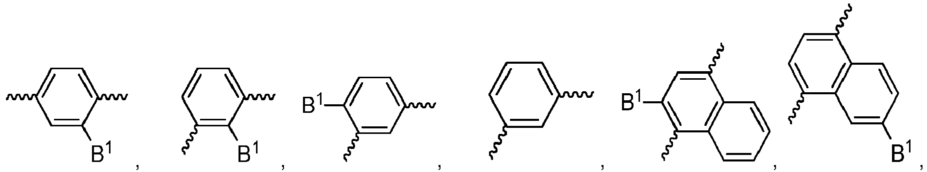 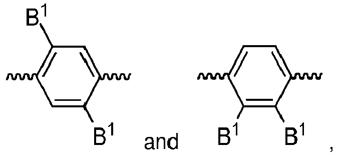 wherein, when B is a phenyl, B¹ is selected from the group consisting of -F, -Cl , -SCH₃, -SCH₂CH₃, -CH₃,-CF₃, -OCH₃, and -OCF₃ and, when B is a naphtyl, B¹ is -H, and
C is selected from the group consisting of 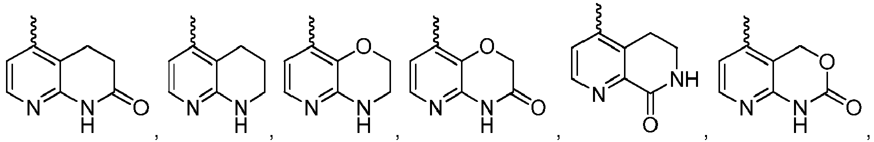 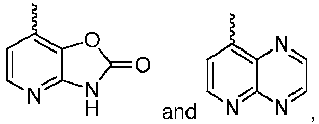 optionally C being selected from the group consisting of 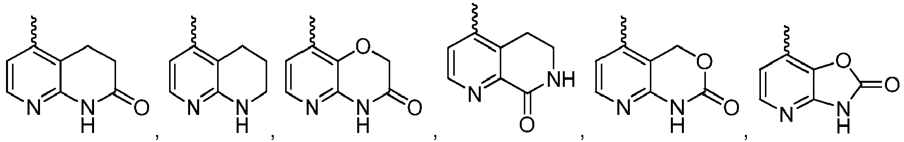 and

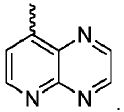

More preferably,
A is a ring selected from the group consisting of 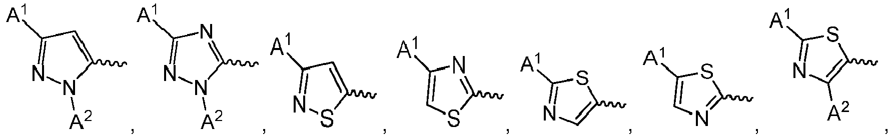 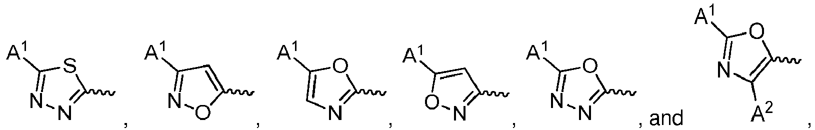 A¹ is tert-butyl,
   A² is a phenyl or 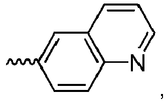 optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 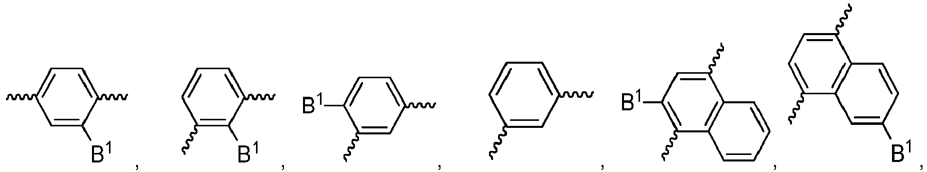 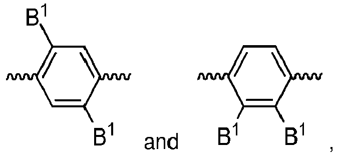 wherein, when B is a phenyl, B¹ is selected from the group consisting of -F, -SCH₃, -SCH₂CH₃, -CF₃, -OCH₃, and -OCF₃ and, when B is a naphtyl, B¹ is -H, and
C is selected from the group consisting of 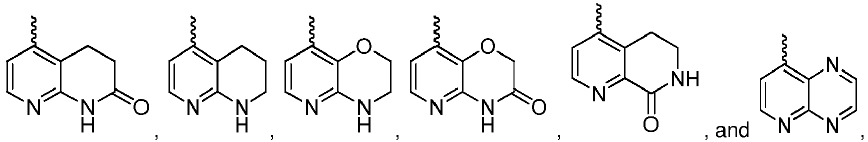 optionally C being selected from the group consisting of

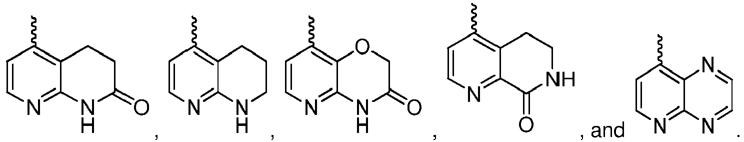

Still more preferably,
A is a ring selected from the group consisting of 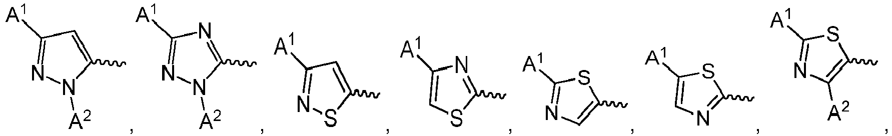 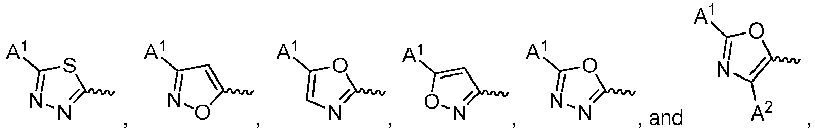 A¹ is tert-butyl,
   A² is a phenyl or 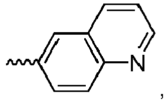 optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 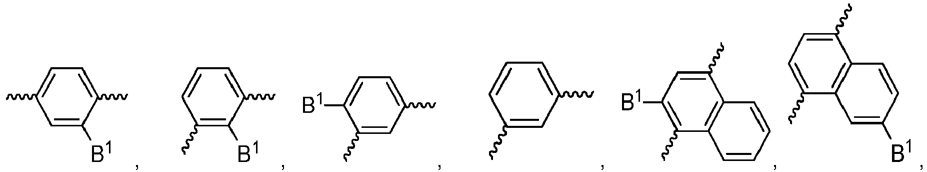 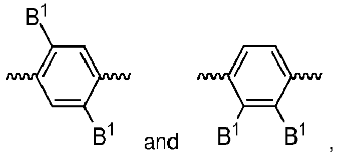 wherein, when B is a phenyl, B¹ is selected from the group consisting of -F, -SCH₃, -SCH₂CH₃, -CF₃, -OCH₃, and -OCF₃ and, when B is a naphtyl, B¹ is -H, and
C is selected from the group consisting of 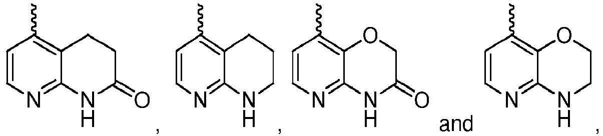 optionally C being selected from the group consisting of

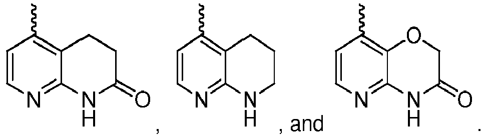

More specifically,
A is a ring selected from the group consisting of 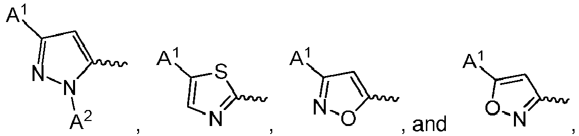 A¹ is tert-butyl,
   A² is a phenyl optionally substituted with 1, 2 or 3 substituents selected from the group consisting of methyl, -Cl, -F, -CN, methoxy, -O-(CH₂)₂-piperidinyl, -O-(CH₂)₂-morpholinyl, -O-(CH₂)₃-N(CH₃)₂, and -CH₂-morpholinyl, or A² is 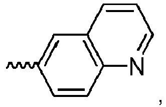
B is a ring system selected in the group consisting of 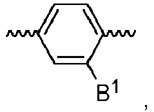 with B¹ being selected from the group consisting of -F, -SCH₃, -SCH₂CH₃, -CF₃, -OCH₃, and -OCF₃, preferably -SCH₃, 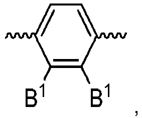 with B¹ being F, and 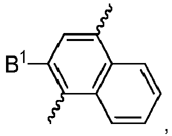 with B¹ being -H, and
C is selected from the group consisting of 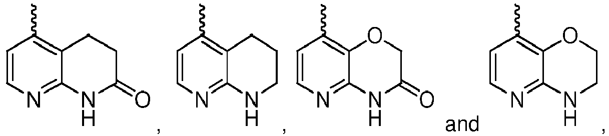 optionally C being selected from the group consisting of

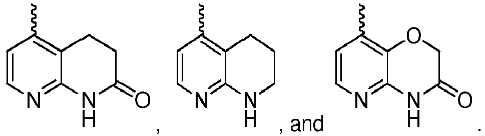

In a very specific aspect, the compound is selected in the group consisting of
1-(3-(tert-butyl)-1-phenyl-1H-pyrazol-5-yl)-3-(2-(methylthio)-4-((7-oxo-5,6,7,8-tetrahydro-1,8-naphthyridin-4-yl)oxy)phenyl)urea;
1-(3-(tert-butyl)-1-phenyl-1H-pyrazol-5-yl)-3-(2-(methylthio)-4-((5,6,7,8-tetrahydro-1,8-naphthyridin-4-yl)oxy)phenyl)urea;
1-(3-(tert-butyl)-1-(p-tolyl)-1H-pyrazol-5-yl)-3-(2-(methylthio)-4-((7-oxo-5,6,7,8-tetrahydro-1,8-naphthyridin-4-yl)oxy)phenyl)urea;
1-(5-(tert-butyl)thiazol-2-yl)-3-(2-(methylthio)-4-((7-oxo-5,6,7,8-tetrahydro-1,8-naphthyridin-4-yl)oxy)phenyl)urea;
1-(3-(tert-butyl)-1-phenyl-1H-pyrazol-5-yl)-3-(2-(methylthio)-4-((3-oxo-3,4-dihydro-2H-pyrido[3,2-b][1,4]oxazin-8-yl)oxy)phenyl)urea;
1-(5-(tert-butyl)thiazol-2-yl)-3-(4-((7-oxo-5,6,7,8-tetrahydro-1,8-naphthyridin-4-yl)oxy)naphthalen-1-yl)urea;
1-(3-(tert-butyl)-1-phenyl-1H-pyrazol-5-yl)-3-(4-((3,4-dihydro-2H-pyrido[3,2-b][1,4]oxazin-8-yl)oxy)-2-(methylthio)phenyl)urea;
1-(3-(tert-butyl)isoxazol-5-yl)-3-(2-(methylthio)-4-((7-oxo-5,6,7,8-tetrahydro-1,8-naphthyridin-4-yl)oxy)phenyl)urea;
1-(3-(tert-butyl)isoxazol-5-yl)-3-(4-((7-oxo-5,6,7,8-tetrahydro-1,8-naphthyridin-4-yl)oxy)naphthalen-1-yl)urea; and
1-(3-(tert-butyl)-1-phenyl-1H-pyrazol-5-yl)-3-(2-(methylthio)-4-((7-oxo-7,8-dihydro-1,8-naphthyridin-4-yl)oxy)phenyl)urea,
or a pharmaceutically acceptable salt thereof.

In a second aspect, the compound is represented by structure (I), 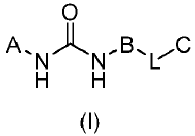
L is -O-
A is a ring system selected from the group consisting of 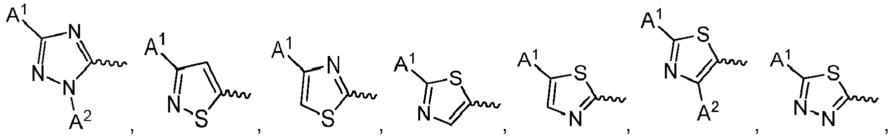 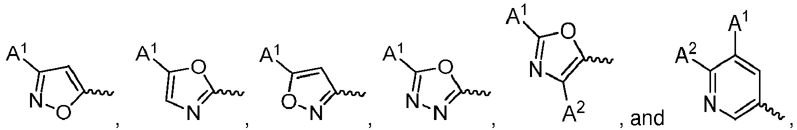 wherein A¹ is selected from the group consisting of C₁-C₄ alkyl, C₁-C₄ cycloalkyl, 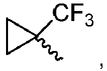 C3-C7 heterocycloalkyl, 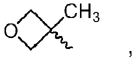 phenyl, benzyl and 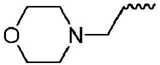 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 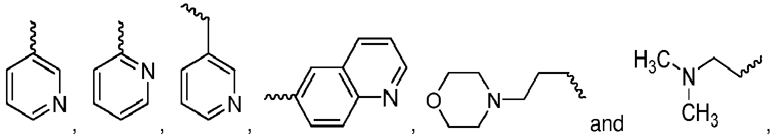 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl, B is a ring system selected from the group consisting of 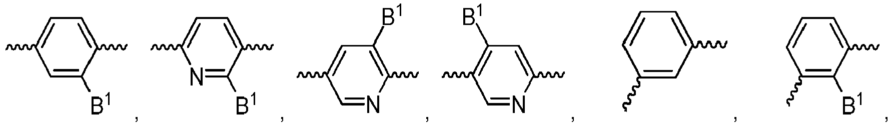 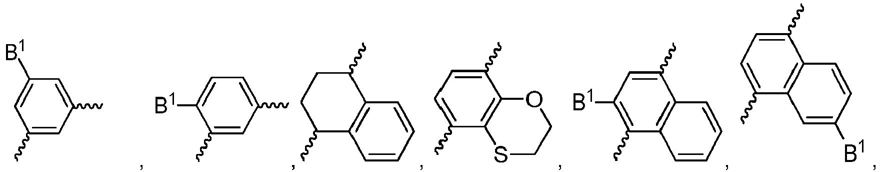 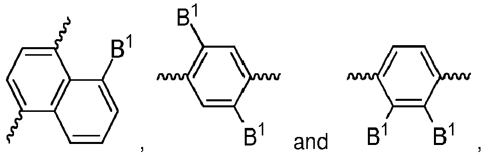 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
C is selected from the group consisting of 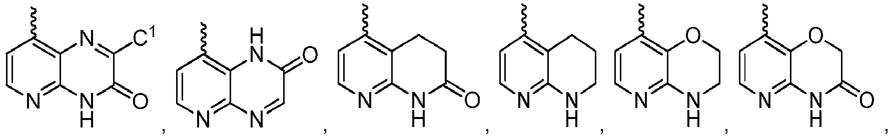 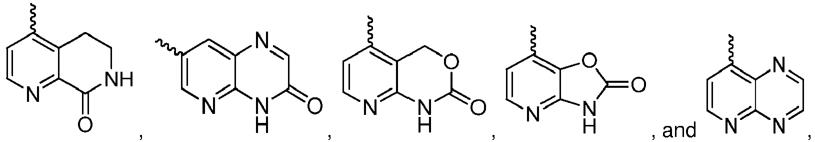 wherein C¹ is selected from the group consisting of -H, -CH₃, -OCH₃, -CN or 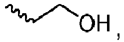 or C is selected from the group consisting of 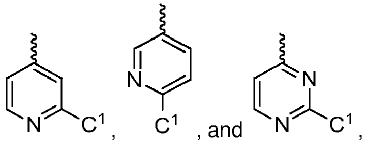 wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR'" or COOR"', wherein R', R" and R'" are independently a C₁-C₆ alkyl.

Optionally, C is selected from the group consisting of 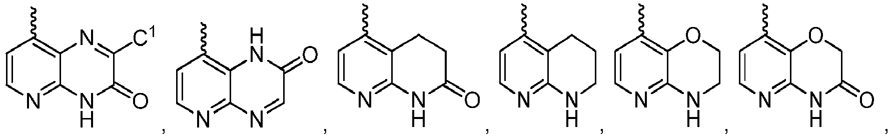 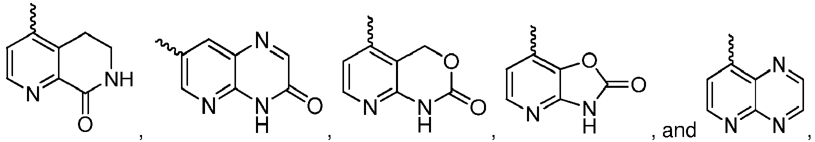 wherein C¹ is selected from the group consisting of -H, -CH₃, -OCH₃, -CN or

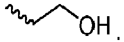

Optionally, B¹ is selected from the group consisting of -H, -SCH₃, -SCH₂CH₃, isopropyl, -CF₃, -OCH₃, -OCF₃, but B¹ is not H when B is a phenyl. Optionally, B¹ is selected from the group consisting of -H, -SCH₃,-SCH₂CH₃, isopropyl, -OCH₃, -OCF₃, but B¹ is not H when B is a phenyl.

Optionally, C is selected from the group consisting of 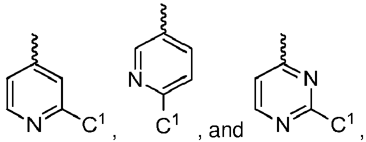 and C1 is selected from the group consisting of -H, -NH₂, 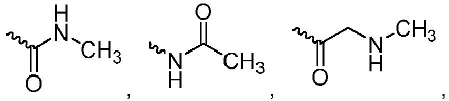 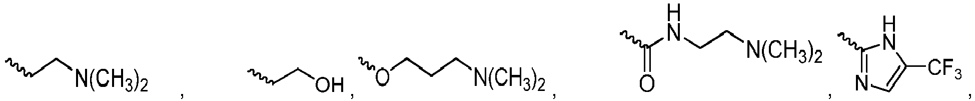 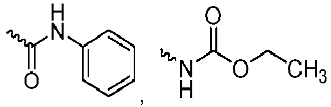 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH_{3;} more preferably from the group consisting of 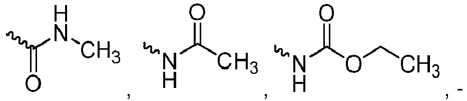 NH-C(O)OtBu, -NH- C(O)N(CH₃)-C(O)OtBu, -NH-C(O)-CH₂-N(CH₃)-C(O)OtBu, -NH-C(O)-CH₂-NHCH₃,-NH-C(O)-Obenzyl, and -NH-C(O)-CH₂-OH.

Optionally,
A is a ring selected from the group consisting of 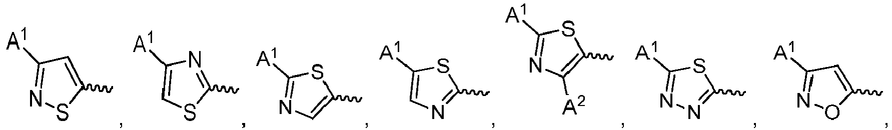 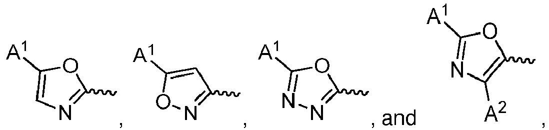 A¹ is selected from the group consisting of methyl, -CF₃, isopropyl, cyclopropyl, isobutyl, sec-butyl, tert-butyl, 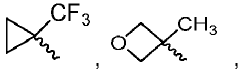 A² is -H,
B is a ring system selected from the group consisting of 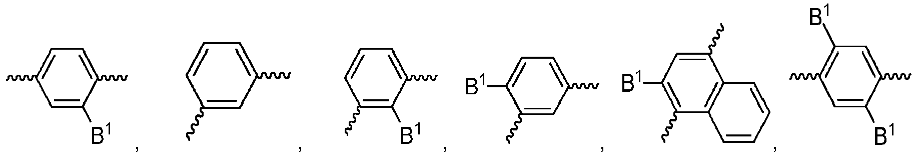 and 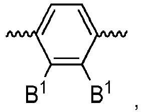 wherein, when B is a phenyl, B¹ is selected from the group consisting of -F, -CI, -SCH₃, -SCH₂CH₃, -CH₃,-CF₃, -OCH₃, and -OCF₃, optionally elected from the group consisting of -SCH₃, -SCH₂CH₃, -CF₃, -OCH₃, and -OCF₃, and, when B is a naphtyl, B¹ is -H, and
C is selected from the group consisting of 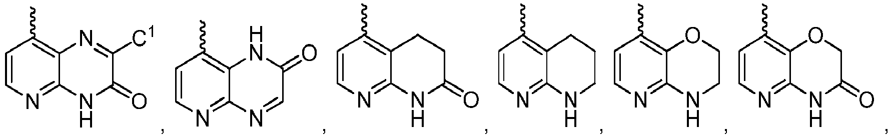 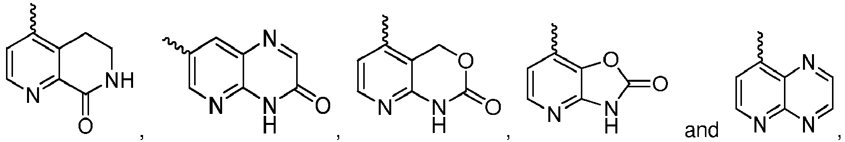 wherein C¹ is selected from the group consisting of -H, -CH₃, -OCH₃, -CN or 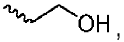 or
C is selected from the group consisting of 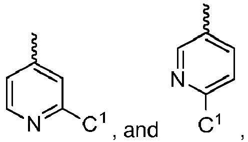 with C¹ being selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₆-C₁₄ aryl, C₃-C₁₃ heteroaryl, and C₇-C₂₄ alkaryl, wherein each hydrogen in each of these groups may be independently replaced by a group selected from halogen, hydroxy, -OR', and -NR'R", each hydrogen in each of R' and R" may be independently replaced by -COR'" or COOR"', wherein R', R" and R'" are independently a C₁-C₆ alkyl.

Optionally, C is selected from the group consisting of 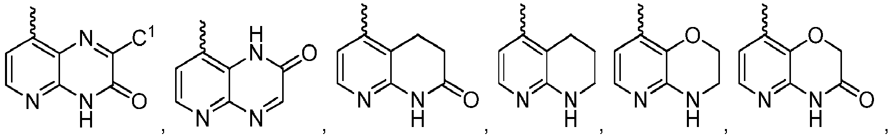 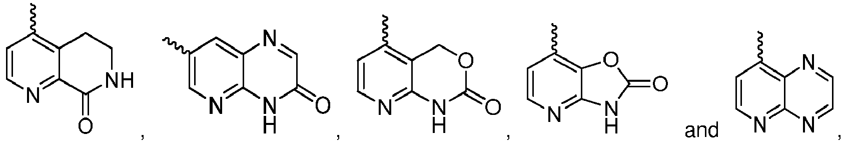 wherein C¹ is selected from the group consisting of -H, -CH₃, -OCH₃, -CN or

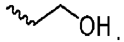

Preferably,
A is a ring selected from the group consisting of 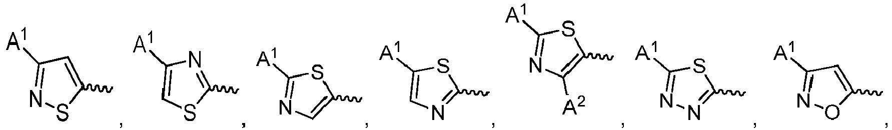 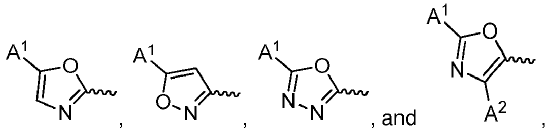
   A¹ is tert-butyl,
   A² is -H,
B is a ring system selected from the group consisting of 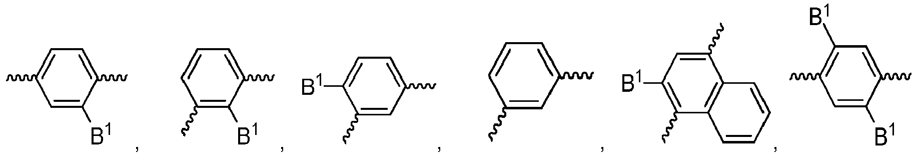 and 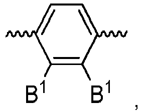 wherein, when B is a phenyl, B¹ is selected from the group consisting of -F, -SCH₃, -SCH₂CH₃, -CF₃, -OCH₃, and -OCF₃ and, when B is a naphtyl, B¹ is -H, and
C is selected from the group consisting of 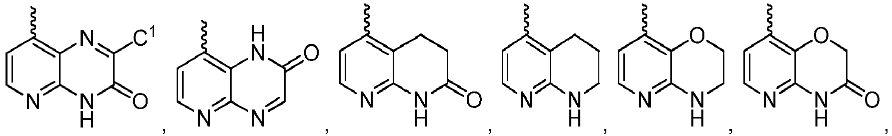 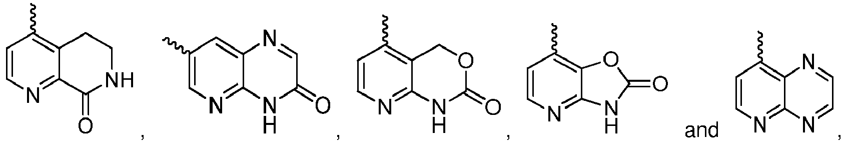 wherein C¹ is selected from the group consisting of -H, -CH₃, -OCH₃, -CN or 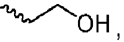 or
C is selected from the group consisting of 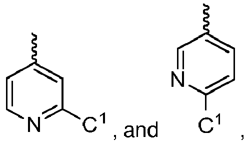 with C¹ being selected from the group consisting of -H, -NH₂, 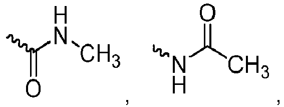 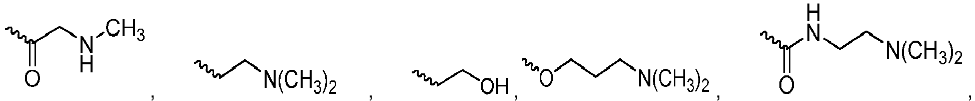 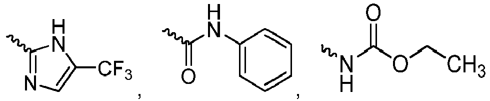 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH_{3;} preferably from the group consisting of 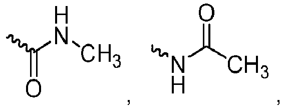 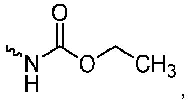 -NH-C(O)OtBu, -NH- C(O)N(CH₃)-C(O)OtBu, -NH-C(O)-CH₂-N(CH₃)-C(O)OtBu, -NH-C(O)-CH₂-NHCH₃, -NH-C(O)-Obenzyl, and -NH-C(O)-CH₂-OH.

Optionally, C is selected from the group consisting of 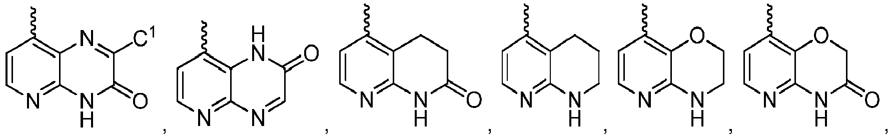 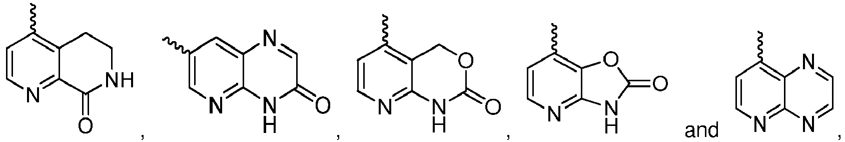 wherein C¹ is selected from the group consisting of -H, -CH₃, -OCH₃, -CN or

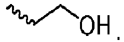

Still more preferably,
A is selected in the group consisting of 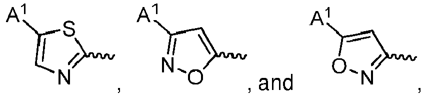 A¹ is tert-butyl,
B is a ring system selected in the group consisting of 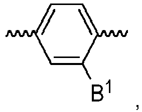 with B¹ being selected from the group consisting of -F, -SCH₃, -SCH₂CH₃, -CF₃, -OCH₃, and -OCF₃, preferably -SCH₃, 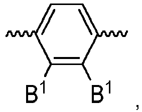 with B¹ being F, and 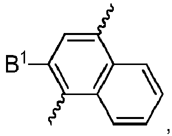 with B¹ being -H, and
C is selected in the group consisting of 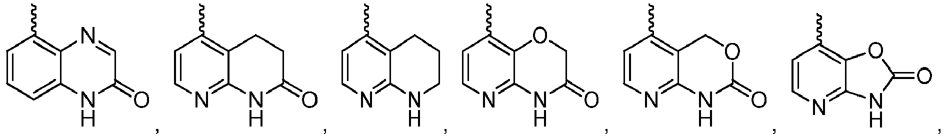 and 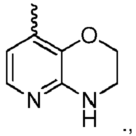 or C is selected from the group consisting of 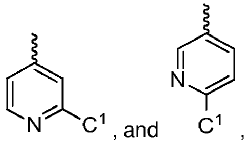 with C¹ being selected from the group consisting of 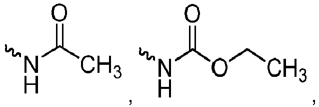 -NH-C(O)OtBu, - NH-C(O)N(CH3)-COOtBu and -NH-C(O)-CH₂-N(CH₃)-C(O)OtBu.

Optionally, C is selected in the group consisting of 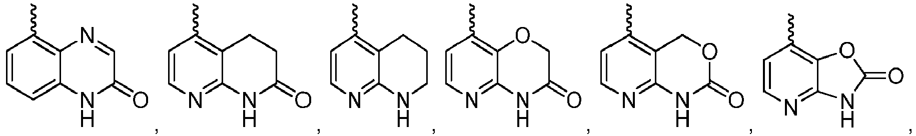 and

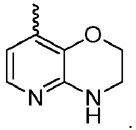

In a very specific aspect, the compound is selected in the group consisting of
1-(5-(tert-butyl)thiazol-2-yl)-3-(4-((3-oxo-3,4-dihydropyrido[2,3-b]pyrazin-8-yl)oxy)naphthalen-1-yl)urea;
1-(3-(tert-butyl)isoxazol-5-yl)-3-(2-(methylthio)-4-((3-oxo-3,4-dihydropyrido[2,3-b]pyrazin-8-yl)oxy)phenyl)urea;
1-(5-(tert-butyl)thiazol-2-yl)-3-(2-(methylthio)-4-((7-oxo-5,6,7,8-tetrahydro-1,8-naphthyridin-4-yl)oxy)phenyl)urea;
1-(5-(tert-butyl)thiazol-2-yl)-3-(4-((7-oxo-5,6,7,8-tetrahydro-1,8-naphthyridin-4-yl)oxy)naphthalen-1-yl)urea;
1-(3-(tert-butyl)isoxazol-5-yl)-3-(2-(methylthio)-4-((7-oxo-5,6,7,8-tetrahydro-1,8-naphthyridin-4-yl)oxy)phenyl)urea;
ethyl (4-(4-(3-(3-(tert-butyl)isoxazol-5-yl)ureido)-3-(methylthio)phenoxy)pyridin-2-yl)carbamate;
ethyl (4-(4-(3-(5-(tert-butyl)thiazol-2-yl)ureido)-3-(methylthio)phenoxy)pyridin-2-yl)carbamate;
1-(3-(tert-butyl)isoxazol-5-yl)-3-(4-((7-oxo-5,6,7,8-tetrahydro-1,8-naphthyridin-4-yl)oxy)naphthalen-1-yl)urea; and
ethyl (4-(4-(3-(5-(tert-butyl)isoxazol-3-yl)ureido)-3-(methylthio)phenoxy)pyridin-2-yl)carbamate; or a pharmaceutically acceptable salt thereof.

In a third aspect, the compound is represented by structure (I), 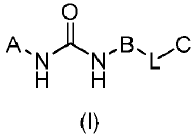
L is -O-
A is a ring system selected from the group consisting of 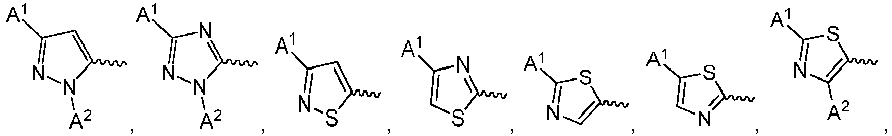 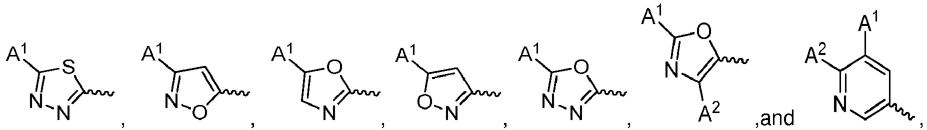 wherein A¹ is selected from the group consisting of C₁-C₄ alkyl, C₁-C₄ cycloalkyl, 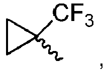 C3-C7 heterocycloalkyl, 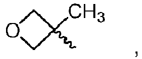 phenyl, benzyl and 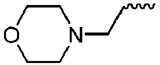 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 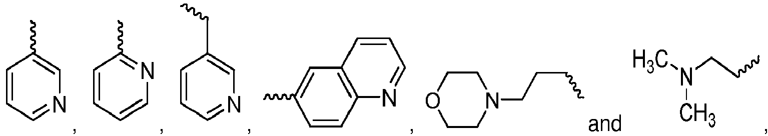 wherein A¹ and A² are optionally fluorinated, and A₂ is optionally substituted with 1, 2 or 3 substituents selected from the group consisting of C₁-C₄ alkyl, -CI, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 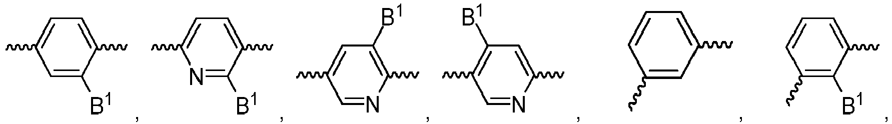 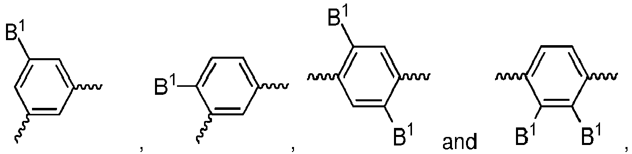 wherein B¹ is selected from the group consisting of -CF₃, -OCF₃, -OCH₃, and SCH₂CH₃, or B has two B¹ groups selected independently from the group consisting of -F, -CI, -SCH₃, -SCH₂CH₃, -CH₃, isopropyl, -CF₃, -OCH₃, -OCF₃,
C is selected from the group consisting of 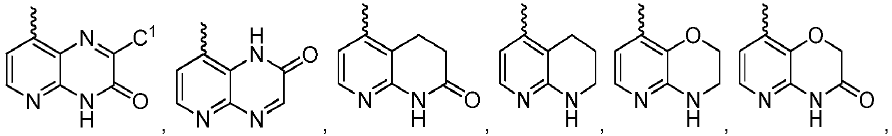 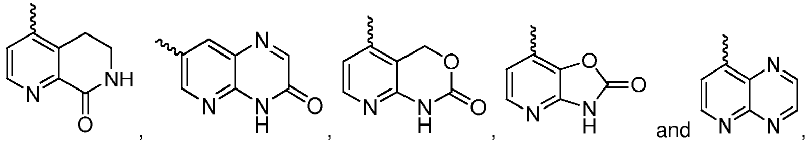 wherein C¹ is selected from the group consisting of -H, -CH₃, -OCH₃, -CN or 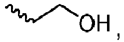
or C is selected from the group consisting of 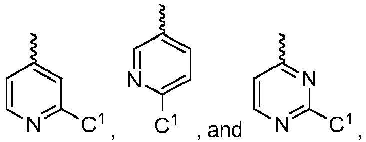 wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR'" or COOR"', wherein R', R" and R'" are independently a C₁-C₆ alkyl.

Optionally, C¹ is selected from the group consisting of
-H, -NH₂, 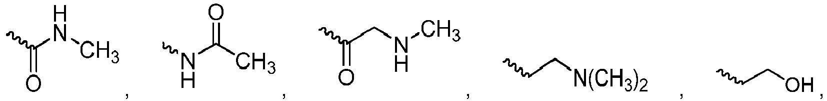 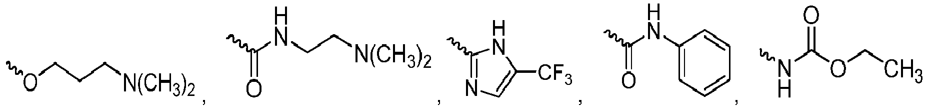 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH_{3;} more preferably from the group consisting of 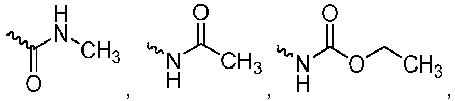 -NH-C(O)OtBu, -NH- C(O)N(CH₃)-C(O)OtBu, -NH-C(O)-CH₂-N(CH₃)-C(O)OtBu, -NH-C(O)-CH₂-NHCH₃, -NH-C(O)-Obenzyl, and -NH-C(O)-CH₂-OH.

Optionally, when B has two B¹ groups, B¹ are independently selected from the group consisting of-SCH₃, -SCH₂CH₃, -CH₃, isopropyl, -CF₃, -OCH₃, and -OCF₃.

Optionally, C is selected from the group consisting of 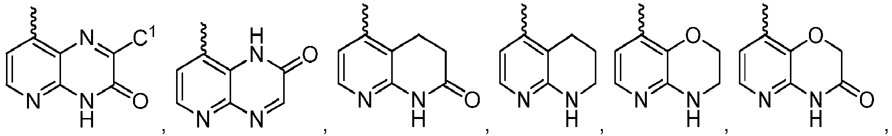 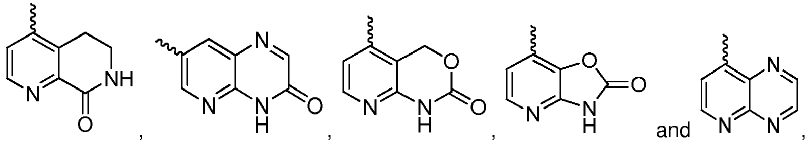 wherein C¹ is selected from the group consisting of -H, -CH₃, -OCH₃, -CN or

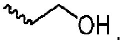

Preferably,
A is a ring selected from the group consisting of 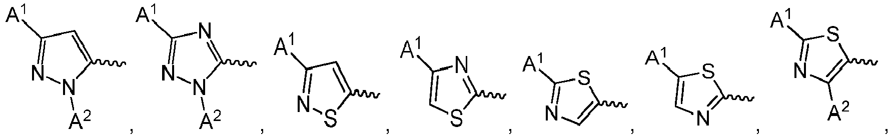 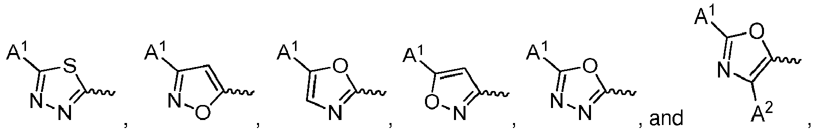 A¹ is tert-butyl,
   A₂ is a phenyl or 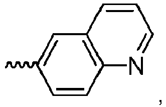 optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 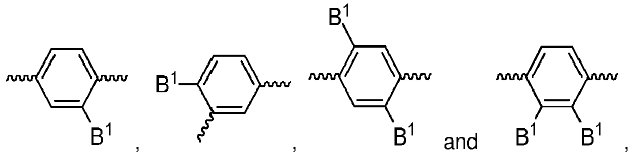 wherein B¹ is selected from the group consisting of -CF₃, -OCF₃, -OCH₃, and SCH₂CH₃, or B has two B¹ groups selected independently from the group consisting of -F, -Cl, -SCH₃, -SCH₂CH₃, -CH₃, isopropyl,-CF₃, -OCH₃, and -OCF₃, optionally from the group consisting of -SCH₃, -SCH₂CH₃, -CH₃, isopropyl, -CF₃,-OCH₃, and -OCF₃,
C is selected from the group consisting of 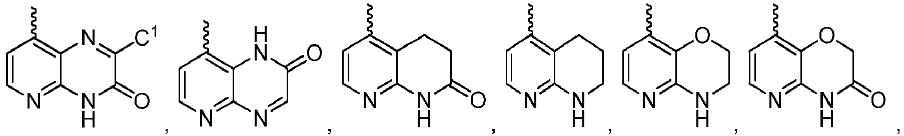 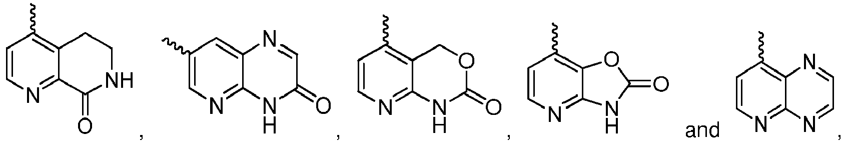 wherein C¹ is selected from the group consisting of -H, -CH₃, -OCH₃, -CN or 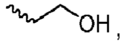 or
C is selected from the group consisting of 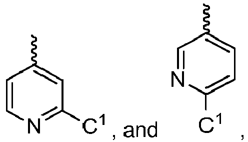 with C¹ being selected from the group consisting of -H, -NH₂, 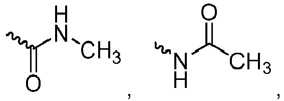 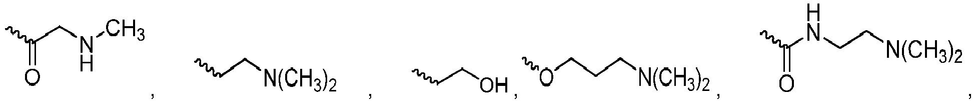 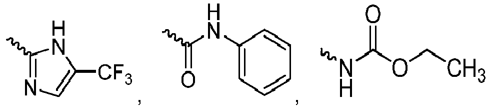 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH_{3;} preferably from the group consisting of 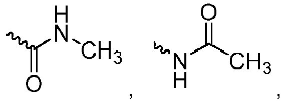 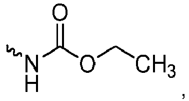 -NH-C(O)OtBu, -NH- C(O)N(CH₃)-C(O)OtBu, -NH-C(O)-CH₂-N(CH₃)-C(O)OtBu, -NH-C(O)-CH₂-NHCH₃, -NH-C(O)-Obenzyl, and -NH-C(O)-CH₂-OH.

Optionally, C is selected from the group consisting of 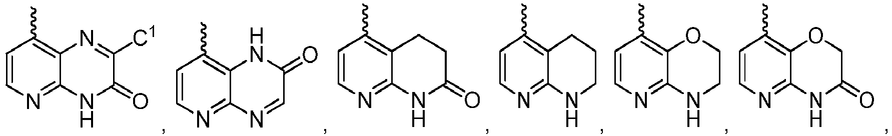 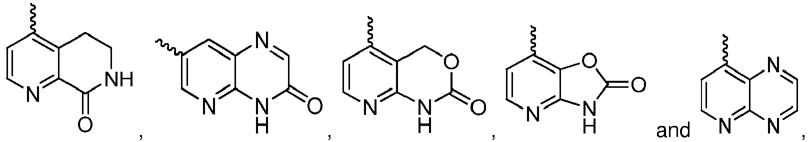 wherein C¹ is selected from the group consisting of -H, -CH₃, -OCH₃, -CN or

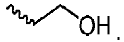

More preferably,
A is a ring selected from the group consisting of 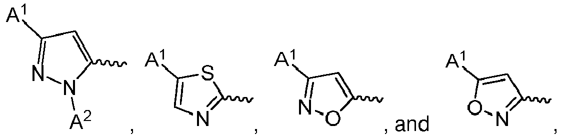 A¹ is tert-butyl,
   A² is a phenyl optionally substituted with 1, 2 or 3 substituents selected from the group consisting of methyl, -Cl, -F, -CN, methoxy, -O-(CH₂)₂-piperidinyl, -O-(CH₂)₂-morpholinyl, -O-(CH₂)₃-N(CH₃)₂, and -CH₂-morpholinyl, or A₂ is 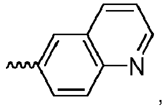
B is a ring system selected in the group consisting of 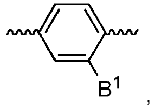 with B¹ being selected from the group consisting of -CF₃, -SCH₂CH₃, -OCH₃, and -OCF₃, preferably-CF₃ or -SCH₂CH₃, 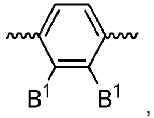 with B¹ being F,
C is selected in the group consisting of 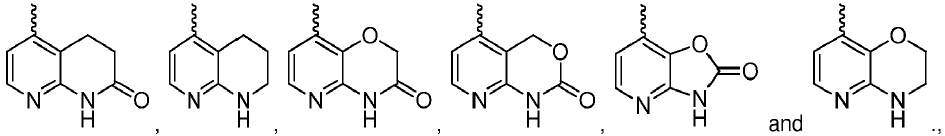 or C is selected from the group consisting of 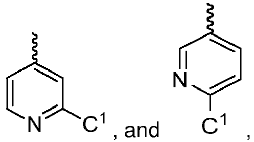 with C¹ being selected from the group consisting of 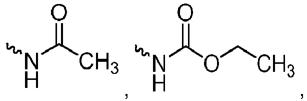 -NH-C(O)OtBu,-NH-C(O)-CH₂-N(CH₃)-C(O)OtBu and -NH-C(O)N(CH3)-COOtBu.

In a very specific aspect, the compound is selected in the group consisting of
1-(3-(tert-butyl)-1-phenyl-1H-pyrazol-5-yl)-3-(4-((3-oxo-3,4-dihydropyrido[2,3-b]pyrazin-8-yl)oxy)-2-(trifluoromethyl)phenyl)urea;
1-(3-(tert-butyl)-1-(p-tolyl)-1H-pyrazol-5-yl)-3-(4-((3-oxo-3,4-dihydropyrido[2,3-b]pyrazin-8-yl)oxy)-2-(trifluoromethyl)phenyl)urea;
1-(3-(tert-butyl)-1-phenyl-1H-pyrazol-5-yl)-3-(2-(ethylthio)-4-((3-oxo-3,4-dihydropyrido[2,3-b]pyrazin-8-yl)oxy)phenyl)urea;
1-(3-(tert-butyl)-1-phenyl-1H-pyrazol-5-yl)-3-(2,3-difluoro-4-((3-oxo-3,4-dihydropyrido[2,3-b]pyrazin-8-yl)oxy)phenyl)urea; and
1-(3-(tert-butyl)-1-phenyl-1H-pyrazol-5-yl)-3-(2,5-difluoro-4-((3-oxo-3,4-dihydropyrido[2,3-b]pyrazin-8-yl)oxy)phenyl)urea;
or a pharmaceutically acceptable salt thereof.

In a fourth aspect, the compound is represented by structure (I), 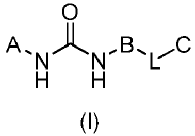
L is -O-
A is 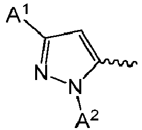 wherein A¹ is selected from the group consisting of C₁-C₄ alkyl, C₁-C₄ cycloalkyl, 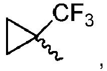 C3-C7 heterocycloalkyl, 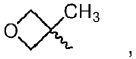 phenyl, benzyl and 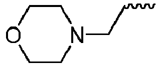 wherein A¹ is optionally fluorinated, and A² is selected from the group consisting of a phenyl substituted with 1, 2 or 3 substituents selected from the group consisting of -Cl, -F, -CN, C₁-C₃ alkyloxy optionally substituted by C₃-C₇ heterocycloalkyl or NRR, with R being a C₁-C₃ alkyl, and C₁-C₄ alkyl substituted by C₃-C₇ heterocycloalkyl or NRR, with R being a C₁-C₃ alkyl; a phenyl or pyridinyl substituted by 2 or 3 C₁-C₃ alkyls; and a pyridinyl substituted with 1, 2 or 3 substituents selected from the group consisting of -CI, -F, -CN, C₁-C₁₀ alkyloxy substituted by C₃-C₇ heterocycloalkyl or NRR, with R being a C₁-C₃ alkyl, and C₁-C₄ alkyl substituted by C₃-C₇ heterocycloalkyl or NRR, with R being a C₁-C₃ alkyl;
B is a ring system selected from the group consisting of 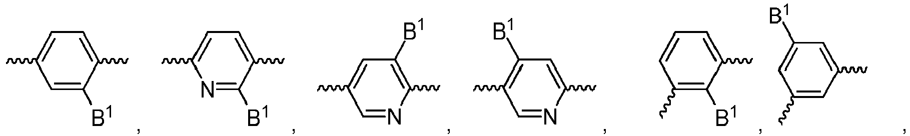 and 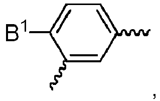 wherein B¹ is -SCH₃,
C is selected from the group consisting of 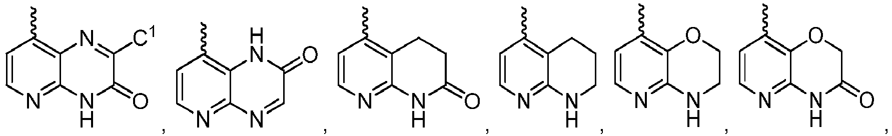 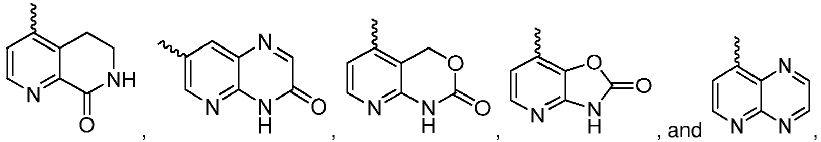 wherein C¹ is selected from the group consisting of -H, -CH₃, -OCH₃, -CN or

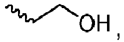

Preferably,
A is 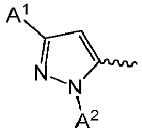 A¹ is tert-butyl,
   and A² is selected from the group consisting of a phenyl substituted with 1, 2 or 3 substituents selected from the group consisting of methoxy, -Cl, -F, -CN, -O-(CH₂)₂-piperidinyl, -O-(CH₂)₂-morpholinyl, -O-(CH₂)₃-N(CH₃)₂, and -CH₂-morpholinyl; a phenyl or pyridinyl substituted with 2 or 3 methyls, and a pyridinyl substituted with 1, 2 or 3 substituents selected from the group consisting of methyl, -Cl, -F, -CN, -O-(CH₂)₂-piperidinyl, -O-(CH₂)₂-morpholinyl, -O-(CH₂)₃-N(CH₃)₂, and -CH₂-morpholinyl;
B is a ring system selected from the group consisting of 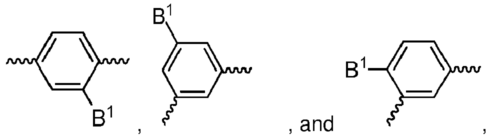 wherein B¹ is -SCH₃,
C is selected from the group consisting of 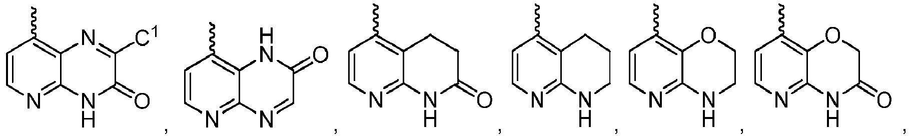 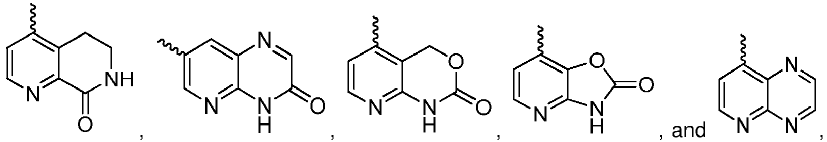 wherein C¹ is selected from the group consisting of -H, -CH₃, -OCH₃, -CN or

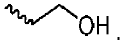

More preferably,
A is 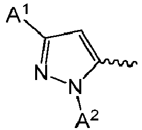 A¹ is tert-butyl,
   and A₂ is selected from the group consisting of a phenyl substituted with 1, 2 or 3 substituents selected from the group consisting of -Cl, -F, -CN, -O-(CH₂)₂-piperidinyl, -O-(CH₂)₃-N(CH₃)₂, and -CH₂-morpholinyl; a pyridinyl substituted with a methyl; and a phenyl substituted by two methyls;
B is 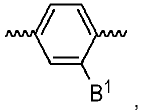 with B¹ being -SCH₃,
C is selected in the group consisting of

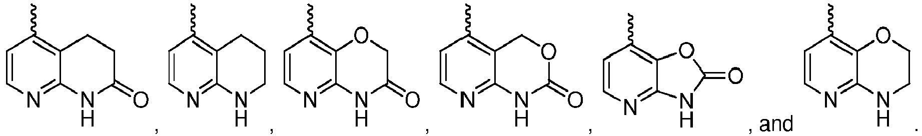

In a very specific aspect, the compound is selected in the group consisting of
1-(3-(tert-butyl)-1-(4-methoxyphenyl)-1 H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(3-chlorophenyl)-1H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(3-methoxyphenyl)-1H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(3-fluorophenyl)-1H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(quinolin-6-yl)-1H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(4-cyanophenyl)-1H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(3,4-dimethylphenyl)-1H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(4-(2-(piperidin-1-yl)ethoxy)phenyl)-1H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(4-(2-morpholinoethoxy)phenyl)-1 H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(3-chloro-4-methylphenyl)-1H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(3-chloro-4-fluorophenyl)-1H-pyrazol-5-yl)-3-(2-(methylthio)-4-((3-oxo-3,4-dihydropyrido[2,3-b]pyrazin-8-yl)oxy)phenyl)urea;
1-(3-(tert-butyl)-1-(4-(3-(dimethylamino)propoxy)phenyl)-1 H-pyrazol-5-yl)-3-(2-(methylthio)-4-((3-oxo-3,4-dihydropyrido[2,3-b]pyrazin-8-yl)oxy)phenyl)urea; and
1-(3-(tert-butyl)-1-(4-(morpholinomethyl)phenyl)-1H-pyrazol-5-yl)-3-(2-(methylthio)-4-((3-oxo-3,4-dihydropyrido[2,3-b]pyrazin-8-yl)oxy)phenyl)urea;
or a pharmaceutically acceptable salt thereof.

In a fifth aspect, the compound is represented by structure (I), 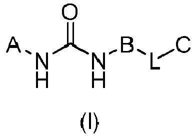
L is -O-
A is 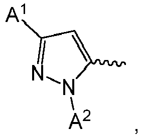 and
   A¹ is selected from the group consisting of C₁-C₄ alkyl, C₃-C₆ cycloalkyl, 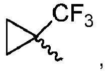 C3-C7 heterocycloalkyl, 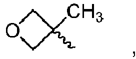 phenyl, benzyl and 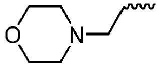 wherein A¹ is optionally fluorinated,
   A² is 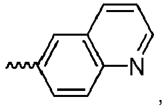
B is a ring system selected from the group consisting of 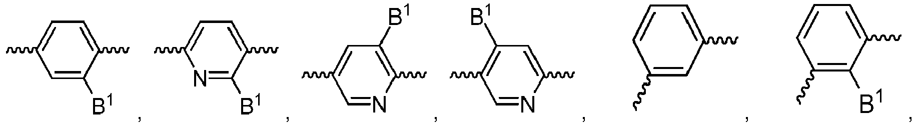 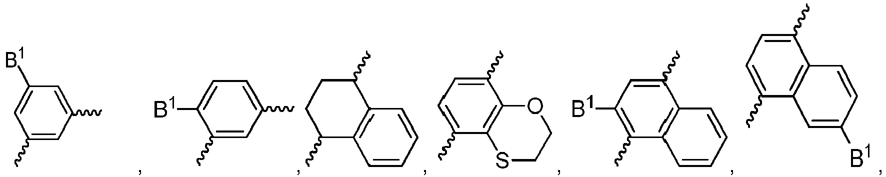 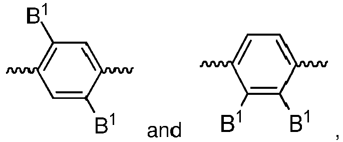 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
C is selected from the group consisting of 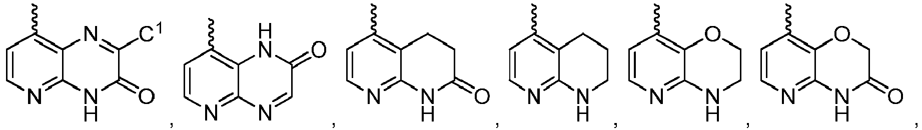 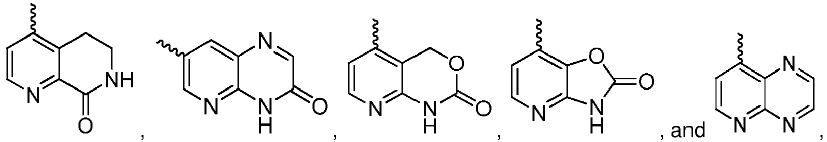 wherein C¹ is selected from the group consisting of -H, -CH₃, -OCH₃, -CN or

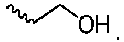

Preferably,
A is 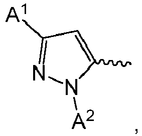
   A¹ is selected from the group consisting of methyl, -CF₃, isopropyl, cyclopropyl, isobutyl, sec-butyl, tert-butyl, 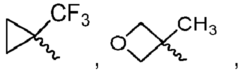
   A² is 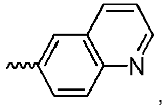
B is a ring system selected from the group consisting of 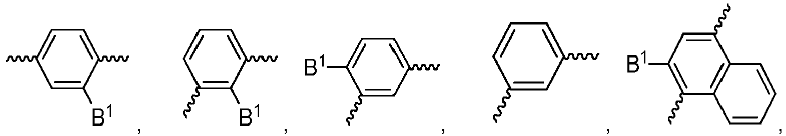 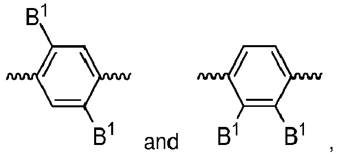 wherein, when B is a phenyl, B¹ is selected from the group consisting of -F, -Cl, -SCH₃, -SCH₂CH₃, -CH₃,-CF₃, -OCH₃, and -OCF₃ and, when B is a naphtyl, B¹ is -H, and
C is selected from the group consisting of 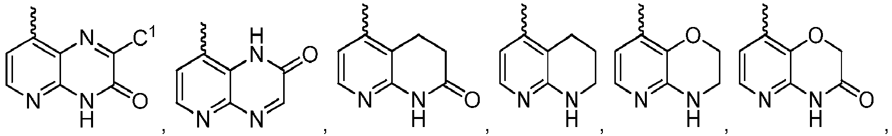 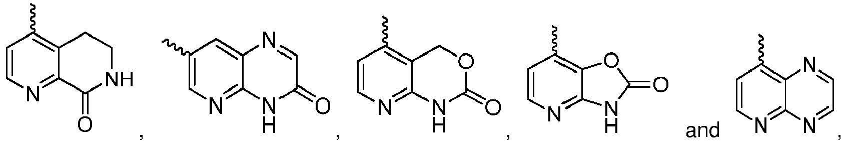 wherein C¹ is selected from the group consisting of -H, -CH₃, -OCH₃, -CN or

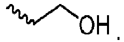

More preferably,
A is 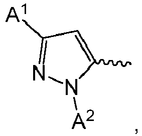
   A¹ is tert-butyl,
   A² is 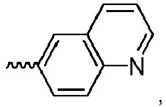
B is a ring system selected from the group consisting of 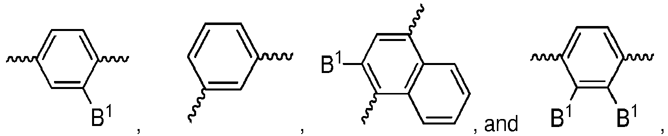 wherein, when B is a phenyl, B¹ is selected from the group consisting of -F, -Cl, -SCH₃, -SCH₂CH₃, -CH₃,-CF₃, -OCH₃, and -OCF₃ and, when B is a naphtyl, B¹ is -H, and
C is selected from the group consisting of

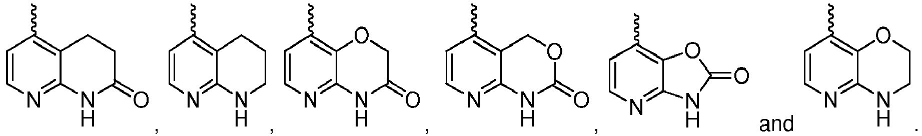

In a very specific aspect, the compound is selected in the group consisting of
1-(3-(tert-butyl)-1-(quinolin-6-yl)-1H-pyrazol-5-yl)-3-(2-fluoro-4-((3-oxo-3,4-dihydropyrido[2,3-b]pyrazin-8-yl)oxy)phenyl)urea;
1-(3-(tert-butyl)-1-(quinolin-6-yl)-1H-pyrazol-5-yl)-3-(4-((3-oxo-3,4-dihydropyrido[2,3-b]pyrazin-8-yl)oxy)naphthalen-1-yl)urea; and
1-(3-(tert-butyl)-1-(quinolin-6-yl)-1H-pyrazol-5-yl)-3-(2-(methylthio)-4-((3-oxo-3,4-dihydropyrido[2,3-b]pyrazin-8-yl)oxy)phenyl)urea;
or a pharmaceutically acceptable salt thereof.

In a sixth aspect, the compound is represented by structure (I), 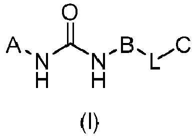
L is -O-
A is a ring system selected from the group consisting of 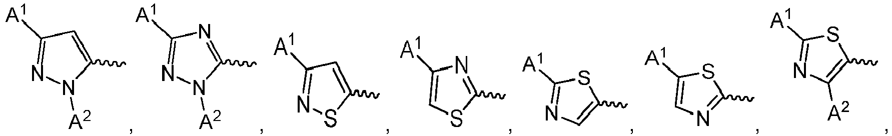 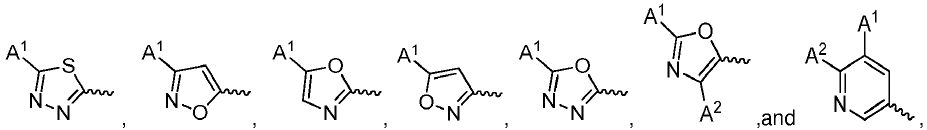 wherein A¹ is selected from the group consisting of C₁-C₄ alkyl, C₁-C₄ cycloalkyl, 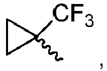 C3-C7 heterocycloalkyl, 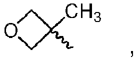 phenyl, benzyl and 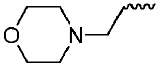 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 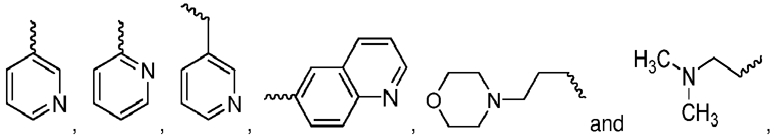 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 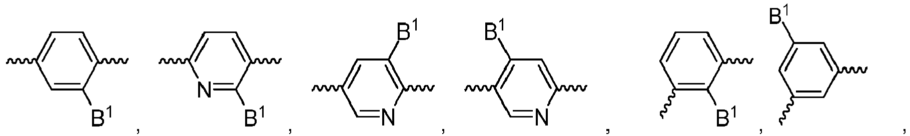 and 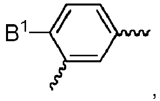 wherein B¹ is -SCH₃,
C is selected from the group consisting of 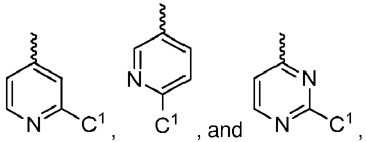 wherein C¹ is selected from the group consisting of wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR'" or COOR"', wherein R', R" and R'" are independently a C₁-C₆ alkyl.

Preferably,
A is a ring selected from the group consisting of 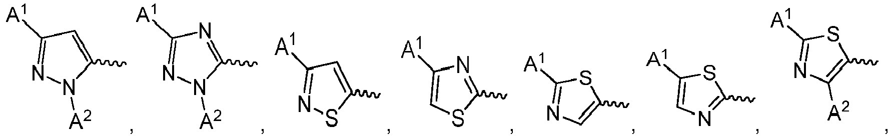 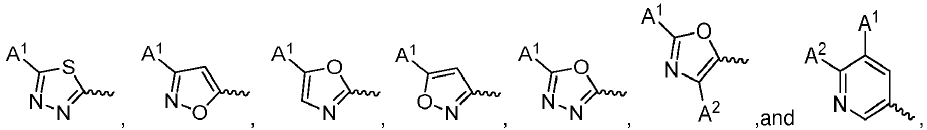 A¹ is selected from the group consisting of methyl, -CF₃, isopropyl, cyclopropyl, isobutyl, sec-butyl, tert-butyl, 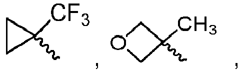 and A₂ is selected from the group consisting of H, methyl, iso-propyl, benzyl, phenyl, chlorine, 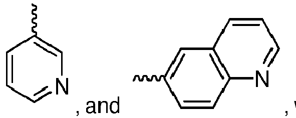 wherein A¹ and A² are optionally fluorinated, and A₂ is optionally substituted with 1, 2 or 3 substituents selected from the group consisting of C₁-C₄ alkyl, -Cl, -F, -CN, -OR^{ABC}, and-SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 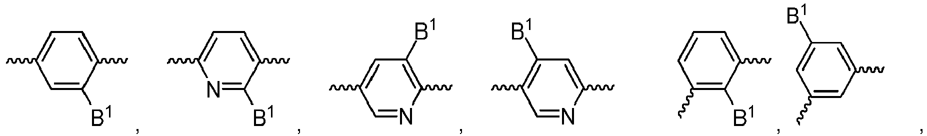 and 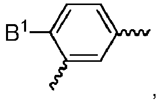 wherein B¹ is -SCH₃,
C is selected from the group consisting of 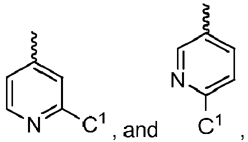 with C¹ being selected C¹ is selected from the group consisting of wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹,-OR¹, -SO₂R¹, with R¹ being selected from the group consisting of C₁-C₁₀ alkyl, C₆-C₁₄ aryl, C₃-C₁₃ heteroaryl, and C₇-C₂₄ alkaryl, wherein each hydrogen in each of these groups may be independently replaced by a group selected from a halogen, a hydroxy, -OR', and -NR'R", each hydrogen in each of R' and R" may be independently replaced by -COR'" or COOR"', wherein R', R" and R'" are independently a C₁-C₆ alkyl.

More preferably,
A is a ring selected from the group consisting of 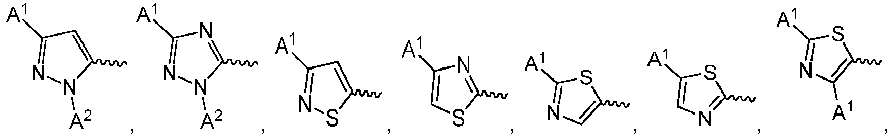 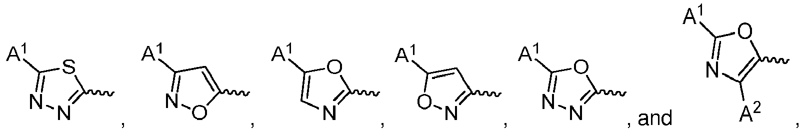
   A¹ is tert-butyl,
   A² is a phenyl or 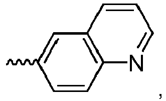 optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 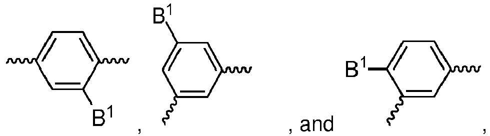 wherein B¹ is -SCH₃,
C is selected from the group consisting of 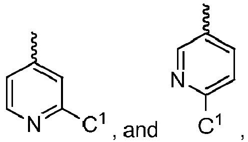 with C¹ being selected from the group consisting of -H, -NH₂, 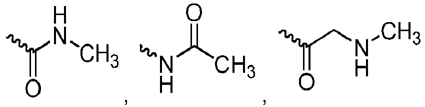 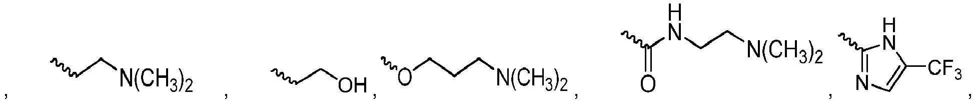 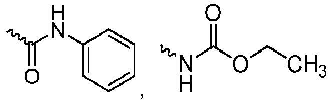 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH₃; preferably from the group consisting of 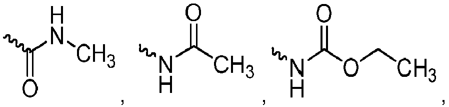 -NH-C(O)OtBu, -NH- C(O)N(CH₃)-C(O)OtBu, -NH-C(O)-CH₂-N(CH₃)-C(O)OtBu, -NH-C(O)-CH₂-NHCH₃, -NH-C(O)-Obenzyl, and -NH-C(O)-CH₂-OH.

Still more preferably,
A is a ring selected from the group consisting of 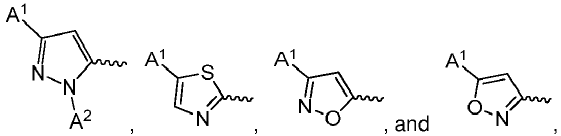
   A¹ is tert-butyl,
   A² is a phenyl optionally substituted with 1, 2 or 3 substituents selected from the group consisting of methyl, -Cl, -F, -CN, methoxy, -O-(CH₂)₂-piperidinyl, -O-(CH₂)₂-morpholinyl, -O-(CH₂)₃-N(CH₃)₂, and -CH₂-morpholinyl, or A² is 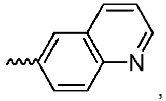
B is 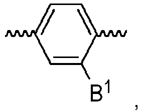 with B¹ being -SCH₃,
C is selected from the group consisting of 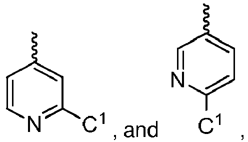 with C¹ being selected from the group consisting of 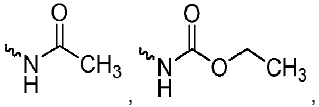 -NH-C(O)OtBu,-NH-C(O)-CH₂-N(CH₃)-C(O)OtBu and -NH-C(O)N(CH3)-COOtBu.

In a very specific aspect, the compound is selected in the group consisting of
5-(4-(3-(3-(tert-butyl)-1-phenyl-1H-pyrazol-5-yl)ureido)-3-(methylthio)phenoxy)-N-methylnicotinamide;
N-(4-(4-(3-(3-(tert-butyl)-1-phenyl-1H-pyrazol-5-yl)ureido)-3-(methylthio)phenoxy)pyridin-2-yl)acetamide;
tert-butyl (4-(4-(3-(3-(tert-butyl)-1-phenyl-1H-pyrazol-5-yl)ureido)-3-(methylthio)phenoxy)pyridin-2-yl)carbamate;
tert-butyl (2-((4-(4-(3-(3-(tert-butyl)-1-phenyl-1H-pyrazol-5-yl)ureido)-3-(methylthio)phenoxy)pyridin-2-yl)amino)-2-oxoethyl)(methyl)carbamate;
N-(4-(4-(3-(3-(tert-butyl)-1-phenyl-1H-pyrazol-5-yl)ureido)-3-(methylthio)phenoxy)pyridin-2-yl)-2-(methylamino)acetamide;
1-(3-(tert-butyl)-1-phenyl-1H-pyrazol-5-yl)-3-(4-((2-((2-(dimethylamino)ethyl)amino)pyridin-4-yl)oxy)-2-(methylthio)phenyl)urea hydrochloride;
ethyl (4-(4-(3-(3-(tert-butyl)-1-phenyl-1H-pyrazol-5-yl)ureido)-3-(methylthio)phenoxy)pyridin-2-yl)carbamate;
benzyl (4-(4-(3-(3-(tert-butyl)-1-phenyl-1H-pyrazol-5-yl)ureido)-3-(methylthio)phenoxy)pyridin-2-yl)carbamate;
ethyl (4-(4-(3-(3-(tert-butyl)isoxazol-5-yl)ureido)-3-(methylthio)phenoxy)pyridin-2-yl)carbamate;
ethyl (4-(4-(3-(5-(tert-butyl)thiazol-2-yl)ureido)-3-(methylthio)phenoxy)pyridin-2-yl)carbamate; and
ethyl (4-(4-(3-(5-(tert-butyl)isoxazol-3-yl)ureido)-3-(methylthio)phenoxy)pyridin-2-yl)carbamate;
or a pharmaceutically acceptable salt thereof.

In a seventh aspect, the compound is represented by structure (I), 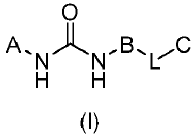
L is -O-
A is a ring system selected from the group consisting of 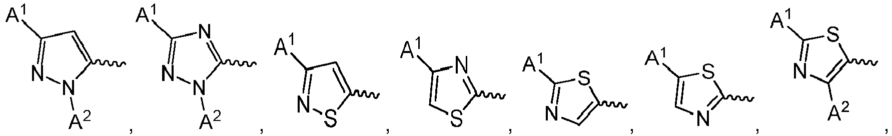 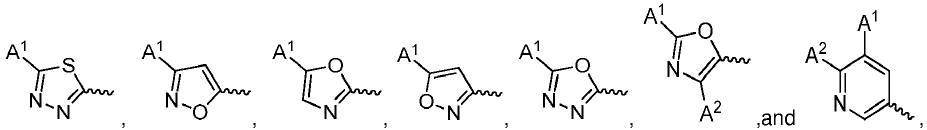 wherein A¹ is selected from the group consisting of C₁-C₄ alkyl, C₁-C₄ cycloalkyl, 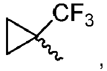 C3-C7 heterocycloalkyl, 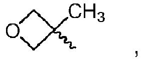 phenyl, benzyl and 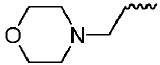 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 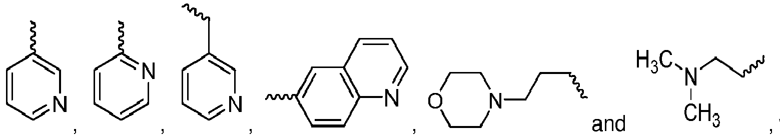 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 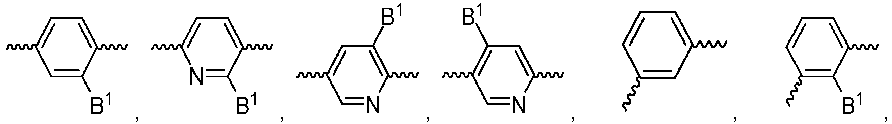 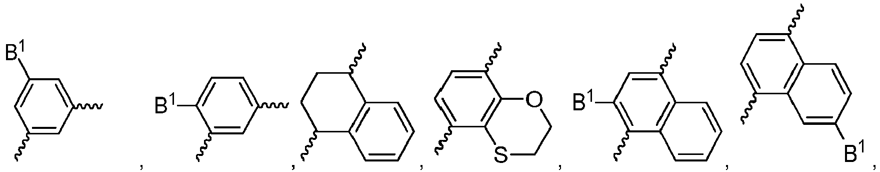 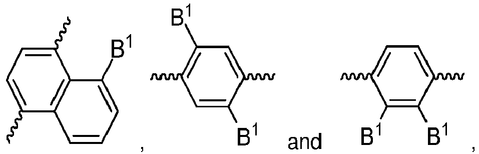 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
C is selected from the group consisting of 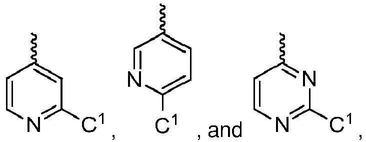 wherein C¹ is selected from the group consisting of -NH-C(O)R¹ or -NH-C(O)-OR¹ with R¹ being selected from the group consisting of C₁-C₁₀ alkyl, C₆-C₁₄ aryl, C₃-C₁₃ heteroaryl, and C₇-C₂₄ alkaryl, wherein each hydrogen in each of these groups may be independently replaced by a group selected from halogen, hydroxy, -OR', and -NR'R", each hydrogen in each of R' and R" may be independently replaced by -COR'" or COOR"', wherein R', R" and R'" are independently a C₁-C₆ alkyl.

Optionally, C¹ is selected from the group consisting of -H, -NH₂, 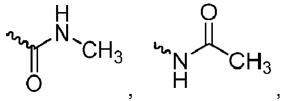 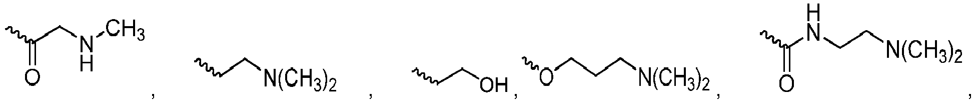 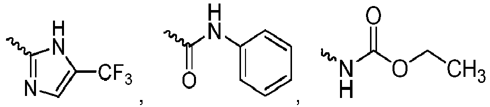 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH_{3;} more preferably from the group consisting of 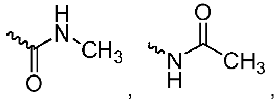 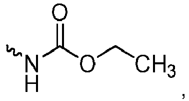 -NH-C(O)OtBu, -NH- C(O)N(CH₃)-C(O)OtBu, -NH-C(O)-CH₂-N(CH₃)-C(O)OtBu, -NH-C(O)-CH₂-NHCH₃, -NH-C(O)-Obenzyl, and -NH-C(O)-CH₂-OH.

Preferably,
A is a ring selected from the group consisting of 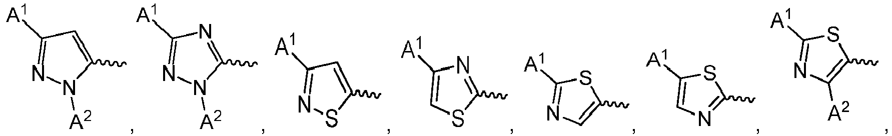 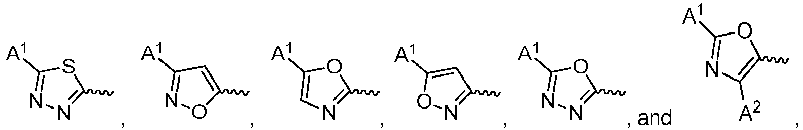
   A¹ is tert-butyl,
   A² is a phenyl or 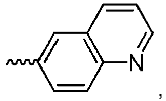 optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 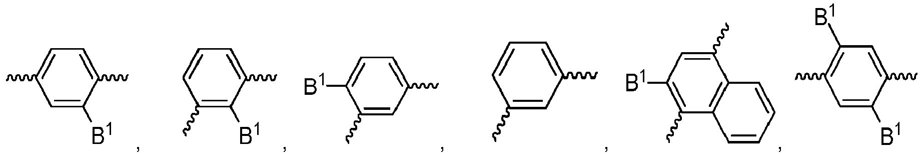 and 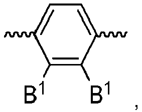 wherein, when B is a phenyl, B¹ is selected from the group consisting of -F, -SCH₃, -SCH₂CH₃, -CF₃, -OCH₃, and -OCF₃ and, when B is a naphtyl, B¹ is -H, and
C is selected from the group consisting of 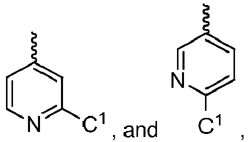 with C¹ being selected from the group consisting of 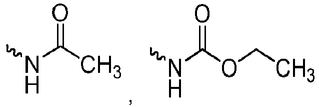 -NH-C(O)OtBu, - NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, preferably

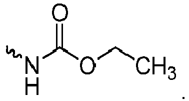

In any aspect as disclosed above, when possible, A can be a ring selected from the group consisting of 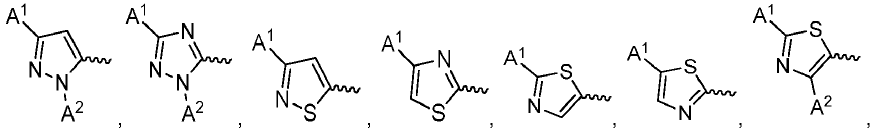 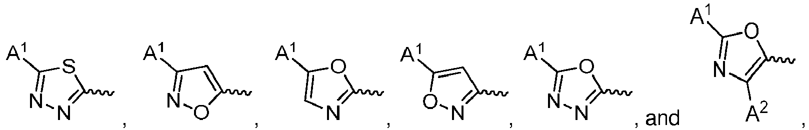 A¹ is tert-butyl, A² is a phenyl or 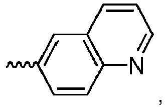 optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl.

In another particular aspect, A is a ring selected from the group consisting of 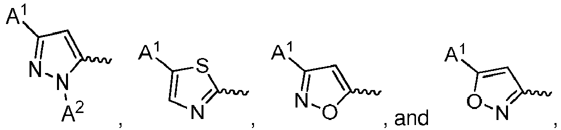 A¹ is tert-butyl,
A² is a phenyl optionally substituted with 1, 2 or 3 substituents selected from the group consisting of methyl, -Cl, -F, -CN, methoxy, -O-(CH₂)₂-piperidinyl, -O-(CH₂)₂-morpholinyl, -O-(CH₂)₃-N(CH₃)₂, and -CH₂-morpholinyl, or A² is

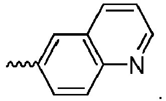

In any aspect as disclosed above, when possible, B can be a ring system selected from the group consisting of

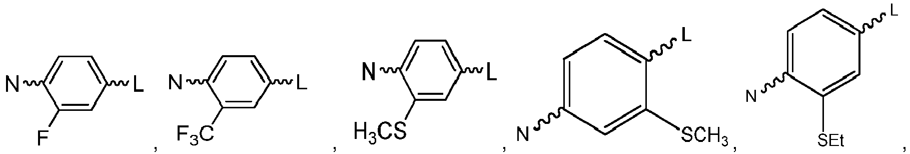

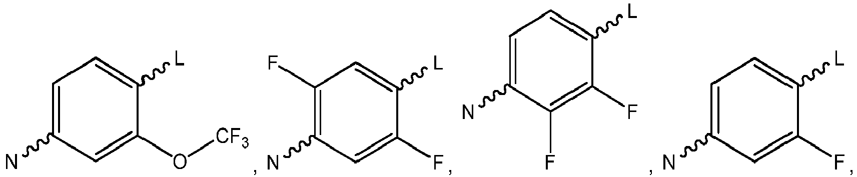

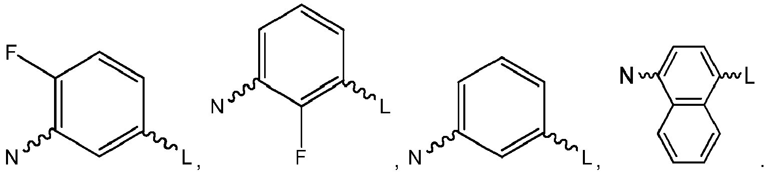

In a specific aspect, B is a ring system selected from the group consisting of

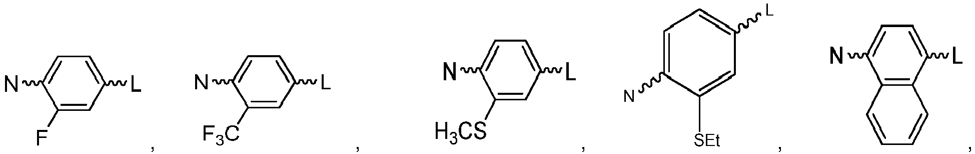

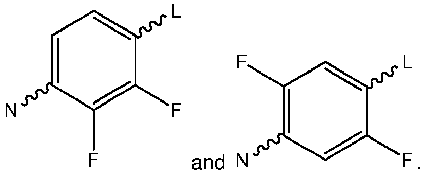

In a very specific aspect, B is a ring system selected from the group consisting of

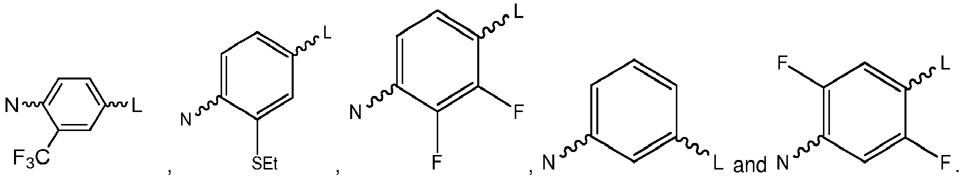

Hence, in a preferred embodiment is provided a compound according to the invention, wherein said compound is represented by structures (III-a)-(III-m), preferably wherein L is O,

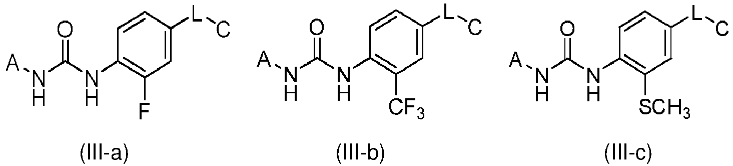

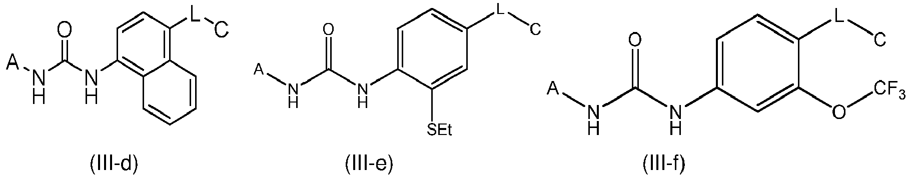

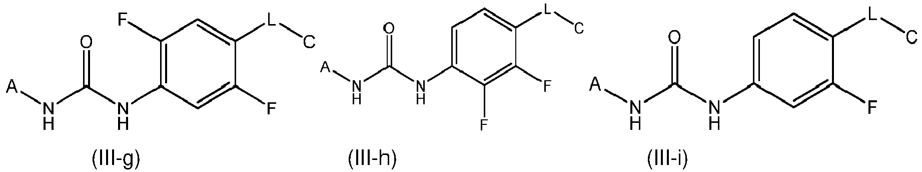

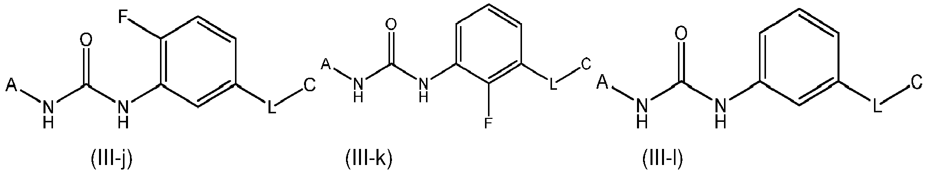

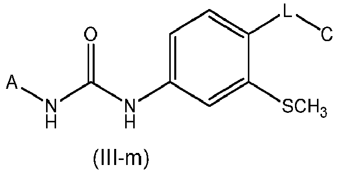

In a specific aspect, the compound may be represented by the structures (III-a), (III-b), (III-c), (III-d), (III-e), (III-g), and (III-h).

In another specific aspect, the compound may be represented by the structures (III-b), (III-e), (III-g), (III-h) and (III-l).

In another specific aspect, the compound may be represented by the structures (III-a)-(III-d).

In any aspect as disclosed above, when possible, C can be selected from the group consisting of 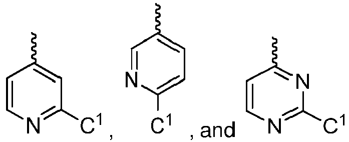 wherein C¹ is selected from the group consisting of wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR'" or COOR"', wherein R', R" and R'" are independently a C₁-C₆ alkyl.

Preferably, C is 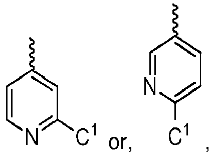 more preferably

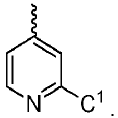

Preferably, C¹ is selected from the group consisting of -H, -NH₂, 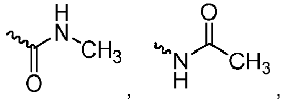 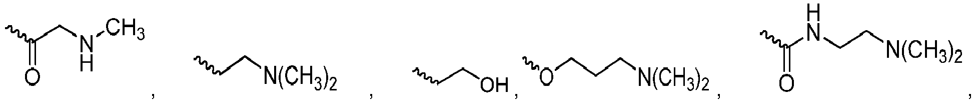 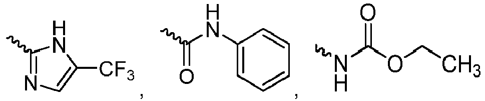 -NH-C(O)-OtBu, -NH-C(O)-N(CH₃)-C(O)-OtBu, -NH-C(O)-CH₂-N(CH₃)-C(O)-OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH_{3;} more preferably from the group consisting of 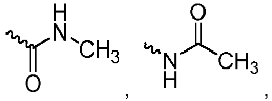 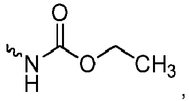 -NH-C(O)-OtBu, -NH-C(O)-N(CH₃)-C(O)-OtBu, -NH-C(O)-CH₂-N(CH₃)-C(O)-OtBu,-NH-C(O)-CH₂-NHCH₃, -NH-C(O)-Obenzyl, and -NH-C(O)-CH₂-OH.

In a very specific aspect, C is selected from the group consisting of 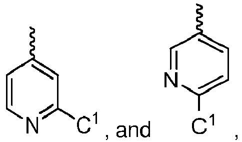 with C¹ being selected from the group consisting of 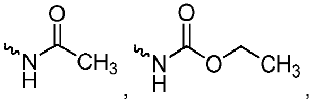 -NH-C(O)-OtBu, -NH-C(O)-N(CH₃)-CO-OtBu and -NH-C(O)-CH₂-N(CH₃)-C(O)-OtBu.

Alternatively, in any aspect as disclosed above, when possible, C can be selected from the group consisting of 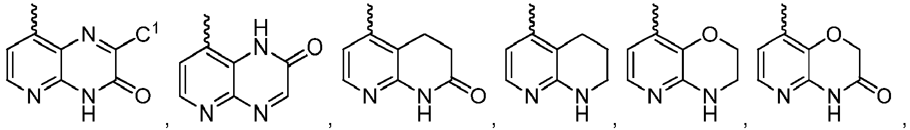 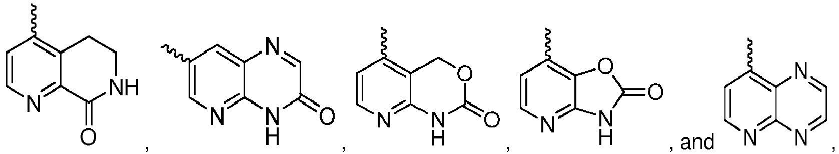 wherein C¹ is selected from the group consisting of -H, -CH₃, -OCH₃, -CN or

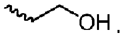

Optionally, C is selected in the group consisting of 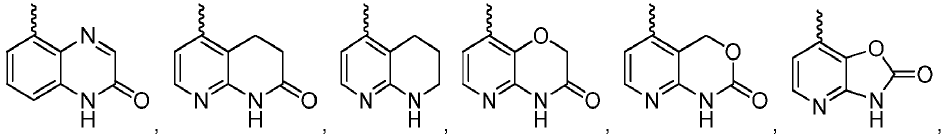 and

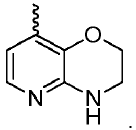

Optionally, C is selected in the group consisting of

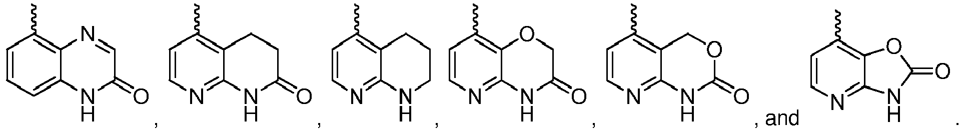

Optionally, C is selected from the group consisting of

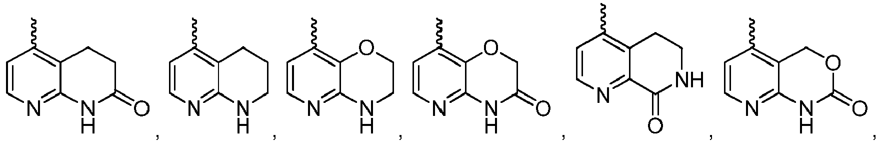

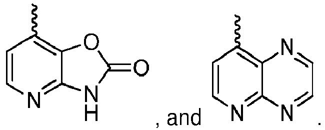

Optionally, C is selected from the group consisting of

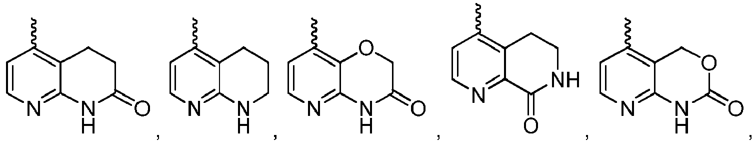

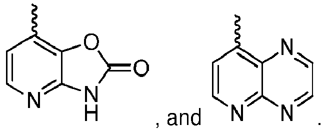

Optionally, C is selected from the group consisting of

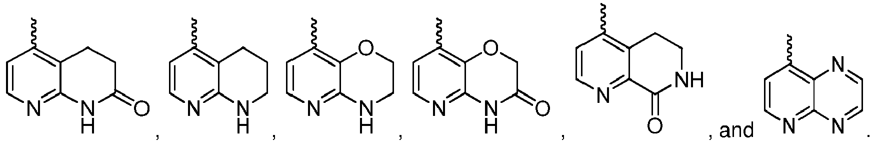

Optionally, C is selected from the group consisting of

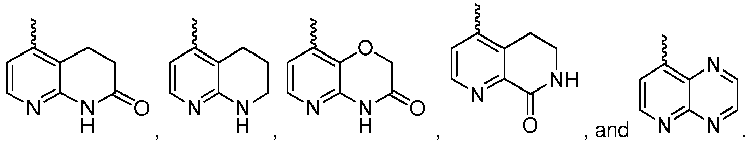

Optionally, C is selected from the group consisting of

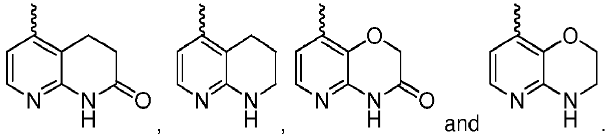

Optionally, C is selected from the group consisting of

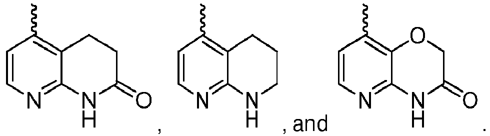

In a specific aspect, B¹ is selected from the group consisting of -SCH₃, -CF₃, -OCF₃, -OCH₃, and SCH₂CH₃, or B has two B¹ groups selected independently from the group consisting of -F, -CI, -SCH₃, -SCH₂CH₃, - CH₃, isopropyl, -CF₃, -OCH₃, -OCF₃ and
A¹ is selected from the group consisting of methyl, -CF₃, isopropyl, cyclopropyl, isobutyl, sec-butyl, tert-butyl, 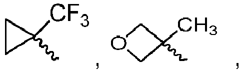 preferably tert-butyl,
and A² is selected from the group consisting of a phenyl substituted with 1, 2 or 3 substituents selected from the group consisting of methoxy, -Cl, -F, -CN, -O-(CH₂)₂-piperidinyl, -O-(CH₂)₂-morpholinyl, -O-(CH₂)₃-N(CH₃)₂, and -CH₂-morpholinyl; a phenyl or pyridinyl substituted with 2 or 3 methyl, and a pyridinyl substituted with 1, 2 or 3 substituents selected from the group consisting of methyl, -Cl, -F, -CN, -O-(CH₂)₂-piperidinyl, -O-(CH₂)₂-morpholinyl, -O-(CH₂)₃-N(CH₃)₂, and -CH₂-morpholinyl; preferably selected from the group consisting of -Cl, -F, -CN, -O-(CH₂)₂-piperidinyl, -O-(CH₂)₃-N(CH₃)₂, and -CH₂-morpholinyl; a pyridinyl substituted with a methyl; and a phenyl substituted by two methyl and
C is selected from the group consisting of 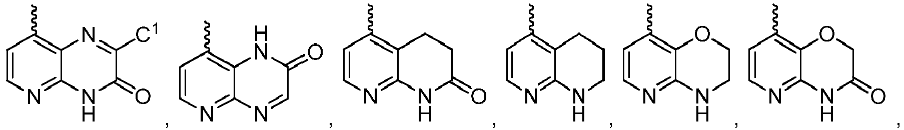 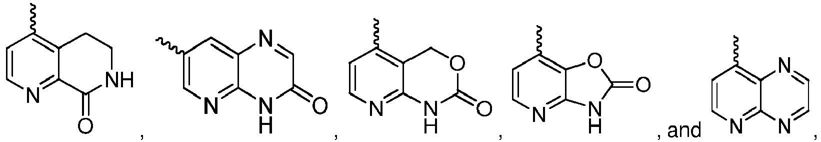 wherein C¹ is selected from the group consisting of -H, -CH₃, -OCH₃, -CN or 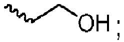 preferably from the group consisting of

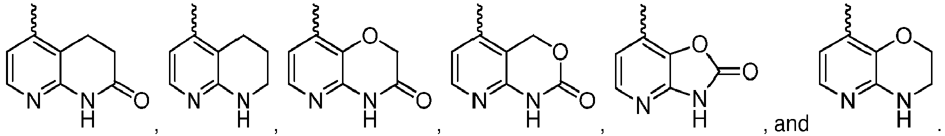

In another specific aspect, B¹ is selected from the group consisting of -SCH₃, -CF₃, -OCF₃, -OCH₃, and SCH₂CH₃, or B has two B¹ groups selected independently from the group consisting of -F, -Cl, -SCH₃,-SCH₂CH₃, -CH₃, isopropyl, -CF₃, -OCH₃, -OCF₃
A¹ is selected from the group consisting of methyl, -CF₃, isopropyl, cyclopropyl, isobutyl, sec-butyl, tert-butyl, 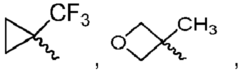 preferably A¹ is tert-butyl,
A² is 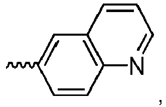 and C is selected from the group consisting of 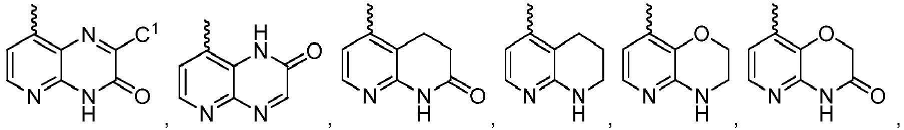 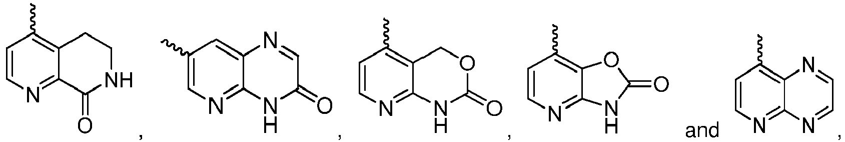 wherein C¹ is selected from the group consisting of -H, -CH₃, -OCH₃, -CN or 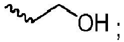 preferably C is selected from the group consisting of

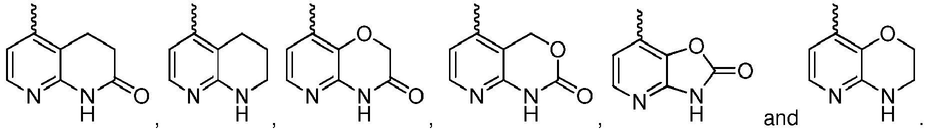

In an additional specific aspect, B¹ is selected from the group consisting of -SCH₃, -CF₃, -OCF₃, -OCH₃, and SCH₂CH₃, or B has two B¹ groups selected independently from the group consisting of -F, -Cl, -SCH₃, -SCH₂CH₃, -CH₃, isopropyl, -CF₃, -OCH₃, -OCF₃,
A¹ is selected from the group consisting of methyl, -CF₃, isopropyl, cyclopropyl, isobutyl, sec-butyl, tert-butyl, 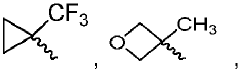 preferably tert-butyl,
A² is a phenyl optionally substituted with 1, 2 or 3 substituents selected from the group consisting of methyl, -Cl, -F, -CN, methoxy, -O-(CH₂)₂-piperidinyl, -O-(CH₂)₂-morpholinyl, -O-(CH₂)₃-N(CH₃)₂, and -CH₂-morpholinyl, or A² is 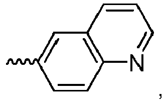 and
C is selected from the group consisting of 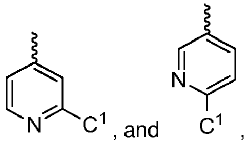 with C¹ being selected from the group consisting of -H, -NH₂, 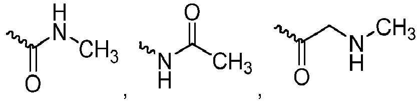 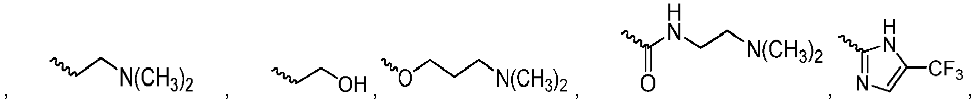 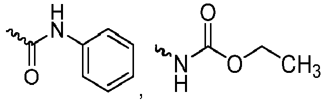 -NH-C(O)-OtBu, -NH-C(O)-N(CH₃)-C(O)-OtBu, -NH-C(O)-CH₂-N(CH₃)-C(O)-OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH₃; preferably from the group consisting of 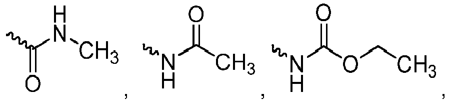 -NH-C(O-)OtBu, -NH-C(O)-N(CH₃)-C(O)-OtBu, -NH-C(O)-CH₂-N(CH₃)-C(O)-OtBu, -NH-C(O)-CH₂-NHCH₃,-NH-C(O)-Obenzyl, and -NH-C(O)-CH₂-OH.

More specifically, B¹ is -SCH₃ in any of these specific aspects.

In a particular aspect, the compound is any compound as disclosed in Table 1.

**Table 1**

| Compound | Structure | Name |
|---|---|---|
| (a) | 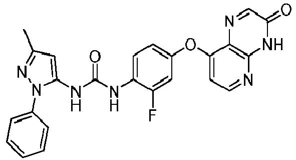 | 1-(2-fluoro-4-((3-oxo-3,4-dihydropyrido[2,3-b]pyrazin-8-yl)oxy)phenyl)-3-(3-methyl-1-phenyl-1 H-pyrazol-5-yl)urea |
| (b) | 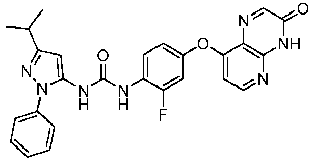 | 1-(2-fluoro-4-((3-oxo-3,4-dihydropyrido[2,3-b]pyrazin-8-yl)oxy)phenyl)-3-(3-isopropyl-1-phenyl-1 H-pyrazol-5-yl)urea |
| (c) | 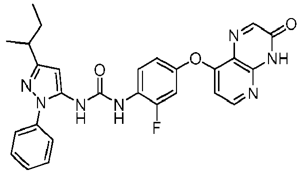 | 1-(3-(sec-butyl)-1-phenyl-1 H-pyrazol-5-yl)-3-(2-fluoro-4-((3-oxo-3,4-dihydropyrido[2,3-b]pyrazin-8-yl)oxy)phenyl)urea |
| (d) | 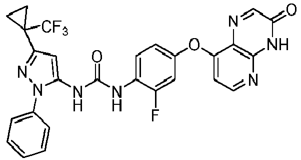 | 1-(2-fluoro-4-((3-oxo-3,4-dihydropyrido[2,3-b]pyrazin-8-yl)oxy)phenyl)-3-(1-phenyl-3-(1-(trifluoromethyl)cyclopropyl)-1 H-pyrazol-5-yl)urea |
| (e) | 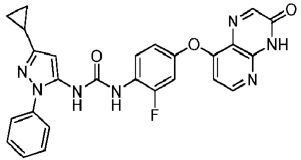 | 1-(3-cyclopropyl-1-phenyl-1 H-pyrazol-5-yl)-3-(2-fluoro-4-((3-oxo-3,4-dihydropyrido[2,3-b]pyrazin-8-yl)oxy)phenyl)urea |
| (f) | 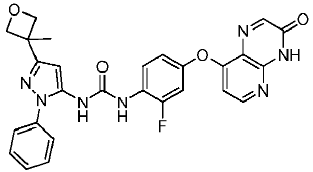 | 1-(2-fluoro-4-((3-oxo-3,4-dihydropyrido[2,3-b]pyrazin-8-yl)oxy)phenyl)-3-(3-(3-methyloxetan-3-yl)-1-phenyl-1 H-pyrazol-5-yl)urea |
| (g) | 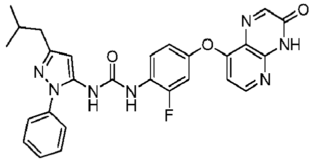 | 1-(2-fluoro-4-((3-oxo-3,4-dihydropyrido[2,3-b]pyrazin-8-yl)oxy)phenyl)-3-(3-isobutyl-1-phenyl-1H-pyrazol-5-yl)urea |
| (h) | 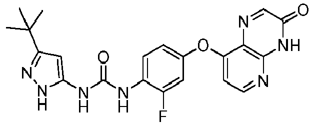 | 1-(3-(tert-butyl)-1H-pyrazol-5-yl)-3-(2-fluoro-4-((3-oxo-3,4-dihydropyrido[2,3-b]pyrazin-8-yl)oxy)phenyl)urea |
| (i) | 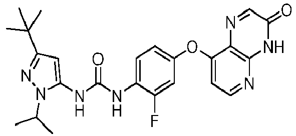 | 1-(3-(tert-butyl)-1-isopropyl-1H-pyrazol-5-yl)-3-(2-fluoro-4-((3-oxo-3,4-dihydropyrido[2,3-b]pyrazin-8-yl)oxy)phenyl)urea |
| (j) | 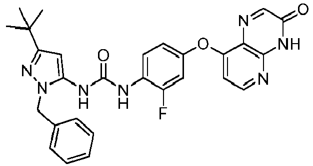 | 1-(1-benzyl-3-(tert-butyl)-1 H-pyrazol-5-yl)-3-(2-fluoro-4-((3-oxo-3,4-dihydropyrido[2,3-b]pyrazin-8-yl)oxy)phenyl)urea |
| (k) | 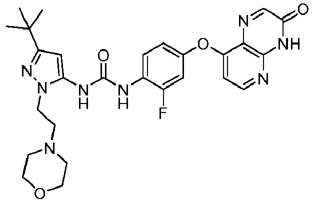 | 1-(3-(tert-butyl)-1-(2-morpholinoethyl)-1 H-pyrazol-5-yl)-3-(2-fluoro-4-((3-oxo-3,4-dihydropyrido[2,3-b]pyrazin-8-yl)oxy)phenyl)urea |
| (l) | 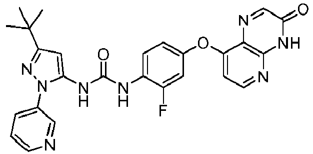 | 1-(3-(tert-butyl)-1-(pyridin-3-yl)-1 H-pyrazol-5-yl)-3-(2-fluoro-4-((3-oxo-3,4-dihydropyrido[2,3-b]pyrazin-8-yl)oxy)phenyl)urea |
| (m) | 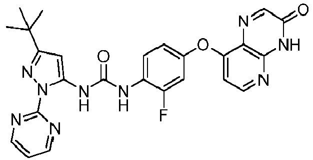 | 1-(3-(tert-butyl)-1-(pyrimidin-2-yl)-1 H-pyrazol-5-yl)-3-(2-fluoro-4-((3-oxo-3,4-dihydropyrido[2,3-b]pyrazin-8-yl)oxy)phenyl)urea |
| (n) | 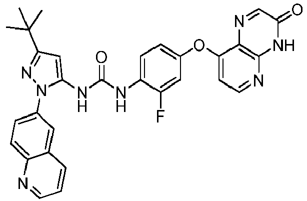 | 1-(3-(tert-butyl)-1-(quinolin-6-yl)-1 H-pyrazol-5-yl)-3-(2-fluoro-4-((3-oxo-3,4-dihydropyrido[2,3-b]pyrazin-8-yl)oxy)phenyl)urea |
| (o) | 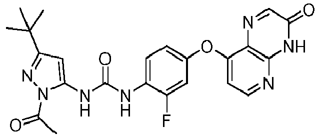 | 1-(1-acetyl-3-(tert-butyl)-1 H-pyrazol-5-yl)-3-(2-fluoro-4-((3-oxo-3,4-dihydropyrido[2,3-b]pyrazin-8-yl)oxy)phenyl)urea |
| (p) | 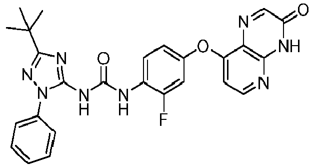 | 1-(3-(tert-butyl)-1-phenyl-1 H-1,2,4-triazol-5-yl)-3-(2-fluoro-4-((3-oxo-3,4-dihydropyrido[2,3-b]pyrazin-8-yl)oxy)phenyl)urea |
| (q) | 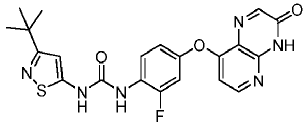 | 1-(3-(tert-butyl)isothiazol-5-yl)-3-(2-fluoro-4-((3-oxo-3,4-dihydropyrido[2,3-b]pyrazin-8-yl)oxy)phenyl)urea |
| (r) | 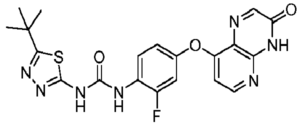 | 1-(5-(tert-butyl)-1,3,4-thiadiazol-2-yl)-3-(2-fluoro-4-((3-oxo-3,4-dihydropyrido[2,3-b]pyrazin-8-yl)oxy)phenyl)urea |
| (s) | 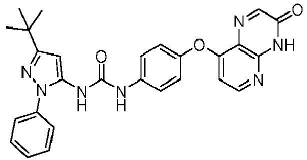 | 1-(3-(tert-butyl)-1-phenyl-1 H-pyrazol-5-yl)-3-(4-((3-oxo-3,4-dihydropyrido[2,3-b]pyrazin-8-yl)oxy)phenyl)urea |
| (t) | 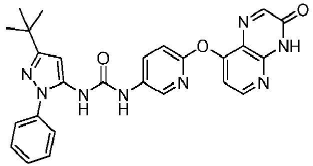 | 1-(3-(tert-butyl)-1-phenyl-1 H-pyrazol-5-yl)-3-(6-((3-oxo-3,4-dihydropyrido[2,3-b]pyrazin-8-yl)oxy)pyridin-3-yl)urea |
| (u) | 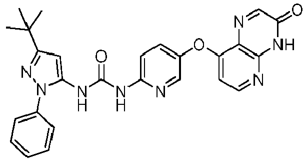 | 1-(3-(tert-butyl)-1-phenyl-1 H-pyrazol-5-yl)-3-(5-((3-oxo-3,4-dihydropyrido[2,3-b]pyrazin-8-yl)oxy)pyridin-2-yl)urea |
| (v) | 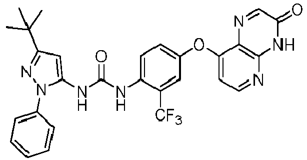 | 1-(3-(tert-butyl)-1-phenyl-1H-pyrazol-5-yl)-3-(4-((3-oxo-3,4-dihydropyrido[2,3-b]pyrazin-8-yl)oxy)-2-(trifluoromethyl)phenyl)urea |
| (w) | 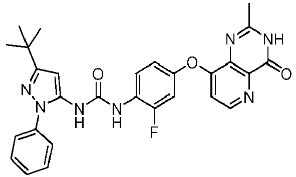 | 1-(3-(tert-butyl)-1-phenyl-1 H-pyrazol-5-yl)-3-(2-fluoro-4-((2-methyl-4-oxo-3,4-dihydropyrido[3,2-d]pyrimidin-8-yl)oxy)phenyl)urea |
| (x) | 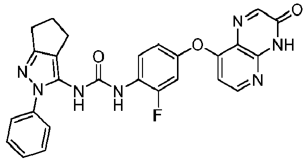 | 1-(2-fluoro-4-((3-oxo-3,4-dihydropyrido[2,3-b]pyrazin-8-yl)oxy)phenyl)-3-(2-phenyl-2,4,5,6-tetrahydrocyclopenta[c]pyrazol-3-yl)urea |
| (y) | 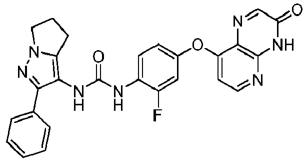 | 1-(2-fluoro-4-((3-oxo-3,4-dihydropyrido[2,3-b]pyrazin-8-yl)oxy)phenyl)-3-(2-phenyl-5,6-dihydro-4H-pyrrolo[1,2-b]pyrazol-3-yl)urea |
| (z) | 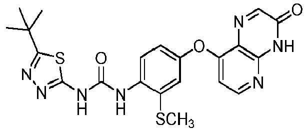 | 1-(5-(tert-butyl)-1,3,4-thiadiazol-2-yl)-3-(2-(methylthio)-4-((3-oxo-3,4-dihydropyrido[2,3-b]pyrazin-8-yl)oxy)phenyl)urea |
| (ab) | 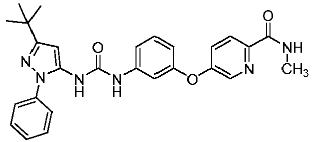 | 5-(3-(3-(3-(tert-butyl)-1-phenyl-1H-pyrazol-5-yl)ureido)phenoxy)-N-methylpicolinamide |
| (ac) | 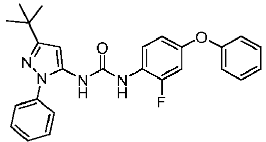 | 1-(3-(tert-butyl)-1-phenyl-1H-pyrazol-5-yl)-3-(2-fluoro-4-phenoxyphenyl)urea |
| (ad) | 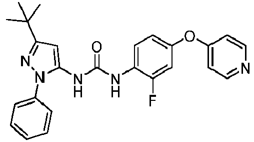 | 1-(3-(tert-butyl)-1-phenyl-1H-pyrazol-5-yl)-3-(2-fluoro-4-(pyridin-4-yloxy)phenyl)urea |
| (ae) | 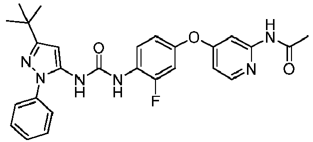 | N-(4-(4-(3-(3-(tert-butyl)-1-phenyl-1H-pyrazol-5-yl)ureido)-3-fluorophenoxy)pyridin-2-yl)acetamide |
| (af) | 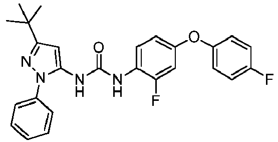 | 1-(3-(tert-butyl)-1-phenyl-1 H-pyrazol-5-yl)-3-(2-fluoro-4-(4-fluorophenoxy)phenyl)urea |
| (ag) | 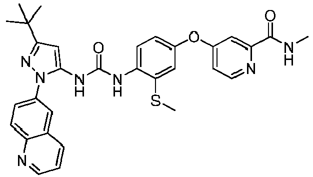 | 4-(4-(3-(3-(tert-butyl)-1-(quinol in-6-yl)-1H-pyrazol-5-yl)ureido)-3-(methylthio)phenoxy)-N-methylpicolinamide |
| (ah) | 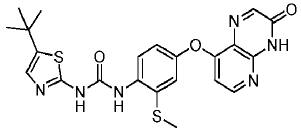 | 1-(5-(tert-butyl)thiazol-2-yl)-3-(2-(methylthio)-4-((3-oxo-3,4-dihydropyrido[2,3-b]pyrazin-8-yl)oxy)phenyl)urea |
| (ai) | 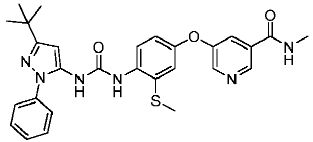 | 5-(4-(3-(3-(tert-butyl)-1-phenyl-1H-pyrazol-5-yl)ureido)-3-(methylthio)phenoxy)-N-methylnicotinamide |
| (aj) | 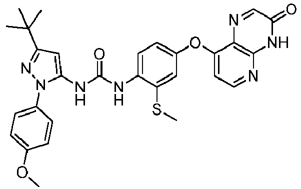 | 1-(3-(tert-butyl)-1-(4-methoxyphenyl)-1 H-pyrazol-5-yl)-3-(2-(methylthio)-4-((3-oxo-3,4-dihydropyrido[2,3-b]pyrazin-8-yl)oxy)phenyl)urea |
| (ak) | 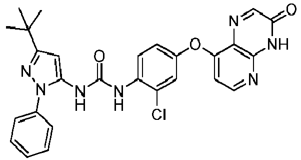 | 1-(3-(tert-butyl)-1-phenyl-1H-pyrazol-5-yl)-3-(2-chloro-4-((3-oxo-3,4-dihydropyrido[2,3-b]pyrazin-8-yl)oxy)phenyl)urea |
| (al) | 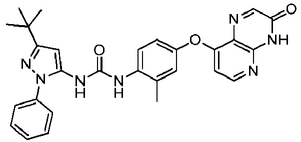 | 1-(3-(tert-butyl)-1-phenyl-1 H-pyrazol-5-yl)-3-(2-methyl-4-((3-oxo-3,4-dihydropyrido[2,3-b]pyrazin-8-yl)oxy)phenyl)urea |
| (am) | 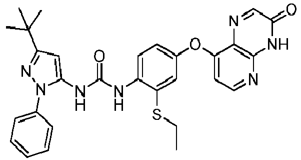 | 1-(3-(tert-butyl)-1-phenyl-1 H-pyrazol-5-yl)-3-(2-(ethylthio)-4-((3-oxo-3,4-dihydropyrido[2,3-b]pyrazin-8-yl)oxy)phenyl)urea |
| (an) | 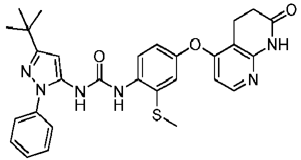 | 1-(3-(tert-butyl)-1-phenyl-1H-pyrazol-5-yl)-3-(2-(methylthio)-4-((7-oxo-5,6,7,8-tetrahydro-1,8-naphthyridin-4-yl)oxy)phenyl)urea |
| (ao) | 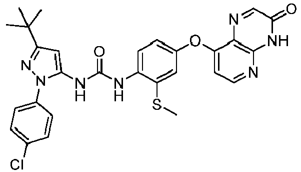 | 1-(3-(tert-butyl)-1-(4-chlorophenyl)-1H-pyrazol-5-yl)-3-(2-(methylthio)-4-((3-oxo-3,4-dihydropyrido[2,3-b]pyrazin-8-yl)oxy)phenyl)urea |
| (ap) | 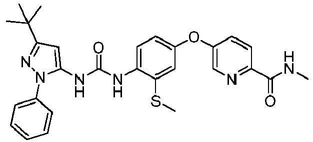 | 5-(4-(3-(3-(tert-butyl)-1-phenyl-1H-pyrazol-5-yl)ureido)-3-(methylthio)phenoxy)-N-methylpicolinamide |
| (aq) | 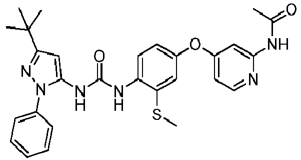 | N-(4-(4-(3-(3-(tert-butyl)-1-phenyl-1H-pyrazol-5-yl)ureido)-3-(methylthio)phenoxy)pyridin-2-yl)acetamide |
| (ar) | 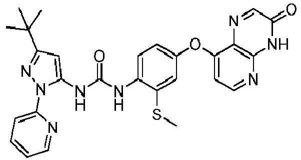 | 1-(3-(tert-butyl)-1-(pyridin-2-yl)-1H-pyrazol-5-yl)-3-(2-(methylthio)-4-((3-oxo-3,4-dihydropyrido[2,3-b]pyrazin-8-yl)oxy)phenyl)urea |
| (as) | 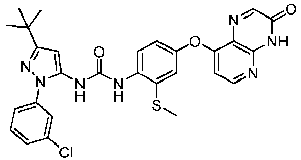 | 1-(3-(tert-butyl)-1-(3-chlorophenyl)-1H-pyrazol-5-yl)-3-(2-(methylthio)-4-((3-oxo-3,4-dihydropyrido[2,3-b]pyrazin-8-yl)oxy)phenyl)urea |
| (at) | 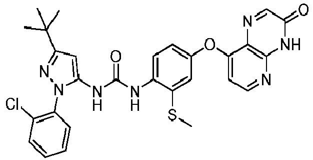 | 1-(3-(tert-butyl)-1-(2-chlorophenyl)-1H-pyrazol-5-yl)-3-(2-(methylthio)-4-((3-oxo-3,4-dihydropyrido[2,3-b]pyrazin-8-yl)oxy)phenyl)urea |
| (au) | 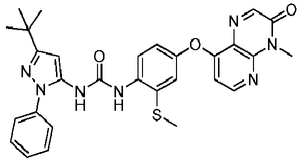 | 1-(3-(tert-butyl)-1-phenyl-1H-pyrazol-5-yl)-3-(4-((4-methyl-3-oxo-3,4-dihydropyrido[2,3-b]pyrazin-8-yl)oxy)-2-(methylthio)phenyl)urea |
| (av) | 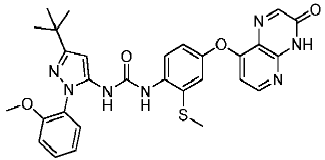 | 1-(3-(tert-butyl)-1-(2-methoxyphenyl)-1H-pyrazol-5-yl)-3-(2-(methylthio)-4-((3-oxo-3,4-dihydropyrido[2,3-b]pyrazin-8-yl)oxy)phenyl)urea |
| (aw) | 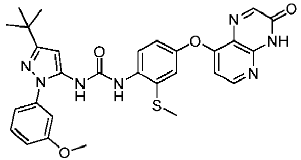 | 1-(3-(tert-butyl)-1-(3-methoxyphenyl)-1 H-pyrazol-5-yl)-3-(2-(methylthio)-4-((3-oxo-3,4-dihydropyrido[2,3-b]pyrazin-8-yl)oxy)phenyl)urea |
| (ax) | 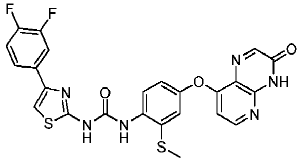 | 1-(4-(3,4-difluorophenyl)thiazol-2-yl)-3-(2-(methylthio)-4-((3-oxo-3,4-dihydropyrido[2,3-b]pyrazin-8-yl)oxy)phenyl)urea |
| (ay) | 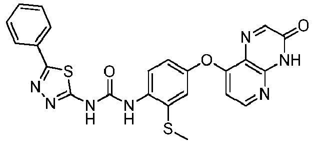 | 1-(2-(methylthio)-4-((3-oxo-3,4-dihydropyrido[2,3-b]pyrazin-8-yl)oxy)phenyl)-3-(5-phenyl-1,3,4-thiadiazol-2-yl)urea |
| (az) | 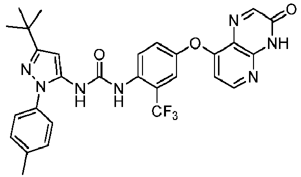 | 1-(3-(tert-butyl)-1-(p-tolyl)-1H-pyrazol-5-yl)-3-(4-((3-oxo-3,4-dihydropyrido[2,3-b]pyrazin-8-yl)oxy)-2-(trifluoromethyl)phenyl)urea |
| (ba) | 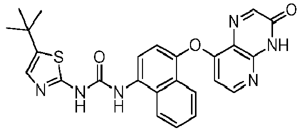 | 1-(5-(tert-butyl)thiazol-2-yl)-3-(4-((3-oxo-3,4-dihydropyrido[2,3-b]pyrazin-8-yl)oxy)naphthalen-1-yl)urea |
| (bb) | 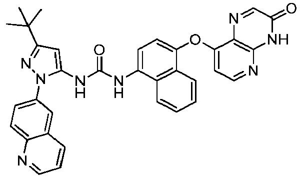 | 1-(3-(tert-butyl)-1-(quinolin-6-yl)-1 H-pyrazol-5-yl)-3-(4-((3-oxo-3,4-dihydropyrido[2,3-b]pyrazin-8-yl)oxy)naphthalen-1-yl)urea |
| (bc) | 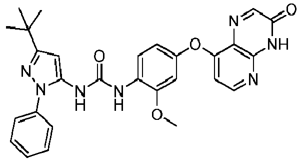 | 1-(3-(tert-butyl)-1-phenyl-1 H-pyrazol-5-yl)-3-(2-methoxy-4-((3-oxo-3,4-dihydropyrido[2,3-b]pyrazin-8-yl)oxy)phenyl)urea |
| (bd) | 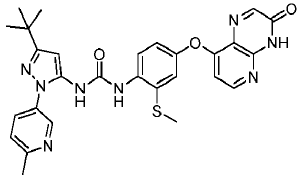 | 1-(3-(tert-butyl)-1-(6-methylpyridin-3-yl)-1 H-pyrazol-5-yl)-3-(2-(methylthio)-4-((3-oxo-3,4-dihydropyrido[2,3-b]pyrazin-8-yl)oxy)phenyl)urea |
| (be) | 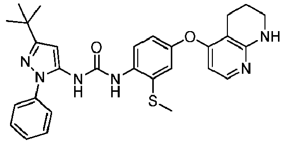 | 1-(3-(tert-butyl)-1-phenyl-1H-pyrazol-5-yl)-3-(2-(methylthio)-4-((5,6,7,8-tetrahydro-1,8-naphthyridin-4-yl)oxy)phenyl)urea |
| (bf) | 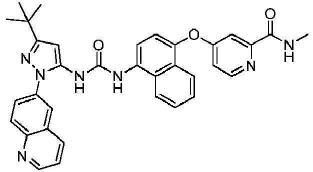 | 4-((4-(3-(3-(tert-butyl)-1-(quinolin-6-yl)-1 H-pyrazol-5-yl)ureido)naphthalen-1-yl)oxy)-N-methylpicolinamide |
| (bg) | 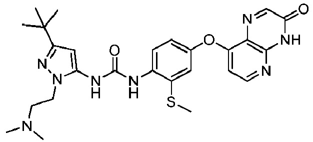 | 1-(3-(tert-butyl)-1-(2-(dimethylamino)ethyl)-1H-pyrazol-5-yl)-3-(2-(methylthio)-4-((3-oxo-3,4-dihydropyrido[2,3-b]pyrazin-8-yl)oxy)phenyl)urea |
| (bh) | 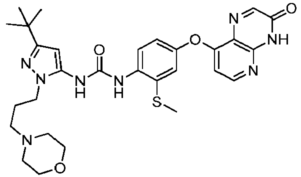 | 1-(3-(tert-butyl)-1-(3-morpholinopropyl)-1 H-pyrazol-5-yl)-3-(2-(methylthio)-4-((3-oxo-3,4-dihydropyrido[2,3-b]pyrazin-8-yl)oxy)phenyl)urea |
| (bi) | 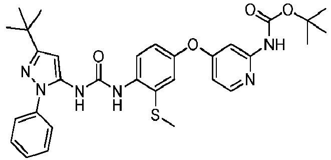 | tert-butyl (4-(4-(3-(3-(tert-butyl)-1-phenyl-1H-pyrazol-5-yl)ureido)-3-(methylthio)phenoxy)pyridin-2-yl)carbamate |
| (bj) | 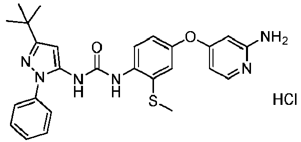 | 1-(4-((2-aminopyridin-4-yl)oxy)-2-(methylthio)phenyl)-3-(3-(tert-butyl)-1-phenyl-1H-pyrazol-5-yl)urea hydrochloride |
| (bk) | 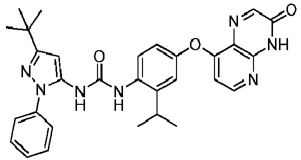 | 1-(3-(tert-butyl)-1-phenyl-1H-pyrazol-5-yl)-3-(2-isopropyl-4-((3-oxo-3,4-dihydropyrido[2,3-b]pyrazin-8-yl)oxy)phenyl)urea |
| (bl) | 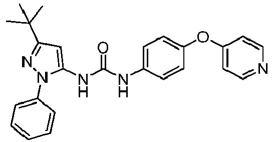 | 1-(3-(tert-butyl)-1-phenyl-1 H-pyrazol-5-yl)-3-(4-(pyridin-4-yloxy)phenyl)urea |
| (bm) | 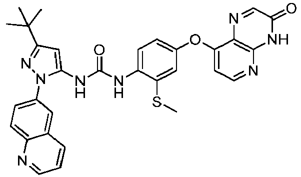 | 1-(3-(tert-butyl)-1-(quinolin-6-yl)-1 H-pyrazol-5-yl)-3-(2-(methylthio)-4-((3-oxo-3,4-dihydropyrido[2,3-b]pyrazin-8-yl)oxy)phenyl)urea |
| (bn) | 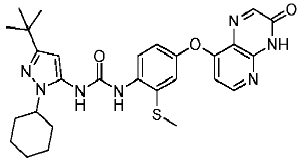 | 1-(3-(tert-butyl)-1-cyclohexyl-1H-pyrazol-5-yl)-3-(2-(methylthio)-4-((3-oxo-3,4-dihydropyrido[2,3-b]pyrazin-8-yl)oxy)phenyl)urea |
| (bo) | 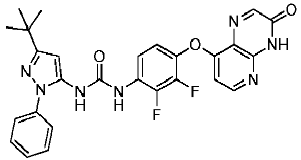 | 1-(3-(tert-butyl)-1-phenyl-1 H-pyrazol-5-yl)-3-(2,3-difluoro-4-((3-oxo-3,4-dihydropyrido[2,3-b]pyrazin-8-yl)oxy)phenyl)urea |
| (bp) | 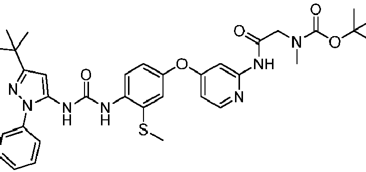 | tert-butyl (2-((4-(4-(3-(3-(tert-butyl)-1-phenyl-1H-pyrazol-5-yl)ureido)-3-(methylthio)phenoxy)pyridin-2-yl)amino)-2-oxoethyl)(methyl)carbamate |
| (bq) | 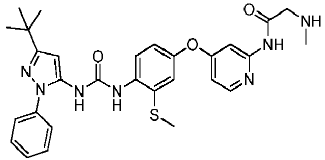 | N-(4-(4-(3-(3-(tert-butyl)-1-phenyl-1H-pyrazol-5-yl)ureido)-3-(methylthio)phenoxy)pyridin-2-yl)-2-(methylamino)acetamide |
| (br) | 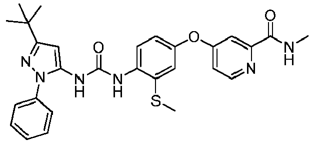 | 4-(4-(3-(3-(tert-butyl)-1-phenyl-1H-pyrazol-5-yl)ureido)-3-(methylthio)phenoxy)-N-methylpicolinamide |
| (bs) | 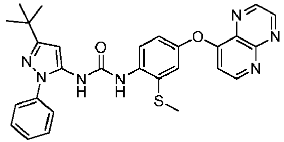 | 1-(3-(tert-butyl)-1-phenyl-1 H-pyrazol-5-yl)-3-(2-(methylthio)-4-(pyrido[2,3-b]pyrazin-8-yloxy)phenyl)urea |
| (bt) | 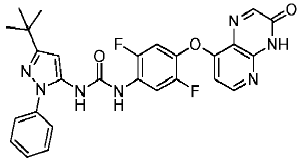 | 1-(3-(tert-butyl)-1-phenyl-1 H-pyrazol-5-yl)-3-(2,5-difluoro-4-((3-oxo-3,4-dihydropyrido[2,3-b]pyrazin-8-yl)oxy)phenyl)urea |
| (bu) | 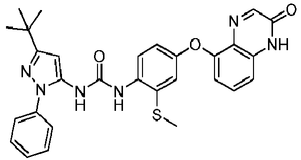 | 1-(3-(tert-butyl)-1-phenyl-1H-pyrazol-5-yl)-3-(2-(methylthio)-4-((2-oxo-1,2-dihydroquinoxalin-5-yl)oxy)phenyl)urea |
| (bv) | 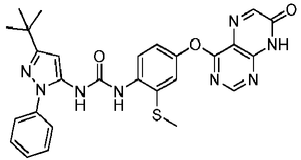 | 1-(3-(tert-butyl)-1-phenyl-1H-pyrazol-5-yl)-3-(2-(methylthio)-4-((7-oxo-7,8-dihydropteridin-4-yl)oxy)phenyl)urea |
| (bw) | 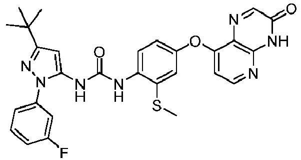 | 1-(3-(tert-butyl)-1-(3-fluorophenyl)-1H-pyrazol-5-yl)-3-(2-(methylthio)-4-((3-oxo-3,4-dihydropyrido[2,3-b]pyrazin-8-yl)oxy)phenyl)urea |
| (bx) | 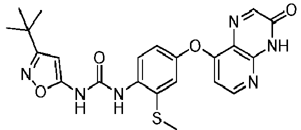 | 1-(3-(tert-butyl)isoxazol-5-yl)-3-(2-(methylthio)-4-((3-oxo-3,4-dihydropyrido[2,3-b]pyrazin-8-yl)oxy)phenyl)urea |
| (by) | 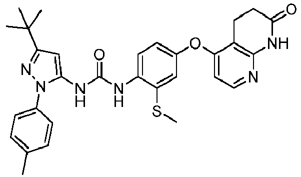 | 1-(3-(tert-butyl)-1-(p-tolyl)-1H-pyrazol-5-yl)-3-(2-(methylthio)-4-((7-oxo-5,6,7,8-tetrahydro-1,8-naphthyridin-4-yl)oxy)phenyl)urea |
| (bz) | 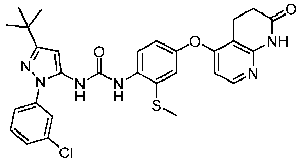 | 1-(3-(tert-butyl)-1-(3-chlorophenyl)-1H-pyrazol-5-yl)-3-(2-(methylthio)-4-((7-oxo-5,6,7,8-tetrahydro-1,8-naphthyridin-4-yl)oxy)phenyl)urea |
| (ca) | 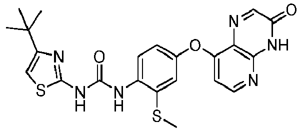 | 1-(4-(tert-butyl)thiazol-2-yl)-3-(2-(methylthio)-4-((3-oxo-3,4-dihydropyrido[2,3-b]pyrazin-8-yl)oxy)phenyl)urea |
| (cb) | 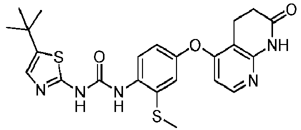 | 1-(5-(tert-butyl)thiazol-2-yl)-3-(2-(methylthio)-4-((7-oxo-5,6,7,8-tetrahydro-1,8-naphthyridin-4-yl)oxy)phenyl)urea |
| (cc) | 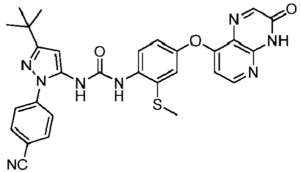 | 1-(3-(tert-butyl)-1-(4-cyanophenyl)-1H-pyrazol-5-yl)-3-(2-(methylthio)-4-((3-oxo-3,4-dihydropyrido[2,3-b]pyrazin-8-yl)oxy)phenyl)urea |
| (cd) | 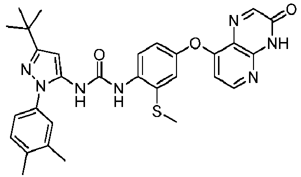 | 1-(3-(tert-butyl)-1-(3,4-dimethylphenyl)-1H-pyrazol-5-yl)-3-(2-(methylthio)-4-((3-oxo-3,4-dihydropyrido[2,3-b]pyrazin-8-yl)oxy)phenyl)urea |
| (ce) | 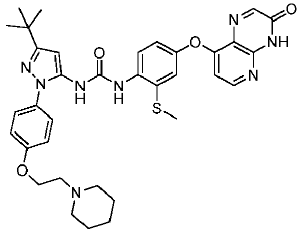 | 1-(3-(tert-butyl)-1-(4-(2-(piperidin-1-yl)ethoxy)phenyl)-1H-pyrazol-5-yl)-3-(2-(methylthio)-4-((3-oxo-3,4-dihydropyrido[2,3-b]pyrazin-8-yl)oxy)phenyl)urea |
| (cf) | 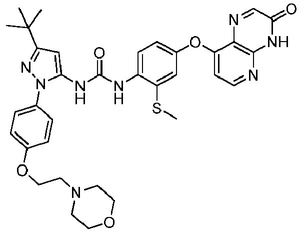 | 1-(3-(tert-butyl)-1-(4-(2-morpholinoethoxy)phenyl)-1 H-pyrazol-5-yl)-3-(2-(methylthio)-4-((3-oxo-3,4-dihydropyrido[2,3-b]pyrazin-8-yl)oxy)phenyl)urea |
| (eg) | 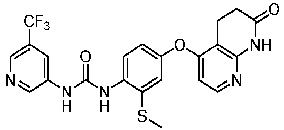 | 1-(2-(methylthio)-4-((7-oxo-5,6,7,8-tetrahydro-1,8-naphthyridin-4-yl)oxy)phenyl)-3-(5-(trifluoromethyl)pyridin-3-yl)urea |
| (ch) | 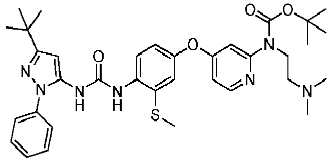 | tert-butyl (4-(4-(3-(3-(tert-butyl)-1-phenyl-1H-pyrazol-5-yl)ureido)-3-(methylthio)phenoxy)pyridin-2-yl)(2-(dimethylamino)ethyl)carbamate |
| (ci) | 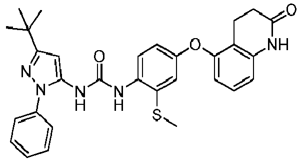 | 1-(3-(tert-butyl)-1-phenyl-1H-pyrazol-5-yl)-3-(2-(methylthio)-4-((2-oxo-1,2,3,4-tetrahydroquinolin-5-yl)oxy)phenyl)urea |
| (cj) | 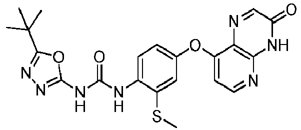 | 1-(5-(tert-butyl)-1,3,4-oxadiazol-2-yl)-3-(2-(methylthio)-4-((3-oxo-3,4-dihydropyrido[2,3-b]pyrazin-8-yl)oxy)phenyl)urea |
| (ck) | 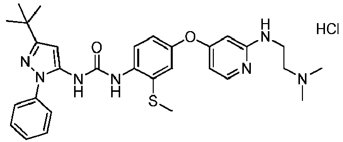 | 1-(3-(tert-butyl)-1-phenyl-1 H-pyrazol-5-yl)-3-(4-((2-((2-(dimethylamino)ethyl)amino)pyridin-4-yl)oxy)-2-(methylthio)phenyl)urea hydrochloride |
| (cl) | 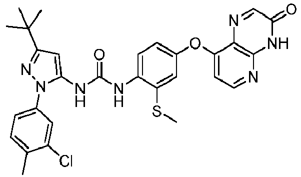 | 1-(3-(tert-butyl)-1-(3-chloro-4-methylphenyl)-1H-pyrazol-5-yl)-3-(2-(methylthio)-4-((3-oxo-3,4-dihydropyrido[2,3-b]pyrazin-8-yl)oxy)phenyl)urea |
| (cm) | 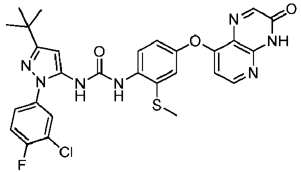 | 1-(3-(tert-butyl)-1-(3-chloro-4-fluorophenyl)-1H-pyrazol-5-yl)-3-(2-(methylthio)-4-((3-oxo-3,4-dihydropyrido[2,3-b]pyrazin-8-yl)oxy)phenyl)urea |
| (cn) | 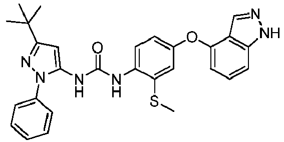 | 1-(4-((1H-indazol-4-yl)oxy)-2-(methylthio)phenyl)-3-(3-(tert-butyl)-1-phenyl-1H-pyrazol-5-yl)urea |
| (co) | 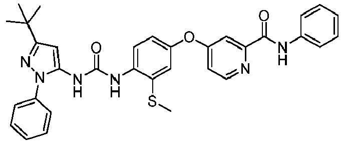 | 4-(4-(3-(3-(tert-butyl)-1-phenyl-1H-pyrazol-5-yl)ureido)-3-(methylthio)phenoxy)-N-phenylpicolinamide |
| (cp) | 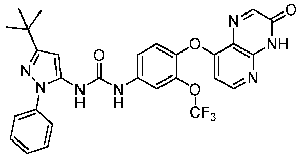 | 1-(3-(tert-butyl)-1-phenyl-1H-pyrazol-5-yl)-3-(4-((3-oxo-3,4-dihydropyrido[2,3-b]pyrazin-8-yl)oxy)-3-(trifluoromethoxy)phenyl)urea |
| (cq) | 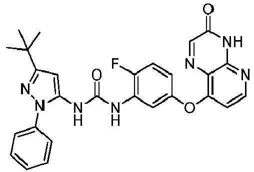 | 1-(3-(tert-butyl)-1-phenyl-1 H-pyrazol-5-yl)-3-(2-fluoro-5-((3-oxo-3,4-dihydropyrido[2,3-b]pyrazin-8-yl)oxy)phenyl)urea |
| (cr) | 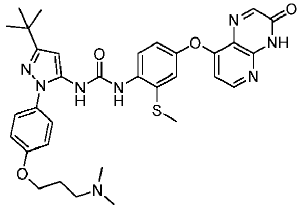 | 1-(3-(tert-butyl)-1-(4-(3-(dimethylamino)propoxy)phenyl)-1H-pyrazol-5-yl)-3-(2-(methylthio)-4-((3-oxo-3,4-dihydropyrido[2,3-b]pyrazin-8-yl)oxy)phenyl)urea |
| (cs) | 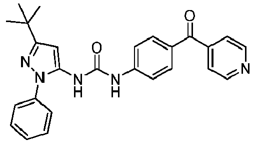 | 1-(3-(tert-butyl)-1-phenyl-1 H-pyrazol-5-yl)-3-(4-isonicotinoylphenyl)urea |
| (ct) | 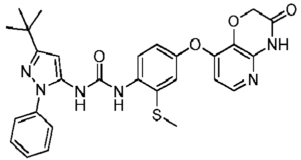 | 1-(3-(tert-butyl)-1-phenyl-1H-pyrazol-5-yl)-3-(2-(methylthio)-4-((3-oxo-3,4-dihydro-2H-pyrido[3,2-b][1,4]oxazin-8-yl)oxy)phenyl)urea |
| (cu) | 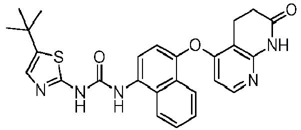 | 1-(5-(tert-butyl)thiazol-2-yl)-3-(4-((7-oxo-5,6,7,8-tetrahydro-1,8-naphthyridin-4-yl)oxy)naphthalen-1-yl)urea |
| (cv) | 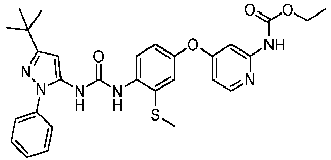 | ethyl (4-(4-(3-(3-(tert-butyl)-1-phenyl-1H-pyrazol-5-yl)ureido)-3-(methylthio)phenoxy)pyridin-2-yl)carbamate |
| (cw) | 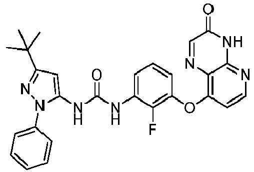 | 1-(3-(tert-butyl)-1-phenyl-1 H-pyrazol-5-yl)-3-(2-fluoro-3-((3-oxo-3,4-dihydropyrido[2,3-b]pyrazin-8-yl)oxy)phenyl)urea |
| (cx) | 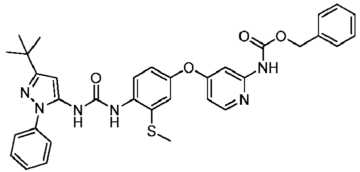 | benzyl (4-(4-(3-(3-(tert-butyl)-1-phenyl-1H-pyrazol-5-yl)ureido)-3-(methylthio)phenoxy)pyridin-2-yl)carbamate |
| (cy) | 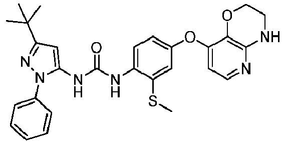 | 1-(3-(tert-butyl)-1-phenyl-1H-pyrazol-5-yl)-3-(4-((3,4-dihydro-2H-pyrido[3,2-b][1,4]oxazin-8-yl)oxy)-2-(methylthio)phenyl)urea |
| (cz) | 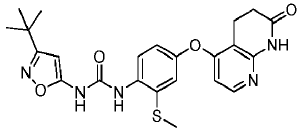 | 1-(3-(tert-butyl)isoxazol-5-yl)-3-(2-(methylthio)-4-((7-oxo-5,6,7,8-tetrahydro-1,8-naphthyridin-4-yl)oxy)phenyl)urea |
| (da) | 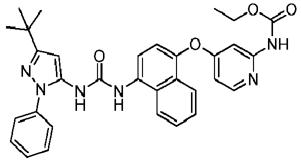 | ethyl (4-((4-(3-(3-(tert-butyl)-1-phenyl-1 H-pyrazol-5-yl)ureido)naphthalen-1-yl)oxy)pyridin-2-yl)carbamate |
| (db) | 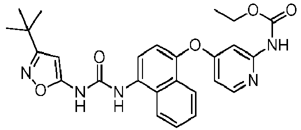 | ethyl (4-((4-(3-(3-(tert-butyl)isoxazol-5-yl)ureido)naphthalen-1-yl)oxy)pyridin-2-yl)carbamate |
| (dc) | 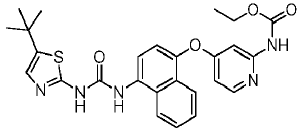 | ethyl (4-((4-(3-(5-(tert-butyl)thiazol-2-yl)ureido)naphthalen-1-yl)oxy)pyridin-2-yl)carbamate |
| (dd) | 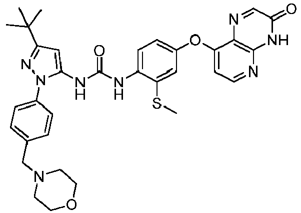 | 1-(3-(tert-butyl)-1-(4-(morpholinomethyl)phenyl)-1 H-pyrazol-5-yl)-3-(2-(methylthio)-4-((3-oxo-3,4-dihydropyrido[2,3-b]pyrazin-8-yl)oxy)phenyl)urea |
| (de) | 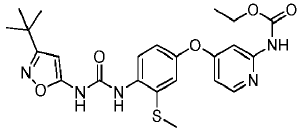 | ethyl (4-(4-(3-(3-(tert-butyl)isoxazol-5-yl)ureido)-3-(methylthio)phenoxy)pyridin-2-yl)carbamate |
| (df) | 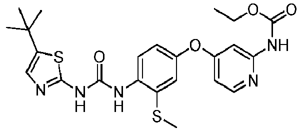 | ethyl (4-(4-(3-(5-(tert-butyl)thiazol-2-yl)ureido)-3-(methylthio)phenoxy)pyridin-2-yl)carbamate |
| (dg) | 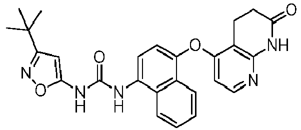 | 1-(3-(tert-butyl)isoxazol-5-yl)-3-(4-((7-oxo-5,6,7,8-tetrahydro-1,8-naphthyridin-4-yl)oxy)naphthalen-1-yl)urea |
| (dh) | 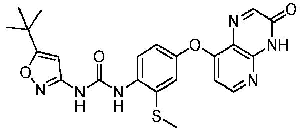 | 1-(5-(tert-butyl)isoxazol-3-yl)-3-(2-(methylthio)-4-((3-oxo-3,4-dihydropyrido[2,3-b]pyrazin-8-yl)oxy)phenyl)urea |
| (di) | 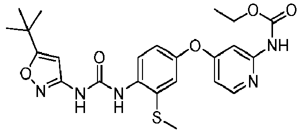 | ethyl (4-(4-(3-(5-(tert-butyl)isoxazol-3-yl)ureido)-3-(methylthio)phenoxy)pyridin-2-yl)carbamate |
| (dj) | 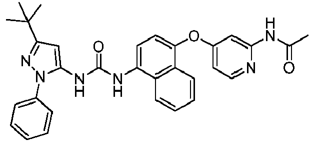 | N-(4-((4-(3-(3-(tert-butyl)-1-phenyl-1H-pyrazol-5-yl)ureido)naphthalen-1-yl)oxy)pyridin-2-yl)acetamide |
| (dk) | 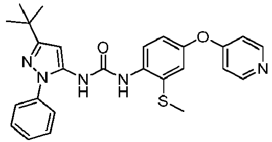 | 1-(3-(tert-butyl)-1-phenyl-1 H-pyrazol-5-yl)-3-(2-(methylthio)-4-(pyridin-4-yloxy)phenyl)urea |
| (dl) | 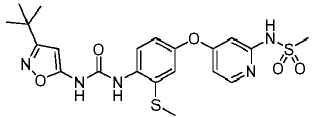 | N-(4-(4-(3-(3-(tert-butyl)isoxazol-5-yl)ureido)-3-(methylthio)phenoxy)pyridin-2-yl)methanesulfonamide |
| (dm) | 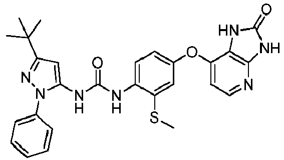 | 1-(3-(tert-butyl)-1-phenyl-1H-pyrazol-5-yl)-3-(2-(methylthio)-4-((2-oxo-2,3-dihydro-1H-imidazo[4,5-b]pyridin-7-yl)oxy)phenyl)urea |
| (dn) | 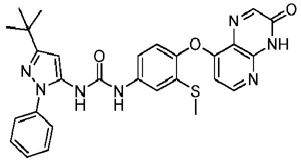 | 1-(3-(tert-butyl)-1-phenyl-1H-pyrazol-5-yl)-3-(3-(methylthio)-4-((3-oxo-3,4-dihydropyrido[2,3-b]pyrazin-8-yl)oxy)phenyl)urea |
| (do) | 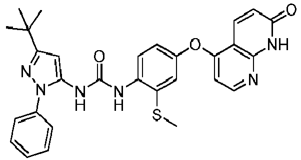 | 1-(3-(tert-butyl)-1-phenyl-1H-pyrazol-5-yl)-3-(2-(methylthio)-4-((7-oxo-7,8-dihydro-1,8-naphthyridin-4-yl)oxy)phenyl)urea |
| (dp) | 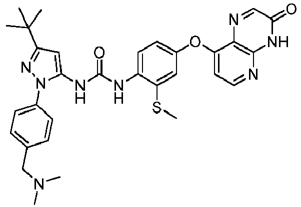 | 1-(3-(tert-butyl)-1-(4-((dimethylamino)methyl)phenyl)-1H-pyrazol-5-yl)-3-(2-(methylthio)-4-((3-oxo-3,4-dihydropyrido[2,3-b]pyrazin-8-yl)oxy)phenyl)urea |
| (dq) | 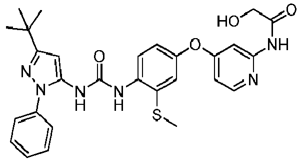 | N-(4-(4-(3-(3-(tert-butyl)-1-phenyl-1H-pyrazol-5-yl)ureido)-3-(methylthio)phenoxy)pyridin-2-yl)-2-hydroxyacetamide |

In a particular aspect, the compound has a structure selected in the group consisting of the following list or a pharmaceutically acceptable salt thereof:
(g), (I), (n), (s), (v), (ab), (ai), (aj), (am), (an), (aq), (as), (aw), (az), (ba), (bb), (bd), (be), (bi), (bl), (bm), (bo), (bp), (bq), (bt), (bw), (bx), (by), (bz), (cb), (cc), (cd), (ce), (cf), (ck), (cl), (cm), (cp), (cq), (cr), (ct), (cu), (cv), (cx), (cy), (cz), (da), (db), (dc), (dd), (de), (df), (dg), (dh), (di), (dj), (dn) and (do)
preferably
(n), (v), (am), (an), (as), (az), (ba), (bb), (bd), (be), (bi), (bm), (bo), (bp), (bq), (bw), (by), (bz), (cb), (cc), (cd), (ce), (cl), (cr), (ct), (cu), (cv), (da), (db), (dc), (dd), (de), (df), (dg), (dh), (di), (dj), and (do).

In a particular aspect, the compound has a structure selected in the group consisting of the following list or a pharmaceutically acceptable salt thereof:
(q), (az) and (bw).

In a particular aspect, the compound is selected in one of the following lists:
1)
   1-(3-(tert-butyl)-1-phenyl-1H-pyrazol-5-yl)-3-(2-(methylthio)-4-((7-oxo-5,6,7,8-tetrahydro-1,8-naphthyridin-4-yl)oxy)phenyl)urea;
   1-(3-(tert-butyl)-1-phenyl-1H-pyrazol-5-yl)-3-(2-(methylthio)-4-((5,6,7,8-tetrahydro-1,8-naphthyridin-4-yl)oxy)phenyl)urea;
   1-(3-(tert-butyl)-1-(p-tolyl)-1H-pyrazol-5-yl)-3-(2-(methylthio)-4-((7-oxo-5,6,7,8-tetrahydro-1,8-naphthyridin-4-yl)oxy)phenyl)urea;
   1-(5-(tert-butyl)thiazol-2-yl)-3-(2-(methylthio)-4-((7-oxo-5,6,7,8-tetrahydro-1,8-naphthyridin-4-yl)oxy)phenyl)urea;
   1-(3-(tert-butyl)-1-phenyl-1H-pyrazol-5-yl)-3-(2-(methylthio)-4-((3-oxo-3,4-dihydro-2H-pyrido[3,2-b][1,4]oxazin-8-yl)oxy)phenyl)urea;
   1-(5-(tert-butyl)thiazol-2-yl)-3-(4-((7-oxo-5,6,7,8-tetrahydro-1,8-naphthyridin-4-yl)oxy)naphthalen-1-yl)urea;
   1-(3-(tert-butyl)-1-phenyl-1H-pyrazol-5-yl)-3-(4-((3,4-dihydro-2H-pyrido[3,2-b][1,4]oxazin-8-yl)oxy)-2-(methylthio)phenyl)urea;
   1-(3-(tert-butyl)isoxazol-5-yl)-3-(2-(methylthio)-4-((7-oxo-5,6,7,8-tetrahydro-1,8-naphthyridin-4-yl)oxy)phenyl)urea;
   1-(3-(tert-butyl)isoxazol-5-yl)-3-(4-((7-oxo-5,6,7,8-tetrahydro-1,8-naphthyridin-4-yl)oxy)naphthalen-1-yl)urea;
   1-(3-(tert-butyl)-1-phenyl-1H-pyrazol-5-yl)-3-(2-(methylthio)-4-((7-oxo-7,8-dihydro-1,8-naphthyridin-4-yl)oxy)phenyl)urea;
   1-(5-(tert-butyl)thiazol-2-yl)-3-(4-((3-oxo-3,4-dihydropyrido[2,3-b]pyrazin-8-yl)oxy)naphthalen-1-yl)urea; 1-(3-(tert-butyl)isoxazol-5-yl)-3-(2-(methylthio)-4-((3-oxo-3,4-dihydropyrido[2,3-b]pyrazin-8-yl)oxy)phenyl)urea;
   1-(5-(tert-butyl)thiazol-2-yl)-3-(2-(methylthio)-4-((7-oxo-5,6,7,8-tetrahydro-1,8-naphthyridin-4-yl)oxy)phenyl)urea;
   1-(5-(tert-butyl)thiazol-2-yl)-3-(4-((7-oxo-5,6,7,8-tetrahydro-1,8-naphthyridin-4-yl)oxy)naphthalen-1-yl)urea;
   1-(3-(tert-butyl)isoxazol-5-yl)-3-(2-(methylthio)-4-((7-oxo-5,6,7,8-tetrahydro-1,8-naphthyridin-4-yl)oxy)phenyl)urea;
   ethyl (4-(4-(3-(3-(tert-butyl)isoxazol-5-yl)ureido)-3-(methylthio)phenoxy)pyridin-2-yl)carbamate;
   ethyl (4-(4-(3-(5-(tert-butyl)thiazol-2-yl)ureido)-3-(methylthio)phenoxy)pyridin-2-yl)carbamate;
   1-(3-(tert-butyl)isoxazol-5-yl)-3-(4-((7-oxo-5,6,7,8-tetrahydro-1,8-naphthyridin-4-yl)oxy)naphthalen-1-yl)urea;
   ethyl (4-(4-(3-(5-(tert-butyl)isoxazol-3-yl)ureido)-3-(methylthio)phenoxy)pyridin-2-yl)carbamate;
   1-(3-(tert-butyl)-1-phenyl-1H-pyrazol-5-yl)-3-(4-((3-oxo-3,4-dihydropyrido[2,3-b]pyrazin-8-yl)oxy)-2-(trifluoromethyl)phenyl)urea;
   1-(3-(tert-butyl)-1-(p-tolyl)-1H-pyrazol-5-yl)-3-(4-((3-oxo-3,4-dihydropyrido[2,3-b]pyrazin-8-yl)oxy)-2-(trifluoromethyl)phenyl)urea;
   1-(3-(tert-butyl)-1-phenyl-1H-pyrazol-5-yl)-3-(2-(ethylthio)-4-((3-oxo-3,4-dihydropyrido[2,3-b]pyrazin-8-yl)oxy)phenyl)urea;
   1-(3-(tert-butyl)-1-phenyl-1H-pyrazol-5-yl)-3-(2,3-difluoro-4-((3-oxo-3,4-dihydropyrido[2,3-b]pyrazin-8-yl)oxy)phenyl)urea;
   1-(3-(tert-butyl)-1-phenyl-1H-pyrazol-5-yl)-3-(2,5-difluoro-4-((3-oxo-3,4-dihydropyrido[2,3-b]pyrazin-8-yl)oxy)phenyl)urea;
   1-(3-(tert-butyl)-1-(4-methoxyphenyl)-1 H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-chlorophenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-methoxyphenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-fluorophenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(quinolin-6-yl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(4-cyanophenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3,4-dimethylphenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(4-(2-(piperidin-1-yl)ethoxy)phenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(4-(2-morpholinoethoxy)phenyl)-1 H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-chloro-4-methylphenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-chloro-4-fluorophenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(4-(3-(dimethylamino)propoxy)phenyl)-1 H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(4-(morpholinomethyl)phenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(quinolin-6-yl)-1H-pyrazol-5-yl)-3-(2-fluoro-4-((3-oxo-3,4-dihydropyrido[2,3-b]pyrazin-8-yl)oxy)phenyl)urea;
   1-(3-(tert-butyl)-1-(quinolin-6-yl)-1H-pyrazol-5-yl)-3-(4-((3-oxo-3,4-dihydropyrido[2,3-b]pyrazin-8-yl)oxy)naphthalen-1-yl)urea;
   1-(3-(tert-butyl)-1-(quinolin-6-yl)-1H-pyrazol-5-yl)-3-(2-(methylthio)-4-((3-oxo-3,4-dihydropyrido[2,3-b]pyrazin-8-yl)oxy)phenyl)urea;
   5-(4-(3-(3-(tert-butyl)-1-phenyl-1H-pyrazol-5-yl)ureido)-3-(methylthio)phenoxy)-N-methylnicotinamide;
   N-(4-(4-(3-(3-(tert-butyl)-1-phenyl-1H-pyrazol-5-yl)ureido)-3-(methylthio)phenoxy)pyridin-2-yl)acetamide;
   tert-butyl (4-(4-(3-(3-(tert-butyl)-1-phenyl-1H-pyrazol-5-yl)ureido)-3-(methylthio)phenoxy)pyridin-2-yl)carbamate;
   tert-butyl (2-((4-(4-(3-(3-(tert-butyl)-1-phenyl-1H-pyrazol-5-yl)ureido)-3-(methylthio)phenoxy)pyridin-2-yl)amino)-2-oxoethyl)(methyl)carbamate;
   N-(4-(4-(3-(3-(tert-butyl)-1-phenyl-1H-pyrazol-5-yl)ureido)-3-(methylthio)phenoxy)pyridin-2-yl)-2-(methylamino)acetamide;
   1-(3-(tert-butyl)-1-phenyl-1H-pyrazol-5-yl)-3-(4-((2-((2-(dimethylamino)ethyl)amino)pyridin-4-yl)oxy)-2-(methylthio)phenyl)urea hydrochloride;
   ethyl (4-(4-(3-(3-(tert-butyl)-1-phenyl-1H-pyrazol-5-yl)ureido)-3-(methylthio)phenoxy)pyridin-2-yl)carbamate;
   benzyl (4-(4-(3-(3-(tert-butyl)-1-phenyl-1H-pyrazol-5-yl)ureido)-3-(methylthio)phenoxy)pyridin-2-yl)carbamate;
   ethyl (4-(4-(3-(3-(tert-butyl)isoxazol-5-yl)ureido)-3-(methylthio)phenoxy)pyridin-2-yl)carbamate;
   ethyl (4-(4-(3-(5-(tert-butyl)thiazol-2-yl)ureido)-3-(methylthio)phenoxy)pyridin-2-yl)carbamate;
   ethyl (4-(4-(3-(5-(tert-butyl)isoxazol-3-yl)ureido)-3-(methylthio)phenoxy)pyridin-2-yl)carbamate;
   N-(4-(4-(3-(3-(tert-butyl)-1-phenyl-1H-pyrazol-5-yl)ureido)-3-fluorophenoxy)pyridin-2-yl)acetamide;
   N-(4-(4-(3-(3-(tert-butyl)-1-phenyl-1H-pyrazol-5-yl)ureido)-3-(methylthio)phenoxy)pyridin-2-yl)acetamide;
   tert-butyl (4-(4-(3-(3-(tert-butyl)-1-phenyl-1H-pyrazol-5-yl)ureido)-3-(methylthio)phenoxy)pyridin-2-yl)carbamate;
   tert-butyl (2-((4-(4-(3-(3-(tert-butyl)-1-phenyl-1H-pyrazol-5-yl)ureido)-3-(methylthio)phenoxy)pyridin-2-yl)amino)-2-oxoethyl)(methyl)carbamate;
   ethyl (4-(4-(3-(3-(tert-butyl)-1-phenyl-1H-pyrazol-5-yl)ureido)-3-(methylthio)phenoxy)pyridin-2-yl)carbamate
   ethyl (4-((4-(3-(3-(tert-butyl)-1-phenyl-1H-pyrazol-5-yl)ureido)naphthalen-1-yl)oxy)pyridin-2-yl)carbamate;
   ethyl (4-((4-(3-(3-(tert-butyl)isoxazol-5-yl)ureido)naphthalen-1-yl)oxy)pyridin-2-yl)carbamate;
   ethyl (4-((4-(3-(5-(tert-butyl)thiazol-2-yl)ureido)naphthalen-1-yl)oxy)pyridin-2-yl)carbamate;
   ethyl (4-(4-(3-(3-(tert-butyl)isoxazol-5-yl)ureido)-3-(methylthio)phenoxy)pyridin-2-yl)carbamate;
   ethyl (4-(4-(3-(5-(tert-butyl)thiazol-2-yl)ureido)-3-(methylthio)phenoxy)pyridin-2-yl)carbamate;
   benzyl (4-(4-(3-(3-(tert-butyl)-1-phenyl-1H-pyrazol-5-yl)ureido)-3-(methylthio)phenoxy)pyridin-2-yl)carbamate;
   N-(4-(4-(3-(3-(tert-butyl)-1-phenyl-1H-pyrazol-5-yl)ureido)-3-(methylthio)phenoxy)pyridin-2-yl)-2-hydroxyacetamide; and
   tert-butyl (4-(4-(3-(3-(tert-butyl)-1-phenyl-1H-pyrazol-5-yl)ureido)-3-(methylthio)phenoxy)pyridin-2-yl)(2-(dimethylamino)ethyl)carbamate;
2)
   1-(3-(tert-butyl)-1-phenyl-1H-pyrazol-5-yl)-3-(2-(methylthio)-4-((7-oxo-5,6,7,8-tetrahydro-1,8-naphthyridin-4-yl)oxy)phenyl)urea;
   1-(3-(tert-butyl)-1-phenyl-1H-pyrazol-5-yl)-3-(2-(methylthio)-4-((5,6,7,8-tetrahydro-1,8-naphthyridin-4-yl)oxy)phenyl)urea;
   1-(3-(tert-butyl)-1-(p-tolyl)-1H-pyrazol-5-yl)-3-(2-(methylthio)-4-((7-oxo-5,6,7,8-tetrahydro-1,8-naphthyridin-4-yl)oxy)phenyl)urea;
   1-(5-(tert-butyl)thiazol-2-yl)-3-(2-(methylthio)-4-((7-oxo-5,6,7,8-tetrahydro-1,8-naphthyridin-4-yl)oxy)phenyl)urea;
   1-(3-(tert-butyl)-1-phenyl-1H-pyrazol-5-yl)-3-(2-(methylthio)-4-((3-oxo-3,4-dihydro-2H-pyrido[3,2-b][1,4]oxazin-8-yl)oxy)phenyl)urea;
   1-(5-(tert-butyl)thiazol-2-yl)-3-(4-((7-oxo-5,6,7,8-tetrahydro-1,8-naphthyridin-4-yl)oxy)naphthalen-1-yl)urea;
   1-(3-(tert-butyl)-1-phenyl-1H-pyrazol-5-yl)-3-(4-((3,4-dihydro-2H-pyrido[3,2-b][1,4]oxazin-8-yl)oxy)-2-(methylthio)phenyl)urea;
   1-(3-(tert-butyl)isoxazol-5-yl)-3-(2-(methylthio)-4-((7-oxo-5,6,7,8-tetrahydro-1,8-naphthyridin-4-yl)oxy)phenyl)urea;
   1-(3-(tert-butyl)isoxazol-5-yl)-3-(4-((7-oxo-5,6,7,8-tetrahydro-1,8-naphthyridin-4-yl)oxy)naphthalen-1-yl)urea; and
   1-(3-(tert-butyl)-1-phenyl-1H-pyrazol-5-yl)-3-(2-(methylthio)-4-((7-oxo-7,8-dihydro-1,8-naphthyridin-4-yl)oxy)phenyl)urea
3)
   1-(5-(tert-butyl)thiazol-2-yl)-3-(4-((3-oxo-3,4-dihydropyrido[2,3-b]pyrazin-8-yl)oxy)naphthalen-1-yl)urea;
   1-(3-(tert-butyl)isoxazol-5-yl)-3-(2-(methylthio)-4-((3-oxo-3,4-dihydropyrido[2,3-b]pyrazin-8-yl)oxy)phenyl)urea;
   1-(5-(tert-butyl)thiazol-2-yl)-3-(2-(methylthio)-4-((7-oxo-5,6,7,8-tetrahydro-1,8-naphthyridin-4-yl)oxy)phenyl)urea;
   1-(5-(tert-butyl)thiazol-2-yl)-3-(4-((7-oxo-5,6,7,8-tetrahydro-1,8-naphthyridin-4-yl)oxy)naphthalen-1-yl)urea;
   1-(3-(tert-butyl)isoxazol-5-yl)-3-(2-(methylthio)-4-((7-oxo-5,6,7,8-tetrahydro-1,8-naphthyridin-4-yl)oxy)phenyl)urea;
   ethyl (4-(4-(3-(3-(tert-butyl)isoxazol-5-yl)ureido)-3-(methylthio)phenoxy)pyridin-2-yl)carbamate;
   ethyl (4-(4-(3-(5-(tert-butyl)thiazol-2-yl)ureido)-3-(methylthio)phenoxy)pyridin-2-yl)carbamate;
   1-(3-(tert-butyl)isoxazol-5-yl)-3-(4-((7-oxo-5,6,7,8-tetrahydro-1,8-naphthyridin-4-yl)oxy)naphthalen-1-yl)urea; and
   ethyl (4-(4-(3-(5-(tert-butyl)isoxazol-3-yl)ureido)-3-(methylthio)phenoxy)pyridin-2-yl)carbamate;
4)
   1-(3-(tert-butyl)-1-phenyl-1H-pyrazol-5-yl)-3-(4-((3-oxo-3,4-dihydropyrido[2,3-b]pyrazin-8-yl)oxy)-2-(trifluoromethyl)phenyl)urea;
   1-(3-(tert-butyl)-1-(p-tolyl)-1H-pyrazol-5-yl)-3-(4-((3-oxo-3,4-dihydropyrido[2,3-b]pyrazin-8-yl)oxy)-2-(trifluoromethyl)phenyl)urea;
   1-(3-(tert-butyl)-1-phenyl-1H-pyrazol-5-yl)-3-(2-(ethylthio)-4-((3-oxo-3,4-dihydropyrido[2,3-b]pyrazin-8-yl)oxy)phenyl)urea;
   1-(3-(tert-butyl)-1-phenyl-1H-pyrazol-5-yl)-3-(2,3-difluoro-4-((3-oxo-3,4-dihydropyrido[2,3-b]pyrazin-8-yl)oxy)phenyl)urea; and
   1-(3-(tert-butyl)-1-phenyl-1H-pyrazol-5-yl)-3-(2,5-difluoro-4-((3-oxo-3,4-dihydropyrido[2,3-b]pyrazin-8-yl)oxy)phenyl)urea;
5)
   1-(3-(tert-butyl)-1-(4-methoxyphenyl)-1 H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-chlorophenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-methoxyphenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-fluorophenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(quinolin-6-yl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(4-cyanophenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3,4-dimethylphenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(4-(2-(piperidin-1-yl)ethoxy)phenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(4-(2-morpholinoethoxy)phenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-chloro-4-methylphenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(3-chloro-4-fluorophenyl)-1H-pyrazol-5-yl)-3-(2-(methylthio)-4-((3-oxo-3,4-dihydropyrido[2,3-b]pyrazin-8-yl)oxy)phenyl)urea;
   1-(3-(tert-butyl)-1-(4-(3-(dimethylamino)propoxy)phenyl)-1 H-pyrazol-5-yl)-3-(2-(methylthio)-4-((3-oxo-3,4-dihydropyrido[2,3-b]pyrazin-8-yl)oxy)phenyl)urea; and
   1-(3-(tert-butyl)-1-(4-(morpholinomethyl)phenyl)-1H-pyrazol-5-yl)-3-(2-(methylthio)-4-((3-oxo-3,4-dihydropyrido[2,3-b]pyrazin-8-yl)oxy)phenyl)urea;
6)
   1-(3-(tert-butyl)-1-(quinolin-6-yl)-1H-pyrazol-5-yl)-3-(2-fluoro-4-((3-oxo-3,4-dihydropyrido[2,3-b]pyrazin-8-yl)oxy)phenyl)urea;
   1-(3-(tert-butyl)-1-(quinolin-6-yl)-1H-pyrazol-5-yl)-3-(4-((3-oxo-3,4-dihydropyrido[2,3-b]pyrazin-8-yl)oxy)naphthalen-1-yl)urea; and
   1-(3-(tert-butyl)-1-(quinolin-6-yl)-1H-pyrazol-5-yl)-3-(2-(methylthio)-4-((3-oxo-3,4-dihydropyrido[2,3-b]pyrazin-8-yl)oxy)phenyl)urea;
7)
   5-(4-(3-(3-(tert-butyl)-1-phenyl-1H-pyrazol-5-yl)ureido)-3-(methylthio)phenoxy)-N-methylnicotinamide;
   N-(4-(4-(3-(3-(tert-butyl)-1-phenyl-1H-pyrazol-5-yl)ureido)-3-(methylthio)phenoxy)pyridin-2-yl)acetamide;
   tert-butyl (4-(4-(3-(3-(tert-butyl)-1-phenyl-1H-pyrazol-5-yl)ureido)-3-(methylthio)phenoxy)pyridin-2-yl)carbamate;
   tert-butyl (2-((4-(4-(3-(3-(tert-butyl)-1-phenyl-1H-pyrazol-5-yl)ureido)-3-(methylthio)phenoxy)pyridin-2-yl)amino)-2-oxoethyl)(methyl)carbamate;
   N-(4-(4-(3-(3-(tert-butyl)-1-phenyl-1H-pyrazol-5-yl)ureido)-3-(methylthio)phenoxy)pyridin-2-yl)-2-(methylamino)acetamide;
   1-(3-(tert-butyl)-1-phenyl-1H-pyrazol-5-yl)-3-(4-((2-((2-(dimethylamino)ethyl)amino)pyridin-4-yl)oxy)-2-(methylthio)phenyl)urea hydrochloride;
   ethyl (4-(4-(3-(3-(tert-butyl)-1-phenyl-1H-pyrazol-5-yl)ureido)-3-(methylthio)phenoxy)pyridin-2-yl)carbamate;
   benzyl (4-(4-(3-(3-(tert-butyl)-1-phenyl-1H-pyrazol-5-yl)ureido)-3-(methylthio)phenoxy)pyridin-2-yl)carbamate;
   ethyl (4-(4-(3-(3-(tert-butyl)isoxazol-5-yl)ureido)-3-(methylthio)phenoxy)pyridin-2-yl)carbamate;
   ethyl (4-(4-(3-(5-(tert-butyl)thiazol-2-yl)ureido)-3-(methylthio)phenoxy)pyridin-2-yl)carbamate; and
   ethyl (4-(4-(3-(5-(tert-butyl)isoxazol-3-yl)ureido)-3-(methylthio)phenoxy)pyridin-2-yl)carbamate;
8)
   N-(4-(4-(3-(3-(tert-butyl)-1-phenyl-1H-pyrazol-5-yl)ureido)-3-fluorophenoxy)pyridin-2-yl)acetamide;
   N-(4-(4-(3-(3-(tert-butyl)-1-phenyl-1H-pyrazol-5-yl)ureido)-3-(methylthio)phenoxy)pyridin-2-yl)acetamide;
   tert-butyl (4-(4-(3-(3-(tert-butyl)-1-phenyl-1H-pyrazol-5-yl)ureido)-3-(methylthio)phenoxy)pyridin-2-yl)carbamate;
   tert-butyl (2-((4-(4-(3-(3-(tert-butyl)-1-phenyl-1H-pyrazol-5-yl)ureido)-3-(methylthio)phenoxy)pyridin-2-yl)amino)-2-oxoethyl)(methyl)carbamate;
   ethyl (4-(4-(3-(3-(tert-butyl)-1-phenyl-1H-pyrazol-5-yl)ureido)-3-(methylthio)phenoxy)pyridin-2-yl)carbamate;
   ethyl (4-((4-(3-(3-(tert-butyl)-1-phenyl-1H-pyrazol-5-yl)ureido)naphthalen-1-yl)oxy)pyridin-2-yl)carbamate;
   ethyl (4-((4-(3-(3-(tert-butyl)isoxazol-5-yl)ureido)naphthalen-1-yl)oxy)pyridin-2-yl)carbamate;
   ethyl (4-((4-(3-(5-(tert-butyl)thiazol-2-yl)ureido)naphthalen-1-yl)oxy)pyridin-2-yl)carbamate;
   ethyl (4-(4-(3-(3-(tert-butyl)isoxazol-5-yl)ureido)-3-(methylthio)phenoxy)pyridin-2-yl)carbamate;
   ethyl (4-(4-(3-(5-(tert-butyl)thiazol-2-yl)ureido)-3-(methylthio)phenoxy)pyridin-2-yl)carbamate;
   benzyl (4-(4-(3-(3-(tert-butyl)-1-phenyl-1H-pyrazol-5-yl)ureido)-3-(methylthio)phenoxy)pyridin-2-yl)carbamate;
   N-(4-(4-(3-(3-(tert-butyl)-1-phenyl-1H-pyrazol-5-yl)ureido)-3-(methylthio)phenoxy)pyridin-2-yl)-2-hydroxyacetamide; and
   tert-butyl (4-(4-(3-(3-(tert-butyl)-1-phenyl-1H-pyrazol-5-yl)ureido)-3-(methylthio)phenoxy)pyridin-2-yl)(2-(dimethylamino)ethyl)carbamate.

In a specific aspect, the compound is represented by a structure selected from the group consisting of: (ab), (ac), (ad), (ae), (ai), preferably (ab), (ae) and (ai), more preferably (ab) or (ai).

### Corticosteroids and HDAC inhibitors

The compound as disclosed herein promoting muscle progenitor differentiation is used in combination with a corticosteroid and/or a HDAC inhibitor.

The corticosteroid is a well-known family of drugs. In a specific aspect, the corticosteroid is selected from the group consisting of prednisone, prednisolone, methylprednisolone, oxandrolone, dexamethasone, deflazacort and valmorolone preferably selected from the group consisting of prednisolone, deflazacort and valmorolone. In a very specific aspect, the corticosteroid is prednisolone.

The histone deacetylase (HDAC) inhibitor is also a well-known family of drugs.

In one aspect, the HDAC inhibitor can be a class I HDAC inhibitor. For instance, the HDAC inhibitor can be selected from the group consisting of Abexinostat (PCI- 24781), Apicidin (OS 12040), AR-42, Belinostat (PXD101), BG45, BML-210, BML-281, BMN290, BRD0302, BRD2283, BRD3227, BRD3308, BRD3349, BRD3386, BRD3493, BRD4161, BRD4884, BRD6688, BRD8951, BRD9757, BRD9757, CBHA, Chromopeptide A, Citarinostat (ACY-214), CM-414, compound 25, CRA-026440, Crebinostat, CUDC-101, CUDC-907, Curcumin, Dacinostat (LAQ824), Depudecin, Domatinostat (4SC-202), Droxinostat, Entinostat (MS0275), EVX001688, FR901228, FRM-0334, Givinostat, HDACi-4b, HDACi-109, HPOB, 12, KD5170, LB-205, M344, Martinostat, Merck60 (BRD6929), Mocetinostat (MGCD0103), OBP-801, Oxamflatin, Panobinostat (LBH589), PCI-34051, PCI-48000, Pracinostat (SB939), Pyroxamide, Quisinostat (JNJ-26481585), Resminostat, RG2833 (RGFP109), RGFP963, RGFP966, RGFP968, Rocilinostat (ACY-1215), Romidepsin (FK228), Scriptaid, sodium phenylbutyrate, Splitomicin, T247, Tacedinaline (CI994), Trapoxin, Trichostatin A (TSA), Tucidinostat (chidamide), Valproic acid, vorinostat (SAHA), W2, MC1742, MC2625, A8B4, A14B3, A12B4, A14B4, A7B4, or any combination thereof.

Preferably, the HDAC inhibitor is givinostat.

Optionally, the combined treatment of a corticosteroid and/or a HDAC inhibitor with a compound as disclosed herein promoting muscle progenitor differentiation allows to use a dose of corticosteroid or HDAC inhibitor lower than the standard dose, thereby decreasing the side effect or the toxicity of corticosteroid or HDAC inhibitor. In one aspect, corticosteroid or HDAC inhibitor is administered at a low dose.

Reference to a low dose corticosteroid includes 90, 80, 75, 70, 65, 60, 55, 50, 45, 40, 35, 30, 25 or 20 percent of the standard dose.

For instance, the corticosteroid dose is 10, 20, 30, 40, 50, 60, 70mg/kg/day oral prednisolone, or 10, 20, 40, 40, 50, 60, 70 or 80mg of deflazacort.

The pharmaceutical composition may comprise a pharmaceutically acceptable excipient or carrier. The compound as disclosed herein promoting muscle progenitor differentiation or the pharmaceutical composition according to the invention may be administered by any conventional route of administration. In particular, the compound or the pharmaceutical composition of the invention can be administered by a topical, enteral, oral, parenteral, intranasal, intravenous, intra-arterial, intramuscular, or subcutaneous administration and the like.

Preferably, the compound as disclosed herein promoting muscle progenitor differentiation or the pharmaceutical composition according to the invention is administered by enteral or parenteral route of administration. When administered parenterally, the compound as disclosed herein promoting muscle progenitor differentiation or the pharmaceutical composition according to the invention is preferably administered by intravenous route of administration. When administered enterally, the compound as disclosed herein promoting muscle progenitor differentiation or the pharmaceutical composition according to the invention is preferably administered by oral route of administration.

The pharmaceutical composition comprising the molecule is formulated in accordance with standard pharmaceutical practice (Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

For oral administration, the composition can be formulated into conventional oral dosage forms such as tablets, capsules, powders, granules and liquid preparations such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, polysorbate (e.g., 20, 40, 65, 80, 100, 120), and the like. For compressed tablets, binders, which are agents which impart cohesive qualities to powdered materials, are also necessary. For example, starch, gelatin, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. Detergents such as dimethyl sulfoxide, dimethyl acetamide and dimethylformamide could be added.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Pharmaceutical compositions according to the invention may be formulated to release the active drug substantially immediately upon administration or at any predetermined time or time period after administration.

The amount of compound as disclosed herein promoting muscle progenitor differentiation or of pharmaceutical composition according to the invention to be administered has to be determined by standard procedure well known by those of ordinary skills in the art. Physiological data of the patient (e.g. age, size, and weight) and the routes of administration have to be taken into account to determine the appropriate dosage, so as a therapeutically effective amount will be administered to the patient.

In a preferred embodiment, the total dose of the compound as disclosed herein promoting muscle progenitor differentiation for each administration of the compound promoting muscle progenitor differentiation as defined herein or of the pharmaceutical composition according to the invention is comprised between 0.00001 and 1 g.

A compound as disclosed herein promoting muscle progenitor differentiation in combination with a corticosteroid or a HDAC inhibitor or the pharmaceutical composition is for use in treating, ameliorating, delaying, curing and/or preventing a disease or condition associated with muscle cells and/or satellite cells. A muscle cell is a type of cell found in muscle tissue. Optionally, a muscle cell can be a striated muscle cell. Preferably, a muscle cell is a skeletal muscle cell. During myogenesis, muscle cells are formed from muscle progenitors, including Pax7 positive cells. Diseases or conditions associated with muscle cells include myopathies including congenital myopathies and acquired myopathies, sarcopenia and cachexia. Examples of congenital myopathies are (muscular) dystrophies such as Duchenne muscular dystrophy (DMD), Becker muscular dystrophy (BMD), congenital muscular dystrophy, myotonic muscular dystrophy, limb-girdle muscular dystrophy, facioscapulohumeral muscular dystrophy (FSHD) and LAMA2 and SEPN1 myopathies; and non-dystrophic congenital myopathies such as nemaline myopathy, spinal muscular atrophy, SMARD1, multi/minicore myopathy and centronuclear myopathy. Examples of acquired myopathies include myopathies induces by drugs, alcohol and toxic agents, dermatomyositis and polymyositis.

Preferably, a disease or a condition associated with a muscle cell and/or a satellite cell is not facioscapulohumeral muscular dystrophy (FSHD).

Satellite cells, also known as muscle stem cells, are small multipotent cells, present in muscle tissue, which are precursors of skeletal muscle cells. Although these cells are quiescent under normal physiological conditions, they are activated in response to trauma and therefore play an important role in muscle repair and regeneration. Hence, diseases or conditions associated with satellite cells are often diseases or conditions wherein muscle cells are unable to repair and/or regenerate. Optionally, diseases or conditions associated with satellite cells include aging. Important markers for satellite cells which are able to play role in muscle repair and regeneration are Pax3 and Pax7, preferably Pax7. Preferably, the presence of Pax7 is detected via immunostaining using the anti-Pax7 antibody from the Developmental Studies Hybridoma Bank (DSHB).

In diseases or conditions associated with muscle cells, said muscle cells may have a decreased functionality, integrity and/or survival (i.e. said muscle cells have an increased degeneration) compared to a corresponding muscle cell from a healthy individual. Functionality in this context refers to, amongst other parameters, the ability of the muscle cell to contract. Preferably, the satellite cells in the muscle tissue wherein said muscle cells are comprised have a decreased capability in muscle repair and regeneration compared to corresponding satellite cells in a healthy individual.

In diseases or conditions associated with satellite cells, said satellite cells have a decreased capability in muscle repair and regeneration compared to corresponding satellite cells in a healthy individual. Preferably, the muscle cells comprised in the muscle tissue comprising said satellite cells have a decreased functionality, integrity and/or survival (i.e. said muscle cells have an increased degeneration) compared to a corresponding muscle cell from a healthy individual. Functionality in this context refers to, amongst other parameters, the ability of the muscle cell to contract.

A compound as disclosed herein promoting muscle progenitor differentiation in combination with a corticosteroid or a HDAC inhibitor or the pharmaceutical composition is for use in treating, ameliorating, delaying, curing and/or preventing a disease or condition associated with muscle cells and/or satellite cells. Hence, the administration to a patient of a compound according to the invention is associated with the alleviation of one or more characteristics of said muscle cell, or of a muscle cell comprised in the muscle tissue comprising said satellite cell, from said patient, preferably with the improvement of muscle fiber functionality, integrity and/or survival. Moreover, the administration of said compound may increase the number of satellite cells, or the number of satellite cells comprised in the muscle tissue comprising said muscle cell, relative to the number before administration; and preferably also increases the capability of said satellite cell, or the satellite cells comprised in the muscle tissue comprising said muscle cell, in muscle repair and regeneration. The number of satellite cells is preferably determined by measuring the number of Pax7 positive cells. Said increase of the number of satellite cells is preferably at least 5%, more preferably 10%, even more preferably 20%, most preferably 30, 32, 34; 36, 38, 40, 42, 44, 46, 48 or 50%, relative to the number of satellite cells in the assay before administration of the compound. In a preferred embodiment, the increase of the number of satellite cells is assessed *in vitro,* more preferably *in vitro* in an assay as carried out in the experimental part in the myotube assay of examples 78 and 79. Said increase of the number of satellite cells *in vitro* is preferably at least 5%, more preferably 10%, even more preferably 20%, most preferably 30, 32, 34; 36, 38, 40, 42, 44, 46, 48 or 50%, relative to the number of satellite cells in the assay before administration of the compound

Hence, in a preferred embodiment is provided a compound according to the invention, wherein said compound is able to induce the generation of myotubes and/or increase the number of Pax7 positive cells in muscle tissue comprising said muscle cell and/or said satellite cell, relative to the number of Pax7 positive cells in said muscle tissue before administration of said compound. Preferably said increase in the number of Pax7 positive cells is at least 5 %, more preferably said increase in the number of Pax7 positive cells is 10%, even more preferably said increase in the number of Pax7 positive cells is at least 20%, most preferably said increase in the number of Pax7 positive cells is at least 30, 32, 34; 36, 38, 40, 42, 44, 46, 48 or 50%.

An alleviation of one or more characteristics of a muscle cell from a patient may be assessed by a variety of assays on a myogenic cell or muscle cell from a patient. The following test results, obtained from an assay on a myogenic cell or muscle cell from a patient, correlate with improved characteristics of a muscle cell from said patient: reduced cytosolic calcium concentration in muscle cells, decreased collagen synthesis, modified morphology, altered lipid biosynthesis, decreased oxidative stress, and/or improved muscle fiber function, integrity, and/or survival. These parameters are usually assessed using immunofluorescence and/or histochemical analyses of cross sections of muscle biopsies or primary cultures of patient muscle cells.

The improvement of muscle fiber function, integrity and/or survival may be assessed using at least one of the following assays: a detectable decrease of creatine kinase in blood, a detectable decrease of necrosis of muscle fibers in a biopsy cross-section of a muscle suspected to be dystrophic, a detectable decrease of fibrosis in a muscle suspected to be dystrophic, a detectable decrease in fat accumulation in a muscle suspected to by dystrophic, and/or a detectable increase of the homogeneity of the diameter of muscle fibers in a biopsy cross-section of a muscle suspected to be dystrophic. Each of these assays is known to the skilled person.

Creatine kinase may be detected in blood as described in Hodgetts et al. (2006). A detectable decrease in creatine kinase may mean a decrease of 5%, 10%, 20%, 30%, 40%, 50%, 60%, 70%, 80%, 90% or more compared to the concentration of creatine kinase in blood before administration of a compound according to the invention.

A detectable decrease of necrosis of muscle fibers is preferably assessed in a muscle biopsy, more preferably as described in Hodgetts et al. (2006), using biopsy cross-sections. A detectable decrease of necrosis may be a decrease of 5%, 10%, 20%, 30%, 40%, 50%, 60%, 70%, 80%, 90% or more of the area wherein necrosis has been identified using biopsy cross-sections. The decrease is measured by comparison to the necrosis as assessed before administration of a compound according to the invention.

A detectable increase of the homogeneity of the diameter of a muscle fiber is preferably assessed in a muscle biopsy cross-section, more preferably as described in Hodgetts et al. (2006). The increase is measured by comparison to the homogeneity of the diameter of a muscle fiber before administration of a compound according to the invention.

An *in vitro* assay for assessing the efficacy of compounds for use in treating, ameliorating, delaying, curing and/or preventing a disease or condition associated with muscle cells and/or satellite cells is described in example 78. Herein, the generation of myotubes and satellite cells after adding a compound to a myotube assay workflow is assessed. Said myotube assay workflow is derived from wild-type or DMD human induced pluripotent stem cells (WT hiPSC or DMD hiPSC). Before the addition of said compound, the assay comprises satellite-like cells, but no significant amount of myotubes.

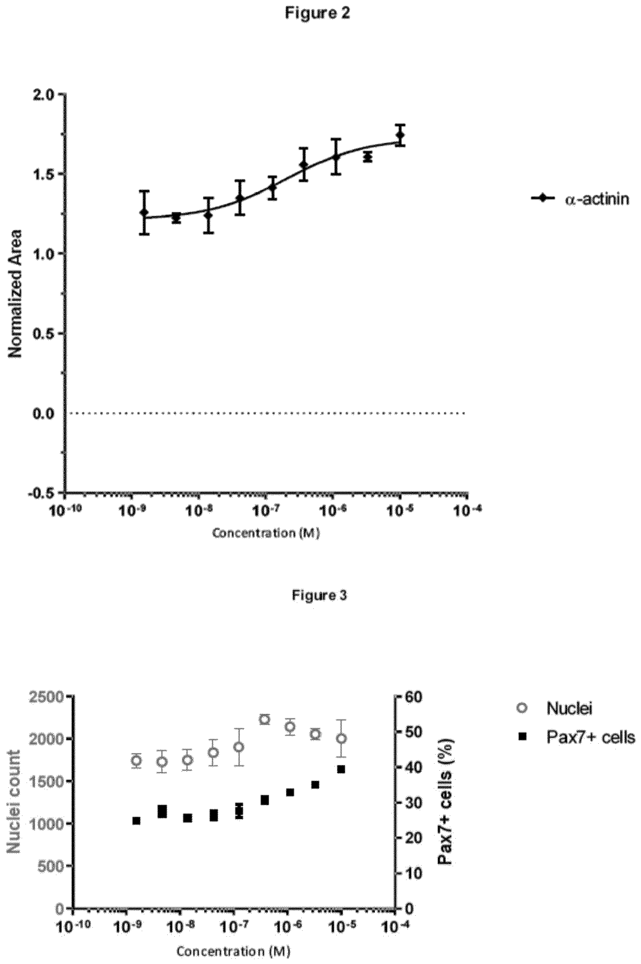

The efficacy of said compound for use in treating, ameliorating, delaying, curing and/or preventing a disease or condition associated with muscle cells and/or satellite cells is positively correlated with the generation of myotubes (i.e. the increase of the area occupied by myotubes on the assay) and/or satellite cells (i.e. the increase of the number of satellite cells) in the assay after adding said compound. The generation of myotubes by the addition of said compound is expressed as the EC₅₀ value, the concentration of said compound that corresponds with the half-maximal generation of myotubes, and the MA (normalized myotube area) value, the normalized area occupied by generated myotubes on the assay. The EC₅₀ value is determined using the GraphPad Prism software after fitting a 10 points dose-response 4 parameter logistic regression curve (ranging from 1.5 nM to 30 µM). Since muscle cells are formed from myotubes during myogenesis, the generation of myotubes in the assay is a good indicator of the ability of said compound to induce the formation of functional muscle cells *in vivo.* The number of satellite cells is measured as the percentage of Pax7 positive cells, relative to the total number of cells in the assay, as shown in Figure 3. The percentage of Pax7 is a good indicator of the ability of said compound to induce the formation of satellite cells which are able to play a role in muscle repair and regeneration *in vivo.*

In example 2, it is shown that all tested compounds according to the invention are suitable for use in treating, ameliorating, delaying, curing and/or preventing a disease or condition associated with muscle cells and/or satellite cells.

The efficacy of a compound according to the invention for use in treating, ameliorating, delaying, curing and/or preventing a disease or condition associated with muscle cells and/or satellite cells is preferably assessed by the myotube assay workflow described above and in Example 2.

In a preferred embodiment is provided a compound as disclosed herein promoting muscle progenitor differentiation in combination with a corticosteroid or a HDAC inhibitor or a pharmaceutical composition for use in treating, ameliorating, delaying, curing and/or preventing a disease or condition associated with muscle cells and/or satellite cells, wherein said compound is able to induce the generation of myotubes and/or Pax7 positive cells in muscle tissue comprising said muscle cells and/or said satellite cells, preferably assessed by the myotube assay workflow described above and in Example 2. In an even more preferred embodiment, said disease or condition is not facioscapulohumeral muscular dystrophy (FSHD).

In a preferred embodiment is provided a compound as disclosed herein promoting muscle progenitor differentiation in combination with a corticosteroid or a HDAC inhibitor or a pharmaceutical composition for use in treating, ameliorating, delaying, curing and/or preventing a disease or condition selected from the group consisting of sarcopenia, cachexia, Duchenne muscular dystrophy and Becker muscular dystrophy, preferably wherein said compound is able to induce the generation of myotubes and Pax7 positive cells in muscle tissue, preferably assessed by the myotube assay workflow described above and in Example 2.

In a preferred embodiment is provided a compound as disclosed herein promoting muscle progenitor differentiation in combination with a corticosteroid or a HDAC inhibitor or a pharmaceutical composition for use in treating, ameliorating, delaying, curing and/or preventing a disease or condition selected from the group consisting of Duchenne muscular dystrophy and Becker muscular dystrophy, preferably wherein said compound is able to induce the generation of myotubes and Pax7 positive cells in muscle tissue, preferably assessed by the myotube assay workflow described above and in Example 2.

In a preferred embodiment is provided a compound as disclosed herein promoting muscle progenitor differentiation in combination with a corticosteroid or a HDAC inhibitor or a pharmaceutical composition for use in treating, ameliorating, delaying, curing and/or preventing a disease or condition selected from the group consisting of sarcopenia and cachexia, preferably wherein said compound is able to induce the generation of myotubes and Pax7 positive cells in muscle tissue, preferably assessed by the myotube assay workflow described above and in Example 2.

Duchenne muscular dystrophy is a severe type of muscular dystrophy caused by a mutation in the dystrophin gene, resulting in muscle cells with a reduced biological functionality, leading to progressive muscle weakness. Becker muscular dystrophy is a similar disorder, albeit with less severe symptoms. Sarcopenia is the loss of skeletal muscle mass due to aging. Cachexia, in the context of this application, is the loss of skeletal muscle mass associated with a chronic illness such as cancer, acquired immune deficiency syndrome (AIDS) or chronic obstructive pulmonary disease (COPD).

Duchenne muscular dystrophy and Becker muscular dystrophy are diseases associated with muscle cells and satellite cells. Without being bound to this theory, satellite cells comprised in muscle tissue of a patient suffering from DMD or BMD are not able to undergo asymmetric division, and are therefore not able to play a role in the regeneration and/or repair of said muscle, due to lack of a functional dystrophin in said satellite cells (Chang et al., 2016, Trends Mol Med., 22(6): 479-496). In individuals not suffering from DMD or BMD, said satellite cells are able to undergo asymmetric division, eventually leading to a new satellite cell and a myotube (Chang et al., 2016, Trends Mol Med., 22(6): 479-496). Additionally or alternatively, without being bound to this theory, the number of satellite cells in muscle tissue of a patient suffering from DMD or BMD is significantly decreased relative to muscle tissue of individuals not suffering from DMD or BMD.

Alleviating one or more symptom(s) of Duchenne muscular dystrophy and/or Becker muscular dystrophy in an individual using a compound according to the invention may be assessed by any of the following assays: prolongation of time to loss of walking, improvement of muscle strength, improvement of the ability to lift weight, improvement of the time taken to rise from the floor, improvement in the 6-minute walk test (6MWT), improvement in the time taken for four-stairs climbing, improvement of the leg function grade, improvement of the pulmonary function, improvement of cardiac function, improvement of the quality of life. Each of these assays is known to the skilled person. As an example, the publication of Manzur et al. (2008), gives an extensive explanation of each of these assays. For each of these assays, as soon as a detectable improvement or prolongation of a parameter measured in an assay has been found, it will preferably mean that one or more symptoms of Duchenne and/or Becker muscular dystrophy has been alleviated in an individual using a compound according to the invention. Detectable improvement or prolongation is preferably a statistically significant improvement or prolongation as described in Hodgetts et al. (2006). Alternatively, the alleviation of one or more symptom(s) of Duchenne and/or Becker muscular dystrophy may be assessed by measuring an improvement of a muscle fiber function, integrity and/or survival. In a preferred method, one or more symptom(s) of a DMD and/or BMD is/are alleviated and/or one or more characteristic(s) of one or more muscle cells from a DMD and/or BMD is/are improved. Such symptoms or characteristics may be assessed at the cellular, tissue level or on the patient self.

Alleviating one or more symptom(s) of sarcopenia and/or cachexia in an individual using a compound according to the invention may be assessed by any of the following assays: prolongation of time to loss of walking, improvement of muscle strength, improvement of the ability to lift weight, improvement of the time taken to rise from the floor, improvement in the ten-meter walking time, improvement in the time taken for four-stairs climbing, improvement of the leg function grade, improvement of the quality of life. For each of these assays, as soon as a detectable improvement or prolongation of a parameter measured in an assay has been found, it will preferably mean that one or more symptoms of sarcopenia and/or cachexia has been alleviated in an individual using a compound according to the invention. Alternatively, the alleviation of one or more symptom(s) of sarcopenia and/or cachexia may be assessed by measuring an improvement of a muscle fiber function, integrity and/or survival. In a preferred method, one or more symptom(s) of sarcopenia and/or cachexia is/are alleviated and/or one or more characteristic(s) of one or more muscle cells from sarcopenia is/are improved. Such symptoms or characteristics may be assessed at the cellular, tissue level or on the patient self.

Optionally, the present invention may further relate to a compound selected from the group consisting of Rebastinib or CCT196969 or a pharmaceutical composition comprising it for use in treating, ameliorating, delaying, curing and/or preventing a disease or condition associated with muscle cells and/or satellite cells, in combination with a corticosteroid or a HDAC inhibitor, a pharmaceutical composition comprising a compound selected from the group consisting of Rebastinib or CCT196969 and a corticosteroid or a HDAC inhibitor, and a pharmaceutical composition comprising a compound selected from the group consisting of Rebastinib or CCT196969 and a corticosteroid or a HDAC inhibitor for use in treating, ameliorating, delaying, curing and/or preventing a disease or condition associated with muscle cells and/or satellite cells. The present invention also relates to the use of compound selected from the group consisting of Rebastinib or CCT196969 for the manufacture of a medicament for treating, ameliorating, delaying, curing and/or preventing a disease or condition associated with muscle cells and/or satellite cells in combination with a corticosteroid or a HDAC inhibitor; or to the use of a pharmaceutical composition comprising compound selected from the group consisting of Rebastinib or CCT196969 and a corticosteroid or a HDAC inhibitor for the manufacture of a medicament for treating, ameliorating, delaying, curing and/or preventing a disease or condition associated with muscle cells and/or satellite cells. In addition, it relates to a method of treating, ameliorating, delaying, curing and/or preventing a disease or condition associated with muscle cells and/or satellite cells in a subject in need thereof, comprising administering a therapeutic amount of compound selected from the group consisting of Rebastinib or CCT196969 and administering a therapeutic amount of a corticosteroid or a HDAC inhibitor. Alternatively, it relates to a method of treating, ameliorating, delaying, curing and/or preventing a disease or condition associated with muscle cells and/or satellite cells in a subject in need thereof, comprising administering a therapeutic amount of a pharmaceutical composition comprising compound selected from the group consisting of Rebastinib or CCT196969 and a corticosteroid or a HDAC inhibitor. The corticosteroid or a HDAC inhibitor can be any of those disclosed in the present application. The disease or condition can be selected from the group consisting of Duchenne muscular dystrophy, Becker muscular dystrophy, sarcopenia and cachexia, preferably selected from the group consisting of Duchenne muscular dystrophy, Becker muscular dystrophy, and sarcopenia. In a particular aspect, the disease or condition is a muscular dystrophy such as Duchenne muscular dystrophy or Becker muscular dystrophy.

### Examples

The following examples are offered for illustrative purposes only, and are not intended to limit the scope of the present invention in any way.

### Example 1: Synthesis of the compounds

### General synthetic protocol

The reagents and starting materials are commercially available and/or, using well-known techniques, can be readily synthesized by one of ordinary skill in the art. Unless otherwise noted, all commercially starting materials were used without further purification.

The following abbreviation may be used in the examples and throughout the specification: g (grams), mg (milligrams), L (liter), mL (milliliters), µL (microliters), M (molar), % (percentage), MHz (megahertz), mmol (millimoles), min (minutes), AcOEt or EtOAc or EA (Ethyl acetate), Et₂O (diethyl ether), % (percent), DCM (dichloromethane), DMSO (dimethyl sulfoxide), NEt₃ or TEA (triethylamine), TBDMS-CI (tert-butyldimethylsilyl chloride), CHCl₃ (chloroform), DMF (dimethylformamide), EtOH (ethyl alcohol), Pd/C (Pd/C), H₂ (hydrogen gas), NaN₃ (sodium azide), DIPEA (diisopropyl ethyl amine), NaH (sodium hydride), Na₂CO₃ (sodium carbonate), NaHCO₃ (sodium hydrogenocarbonate), MeONa (sodium methoxide), CBr₄ (carbon tetrabromide), PPh₃ (triphenylphosphine), CO₂ (carbon dioxide), DBU (1,8-Diazabicyclo[5.4.0]undec-7-ene), TFA (trifluoroacetic acid), MeCN or CH₃CN or ACN (acetonitrile), K₂CO₃ (potassium carbonate), NMP (1-methyl-2-pyrrolidinone), CHCl₃ (chloroform), Cul (copper iodide), t-BuOK (potassium tert-butoxide), Cs₂CO₃ (cesium carbonate), CuCN (copper cyanide), i-AmONO (isoamyl nitrite), SnCl₂.2H₂O (stannous chloride dihydrate), AC₂O (acetic anhydride), N₂H₄.H₂O (hydrazine monohydrate), PE (petroleum ether), NEt₃.3HF (triethylamine trifluoride), p-TSA (p-toluenesulfonic acid), NH₃ (ammoniac), NaHCO₃ (sodium hydrogen carbonate), Na₂CO₃ (sodium carbonate), Me₂CO (acetone), SOCl₂ (thionyl chloride), DCE (dichloroethane), DMAP (N,N-dimethylaminopyridine), (Boc)₂O (Di-tert-butyl dicarbonate), tBuXPhos Pd G3 ([(2-Di-*tert*-butylphosphino-2',4',6'-triisopropyl-1,1'-biphenyl)-2-(2'-amino-1,1'-biphenyl)] palladium(II) methanesulfonate), °C (Celsius degrees), MeOH (methyl alcohol), Hz (Hertz), LCMS (Liquid Chromatography Mass Spectrum), MS (Mass Spectrum), ES (Electrospray), HPLC (High Pressure Liquid Chromatography), NMR (Nuclear Magnetic Reasonance), 1H (proton), MgSO₄ (magnesium sulphate), R.T. or r.t. (room temperature), KOH (potassium hydroxide), NaOH (sodium hydroxide), h (hour), HCl (hydrochloric acid), THF (tetrahydrofuran), N₂ (nitrogen), eq (equivalent), mm (millimeters), s (singulet), d (doublet), t (triplet), m (multiplet), q (quintuplet), bs (broad singulet), dd (doublet of doublet), dt (doublet of triplet), td (triplet of doublet), dq (doublet of quintuplet).

NMR spectra were registered on a 400 MHz Bruker DPX and processed using Bruker XWinNMR software. All commercially obtained reagents were used without further purification.

All references to brine refer to a saturated aqueous solution of NaCl. Unless otherwise indicated, all temperatures are expressed in °C. All reactions are not conducted under an inert atmosphere at r.t. unless otherwise noted.

### Synthesis of compounds according to the invention

Compounds represented by structure (I) may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (T.W. Green and P.G.M. Wuts (1991) Protecting Groups in Organic Synthesis, John Wiley et Sons). These groups are removed at a convenient stage of the compound synthesis using methods that are readily apparent to those skilled in the art. The selection of process as well as the reaction conditions and order of their execution shall be consistent with the preparation of compounds of structure (I).

The compound of structure (I) may be represented as a mixture of enantiomers, which may be resolved into the individual pure *R*- or S-enantiomers. If for instance, a particular enantiomer of the compound of structure (I) is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group such as amino, or an acidic functional group such as carboxyl, this resolution may be conveniently performed by fractional crystallization from various solvents, of the salts of the compounds of structure (I) with optical active acid or by other methods known in the literature, e.g. chiral column chromatography.

Resolution of the final product, an intermediate or a starting material may be performed by any suitable method known in the art as described by E.L. Eliel, S.H. Wilen and L.N. Mander (1984) Stereochemistry of Organic Compounds, Wiley-Interscience.

All the products generated from the following reactions can be isolated and purified employing standard techniques, such as extraction, chromatography, crystallization, distillation, and the like.

General routes to prepare the Examples of the present invention are shown in the Schemes and examples that follow.

All substituents in the synthetic Schemes unless otherwise indicated, are as previously defined.

The compounds of structure (I) may be prepared by general route of synthesis as disclosed in the following methods.

The compounds of structure (I) wherein A, B, L and -C are as described above may be prepared following the Scheme 1. The amine **1.1** react with diphosgene or triphosgene to obtain the corresponding isocyanate **1.2** (Liu, Dazhi & al, Bioorganic & Medicinal Chemistry, 2013, 21(11), 2960-2967; Snorri Th. Sigurdsson & al, J. Org. Chem., 1996, 61, 3883-3884). Then **1.2** can react with multiple aryl or heteroaryl amine **1.3** to generate the final compound **1.4** by reaction in DMF or dioxane (WO2009034308).

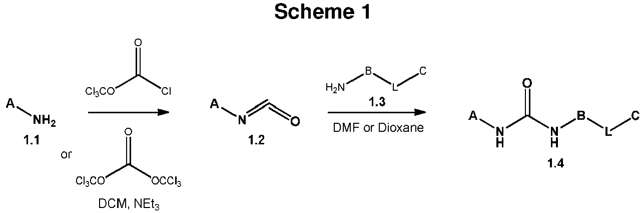

A similar procedure as those described in Scheme 1 can be used starting from commercially available amine **1.1.** Following the procedure described in Pavia, Michael & al, Journal of Medicinal Chemistry, 1990, 33(2), 854-861, **1.6** is obtained after deprotection of **1.5** in presence of triethylamine trihydrofluoride (WO2001029025). Then the phenol **1.6** can give compound **1.7** in presence of the chloropyridine to be subsequently reduced to the compound **1.8** (Menard, Delphine & al, Journal of Medicinal Chemistry, 2009, 52(13), 3881-3891). In a presence of ethyl glyoxalate compound (Zambon, Alfonso & al, Journal of Medicinal Chemistry, 2010, 53(15), 5639-5655) give final derivative **1.9** and in presence of triphosgene the final derivative **1.15** (Menard, Delphine & al, Journal of Medicinal Chemistry, 2009, 52(13), 3881-3891).

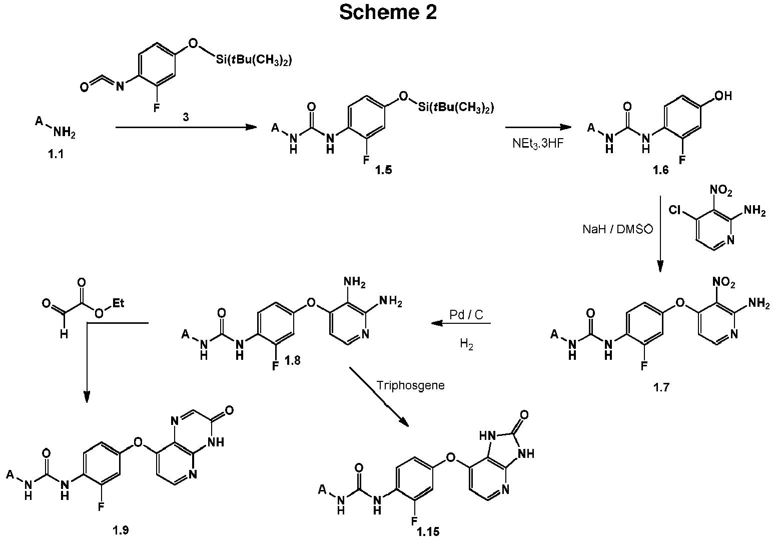

Alternatively, the aryl or heteroarylamine **1.3** can be first transformed in isocyanate **1.10** by reaction with diphosgene or triphosgene (Kurita, Keissuke & al, Journal of Organic Chemistry, 1976, 41(11), 2070-2071, Baraldi, Pier Giovanni & al, Journal of Medicinal Chemistry, 2001, 44(17), 2735-2742 or Liu, Dazhi & al, Bioorganic & Medicinal Chemistry, 2013, 21(11), 2960-2967). The corresponding isocyanate **1.10** react then with the amine **1.1** (Pavia, Michael & al, Journal of Medicinal Chemistry, 1990, 33(2), 854-861) to afford **1.4** compounds of structure (I) (Scheme 3).

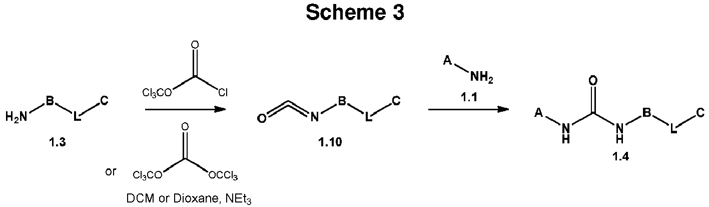

Yet another approach to the compounds of structure (I) can be represented Scheme 4 with the formation of the carbamate **1.11** from the amine **1.1** following the procedure described in Hron, Rebecca J. & al, Bioorganic & Medicinal Chemistry, 2016, 24(23), 6183-6193 or the procedure described in Kono, Mitsunori & al, Bioorganic & Medicinal Chemistry, 2013, 21(1), 28-41. To achieve this procedure the intermediate **1.11** react with the aryl or heteroarylamine **1.3** to get the final compound **1.4** (WO2018132372).

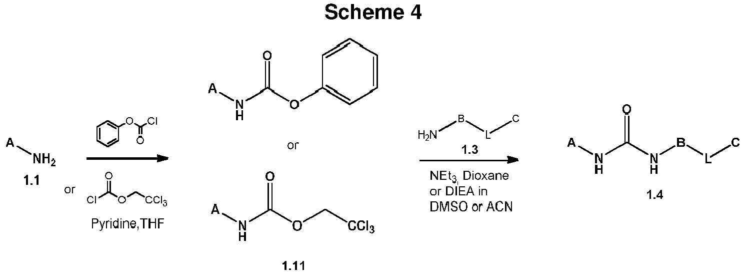

Yet another approach to the compound of structure (I) is described in Scheme 4 in particular for those compounds of structure (I) for which the formation of the acyl azide was particularly indicated. For this particular synthesis the reaction starts with a carboxylic acid **1.12** which is transformed in carbamoyl chloride **1.13** in a presence of oxalyl chloride (Zhou, Yuan & al, Bioorganic & Medicinal Chemistry, 2018, 26(1), 84-95). In the presence of sodium azide the intermediate **1.13** gives the acyl azide **1.14** (Sagandira, Cloudius R. and Watts, Paul, European Journal of Organic Chemistry, 2017, 2017 (44), 6554-6565) which react in DMSO with the aryl or heteroarylamine **1.3** to obtain the final compound **1.4** (Feng, Peng & al, Organic Letters, 2014, 16(12), 3388-3391)

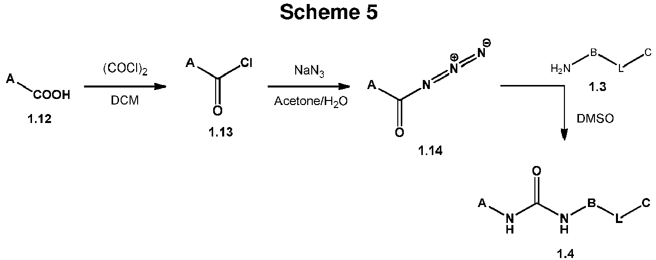

The reagents and starting materials are commercially available and/or, using well-known techniques, can be readily synthesized by one of ordinary skill in the art. Unless otherwise noted, all commercially starting materials were used without further purification.

### Synthesis of key intermediates

### Synthesis of 3: (General Procedure A)

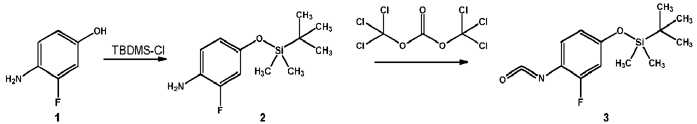

### Step 1. Synthesis of compound (2)

TBDMS-CI (14.23g, 93.6mmol, 1.2eq) was added to a solution of compound **1** (10.0g, 78.6mmol, 1eq) and imidazole (6.42g, 93.6mmol, 1.2eq) in THF (100mL) and the reaction mixture was stirred at ambient temperature for 12h. The formed precipitate was filtered, the solvent was evaporated under reduced pressure, the residue was purified by column chromatography on silica gel (DCM) to afford compound **2** (Yield: 95%, 18g).

### Step 2. Synthesis of compound (3): tert-butyl(3-fluoro-4-isocyanatophenoxy)dimethylsilane

Triphosgene (5.77g, 19.5mmol, 1eq) was added portions to a solution of compound **2** (4.7g, 19.5mmol, 1eq) and Et₃N (8.4mL, 58.5mmol, 3eq) in DCM (50mL) at -20°C, the reaction mixture was stirred at ambient temperature overnight. Solvent was removed under reduced pressure, THF (50mL) was added to the residue. The formed precipitate was filtered off, solvent was evaporated under reduced pressure to give compound **3.** The residue was used directly to the next step without additional purification (Yield: 99%, 5.2g).

### Synthesis of 8: (General Procedure B)

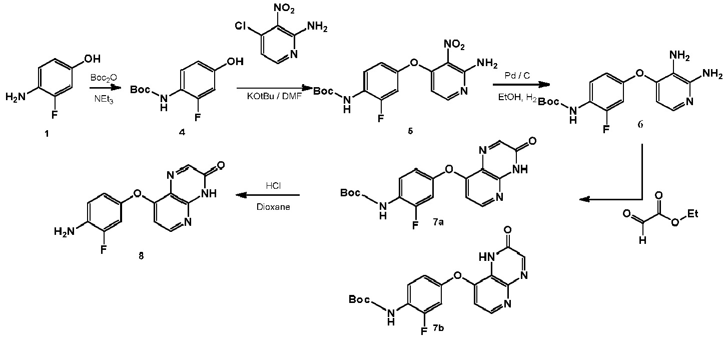

### Step 1. Synthesis of compound (4)

(BOC)₂O (34.0g, 0.15mol) was added by portions to a solution of compound **1** (20.0g, 0.15mol) and Et₃N (22.0mL, 0.15mol) in DCM (200mL). The reaction mixture was stirred at ambient temperature for 2h and evaporated to dryness. The residue was purified by flash chromatography on silica gel with DCM to afford compound **4** (Yield: 56%, 20g).

### Step 2. Synthesis of compound (5)

tBuOK (8.4g, 0.075mol) was added portion wise to a solution of compound **4** (13.0g, 0.057mol) in dry DMF (100mL) and stirred at ambient temperature 30min. 4-Chloro-3-nitropyridin-2-amine (10.0g, 0.057mmol) was added as a solid in one portion and the reaction mixture was subsequently heated at 85°C for 1h. The reaction mixture was cooled, diluted with water (200mL) and the formed precipitate was filtered off, washed with water, hexane and dried to afford compound **5** (Yield: 83%, 17.5g).

### Step 3. Synthesis of compounds (6)

10% Pd/C (2.0 g, ∼10% weight) were added to a solution of compound **5** (17.5g, 0.05 mol) in mixture EtOH/EtOAc (200mL, 2:1). Hydrogen was passed through the reaction mixture under stirring at r.t. over 1 atmosphere for 16h. Then the catalyst was filtered off through celite. The filtrate was evaporated under reduced pressure to afford of compound **6** (Yield: 95%, 15g).

### Step 4. Synthesis of compounds (7a and 7b)

To a solution of compound **6** (15.0g, 0.044mol) in EtOH (200mL) was added ethyl glyoxalate 50% solution in toluene (14mL, 0.067mol). Then reaction mixture was stirred at ambient temperature for 16h. The solvent was evaporated. The residue was subjected to column chromatography on silica gel eluting with DCM/EtOAc (0 → 100%) to afford the compound **7a** (Yield: 33%, 5.5g) and compound **7b** (Yield: 60%, 10.0g).

### Synthesis of compound (8): 8-(4-amino-3-fluorophenoxy)pyrido[2,3-b]pyrazin-3(4H)-one

HCl/dioxane (15mL, 0.04mol, 3M) was added to compound **7a** (5.5g, 0.014mol). The reaction mixture stirred at ambient temperature 2h, then evaporated under reduced pressure, then DCM (100mL) and saturated aqueous solution of KHCO₃ (100mL) was added. The organic layer was separated, washed with brine, dried over sodium sulfate, filtered and concentrated to afford the compound **8** (Yield: 75%, 3g) as a brown solid.

### Synthesis of 11: according to General Procedure A

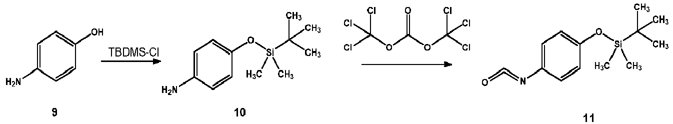

### Step 1. Synthesis of compound (10)

TBDMS-CI (14.23g, 93.6mmol, 1.2eq), compound **9** (8.57g, 78.6mmol, 1eq), imidazole (6.42g, 93.6mmol, 1.2eq), THF (100mL) (Yield of 1**0:** 97%, 17g).

### Step 2. Synthesis of compound (11): tert-butyl(4-isocyanatophenoxy)dimethylsilane

Triphosgene (5.77g, 19.5mmol, 1eq), compound **10** (4.35g, 19.5mmol, 1eq), Et₃N (8.4mL, 58.5mmol, 3eq), DCM (50mL) (Yield of **11:**97%, 4.73g).

### Synthesis of 14: (General Procedure C)

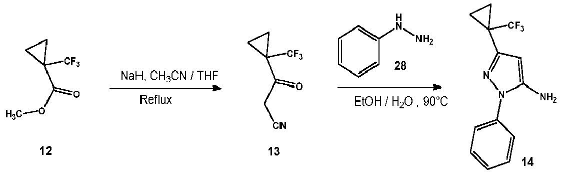

### Step 1. Synthesis of compounds (13)

To a slurry of NaH (1.0g, 26.0mmol) in THF (20mL) heated at reflux, then was added dropwise over 10 minutes a solution compound **12** (2.0g, 17.5mmol) in acetonitrile (1mL, 19mmol) and the reaction mixture heated at reflux overnight. After cooling to r.t., the reaction was partitioned between Et₂O and water, the aqueous layer acidified with 1N HCl(aq), the aqueous layer was extracted with Et₂O (2×50mL), the combined organic layers were dried under MgSO₄, and evaporated to dryness. The residue was purified by flash chromatography on silica gel with hexane/EtOAc (0→50%) as an eluent to afford compound **13** (Yield: 46%, 1g).

### Step 2. Synthesis of compounds (14): 1-phenyl-3-(1-(trifluoromethyl)cyclopropyl)-1H-pyrazol-5-amine

To solution of compound **13** (1g, 8.1 mmol) in EtOH (5mL) was added water (5mL), 1N NaOH(aq) (8mL), and **28** (1.2g, 8.1mmol) and the mixture heated at 90°C overnight. After cooling to r.t., the mixture was diluted with water, extracted with EtOAc, the combined organic layers were dried under MgSO₄, and evaporated to dryness. The residue was purified by flash chromatography on silica gel with DCM/EtOAc (0→10%) as an eluent to afford compound **14** (Yield: 18%, 0.4g).

### Synthesis of 18:

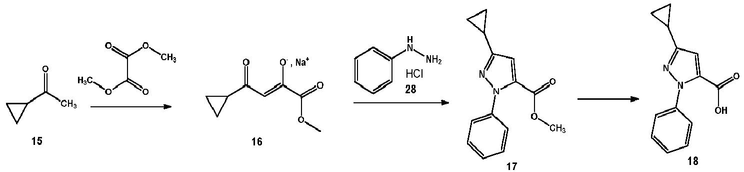

### Step 1. Synthesis of compounds (17)

Compound **15** (5g, 59 mmol), dimethyl oxalate (7g, 59mmol) and MeONa (3.3g, 60mmol) was mixed in MeOH (100mL). After stirring for 2h at 60°C, reaction mixture was cooled to r.t., **28** (8.6g, 59mmol) was added to the reaction mixture. After stirring for 12h at 60°C, reaction mixture was cooled to r.t.. The solvent was then removed under reduced pressure. Water (200mL) was added to the residue, and the resulting mixture was extracted with EtOAc (3×50mL). The combined organic extracts were washed with brine and dried over MgSO₄. The solvent was then removed under reduced pressure. The crude product was purified with column using PE/EtOAc as eluent to give compound **17** (Yield: 32%, 4.6g).

### Step 2. Synthesis of compounds (18): 3-cyclopropyl-1-phenyl-1H-pyrazole-5-carboxylic acid

Compound **17** (4.6g, 3.8mmol) was dissolved in MeOH (50mL) and H₂O (15mL), and NaOH (4g) were then added. After stirring for 12h at 20°C, solvent was then removed under reduced pressure. Water (100mL) was added to the residue, and conc. HCl were then added until reaching pH 3. The precipitate was filtered and dried under reduced pressure to get compound **18** (Yield: 78%, 3.4g).

### Synthesis of 22: according to General Procedure C

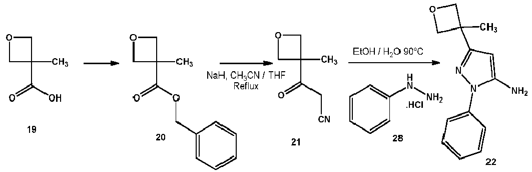

### Step 1. Synthesis of compounds (20)

To a solution of compound **19** (2.44g, 0.021 mol), DIPEA (3.8mL, 0.021 mol) in DCE (50mL) was added benzyl bromide (3.7g, 0.021 mol). The reaction mixture was refluxed for 3h. The solid was removed by filtration and the solution was evaporated to dryness to afford of compound **20** (Yield: 91%, 4g).

### Step 2. Synthesis of compounds (21)

NaH (0.6g, 14.0mmol), THF (20mL), compound **20** (2.0g, 9.7mmol), acetonitrile (0.5mL, 10.0 mmol). Residue was purified by flash chromatography on silica gel with DCM/EtOAc (0→10%) (Yield of **21:** 58%, 0.8g).

### Step 3. Synthesis of compounds (22): 3-(3-methyloxetan-3-yl)-1-phenyl-1H-pyrazol-5-amine

Compound **21** (0.8g, 5.6mmol), EtOH (5mL) was added water (5mL), 1N NaOH(aq) (5.7mL), and **28** (0.8g, 5.6mmol). Residue was purified by flash chromatography on silica gel with DCM/EtOAc (0→10%) (Yield of **22:** 52%, 0.67g).

### Synthesis of 24: 1-(5-amino-3-(tert-butyl)-1H-pyrazol-1-yl)ethenone

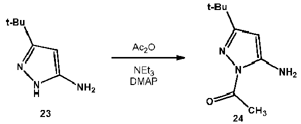

Compound **23** (2.0mmol) was dissolved in DCM (2.5mL). NEt₃ (2.2mmol) and Ac₂O (2.0mmol) were added followed by a catalytic amount of DMAP, and the mixture was stirred for 1h at r.t.. Purification by flash chromatography furnished the corresponding compound **24** as a brown oil (Yield: 41%, 0.15g).

### Synthesis of 26: 3-(tert-butyl)-1-(2-morpholinoethyl)-1H-pyrazol-5-amine (General Procedure D)

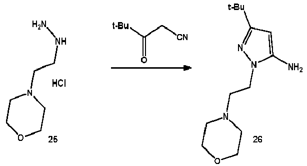

On a suspension of **25** (1g, 3.9mmol) and pivaloyl acetonitrile (0.49g, 3.9mmol) in 100mL EtOH was added HCl 12 N (10.0eq). The reaction mixture was refluxed for 16h. After cooling down, NaHCO₃ solid added until pH was around 7 and the mixture was evaporated off then the residue was purified by column chromatography on silica gel (DCM-MeOH) to afford compound **26** (Yield: 30%, 0.3g).

### Synthesis of 29: phenyl (3-(tert-butyl)-1-(quinolin-6-yl)-1H-pyrazol-5-yl)carbamate (General Procedure E)

### H.-G. Lee, M.-J. Kim, S.-E. Park, J.-J. Kim, S.-G. Lee, Y.-J. Yoon, Synlett, 2009, 2809-2814

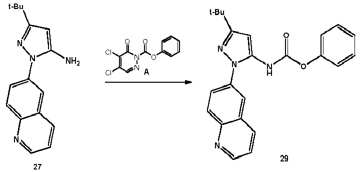

Mixture of compound **27** (0.21g, 1.05mmol) and compound **A** (0.31g, 1.1mmol) in 20mL of dry THF was refluxed overnight. Volatiles was evaporated and product **29** was isolated by column chromatography on silica gel eluting DCM/EtOAc (9:1) (Yield: 49%, 0.2g).

### Synthesis of 34:

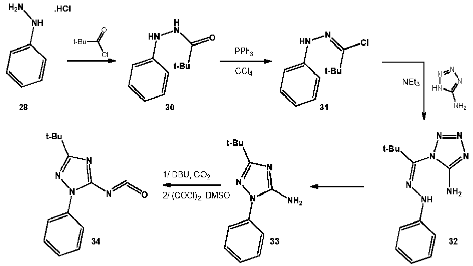

### Step 1. Synthesis of compound (30)

To a solution of **28** (4.0g, 27.68mmol) in 100mL of DCM and Et₃N (8.1mL, 2.1eq) at 5°C was added a solution of pivaloyl chloride (3.5g, 1.05eq) in 20mL DCM dropwise. Mixture stirred at r.t. for 5min and evaporated. Residue was partitioned between EtOAc and water. Organic layer was separated, dried over Na₂CO₃ and evaporated. Product **30** was used in the next step without purification (Yield: 99.6%, 5.3g).

### Step 2. Synthesis of compound (31)

To a solution of compound **30** (5.3g, 27mmol) in dry ACN (100mL), was added CBr₄ (17.9g, 2eq) and PPh₃(14.5g, 2eq). Mixture was stirred at ambient temperature overnight. Volatiles were evaporated and the residue was taken in Et₂O (30mL) and additionally 50mL of hexane were added. After 30min solution was decantated from oily residue and evaporated to afford crude product **31,** which was used in the next step without purification (Yield: 48%, 2.8g)

### Step 3. Synthesis of compound (32)

To a suspension of 5-aminotetrazole monohydrate (1.13g, 11mmol) in EtOH was added Et₃N (1.1g, 11mmol). After dissolution, compound **31** (2.8g, 11mmol) was added in one portion. Precipitate start to form after several minutes. Mixture stirred at r.t. for 2h and filtered, washed with EtOH and precipitate dried on air to afford compound **32** as a white solid (Yield: 52%, 1.5g).

### Step 4. Synthesis of compound (33)

Compound **32** (1.5g, 5.7mmol) suspended in 20mL of o-xylene and subjected to reflux for 20min. Starting material dissolves upon heating and product crystallize from reaction mixture after cooling. Precipitate filtered off, washed with toluene and dried on air to afford compound **33** as a white crystals (Yield: 89%, 1.1g).

### Step 5. Synthesis of compound (34): 3-cyclopropyl-5-isocyanato-1-phenyl-1H-1,2,4-triazole

A solution of oxalyl chloride (200mL, 2.31 mmol) in DCM (5mL) under an atmosphere of CO₂ was cooled to -60°C and DMSO (0.18g, 2.31mmol) in DCM (1mL) was added dropwise to the cold mixture. The resulting mixture was stirred at low temperature for 5min. In a second flask, CO₂ was bubbled through a solution of compound **33** (0.5g, 2.31mmol) and DBU (0.36g, 2.31mmol) in DCM (5mL) at r.t.. This mixture was cannula transferred to the first (cold)solution. The resulting cold mixture was stirred at low temperature for 10min. NEt₃ (0.32mL, 2.31 mmol) was added, and the mixture was stirred for 10min. The solution was allowed to slowly warm to r.t. and was left to stir for an additional 10min. The mixture was concentrated under reduced pressure to afford the crude material, which was purified by flash chromatography on dried silica gel eluting EtOAc/Hex (1:2) to afford isocyanate **34** (Yield: 23%, 130mg) (Probably in dimeric or tetrameric form, ESI/MS M+1=243.6, 485.6, 727.8, 969.7). ¹H-NMR in CDCl₃ ppm: 1.49 (d, 9H), 7.36-7.56 (m, 4H), 8.05 (d, 1H)

### Synthesis of 38: according to General Procedure A

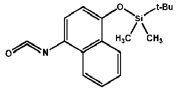

### Step1. Synthesis of compound (36)

TBDMS-CI (2.2g, 14.6mmol, 1.2eq), compound **35** (2.3g, 12.2mmol, 1eq), imidazole (0.99g, 14.6mmol, 1.2eq), THF (100mL) (Yield of **36:** 96%, 3.6g).

### Step2. Synthesis of compound (37)

A mixture of compound **36** (3.6g, 12.1 mmol, 1eq) and 10% Pd/C (10% by weight, 0.4g) in MeOH (20mL) was stirred at r.t. under flow of H₂ overnight. Then Pd/C was filtered, the solvent was evaporated under reduced pressure. The residue containing **37** was used directly to the next step without additional purification (Yield: 96%, 3.2g).

### Step3. Synthesis of compound (38): tert-butyl((4-isocyanatonaphthalen-1-yl)oxy)dimethylsilane

Triphosgene (3.47g, 11.7mmol, 1eq), compound **37** (3.2g, 11.7mmol, 1eq), Et₃N (4.89mL, 35.1mmol, 3eq), DCM (50mL) (Yield of **38:** 100%, 3.5g).

### Synthesis of 50: according to General Procedure B

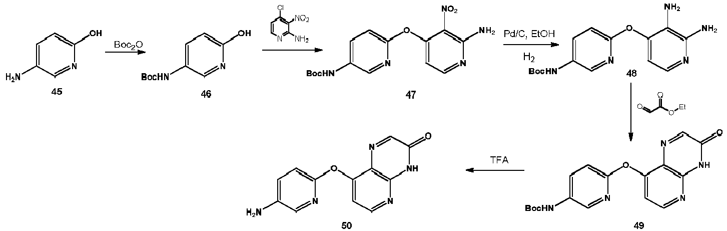

### Step 1. Synthesis of compound (46)

To solution of compound **45** (2g, 18,2mmol) in t-BuOH (40mL) was added (Boc)₂O (4g, 18.2mmol). The solution was stirred and refluxed 18h, then cooled. Then solvent was concentrated under reduced pressure and the residue was crystallized from toluene, dried to afford compound **46** (Yield: 37%, 1.5g).

### Step 2. Synthesis of compound (47)

NaH (0.23g, 5.8mmol) was added portion wise to a solution of compound **46** (0.98g, 5.8mmol) in dry DMSO (30mL) and stirred at ambient temperature 30min. 4-Chloro-3-nitropyridin-2-amine (1.0g, 5.8mmol) was added as a solid in one portion and the reaction mixture was subsequently heated at 70°C for 1h. The reaction mixture was cooled, diluted with water (200mL) and extracted with EtOAc (2x50mL). The organic layer was separated, washed with brine, dried over Na₂CO₃, filtered and concentrated. The residue, after evaporation, was subjected to column chromatography on silica gel eluting with DCM/EtOAc (0 → 30%) to afford compound **47** (Yield: 50%, 1.0g).

### Step 3. Synthesis of compound (48)

10% Pd/C (0.5g, ∼10% weight) were added to a solution of compound **47** (1.0g, 2.88mmol) in mixture EtOH/EtOAc (50mL, 2:1). Hydrogen was passed through the reaction mixture under stirring at room temperature for 16h. Then the catalyst was filtered off through celite. The filtrate was evaporated under reduced pressure to afford compound **48** (Yield: 99%, 0.91g).

### Step 4. Synthesis of compound (49)

To a solution of compound **48** (910mg, 2.87mmol) in EtOH (50mL) was added ethyl glyoxalate 50% solution in toluene (1.5mL, 7mmol). Then reaction mixture refluxed for 2h. The solvent was evaporated. The residue after evaporation was subjected to column chromatography on silica gel eluting with DCM/EtOAc (0 → 100%) to afford the compound **49** (Yield: 11%, 0.11 g).

### Step 5. Synthesis of compound (50): 8-((5-aminopyridin-2-yl)oxy)pyrido[2,3-b]pyrazin-3(4H)-one

To solution of compound **49** (110mg, 0.31mmol) in TFA (2mL) was stirred 1h at ambient temperature, when evaporated under reduced pressure, residue dissolved in water (7mL), pH of solution up to 6 with NaHCO₃, water evaporated under reduced pressure to dryness. Residue **50** was dissolved in EtOH (30mL), filtered, evaporated under reduced pressure to dryness and used on the next stage without separation.

### Synthesis of 52: 6-aminopyridin-3-yl tert-butyl carbonate

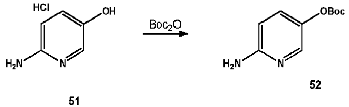

Compound **51** (0.600g, 4.1mmol), (Boc)₂O (0.893g, 4.1mmol), DMAP (30mg, 0.02mmol) and Et₃N (0.414g, 4.1mmol) in ACN (30mL) were stirred at ambient temperature for 12h and evaporated to dryness. The residue was dissolved in DCM, washed with water and brine, dried under Na₂SO₄ and filtrated. The filtrate was evaporated to dryness afford pure compound **52** (Yield: 79%, 0.679g).

### Synthesis of 54: phenyl (3-(tert-butyl)-1-phenyl-1H-pyrazol-5-yl)carbamate according to General Procedure E

### H.-G. Lee, M.-J. Kim, S.-E. Park, J.-J. Kim, S.-G. Lee, Y.-J. Yoon, Synlett, 2009, 2809-2814

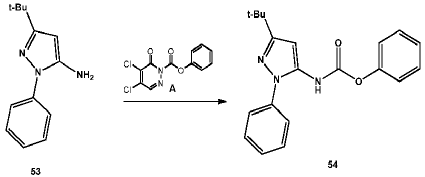

Compound **53** (0.226g, 1.05mmol), compound **A** (0.31 g, 1.1mmol), 20mL of anhydrous THF (Yield of **54:** 57%, 0.2g).

### Synthesis of 56: 3-(tert-butyl)-1-phenyl-1H-pyrazole-5-carbonyl azide

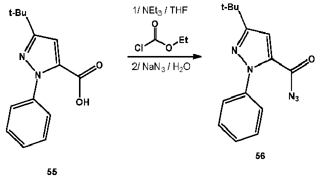

Under inert atmosphere (Argon), a stirred solution of compound **55** (484mg, 1.98mmol) and Et₃N (0.35mL, 2.57mmol) in dry THF (5mL) was cooled to 10°C. Ethyl chloroformate (0.280mL, 2.97mmol) was added drop wise and the resulting mixture was stirred for 0.5h. Afterwards, a solution of NaN₃ (266mg, 3.96mmol) in water (2mL) was added in one portion. After 1 h at 10°C, the reaction was found to be complete (TLC) and was quenched into iced water (5mL). The mixture was extracted with EtOAc (3x10mL) and the combined organic layers were successively dried over MgSO₄, filtered and evaporated to obtain a crude azide **56.**

### Synthesis of 59: according to General Procedure A

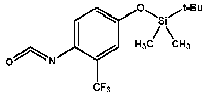

### Step 1. Synthesis of compound (58)

TBDMS-CI (1.022g, 6.78mmol), 4-amino-3-(trifluoromethyl)phenol **57** (1.0g, 5.65mmol), imidazole (0.461 g, 6.78mmol), THF (25mL). Purification by column chromatography, using hexane and hexane/Et₂O 2:1, 1:1. (Yield of **58:** 91.5%, 1.5g).

### Step 2. Synthesis of compound (59): tert-butyl(4-isocyanato-3-(trifluoromethyl)phenoxy)dimethylsilane

Triphosgene (1.531g, 5.15 mmol), compound **58** (1.5g, 5.15mmol), Et₃N (1.4mL, 10.3mmol), DCM (25mL). (Yield of **59:** 100%, 1,62g).

### Synthesis of 61: tert-butyl (3-hydroxyphenyl)carbamate

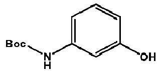

(BOC)₂O (10.0g, 45.9mmol) was added by portions to a solution of 3-aminophenol **60** (5.0g, 45.9 mmol) and Et₃N (6.5mL, 45.9mmol) in DCM (200mL). The reaction mixture was stirred at ambient temperature for 2h and evaporated to dryness. The residue was purified by flash chromatography on silica gel with DCM to afford compound **61** (Yield: 64%, 6.0 g).

### Synthesis of 66: phenyl (3-(tert-butyl)-1-phenyl-1H-1,2,4-triazol-5-yl)carbamate according to General Procedure E

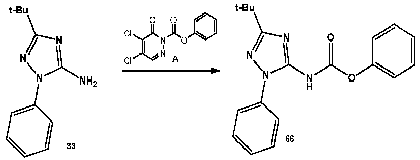

Compound **33** (0.5g, 2.32mmol), compound **A** (0.71g, 2.5mmol), 20mL of dry THF (Yield of **66:** 51%, 400mg).

### Synthesis of 69: (General Procedure F)

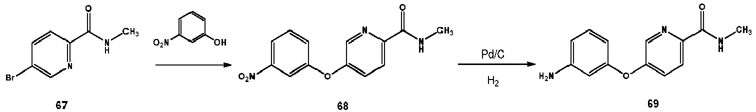

### Step 1. Synthesis of compound (68)

Mixture of compound **67** (1.0g, 4.65mmol, 1.0eq), 3-nitrophenol (1.29g, 9.3mmol, 2.0eq) and K₂CO₃ (0.64g, 4.65mmol) in 30mL of NMP was heating overnight at 150°C, after cooling water was added and product was extracted with CHCl₃. Organic extract was dried over sodium sulfate and evaporated off. Residue was triturated with acetone, brown precipitate **68** was filtered and dried (Yield: 16%, 0.2g).

### Step 2. Synthesis of compound (69): 5-(3-aminophenoxy)-N-methylpicolinamide

0.2g of compound **68** in 100mL of MeOH was hydrogenated on 20mg 10% Pd/C at 10bars for 4h at r.t.. The catalyst was filtered off, solvent was removed under reduced pressure to give compound **69.** The residue was used directly to the next step without additional purification (Yield: 91%, 0.19g).

### Synthesis of 71: 2-fluoro-4-phenoxyaniline (General Procedure G)

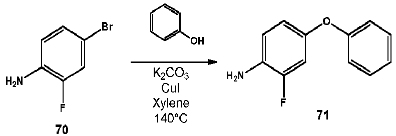

A mixture of aniline **70** (1.0g, 5.26mmol), phenol (1.0g, 10.52mmol), N-methyl-imidazole (0.216g, 2.63mmol), K₂CO₃ (1.452g, 10.52mmol) and Cul (0.05g, 0262 mmol) in xylene (20mL) was stirred in glass "bomb" at 140°C for 30h. After cooling to r.t. the reaction mass was filtered and the mother liquor was concentrated under reduced pressure. The residue was purified by column chromatography, using hexane and hexane/Et₂O 5:1 as eluent, to provide the product **71** (Yield: 9.2%, 0.098g).

### Synthesis of 72: 2-fluoro-4-(pyridin-4-yloxy)aniline

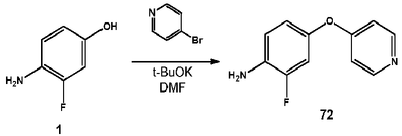

A mixture of **1** (3.41g, 26.8mmol), 4-bromo-pyridine hydrochloride (5.212g, 26.8mmol) and t-BuOK (6.6g, 59.0mmol) in DMF (200mL) was stirred at 50°C for 12h. After cooling to r.t. the reaction mass was filtered, the mother liquor was concentrated under reduced pressure. The residue was purified by column chromatography, using DCM and 2% MeOH in DCM as eluent, to provide the product **72** (Yield: 3.1 %, 0.17g).

### Synthesis of 75:

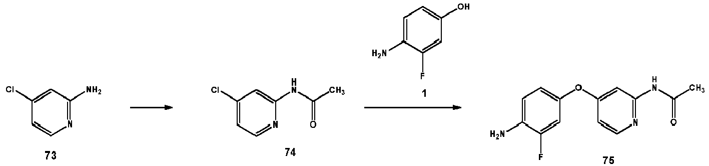

### Step 1. Synthesis of compound (74)

To a compound **73** (1.74g, 13.5mmol) was added acetic anhydride (1.66g, 16mmol) and triethylamine (2.74g, 27mmol). The mixture was stirred overnight at 60°C. The reaction mixture was concentrated under reduced pressure. The crude residue was purified by silica gel chromatography to afford compound **74** (Yield: 67%, 1.55g).

### Step 2. Synthesis of compound (75): N-(4-(4-amino-3-fluorophenoxy)pyridin-2-yl)acetamide (General Procedure H)

Compound **74** (0.62g, 3.65mmol), compound 1 (0.58g, 4.6mmol) and Cs₂CO₃ (1.54g, 4.72mmol) was mixed in NMP (15mL). After stirring for 12h at 100°C, reaction mixture was cooled to r.t., water (100mL) was added to the reaction mixture, and the resulting mixture was extracted with EtOAc (2×50mL). The combined organic extracts were washed with brine and dried over Na₂SO₄. The solvent was then removed under reduced pressure. The crude product was purified with column using PE/EtOAc as eluent to give compound **75** (Yield: 9%, 0.11g).

### Synthesis of 76: 4-(4-amino-3-fluorophenoxy)-N-methylpicolinamide according to General Procedure H

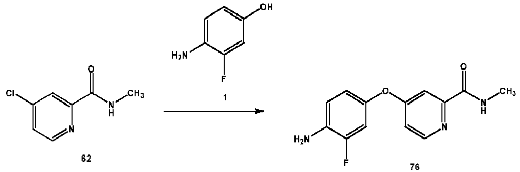

Compound **62** (1.34g, 7.87mmol), compound **1** (1g, 7.8mmol), Cs₂CO₃ (1.54g, 4.72mmol), NMP (15mL) (Yield of **76:** 29%, 0.6g).

### Synthesis of 80:

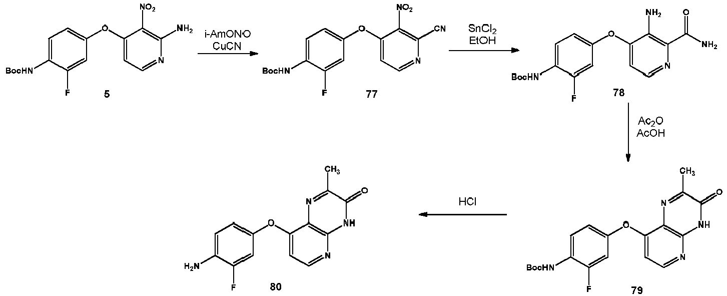

### Step 1. Synthesis of compound (77)

Mixture of compound **5** (5.6g, 15.38mmol) and CuCN (2.07g, 23.07mmol) in 100mL of anhydrous ACN was subjected to reflux and *i*-AmONO (2.34g, 20.0mmol) was added dropwise. Reflux continued for 2h and mixture evaporated to dryness. Residue taken up in EtOAc/THF (9:1) and filtered through celite. Filtrate evaporated and product purified by column chromatography on silica gel using EtOAc as eluent to afford compound **77** (Yield: 26%, 1.5g).

### Step 2. Synthesis of compound (78)

Compound **77** (1.5g, 4.0mmol) dissolved in 30mL of EtOH and SnCl₂.2H₂O (4.51g, 5eq) was added in one portion. Mixture stirred at ambient temperature for 2h. TLC indicated complete conversion. Volatiles evaporated, residue taken up EtOAc and quenched with 100mL of a saturated solution of NaHCO₃. Mixture filtered through celite and organic layer was separated, washed with brine, dried over Na₂CO₃, filtered and concentrated to afford compound **78** (1.45g), which was pure enough to be used in the next step without purification.

### Step 3. Synthesis of compound (79)

Ac₂O (2.0 g, 19.6mmol) were added to a solution of compound **78** (1.45 g, 4.0 mmol) in glacial AcOH (20mL) at r.t.. Mixture stirred at r.t. for 2h and heated at 50°C for 30min. Volatiles evaporated residue taken up EtOAc and quenched with 30mL of a saturated solution of NaHCO₃. Precipitated product filtered, washed with water, EtOAc and dried in air to afford 0.3g of compound **79.** Additional amount of product was left in mother liquor.

### Step 4. Synthesis of compound (80): 8-(4-amino-3-fluorophenoxy)-2-methylpyrido[2,3-b]pyrazin-3(4H)-one

HCl/dioxane (2.1mL, 6.3mmol, 3M) was added to compound **79** (300mg, 0.77mmol). The reaction mixture stirred at ambient temperature overnight with gas-stop bubbler. Mixture evaporated under reduced pressure, then Et₃N (1mL) in 5M MeOH were added. Mixture stirred for 5min and evaporated. Then 1,4-dioxane (5mL) was added and precipitate was filtered off. Filtrate evaporated to dryness to afford the compound **80** (Yield: 91%, 200mg) as a white solid.

### Synthesis of 81: 2-fluoro-4-(4-fluorophenoxy)aniline according to General Procedure G

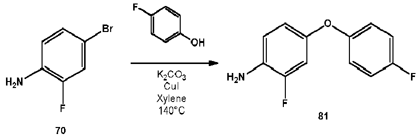

Aniline **70** (1.0g, 5.26mmol), 4-fluoro-phenol (0.707g, 6.31 mmol), N-methyl-imidazole (0.216g, 2.63mmol), K₂CO₃ (1.452g, 10.52mmol) and Cul (0.05g, 0.262mmol), xylene (20mL) (Yield of **81:** 9.5%, 011g).

### Synthesis of 89:

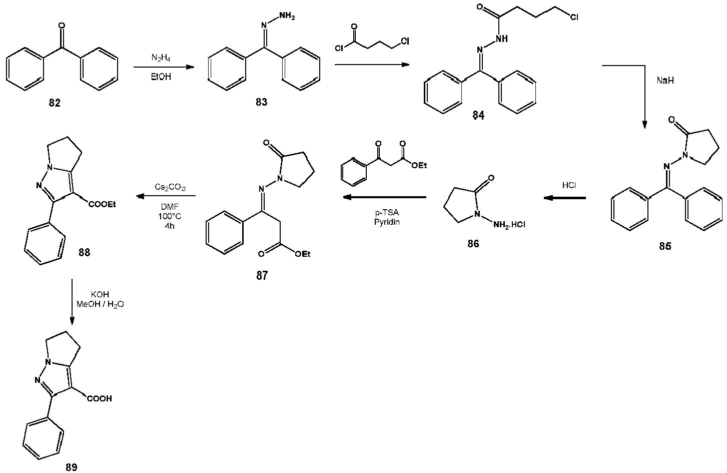

### Step 1. Synthesis of compound (83)

A mixture of **82** (31.0g, 170.3mmol) and N₂H₄.H₂O (11.29g, 238.4mmol) in EtOH (100mL) was stirred at boiling temperature for 36h. The solvent was removed under reduced pressure, the residue was dissolved in DCM (200mL), washed with water (50mL), from aqua layer the product was extracted with DCM (2x30mL), the combined organic layers were dried under Na₂SO₄, the solvent was removed under reduced pressure to provide the product **83** (Yield: 91%, 30.4g).

### Step 2. Synthesis of compound (84)

4-Chlorobutyryl chloride (21.9g, 155mmol) was added dropwise at 0°C to a solution of compound **83** (30.4g, 155mmol) and pyridine (12g, 155mmol) in DCM (250mL), the reaction mass was stirred at r.t. for 12h. Then the mixture was washed with saturated solution of K₂CO₃ (2x100mL), the solvent was removed under reduced pressure, the residue was purified by crystallization from hexane to provide the product **84** (Yield: 79%, 36.9g).

### Step 3. Synthesis of compound (85)

NaH (4.91g, 122.7mmol) was added in portions to a solution of **84** (36.9g, 122.7mmol) in THF (300mL), the reaction mass was stirred at r.t. for 12h. Then saturated solution of NH₄Cl (50mL) was added, the mixture was stirred for 30min., water was added, the product was extracted with EtOAc (2x200mL), the organic layers were dried under Na₂SO₄, the solvents were removed under reduced pressure. The residue was purified by crystallization from hexane/Et₂O 3:1 to provide the product **85** (Yield: 82.4%, 26.7g).

### Step 4. Synthesis of compound (86)

A mixture of **85** (24.3g, 92mmol) and HCl (aqua 37%, 75mL) in THF (150mL) was stirred at r.t. for 12h. The mixture was washed with EtOAc (3x50mL), the residue was reevaporated under reduced pressure with EtOH/toluene 1:1 (3x150mL) and purified by crystallization from ACN to provide the product **86** (Yield: 55.7%, 7g).

### Step 5. Synthesis of compound (87)

A mixture of compound **86** (6.5g, 47.6 mmol), Ethyl benzoylacetate (9.143g, 47.8mmol) and p-TSA (0.05g) in pyridine (100mL) was stirred at boiling temperature for 12h. The solvent was removed under reduced pressure, the residue was reevaporated under reduced pressure with toluene (3x50mL) and then purified by column chromatography, using DCM and 2% MeOH in DCM as eluent, to provide the product **87** (Yield: 31.4%, 4.1g).

### Step 6. Synthesis of compound (88)

A mixture of compound **87** (4.1g, 14.9mmol) and Cs₂CO₃ (9.7g, 29.9mmol) in DMF (100mL) was stirred at r.t. for 30min, at 100°C for 4h. Then hexane (50mL), Et₂O (50mL) and EtOAc (50mL) were added, the precipitate was filtered, the solvents from the mother liquor were removed under reduced pressure, the residue was purified by column chromatography, using hexane/Et₂O 1:1 as eluent, to provide the product **88** (Yield: 22.1%, 0.847g).

### Step 7. Synthesis of compound (89): 2-phenyl-5,6-dihydro-4H-pyrrolo[1,2-b]pyrazole-3-carboxylic acid

A solution of KOH (1g) in water (5mL) was added to a solution of compound **88** (0.847g, 3.31mmol) in MeOH (20mL), the reaction mass was stirred at r.t. for 12h. The solvent was removed under reduced pressure, water (20mL) was added, the solution was acidified with 10% aqua HCl to pH 6, the product was extracted with DCM (2x30mL), the combined organic layers were dried under Na₂SO₄, the solvent was removed under reduced pressure to provide the product **89** (Yield: 66.7%, 0.503g).

### Synthesis of 94: according to General Procedure B

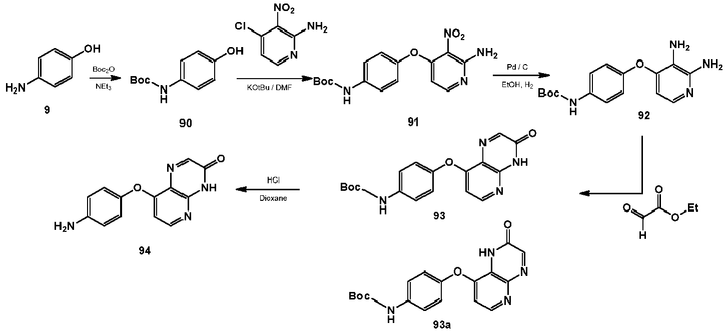

### Step 1. Synthesis of compound (90)

(Boc)₂O (29.9g, 0.14mol), compound **9** (15.0g, 0.14mol) and Et₃N (22.0mL, 0.15mol), THF (500mL). The residue was purified by flash chromatography on silica gel with DCM (Yield of **90:** 59%, 17 g).

### Step 2. Synthesis of compound (91)

tBuOK (2.4g, 0.021 mol), compound **90** (3.7g, 0.017mol), dry DMF (150mL). 4-Chloro-3-nitropyridin-2-amine (3.04g, 0.017mmol) (Yield of **91:** 81%, 5.0g).

### Step 3. Synthesis of compound (92)

10% Pd/C (0.5 g, ∼10% weight), compound **91** (5.0g, 0.014mol), mixture EtOH/EtOAc (200mL, 2:1) (Yield of **92:** 73%, 3.3 g).

### Step 4. Synthesis of compound (93 and 93a)

Compound **92** (3.3g, 0.011 mol) in EtOH (150mL), ethyl glyoxalate 50% solution in toluene (3.2mL, 0.016mol). Residue after evaporation was subjected to column chromatography on silica gel eluting with DCM/ EtOAc (0 → 100%) (Yield of **93:** 21%, 0.77g) and (Yield of **93a:** 56%, 2.1g).

### Step 5. Synthesis of compound (94): 8-(4-aminophenoxy)pyrido[2,3-b]pyrazin-3(4H)-one

HCl/dioxane (3.7mL, 0.01 mol, 3M sol), compound **93** (0.77g, 0.002mol) (Yield of **94:** 86%, 0.48g).

### Synthesis of 98:

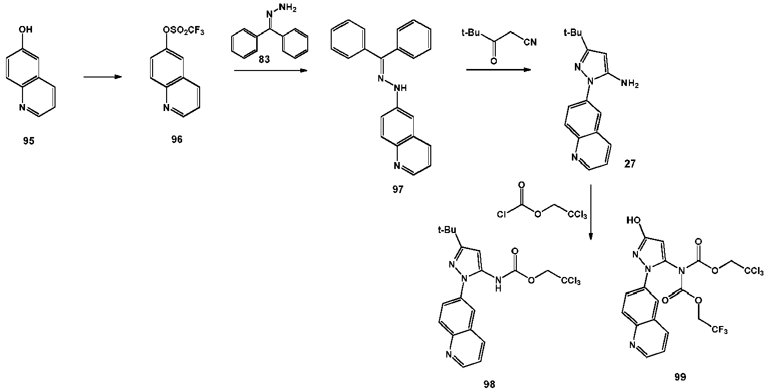

### Step 1. Synthesis of compound (96)

To a solution of compound **95** (1.0g, 6.89mmol, 1.0eq) and pyridine (1.11mL, 13.78mmol, 2.0eq) in 25mL of DCM was added dropwise at 0°C a solution of triflic anhydride (1.26mL, 7.51mmol, 1.09eq) in 5mL of DCM. The mixture was stirred at room temperature for 3h, poured into water and extracted with DCM. The organic layers were dried over MgSO₄, filtered and evaporated under vacuo to afford compound **96** as brown oil (2.0 g, quantitative yield).

### Step 2. Synthesis of compound (97)

In a sealed tube, crude compound **96** (2.0g, 6.9mmol), benzophenone (1.76g, 8.97mmol, 1.3eq), Cs₂CO₃ (3.6g, 11.04mmol, 1.6eq) and dppf (115mg, 0.21mmol, 0.03eq) were put in suspension in 50mL of toluene. The mixture was degassed with Ar for 20min before addition of PdOAc (0.6mg, 0.003mmol, 0.0004eq). The mixture was heated at 90°C for 20h. After cooling down, the mixture was evaporated, adsorbed on silica gel and purified by flash chromatography using cyclohexane and EtOAc to afford compound **97** (Yield: 73%, 1.6g).

### Step 3. Synthesis of compound (27)

To a solution of compound **97** (1.6g, 5.07mmol, 1.0eq) and pivaloyl acetonitrile (952mg, 7.60mmol, 1.5eq) in 25mL of EtOH wad added HCl 12N (4.2mL, 51mmol, 10eq). The mixture was stirred at reflux for 20h. EtOH was evaporated and the residue was triturated in Et₂O, diluted in water and washed with EtOAc. The aqueous layer was basified with a saturated solution of sodium carbonate (pH 8) and extracted with EtOAc. The organic layers were dried over MgSO₄, filtered and evaporated under vacuo. The residue was purified by flash chromatography on silica gel using cyclohexane and EtOAc to afford compound **27** as yellow foam (Yield: 74%, 1.0g).

### Step 4. Synthesis of compound (98): 2,2,2-trichloroethyl (3-(tert-butyl)-1-(quinolin-6-yl)-1H-pyrazol-5-yl)carbamate

At -10°C, to a solution of compound **27** (1.0g, 3.75mmol, 1.0eq) in 15mL of DCM were added pyridine (1.0mL, 12.38mmol, 3.3eq), DMAP (catalytic) and trichloroethyl chloroformate dropwise (0.72mL, 5.25mmol, 1.1 eq). The mixture was stirred at -10°C for 1h. Water was added. After stirring 10min at r.t., the mixture was extracted with dichloromethane. The organic layers were dried over MgSO₄, filtered and evaporated under vacuo. The residue was purified by flash chromatography on silica gel using cyclohexane and EtOAc to afford compound **98** as pale yellow oil (Yield: 43%, 720mg) and compound **99** (1.0g) as by-product.

### Synthesis of 103:

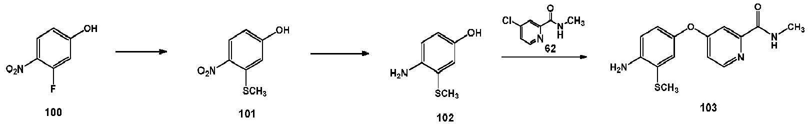

### Step 1. Synthesis of compound (101)

To a solution of compound **100** (5.6g, 35.7mmol, 1eq) in 200mL of dry DMF were added portionwise sodium thiomethoxide (5g, 71.3mmol, 2eq) and potassium carbonate (14.8g, 107mmol, 3eq). The mixture was stirred at r.t. for 48h. The suspension was filtrated on a short pad of celite and rinsed with EtOAc. The filtrate was concentrated. Water was added, the aqueous phase was acidified to pH 4 with a 1N solution of HCl and extracted with EtOAc. The organic layers were dried over MgSO₄, filtered and evaporated under vacuo to afford compound **101** (Yield: 85%, 5.6g, 85%) as yellow solid.

### Step 2. Synthesis of compound (102)

Iron powder (11.9g, 213.8mmol, 3eq) was slowly added to a solution of compound **101** (13.2g, 71.3mmol, 1eq) in 300mL of acetic acid and 30mL of EtOH. The reaction mixture was heated up to 45°C for 18h. After cooling down, the suspension was filtrated on a short pad of celite and rinsed with MeOH. The filtrate was concentrated and adsorbed on silica gel to be purified by flash chromatography using cyclohexane and EtOAc. Compound **102** (Yield: 55%, 6.1g) was obtained as brown solid.

### Step 3. Synthesis of compound (103): 4-(4-amino-3-(methylthio)phenoxy)-N-methylpicolinamide

Compound **102** (500mg, 3.22mmol, 1eq) was solubilized in 7mL of DMA. The mixture was degassed with Ar before addition of tBuOK (434mg, 3.86mmol, 1.2eq). After stirring 15min at r.t., compound **62** (550mg, 3.22mmol, 1eq) was added and the mixture was heated at 100°C for 20h. After cooling down, EtOAc and water were added. The aqueous layer was extracted with EtOAc and the organic phases were washed with brine. The organic layers were dried over MgSO₄, filtered and evaporated under vacuo. The residue was purified by flash chromatography on silica gel using cyclohexane and EtOAc to afford compound **103** (Yield: 41%, 380mg) as pale orange powder.

### Synthesis of 105: 2,2,2-trichloroethyl (5-(tert-butyl)-1,3,4-thiadiazol-2-yl)carbamate (General Procedure I)

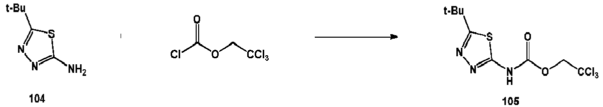

At 0°C, pyridine (0.56mL, 7mmol, 1.1eq) and 2,2,2-trichloroethylchloroformate (0.96mL, 7mmol, 1.1eq) were added dropwise to a solution of compound **104** (1.0g, 6.36mmol, 1.0eq) in 20mL of THF. The reaction mixture was stirred at 0°C for 1h and then at r.t. for 5h. Water was added and the mixture was extracted with EtOAc. The organic layers were washed with water and brine, dried over MgSO₄, filtered and evaporated under vacuo to afford compound **105** (2.26g, quantitative yield) as white solid.

### Synthesis of 111: (General Procedure J)

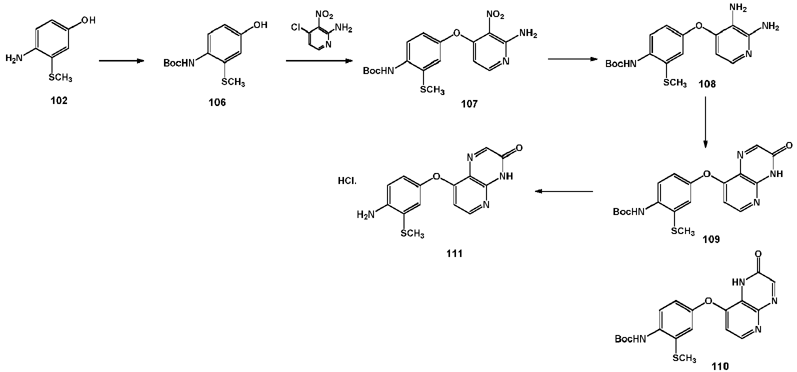

### Step 1. Synthesis of compound (106)

Compound **102** (12.7g, 81.8mmol, 1.0eq) was added to a molten mixture of BoC₂O (17.9g, 81.8mmol, 1.0eq) and InCl₃ (181 mg, 0.82mmol, 0.01 eq) at 35°C. The reaction mixture was stirred at 35°C for 4h. After cooling down, the reaction mixture was directly deposed as liquid on silica gel to be purified by flash chromatography. Flash chromatography was performed using cyclohexane and EtOAc to afford expected compound **106** (Yield: 83%, 17.3g) as off-white solid.

### Step 2. Synthesis of compound (107)

To a solution of compound **106** (15.0g, 58.7mmol, 1.0eq) in 600mL of DMF was added tBuOK (8.6g, 76.9mmol, 1.3eq). After stirring 30min at r.t., 4-chloro-2-amino-3-nitropyridine (10.1g, 58.2mmol, 0.99eq) was added and the mixture was heated at 80°C for 3h. After cooling down, the mixture was evaporated to dryness and EtOAc was added. The organic layer was washed with water, dried over MgSO₄, filtered and evaporated under vacuo. The residue was purified by flash chromatography on silica gel using cyclohexane and EtOAc to afford compound **107** (Yield: 76%, 17.6g) as yellow powder.

### Step 3. Synthesis of compound (108)

Pd/C (around 4.0g) was added to a solution of compound **107** (17.6g, 44.9mmol, 1.0eq) in a mixture of EtOH (650mL) and DCM (150mL). The reaction mixture was hydrogenated at room temperature over 1 atmosphere of hydrogen for 20h. The reaction mixture was filtrated through a short pad of celite and rinsed with EtOAc. The filtrate was evaporated to afford compound **108** as brown foam (17.3g, quantitative yield).

### Step 4. Synthesis of compound (109)

To a solution of compound **108** (16.3g, 45.0mmol, 1.0eq) in 240mL of MeOH was added glyoxylic acid monohydrate (41.4g, 449.7mmol, 10.0eq). The mixture was stirred at r.t. for 20h. The off-white precipitate formed was filtered and washed with a small amount of MeOH to afford compound **109** (Yield: 64%, 11.6g). The undesired regioisomer **110** was contained in the filtrate.

### Step 5. Synthesis of compound (111): 8-(4-amino-3-(methylthio)phenoxy)pyrido[2,3-b]pyrazin-3(4H)-one, hydrochloride

Compound **109** (11.6g, 29.0mmol, 1.0eq) was solubilized in 4N solution of HCl in 1,4-dioxane (250 ml) and the reaction mixture was stirred at room temperature for 3h. The precipitate obtained was filtered and rinsed with dioxane and EtOAc to afford compound **111** (12g, quantitative yield) as beige solid.

### Synthesis of 117: according to General Procedure J

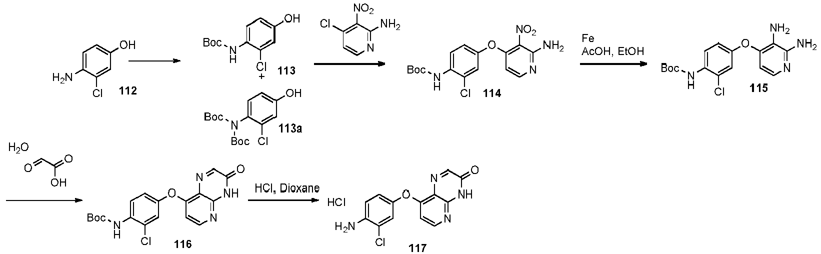

### Step 1. Synthesis of compound (113)

Compound **112** (3.0g, 20.9mmol, 1.0eq), BoC₂O (4.6g, 20.9mmol, 1.0eq) and InCl₃ (46mg, 0.21mmol, 0.01eq). Reaction mixture was stirred at 35°C for 4h. Flash chromatography on silica gel was performed using DCM and EtOAc (Yield of **113:** 9%, 480mg). Diprotected compound **113a** was also isolated (790mg) and used also in the next step.

### Step 2. Synthesis of compound (114)

Compound **113** and by-product **113a** (1.27g, 4.27mmol, 1.0eq), DMF (0.1M), tBuOK (623 mg, 5.5 mmol, 1.3 eq). 4-chloro-2-amino-3-nitropyridine (741mg, 4.27mmol, 1.0eq) added and the mixture heated at 80°C for 3h. Flash chromatography on silica gel was performed using DCM and EtOAc (Yield of **114:** 52%, 850mg).

### Step 3. Synthesis of compound (115)

Compound **114** (850mg, 2.23mmol, 1.0eq) was solubilized in acetic acid (20mL) and EtOH (2mL). Iron powder (374mg, 6.7mmol, 3.0eq) was added and the reaction mixture was stirred at 45°C for 24h. After cooling down, the mixture was filtrated through a short pad of celite and rinsed with MeOH. The filtrate was absorbed on silica gel to be purified by flash chromatography using EtOAc and MeOH to afford the expected compound **115** as colorless oil (Yield: 95%, 740mg).

### Step 4. Synthesis of compound (116)

Compound **115** (740mg, 2.11mmol, 1.0eq), MeOH (15mL),glyoxylic acid monohydrate (1.9g, 21.1mmol, 10.0eq) in MeOH. Mixture stirred at r.t. for 48h (Yield of **116:** 54%, 446mg).

### Step 5. Synthesis of compound (117): 8-(4-amino-3-chlorophenoxy)pyrido[2,3-b]pyrazin-3(4H)-one hyfrochloride

Compound **116** (446mg, 1.15mmol, 1.0eq), 4N solution of hydrochloric acid in 1,4-dioxane (10mL). Mixture stirred at r.t. for 24h. Evaporation to dryness to afford expected compound **117** in mixture with 13% of starting material.

### Synthesis of 123: according to General Procedure J

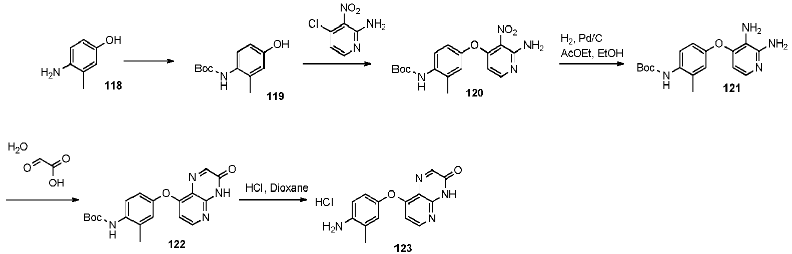

### Step 1. Synthesis of compound (119)

Compound **118** (3.0g, 24.3mmol, 1.0eq), BoC₂O (5.3g, 24.3mmol, 1.0eq), InCl₃ (54mg, 0.24mmol, 0.01eq). Reaction mixture stirred at 35°C for 4h. Flash chromatography on silica gel performed using DCM and EtOAc (Yield of **119:** 98%, 5.3g).

### Step 2. Synthesis of compound (120)

Compound **119** (5.31g, 23.78mmol, 1.0eq), DMF (0.1M), tBuOK (3.47g, 30.91mmol, 1.3eq). 4-chloro-2-amino-3-nitropyridine (4.13g, 23.78mmol, 1.0eq) added and mixture heated at 80°C, 3h. Flash chromatography on silica gel performed using DCM and EtOAc (Yield of **120:** 47%, 4.0g).

### Step 3. Synthesis of compound (121)

Pd/C (around 500mg), compound **120** (4.0g, 11.1mmol, 1.0eq), mixture of EtOH (200mL) and EtOAc (100mL). Hydrogenation at r.t. over 1 atmosphere of hydrogen for 20h. (3.7g, quantitative yield of **121**).

### Step 4. Synthesis of compound (122)

Compound **121** (1.0g, 3.03mmol, 1.0eq) in MeOH, glyoxylic acid monohydrate (1.4g, 15.15mmol, 5.0eq) in MeOH. Mixture was stirred at r.t. for 48h. Off-white precipitate formed filtered and washed with small amount of MeOH (Yield of **122:** 26%, 288mg).

### Step 5. Synthesis of compound (123): 8-(4-amino-3-methylphenoxy)pyrido[2,3-b]pyrazin-3(4H)-one hydrochloride

Compound **122** (288 mg, 0.78 mmol, 1.0 eq), 4N solution of HCl in 1,4-dioxane (6.5mL). Mixture was stirred at r.t. for 24h. Evaporation to dryness to get compound **123.**

### Synthesis of 130: according to General Procedure J

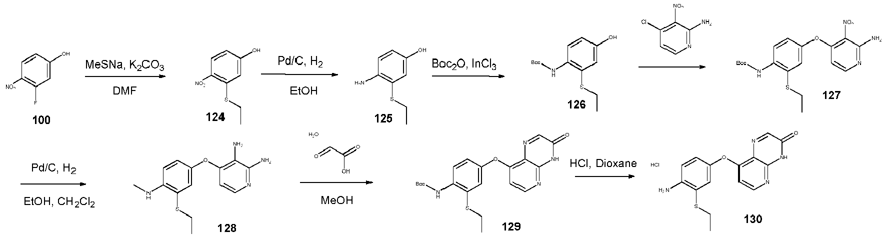

### Step 1. Synthesis of compound (124)

To a compound **100** (9.3g, 59.4mmol, 1.0eq) in dry DMF (350mL) were added portionwise sodium ethanethiolate (10.0g, 118.9mmol, 2.0eq) and K₂CO₃ (24.6g, 178.3mmol, 3.0eq). The mixture was stirred at r.t. for 20h. The suspension was filtrated on a short pad of celite and rinsed with EtOAc. The filtrate was concentrated. Water was added, the aqueous phase was acidified to pH 4 with a 1 N solution of HCl and extracted with EtOAc. The organic layers were dried over MgSO₄, filtered and evaporated under vacuo to afford expected compound **124** (Yield: 88%, 9.6g) as yellow solid.

### Step 2. Synthesis of compound (125)

Iron powder (7.1g, 128.0mmol, 3.0eq), compound **124** (8.5g, 42.7mmol, 1.0eq) in acetic acid (200mL) and EtOH (20mL). The reaction mixture was stirred at r.t. for 48h. The reaction mixture was filtrated through a short pad of celite, rinsed with EtOH and concentrated. The residue was purified by flash chromatography over silica gel using cyclohexane and EtOAc to afford the expected compound **125** as grey solid (Yield: 53%, 3.8g).

### Step 3. Synthesis of compound (126)

Compound **125** (3.8g, 22.5mmol, 1.0eq), Boc₂O(4.9g, 22.5mmol, 1.0eq), InCl₃ (50mg, 0.02mmol, 0.01eq). Reaction mixture stirred at 35°C for 4 hours. Flash chromatography on silica gel was performed using cyclohexane and EtOAc (Yield of **126:** 58%, 3.5g).

### Step 4. Synthesis of compound (127)

Compound **126** (3.5g, 13.0mmol, 1.0eq), DMF (0.1M), tBuOK (1.9g, 16.9mmol, 1.3eq). Stirring 30 minutes 4-chloro-2-amino-3-nitropyridine (2.2g, 12.9mmol, 0.99eq). Reaction mixture heated at 80°C, 3h. Flash chromatography on silica gel was performed using cyclohexane and EtOAc (Yield of **127:** 74%, 3.9g).

### Step 5. Synthesis of compound (128)

Pd/C (around 1g), compound **127** (3g, 9.6mmol, 1.0eq), EtOH (60ml). Hydrogenation at r.t. over 1 atmosphere of hydrogen for 48h. (Yield of **128:** 86%, 3.47g).

### Step 6. Synthesis of compound (129)

Compound **128** (1.0g, 2.66mmol, 1.0eq) in MeOH, glyoxylic acid monohydrate (1.2g, 13.3mmol, 5.0eq) in MeOH. Mixture stirred at r.t. for 48h. The off-white precipitate formed was filtered and washed with a small amount of MeOH (Yield of **129:** 34%, 378mg).

### Step 7. Synthesis of compound (130): 8-(4-amino-3-(ethylthio)phenoxy)pyrido[2,3-b]pyrazin-3(4H)-one hydrochloride

Compound **129** (370mg, 0.89mmol, 1.0eq), 4N solution of hydrochloric acid in 1,4-dioxane (7.5mL). Mixture stirred at r.t. for 3h and then evaporated to dryness (368 mg, quantitative yield of **130**).

### Synthesis of 132: 5-(4-amino-3-(methylthio)phenoxy)-3,4-dihydro-1,8-naphthyridin-2(1H)-one

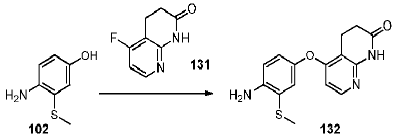

In a sealed tube, tBuOK (159mg, 1.41mmol) was added at r.t. to a solution of compound **102** (200mg, 1.28mmol) and compound **131** (214mg, 1.28mmol) in dry DMF (4mL). The reaction mixture was stirred at 120°C for 1h. After cooling to r.t., water (50mL), aq. sat. NH₄Cl (50mL) and AcOEt (100mL) were added. The aqueous layer was extracted with EtOAc (2x30 mL). The combined organic fractions were dried over MgSO₄, filtered and concentrated in vacuo. The crude obtained was purified by flash chromatography (40 g, SiO₂, cyclohexane/EtOAc 100:0→0:100, 10 CV and AcOEt/MeOH 100:0→95:5, 5 CV) leading to the expected product **132** (Yield: 39%, 150mg) as a brown powder.

### Synthesis of 136:

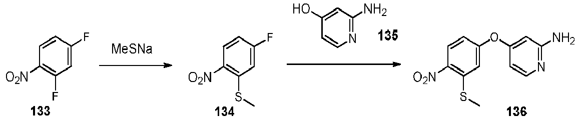

### Step 1. Synthesis of compound (134)

At 0°C, sodium thiomethoxyde (4.4g, 62.9mmol) was added to a solution of compound **133** (6.9ml, 62.9mmol) in dry pyridine (60mL). The reaction mixture was stirred at 0°C for 3h, quenched with cold water (250mL), and extracted with DCM (2x60mL). The combined organic fractions were dried over MgSO₄, filtered and concentrated in vacuo (azeotrope with toluene to remove residual pyridine). The crude obtained was purified by flash chromatography (3x340g, SiO₂, cyclohexane/EtOAc 100:0→90:10, 10 CV) to furnish the expected product **134** (Yield: 39%, 4.6g) as a yellow solid.

### Step 2. Synthesis of compound (136): 4-(3-(methylthio)-4-nitrophenoxy)pyridin-2-amine (General Procedure K)

In a sealed tube, DBU (2.9ml, 19.2mmol) was added at r.t. to a solution compound 135 (1.5g, 13.9mmol) in dry acetonitrile (0.1M). The mixture was stirred at r.t. for 30min before addition of compound **134** (2.0g, 10.7mmol). The reaction mixture was stirred at 80°C for 2h30. After cooling to r.t. the crude obtained was triturated in EtOAc, filtrated off, and dried in vacuo to furnish the expected product (660 mg, 22%) as a yellow powder. The filtrate was purified by flash chromatography (120g, SiO₂, cyclohexane/EtOAc 100:0→0:100, 10 CV and EtOAc/MeOH 100:0→90:10, 5 CV) leading to a new batch of expected product **136** (Yield: 51%, 1.5g) as a yellow solid.

### Synthesis of 140:

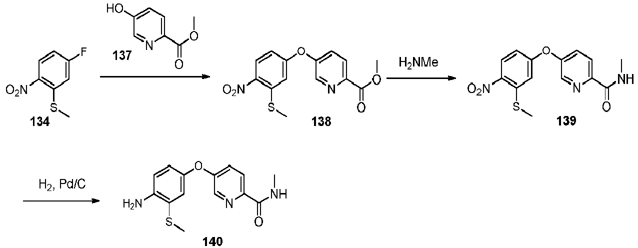

### Step 1. Synthesis of compound (138) according to General Procedure K

DBU (2.9 ml, 19.2 mmol), compound **137** (319mg, 2.08mmol), dry acetonitrile (0.1M). Compound **134** (300mg, 1.60mmol). Reaction mixture stirred at 80°C for 5h. Flash chromatography (40g, SiO₂, cyclohexane/DCM 100:0→0:100,10 CV and DCM/EtOAc 100:0→80:20, 5 CV) (Yield of **138:** 71%, 362mg).

### Step 2. Synthesis of compound (139)

In a sealed tube, a solution of compound **138** (362mg, 1.13mmol) and methylamine 2M in MeOH (11.0ml, 22.0mmol) was heated at 100°C for 4h. After cooling to r.t. and concentration under vacuum, the crude obtained was purified by flash chromatography (40g, SiO₂, DCM/EtOAc 100:0→80:20, 10 CV) leading to the expected product **139** (Yield: 97%, 352mg) as a yellow powder.

### Step 3. Synthesis of compound (140): 5-(4-amino-3-(methylthio)phenoxy)-N-methylpicolinamide

Compound **139** (352mg, 1.10mmol), Pd/C (20mg) in EtOH (20ml) was stirred under hydrogen (1 atm) at r.t. for 18h (Yield of **140:** 35%, 110mg).

### Synthesis of 142:

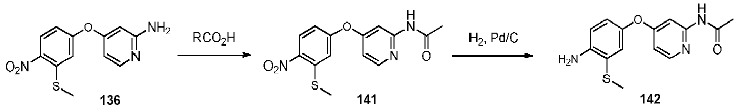

### Step 1. Synthesis of compound (141)

To a stirred solution of compound **136** (320mg, 1.15mmol) in anhydrous DCM (5mL) and DIEA (0.40ml, 2.30mmol), cooled at 0°C, was added acetyl chloride (0.13ml, 1.74mmol). The mixture was allowed to warm to r.t. and stirred at this temperature for 1 h. NH₄Cl sat (30mL) was added. The aqueous layer was extracted with DCM (2x30mL). The combined organic fractions were dried over MgSO₄, filtered and concentrated in vacuo. The crude obtained was purified by flash chromatography (40g, SiO₂, cyclohexane/EtOAc 100:0→20:80, 10 CV) leading to the expected product **141** (Yield: 81%, 300mg) as a yellow solid.

### Step 2. Synthesis of compound (142): N-(4-(4-amino-3-(methylthio)phenoxy)pyridin-2-yl)acetamide

A suspension of compound **141** (300mg, 0.94mmol) and Pd/C (50mg) in EtOH (20mL) was stirred under hydrogen (1 atm) at r.t. for 18h. After work-up, the expected product **142** (Yield: 50%, 136mg) was recovered as a yellow solid.

### Synthesis of 144:

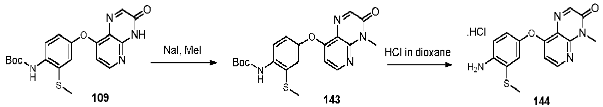

### Step 1. Synthesis of compound (143)

At 0°C, sodium hydride (60% wt., 44mg, 1.10mmol) was added to a suspension of compound **109** (400mg, 1.00mmol) in dry DMF (10mL). After 30min at 0°C, Mel (0.62mL, 10mmol) was added. The reaction mixture was stirred at r.t. for 3h, quenched with water (60mL), and extracted with EtOAc (3x30mL). The combined organic fractions were washed with brine (40mL), dried over MgSO₄, filtered and concentrated in vacuo. The crude obtained was purified by flash chromatography (40g, SiO₂, cyclohexane/EtOAc 100:0→50:50, 10 CV) leading to the expected product **143** (Yield: 64%, 265mg).

### Step 2. Synthesis of compound (144): 8-(4-amino-3-(methylthio)phenoxy)-4-methylpyrido[2,3-b]pyrazin-3(4H)-one hydrochloride

Compound **143** (265 mg, 0.63 mmol) was treated with 4N HCl in dioxane (5 mL) and the reaction mixture was stirred at r.t. for 18h. The suspension obtained was filtered off, washed with dioxane, and dried under vacuum. The pale pink solid recovered **144** (222mg, quantitative yield) was directly used for next step without any purification.

### Synthesis of 149: according to General Procedure J

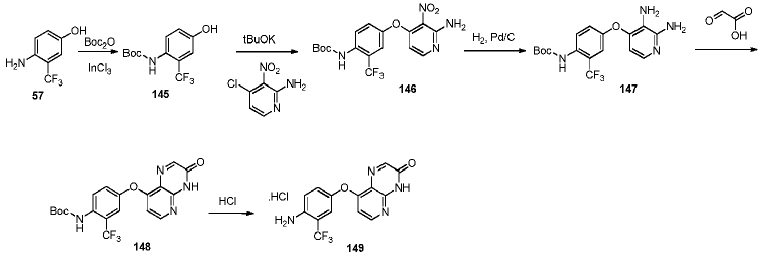

### Step 1. Synthesis of compound (145)

Compound **57** (14.5g, 81.8mmol, 1.0eq), Boc₂O(17.9g, 81.8mmol, 1.0eq) and InCl₃ (181mg, 0.82mmol, 0.01eq). Flash chromatography on silica gel using cyclohexane and EtOAc (Yield of **145:** 83%, 18.8g).

### Step 2. Synthesis of compound (146)

tBuOK (2.2g, 19.2mmol), compound **145** (4.1g, 14.8mmol), DMF (195mL). 4-chloro-3-nitro-pyridin-2-amine (2.5g, 14.6mmol) added and reaction mixture heated at 80°C for 3h. Flash chromatography (80g, SiO₂, DCM/EtOAc 100:0→85:15, 10 CV) (Yield of **146:** 9%, 534mg).

### Step 3. Synthesis of compound (147)

Pd/C(around 50mg), compound **146** (530mg, 1.29mmol), EtOH (60 mL) and DCM (10mL). Hydrogenation at r.t. over 1 atmosphere for 18h (Yield of **147:** 96%, 480mg).

### Step 4. Synthesis of compound (148)

Compound **147** (480mg, 1.24mmol) in MeOH, glyoxylic acid (3.9g, 42mmol) in MeOH (23mL). Mixture stirred at r.t. for 18h (Yield of **148:** 40%, 208mg).

### Step 5. Synthesis of compound (149): 8-(4-amino-3-(trifluoromethyl)phenoxy)pyrido[2,3-b]pyrazin-3(4H)-one hydrochloride

Compound **148** (208mg, 0.49mmol), HCl 4N in dioxane (4mL). Mixture stirred at r.t. for 3h. Concentration under vacuum, the gum recovered (180mg) was directly used for next step without any purification.

### Synthesis of 155: according to General Procedure B

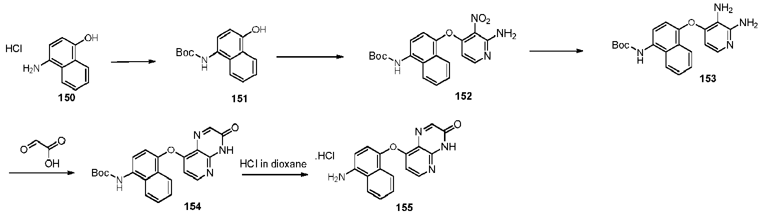

### Step 1. Synthesis of compound (151)

Compound **150** (10.0g, 51.1 mmol, 1.0eq), 75mL of THF, BoC₂O (12.3g, 56.2mmol, 1.1eq), LiOH.H₂O (2.1g, 51.1mmol, 1.0eq). Reaction mixture stirred at r.t. for 18h. THF concentrated and water added. Flash chromatography over silica gel using cyclohexane and EtOAc (Yield of **151:** 79%, 10.5g).

### Step 2. Synthesis of compound (152)

Compound **151** (6.0g, 23.13mmol, 1.0eq), 280mL of DMF, tBuOK (3.4g, 30.03mmol, 1.3eq). 4-chloro-2-amino-3-nitropyridine (0.99eq). Mixture heated at 80°C for 3h. Mixture was evaporated to dryness and EtOAc and water were added. The precipitate obtained filtered, washed with EtOAc and water and dried (Yield of **152:** 73%, 6.75g).

### Step 3. Synthesis of compound (153)

Pd/C (0,7g), compound **152** (6.6g, 16.6mmol, 1.0eq) in EtOH and DCM. Hydrogenation at r.t. over 1 bar of hydrogen for 20h (Yield of **153:** 99%, 6.1g).

### Step 4. Synthesis of compound (154)

Compound **153** (6.1g, 16.53mmol) in MeOH, glyoxylic acid (15.2g, 0.16mmol) in MeOH (100mL). Mixture stirred at r.t. for 20h (Yield of **154:** 60%, 4.0g).

### Step 5. Synthesis of compound (155): 8-((4-aminonaphthalen-1-yl)oxy)pyrido[2,3-b]pyrazin-3(4H)-one hydrochloride

Compound **154** (2.0g, 4.94mmol), HCl 4N in dioxane (12mL, 49mmol, 10.0eq) (1.6g, quantitative yield of **155**).

### Synthesis of 157: 2,2,2-trichloroethyl (5-(tert-butyi)thiazoi-2-yl)carbamate according to General Procedure I

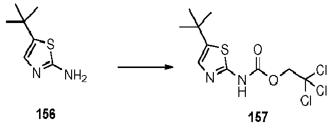

Compound **156** (500mg, 3.2mmol, 1.0eq), dry THF (0.2M), dry pyridine (1.1eq), 2,2,2-trichloroethylchloroformate (0.48mL, 3.52mmol, 1.1eq). Reaction mixture stirred at r.t. for 18h (Yield of **157:** 94%, 1.0g).

### Synthesis of 158: 2,2,2-trichloroethyl (3-(tert-butyl)-1-phenyl-1H-pyrazol-5-yl)carbamate according to General Procedure I

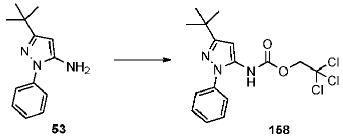

Compound **53** (690mg, 3.2mmol, 1.0eq), dry THF (0.2M), dry pyridine (1.1eq), 2,2,2-trichloroethylchloroformate (0.48mL, 3.52mmol, 1.1eq). Reaction mixture stirred at r.t. for 18h (Yield of **158:** 95%, 1.19g).

### Synthesis of 164: according to General Procedure B

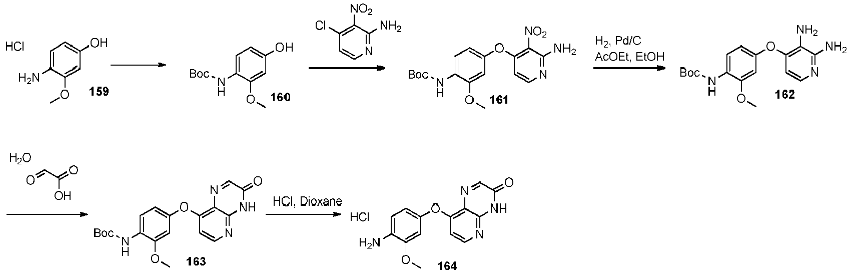

### Step 1. Synthesis of compound (160)

Compound **159** (1.0g, 5.69mmol, 1.0 q), THF (20mL). Triethylamine (1.6mL, 11.38mmol, 2.0eq) and BoC₂O (1.24g, 5.69mmol, 1.0eq) added and mixture stirred for 48h at reflux. After cooling down. Flash chromatography using cyclohexane and EtOAc (Yield of **160:** 90%, 1.23g).

### Step 2. Synthesis of compound (161)

Compound **160** (1.23g, 5.14mmol, 1.0eq), 70mL of DMF, tBuOK (750mg, 6.68mmol, 1.3eq). 4-chloro-2-amino-3-nitropyridine (892mg, 5.14mmol, 1.0eq) added and mixture heated at 80°C for 3h. Flash chromatography on silica gel using DCM and EtOAc (Yield of **160:** 38%, 730 mg).

### Step 3. Synthesis of compound (162)

Pd/C (around 100mg), compound **161** (730mg, 1.94mmol, 1.0eq) in a mixture of EtOH (40mL) and EtOAc (20mL). Hydrogenation at r.t. over 1 atmosphere of hydrogen for 48h (672mg, quantitative yield of **162**).

### Step 4. Synthesis of compound (163)

Compound **162** (672mg, 1.94mmol, 1.0eq) in MeOH, glyoxylic acid (1.8g, 19.4mmol, 10.0eq) in MeOH (15mL). Mixture stirred at r.t. for 18h (Yield of **163:** 54%, 400mg).

### Step 5. Synthesis of compound (164): 8-(4-amino-3-methoxyphenoxy)pyrido[2,3-b]pyrazin-3(4H)-one hydrochloride

Compound **163** (400mg, 1.04mmol, 1.0eq), HCl 4N in dioxane (8mL) and the reaction mixture was stirred at r.t. for 2h (300mg, quantitative yield of **164**).

### Synthesis of 166: 3-(tert-butyl)-1-(6-methylpyridin-3-yl)-1H-pyrazol-5-amine according to General Procedure D

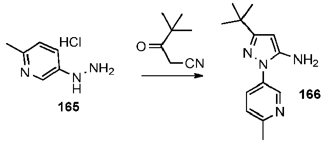

Compound **165** (500mg, 3.13mmol, 1.0eq), pivaloyl acetonitrile (437mg, 3.5mmol, 1.6eq) in EtOH, HCl 12 N (10.0eq). Mixture stirred at reflux for 20h. NaHCO₃ solid added until pH was around 7. Flash chromatography on silica gel using 100% of EtOAc (Yield of **166:** 62%, 447mg).

### Synthesis of 167: 4-((4-aminonaphthalen-1-yl)oxy)-N-methylpicolinamide (General Procedure L)

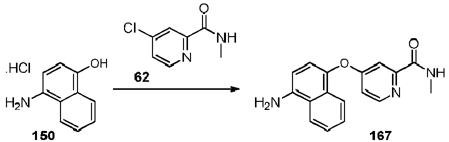

In a sealed tube, tBuOK (660mg, 5.87mmol) followed by compound **62** (436mg, 2.55mmol) were added at r.t. to a solution of compound **150** (500mg, 2.55mmol) in dry DMA (7mL). The reaction mixture was stirred at 100°C for 20h. After cooling to r.t., water (50mL), aq. sat. NH₄Cl (20mL) and EtOAc (50mL) were added. The aqueous layer was extracted with EtOAc (2x30mL). The combined organic fractions were dried over MgSO₄, filtered and concentrated in vacuo. The crude obtained was purified by flash chromatography (80g, SiO₂, cyclohexane/EtOAc 100:0→0:100, 10 CV) leading to the expected product **167** (Yield: 29%, 216mg) as a dark oil.

### Synthesis of 169: 3-(tert-butyl)-1-(2-(dimethylamino)ethyl)-1H-pyrazol-5-amine according to General Procedure D

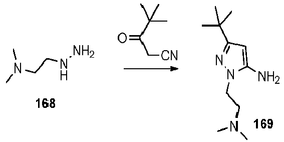

Compound **168** (500mg, 4.84mmol, 1.0eq), pivaloyl acetonitrile (970mg, 7.75mmol, 1.6eq) in EtOH, hydrochloric acid 12 N (10.0eq). NaHCO₃ solid added until pH was around 7. (Yield of **169:** 96%, 980mg).

### Synthesis of 171: 3-(tert-butyl)-1-(3-morpholinopropyl)-1H-pyrazol-5-amine according to General Procedure D

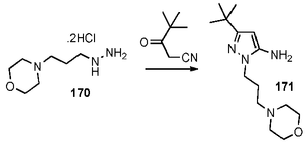

Compound **170** (500mg, 2.15mmol, 1.0eq), pivaloyl acetonitrile (431 mg, 3.44mmol, 1.6eq) in EtOH was added HCl 12 N (10.0eq). NaHCO₃ solid added until pH was around 7. (Yield of **171:** 66%, 381 mg, 66%).

### Synthesis of 173:

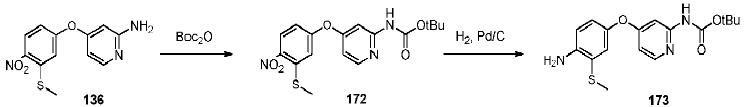

### Step 1. Synthesis of compound (172)

To a stirred suspension of compound **136** (1.10g, 3.97mmol) in tBuOH (9mL) was added at 0°C BoC₂O (1.26g, 5.78mmol). The mixture was heated at 50°C for 6h. After cooling at r.t., EtOH (9mL) was added. The suspension was filtered off and washed with MeOH (4mL). The solid obtained was dried under vacuum leading to the expected product **172** (Yield: 78%, 1.17g) as a yellow solid.

### Step 2. Synthesis of compound (173): tert-butyl (4-(4-amino-3-(methylthio)phenoxy)pyridin-2-yl)carbamate

A suspension of compound **172** (1.17g, 3.12 mmol) and Pd/C (150mg) in a mixture EtOH/DCM (3:1; 200mL) was stirred under hydrogen (4 bars). After 4h at r.t. and work-up, the expected product **173** (Yield: 95%, 1.03g) was recovered as a green powder.

### Synthesis of 178: according to General Procedure B

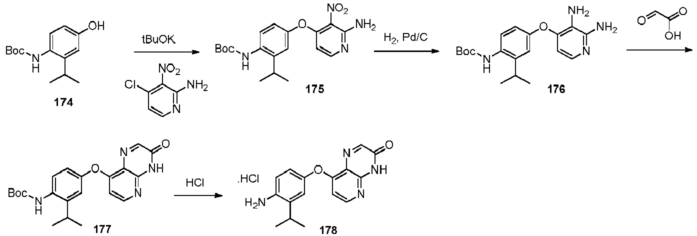

### Step 1. Synthesis of compound (175)

tBuOK (1.1g, 9.8mmol), compound **174** (1.9g, 7.6mmol), DMF (50mL) and 4-chloro-3-nitro-pyridin-2-amine (1.3g, 7.6mmol) was added. Reaction mixture heated at 80°C for 2h30. After cooling at r.t. and evaporation of the solvent under vacuum, the crude obtained was diluted in EtOAc and washed with water. The suspension was filtered off (washing with ether) (Yield of **175:** 74%, 2.1g).

### Step 2. Synthesis of compound (176)

Pd/C (around 200mg), compound **175** (2.1g, 5.56mmol), in EtOH (100 ml) and DCM (10 ml). Hydrogenation at r.t. over 1 atm of hydrogen (Yield of **176:** 79%, 1.58g).

### Step 3. Synthesis of compound (177)

Compound **176** (1.58g, 4.40mmol) in MeOH, glyoxylic acid (4.0g, 44mmol) in MeOH (30mL). Mixture stirred at r.t. for 18h (Yield of **177:** 50%, 870mg).

### Step 4. Synthesis of compound (178): 8-(4-amino-3-isopropylphenoxy)pyrido[2,3-b]pyrazin-3(4H)-one hydrochloride

Compound **177** (870mg, 2.2mmol), HCl 4N in dioxane (10mL). Beige powder **178** recovered (821mg).

### Synthesis of 180: 3-(tert-butyl)-1-eyclohexyl-1H-pyrazol-5-amine according to General Procedure D

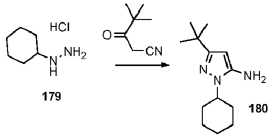

Compound **179** (1.0g, 6.64mmol, 1.0eq), pivaloyl acetonitrile (831mg, 6.64mmol, 1.0eq) in EtOH, HCl 12 N (10.0eq). NaHCO₃ solid added until pH was around 7. (Yield of **180: 95%,** 1.39g).

### Synthesis of 186: according to General Procedure J

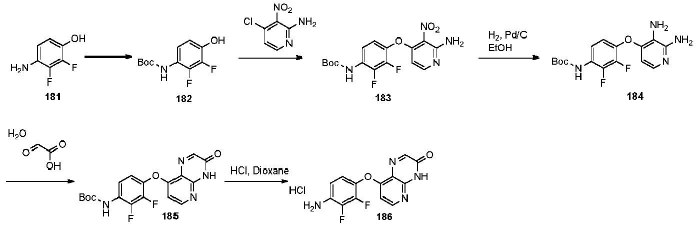

### Step 1. Synthesis of compound (182)

Compound **181** (4.9g, 33.9mmol, 1.0eq), Boc₂O (7.4g, 33.9mmol, 1.0eq), InCl₃ (75 mg, 0.34 mmol, 0.01 eq). Reaction mixture stirred at 35°C for 4h. Flash chromatography on silica gel was performed using cyclohexane and EtOAc to afford expected compound in mixture with 30% of O protected BOC compound (Yield: 14%, 1.68g).

### Step 2. Synthesis of compound (183)

Compound **182** in mixture with 30% of O protected BOC compound (1.68g, 6.85mmol, 1.0eq), 60mL of DMF, tBuOK (999 mg, 8.9 mmol, 1.3 eq). 4-chloro-2-amino-3-nitropyridine (1.19 g, 6.85 mmol, 1.0 eq) added and mixture heated at 80°C for 3h. Flash chromatography on silica gel using cyclohexane and EtOAc (Yield of **183:** 68%, 2.2g).

### Step 3. Synthesis of compound (184)

Pd/C(around 350mg), compound **183** (2.2g, 5.8mmol, 1.0eq) in EtOH (280mL). Hydrogenation at r.t. over 1 atmosphere of hydrogen for 20h (Yield of **184**: 98%, 2.0g).

### Step 4. Synthesis of compound (185)

Compound **184** (2.0g, 5.67mmol, 1.0eq) in MeOH, glyoxylic acid monohydrate (5.2g, 56.7mmol, 10.0eq) in MeOH (40mL). Mixture stirred at r.t. for 18h (Yield of **185:** 47%, 1.04g).

### Step 5. Synthesis of compound (186): 8-(4-amino-2,3-difluorophenoxy)pyrido[2,3-b]pyrazin-3(4H)-one hydrochloride

Compound **185** (1.04g, 2.6mmol, 1.0eq), HCl 4N in dioxane (21mL), reaction mixture was stirred at r.t. for 4h (1.0g of **186**).

### Synthesis of 188:

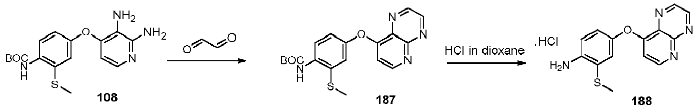

### Step 1. Synthesis of compound (187)

A solution of compound **108** (500mg, 1.38mmol) and glyoxal (40% in water, 0.26mL, 1.79mmol) in THF (10mL) was stirred at r.t. for 18h. After evaporation of the solvent, the crude obtained was purified by flash chromatography (40g, SiO₂, cyclohexane/EtOAc 100:0→0:100, 10 CV and EtOAc/CH₃OH 100:0→90:10, 5 CV) leading to the expected product **187** (Yield: 87%, 530mg) as a beige solid.

### Step 2. Synthesis of compound (188): 2-(methylthio)-4-(pyrido[2,3-b]pyrazin-8-yloxy)aniline hydrochloride

Compound **187** (530mg, 1.38mmol) was treated with 4N HCl in dioxane (15mL) and the reaction mixture was stirred at r.t. for 3h. The suspension obtained was filtered off, washed with ether, and dried under vacuum. The dark blue solid **188** recovered (529mg, quantitative yield) was directly used for next step without any purification.

### Synthesis of 195: according to General Procedure J

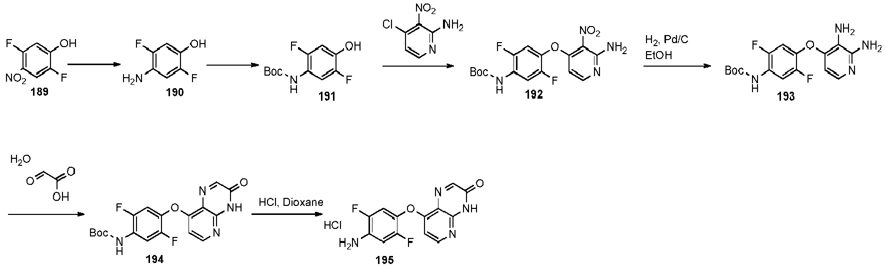

### Step 1. Synthesis of compound (190)

Platinium oxide (around 30mg) was added to a solution of compound **189** (3.73g, 21.30mmol, 1.0eq) in MeOH (150mL). The reaction mixture was hydrogenated at r.t. over 4 atmospheres of hydrogen for 4h. The reaction mixture was filtrated through a short pad of celite and rinsed with MeOH. The filtrate was evaporated to dryness to afford the expected compound **190** as black solid (Yield: 96%, 2.96g). The compound, light and air sensitive, was used directly in the next step without further purification.

### Step 2. Synthesis of compound (191)

Compound **190** (1.7 g, 11.58 mmol, 1.0 eq), Boc₂O (2.5 g, 11.58 mmol, 1.0 eq), InCl₃ (26mg, 0.11 mmol, 0.01eq). Reaction mixture stirred at 35°C for 4h. Flash chromatography on silica gel was performed using cyclohexane and EtOAc (Yield of **191**: 53%, 1.5g, 53%).

### Step 3. Synthesis of compound (192)

Compound **191** (1.5g, 6.1mmol, 1.0eq), 60mL of DMF, tBuOK (892mg, 7.9mmol, 1.3eq). 4-chloro-2-amino-3-nitropyridine (1.06g, 6.1mmol, 1.0eq) added and mixture was heated at 80°C for 3h. Flash chromatography on silica gel using cyclohexane and EtOAc (Yield of **192**: 78%, 2.3g).

### Step 4. Synthesis of compound (193)

Pd/C (around 350mg), compound **192** (2.3g, 6.01 mmol, 1.0eq) in EtOH (150 ml). Hydrogenation at r.t. over 1 atmosphere of hydrogen for 20h (Yield of **193**: 99%, 2.1g).

### Step 5. Synthesis of compound (194)

Compound **193** (2.1g, 5.96mmol, 1.0eq) in MeOH, glyoxylic acid monohydrate (5.5g, 59.6mmol, 10.0eq) in MeOH (42mL). Mixture stirred at r.t. for 18h (Yield of **194**: 56%, 1.3 g).

### Step 6. Synthesis of compound (195): 8-(4-amino-2,5-difluorophenoxy)pyrido[2,3-b]pyrazin-3(4H)-one hydrochloride

Compound **194** (1.3g, 3.33mmol, 1.0eq), 4N HCl in dioxane (27mL) and reaction mixture stirred at r.t. for 3h (1.2g, quantitative yield of **195**).

### Synthesis of 199: according to General Procedure K

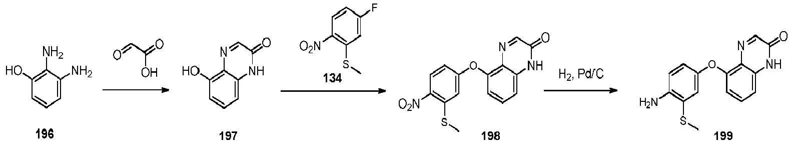

### Step 1. Synthesis of compound (197)

Compound **196** (3.0g, 24.2mmol) in MeOH, glyoxylic acid (22g, 242mmol) in MeOH (20mL). Mixture stirred at r.t. for 18h (Yield of **197:** 61%, 2.4g).

### Step 2. Synthesis of compound (198)

In a sealed tube, DBU (3.7mL, 24.8mmol), compound **197** (2.9g, 17.9mmol) in dry ACN (0.1M). Compound **134** (2.6g, 13.8mmol). Reaction mixture stirred at 80°C for 5h. Flash chromatography (120g, SiO₂, cyclohexane/EtOAc 100:0→0:100, 10 CV and EtOAc/CH₃OH 100:0→90:10, 5 CV) (Yield of **198**: 7%, 322mg).

### Step 3. Synthesis of compound (199): 5-(4-amino-3-(methylthio)phenoxy)quinoxalin-2(1H)-one

Compound **198** (320mg, 0.99mmol), Pd/C (50mg) in a mixture EtOH/DCM (5:2, 70mL) stirred under hydrogen (4 bars) for 5h at r.t. (308mg, quantitative yield of **199**).

### Synthesis of 203:

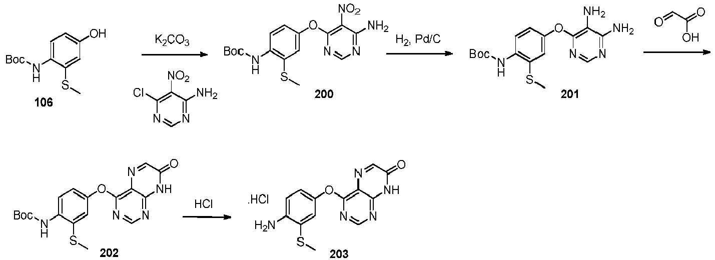

### Step 1. Synthesis of compound (200)

K₂CO₃ (890mg, 6.5mmol) was added at room temperature to a solution of compound **106** (1.1g, 4.3mmol) in DMF (25mL). After 15min, 6-chloro-5-nitro-pyrimidin-4-amine (1.9g, 10.8mmol) was added. The reaction mixture was heated at 90°C for 18h. After cooling at room temperature and evaporation of the solvent under vacuum, the crude obtained was diluted in EtOAc and washed with water. The organic layer was dried over MgSO₄, filtered, and concentrated in vacuo. The crude obtained was purified by flash chromatography (40g, SiO₂, cyclohexane/EtOAc 100:0→60:40, 10 CV), leading to the expected product **200** (Yield: 30%, 498mg) as a yellow powder.

### Step 2. Synthesis of compound (201)

Pd/C (around 100mg) was added to a solution of compound **200** (770mg, 1.97mmol) in EtOH (20mL). The reaction mixture was hydrogenated at room temperature with 5 bars of hydrogen for 20h. After work-up, the expected product **201** (Yield: 89%, 638mg) was recovered.

### Step 3. Synthesis of compound (202)

Compound **201** (638mg, 1.75mmol) in MeOH was added dropwise to a solution of glyoxylic acid (1.6g, 17.5mmol) in MeOH (23mL). The mixture was stirred at room temperature for 18h. After work-up, the crude was purified by flash chromatography (40g, SiO₂, DCM/EtOAc 100:0→80:20, 10 CV) leading to the expected product **202** (Yield: 16%, 112mg).

### Step 4. Synthesis of compound (203): 4-(4-amino-3-(methylthio)phenoxy)pteridin-7(8H)-one hydrochloride

Compound **202** (112mg, 0.28mmol) was treated with HCl 4N in dioxane (10mL) and the reaction mixture was stirred at room temperature for 4h. The suspension was filtered off, washed with ether, and dried under vacuum leading to a beige powder **203** recovered (Yield: 86%, 81mg) which was directly used for next step without any purification.

### Synthesis of 206: according to General Procedure D & I

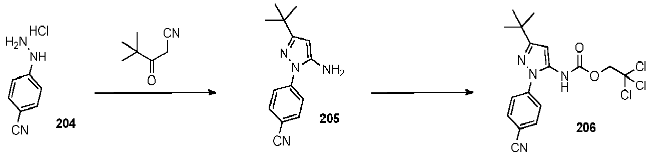

### Step 1. Synthesis of compound (205)

Compound **204** (1.0g, 5.9mmol, 1.0eq), pivaloyl acetonitrile (738mg, 5.9mmol, 1.0eq) in EtOH, HCl 12 N (10.0eq). NaHCO₃ solid added until pH was around 7 (Yield of **205:** 99%, 1.4g).

### Step 2. Synthesis of compound (206): 2,2,2-trichloroethyl (3-(tert-butyl)-1-(4-cyanophenyl)-1H-pyrazol-5-yl)carbamate

Compound **205** (400mg, 1.66mmol, 1.0eq), dry pyridine (1.1eq), 2,2,2-trichloroethylchloroformate (0.25mL, 1.83mmol, 1.1eq). Reaction mixture stirred at r.t. for 18h. Compound **206** was obtained as yellow gum (500mg).

### Synthesis of 209: according to General Procedure D & I

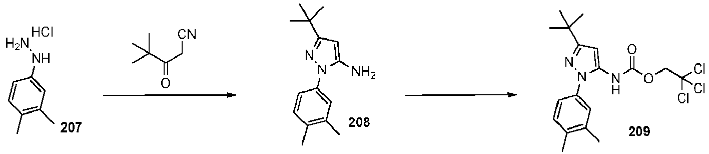

### Step 1. Synthesis of compound (208)

Compound **207** (2.0g, 11.6mmol, 1.0eq), pivaloyl acetonitrile (2.3g, 18.5mmol, 1.0eq) in EtOH, HCl 12 N (10.0eq). NaHCO₃ solid added until pH was around 7 (2.9g, quantitative yield of **208**).

### Step 2. Synthesis of compound (209): 2,2,2-trichloroethyl (3-(tert-butyl)-1-(3,4-dimethylphenyl)-1H-pyrazol-5-yl)carbamate

Compound **208** (1.0g, 4.11 mmol, 1.0eq), dry pyridine (1.1eq), 2,2,2-trichloroethylchloroformate (0.62mL, 4.52mmol, 1.1 eq). Reaction mixture stirred at r.t. for 18h. Compound **209** was obtained as beige solid (1.7g, quantitative yield).

### Synthesis of 211:

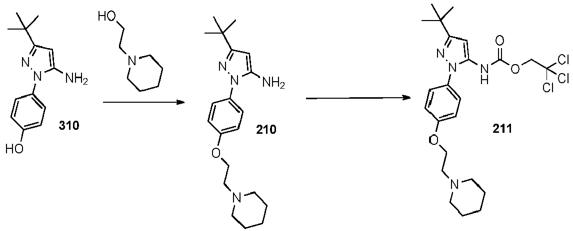

### Step 1. Synthesis of compound (210) (General procedure M)

DIAD (0.53mL, 2.7mmol, 1.9eq) was added at r.t. to a solution of compound **310** (330mg, 1.42mmol, 1.0eq), 2-(1-piperidyl)ethyl alcohol (0.22mL, 1.7mmol, 1.2eq) and PPh₃ (708mg, 2.7mmol, 1.9eq) in 10mL of THF. The reaction mixture was stirred at r.t. for 20h. A 10% solution of citric acid was added, and the mixture was extracted with Et₂O. The aqueous phase was carefully basified with potassium carbonate and extracted with EtOAc. The organic phases were dried over MgSO₄, filtered and evaporated. The residue was purified by flash chromatography over silica gel using EtOAc to afford the expected compound **210** as yellow oil (Yield: 53%, 260mg).

### Step 2. Synthesis of compound (211): 2,2,2-trichloroethyl (3-(tert-butyl)-1-(4-(2-(piperidin-1-yl)ethoxy)phenyl)-1H-pyrazol-5-yl)carbamate according to General Procedure I

Compound **210** (260mg, 0.76mmol, 1.0eq), dry pyridine (1.1eq), 2,2,2-trichloroethylchloroformate (0.11mL, 0.83mmol, 1.1 eq). Reaction mixture stirred at r.t. for 18h (614mg, quantitative yield of **211**).

### Synthesis of 213: according to General Procedure M & J

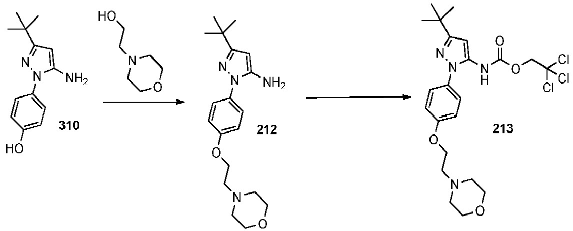

### Step 1. Synthesis of compound (212)

DIAD (0.56mL, 2.8mmol, 2.0eq), compound **310** (330mg, 1.42mmol, 1.0eq), 2-morpholino-ethyl alcohol (0.21mL, 1.7mmol, 1.2eq) and PPh₃ (745mg, 2.8mmol, 2.0eq) in 10mL of THF. Reaction mixture stirred at r.t. for 20h. Flash chromatography over silica gel using EtOAc first and then DCM and MeOH (Yield of **212:** 61%, 300mg).

### Step 2. Synthesis of compound (213): 2,2,2-trichloroethyl (3-(tert-butyl)-1-(4-(2-morpholinoethoxy)phenyl)-1H-pyrazol-5-yl)carbamate

Compound **212** (300mg, 0.87mmol, 1.0eq), dry pyridine (1.1eq), 2,2,2-trichloroethylchloroformate (0.13mL, 0.96mmol, 1.1 eq). Reaction mixture stirred at r.t. for 18h. Compound **213** was obtained as orange oil (606mg, quantitative yield).

### Synthesis of 216: according to General Procedure D & I

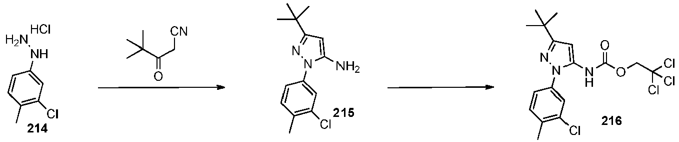

### Step 1. Synthesis of compound (215)

Compound **214** (500mg, 2.6mmol, 1.0eq), pivaloyl acetonitrile (519mg, 4.1mmol, 1.6eq) in EtOH, HCl 12 N (10.0eq). Mixture stirred at reflux for 18h. NaHCO₃ solid added until pH was around 7 (740mg, quantitative yield of **215**).

### Step 2. Synthesis of compound (216): 2,2,2-trichloroethyl (3-(tert-butyl)-1-(3-chloro-4-methylphenyl)-1H-pyrazol-5-yl)carbamate

Compound **215** (740mg, 2.81mmol, 1.0eq), dry pyridine (1.1eq), 2,2,2-trichloroethylchloroformate (0.43mL, 3.09mmol, 1.1 eq). Reaction mixture stirred at r.t. for 18h (1.73 g, quant. Yield of **216**).

### Synthesis of 219: according to General Procedure D & I

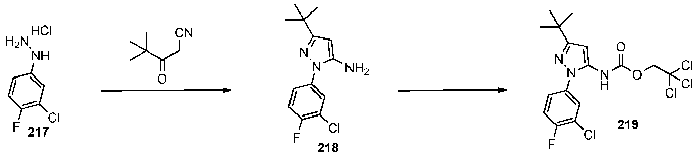

### Step 1. Synthesis of compound (218)

Compound **217** (2.0g, 10.15mmol, 1.0eq), pivaloyl acetonitrile (2.0g, 16.29mmol, 1.6eq) in EtOH, HCl 12N (10.0eq). Mixture stirred at reflux for 18h. NaHCO₃ solid added until pH was around 7. Flash chromatography over silica gel using cyclohexane and EtOAc (2.7g, quantitative yield of **218**).

### Step 2. Synthesis of compound (219): 2,2,2-trichloroethyl (3-(tert-butyl)-1-(3-chloro-4-fluorophenyl)-1H-pyrazol-5-yl)carbamate

Compound **218** (1.0g, 3.74mmol, 1.0eq), dry pyridine (1.1eq), 2,2,2-trichloroethylchloroformate (0.57mL, 4.11mmol, 1.1 eq). Reaction mixture was stirred at r.t. for 18h (1.9g, quantitative yield of **219**).

### Synthesis of 222: according to General Procedure K

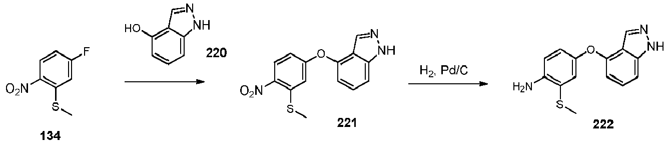

### Step 1. Synthesis of compound (221)

In a sealed tube, DBU (0.28mL, 1.90mmol), 1H-indazol-4-ol **220** (186mg, 1.40mmol) in dry acetonitrile (0.1M). Compound **134** (200mg, 1.06mmol). Reaction mixture stirred at 80°C for 5h (Yield of **221:** 28%, 90mg).

### Step 2. Synthesis of compound (222): 4-((1H-indazol-4-yl)oxy)-2-(methylthio)aniline

Compound **221** (90mg, 0.30mmol), Pd/C (10mg) in EtOH (15mL) and DCM (3mL) was stirred under hydrogen (1 atm) (80mg, quantitative yield of **222**).

### Synthesis of 224: 4-(4-amino-3-(methylthio)phenoxy)-N-phenylpicolinamide according to General Procedure L

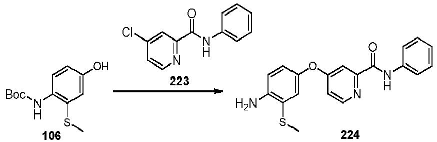

In a sealed tube, tBuOK (262mg, 2.34mmol), compound **223** (363mg, 1.56mmol), compound **106** (242mg, 1.56mmol) in dry DMA (3mL). Reaction mixture stirred at 100°C for 18h. Flash chromatography (40g, SiO₂, cyclohexane/EtOAc 100:0→50:50, 10 CV) (Yield of **224:** 69%, 380mg).

### Synthesis of 231: according to General Procedure J

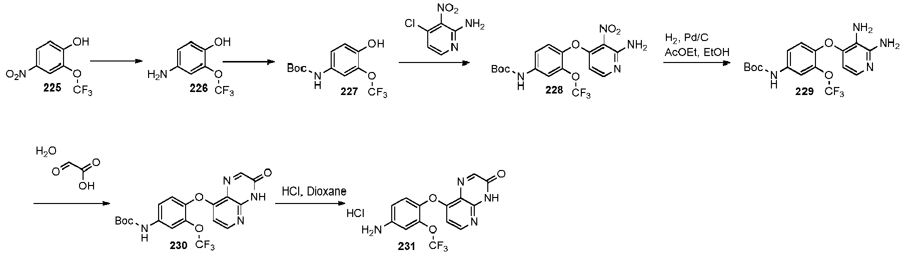

### Step 1. Synthesis of compound (226)

Pd/C (around 100mg) was added to a solution of 4-nitro-2-(trifluoromethoxy)phenol **225** (1.0g, 4.48mmol, 1.0eq) in EtOH (50mL). The reaction mixture was hydrogenated at r.t. over 1 atmosphere of hydrogen for 4h30. The reaction mixture was filtrated through a short pad of celite and rinsed with MeOH. The filtrate was evaporated to dryness to afford the expected compound **226** (970mg, quantitative yield).

### Step 2. Synthesis of compound (227)

Compound **226** (960mg, 4.97mmol, 1.0eq), Boc₂O (1.1g, 4.97mmol, 1.0eq), InCl₃ (11mg, 0.05mmol, 0.01eq). Reaction mixture stirred at 35°C for 4h. Flash chromatography on silica gel using cyclohexane and EtOAc (Yield of **227**: 94%, 1.37g).

### Step 3. Synthesis of compound (228)

Compound **227** (1.4g, 4.8mmol, 1.0eq), 50mL of DMF, tBuOK (695mg, 6.2mmol, 1.3eq). 4-chloro-2-amino-3-nitropyridine (833mg, 4.8mmol, 1.0eq) added and mixture heated at 80°C for 3h. Flash chromatography on silica gel was performed using cyclohexane and EtOAc (Yield to **228**: 77%, 1.6g).

### Step 4. Synthesis of compound (229)

Pd/C (around 300mg), compound **228** (1.6g, 3.8mmol, 1.0eq) in EtOH (150mL). Hydrogenation at r.t. over 1 atmosphere of hydrogen for 20h (1.5g, quantitative yield of **229**).

### Step 5. Synthesis of compound (230)

Compound **229** (1.5g, 3.76mmol, 1.0eq) in MeOH, glyoxylic acid monohydrate (3.5g, 37.6mmol, 10.0eq) in MeOH (54mL). Mixture was stirred at r.t. for 18h (Yield of **230**: 53%, 874mg).

### Step 6. Synthesis of compound (231): 8-(4-amino-2-(trifluoromethoxy)phenoxy)pyrido[2,3-b]pyrazin-3(4H)-one hydrochloride

Compound **230** (874 mg, 1.99 mmol, 1.0 eq), 4N HCl in dioxane (16mL) and reaction mixture stirred at r.t. for 4h (796mg, quantitative yield of **231**).

### Synthesis of 238: according to General Procedure J

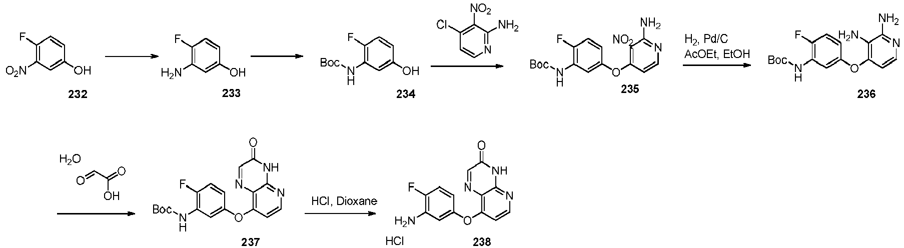

### Step 1. Synthesis of compound (233)

Pd/C (around 300mg) was added to a solution of compound **232** (3.0g, 19.0mmol, 1.0eq) in EtOH (100mL). The reaction mixture was hydrogenated at r.t. over 1 atmosphere of hydrogen for 18h. The reaction mixture was filtrated through a short pad of celite and rinsed with EtOH. The filtrate was evaporated to dryness to afford the expected compound **233** as brown sticky solid (Yield: 90%, 2.18g). The compound was used directly in the next step without further purification.

### Step 2. Synthesis of compound (234)

Compound **233** (2.18g, 17.1mmol, 1.0eq), Boc₂O (3.74g, 17.1mmol, 1.0eq), InCl₃ (38mg, 0.17mmol, 0.01eq). Reaction mixture stirred at 40°C for 4h. Flash chromatography on silica gel using cyclohexane and EtOAc (Yield of **234:** 61%, 2.38g).

### Step 3. Synthesis of compound (235)

Compound **234** (1.96g, 8.6mmol, 1.0eq), 85mL of DMF, tBuOK (1.26g, 11.22mmol, 1.3eq). 4-chloro-2-amino-3-nitropyridine (1.5g, 8.6mmol, 1.0eq) added and mixture heated at 70°C for 4h. Flash chromatography on silica gel was performed using cyclohexane and EtOAc (Yield of **235**: 71%, 2.24g).

### Step 4. Synthesis of compound (236)

Pd/C (around 300mg), compound **235** (2.24g, 6.15 mmol, 1.0 eq) in EtOH (50mL). Hydrogenation at r.t. over 1 atmosphere of hydrogen for 20h (Yield of **236**: 90%, 1.86g).

### Step 5. Synthesis of compound (237)

Compound **236** (2.02g, 6.04mmol, 1.0eq) in MeOH, glyoxylic acid monohydrate (5.56g, 60.4mmol, 10.0eq) in MeOH (100mL). Mixture was stirred at r.t. for 18h (Yield of **237**: 60%, 1.34g).

### Step 6. Synthesis of compound (238): 8-(3-amino-4-fluorophenoxy)pyrido[2,3-b]pyrazin-3(4H)-one hydrochloride

Compound **237** (1.34g, 3.6mmol, 1.0eq), 4N HCl in dioxane (29mL) and reaction mixture stirred at r.t. for 4h (Yield of **238:** 99%, 1.11g).

### Synthesis of 240: according to General Procedure M & I

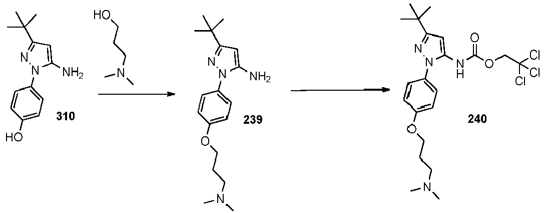

### Step 1. Synthesis of compound (239)

DIAD (0.85mL, 4.3mmol, 2.0eq), compound **310** (500mg, 2.16mmol, 1.0eq), 3-(dimethylamino)propan-1-ol (0.30mL, 2.6mmol, 1.2eq) and PPh₃ (1.13g, 4.3mmol, 2.0eq) in 12mL of THF. Flash chromatography over silica gel using DCM and ammoniac 7N in MeOH (Yield of **239:** 91%, 0.62g).

### Step 2. Synthesis of compound (240): 2,2,2-trichloroethyl (3-(tert-butyl)-1-(4-(3-(dimethylamino)propoxy)phenyl)-1H-pyrazol-5-yl)carbamate

Compound **239** (0.62g, 1.96mmol, 1.0eq), dry pyridine (1.1eq), 2,2,2-trichloroethylchloroformate (0.30mL, 2.15mmol, 1.1eq). Reaction mixture stirred at r.t. for 18h (1.07g, quantitative yield of **240**).

### Synthesis of 246:

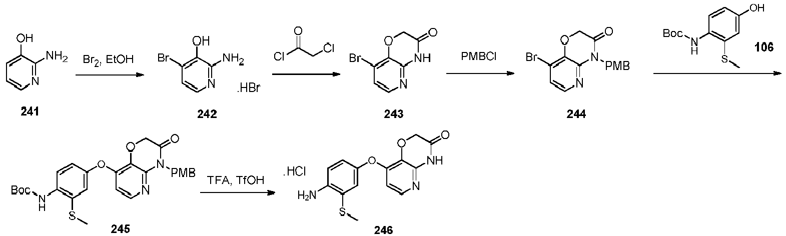

### Step 1. Synthesis of compound (242)

To a solution of compound **241** (10.0g, 91 mmol) in absolute EtOH (50mL) at 0°C was dropwise added bromine (5.2ml, 102mmol) and the mixture was stirred at r.t. for 72h. The solvent was removed under reduced pressure and low temperature. The residue was cooled to 0°C. EtOAc (100mL) was added and the mixture was stirred for 1h. The suspension was filtered off (washing with EtOAc). The red-brown solid recovered was dried under reduced pressure leading to the expected product **242** (Yield: 74%, 18.2g).

### Step 2. Synthesis of compound (243)

In a sealed tube at 0°C, NaHCO₃ (2.34g, 27.9mmol) was added to a solution of compound **242** (2.50g, 9.3mmol) in a mixture of 2-butanone/water (1:1, 18.6mL). After 10min, chloroacetyl chloride (0.74mL, 9.3mmol) was added. The mixture was stirred for 3h at 0°C. Then, the mixture was heated at 80°C for 18h. After cooling and filtration of the suspension, the dark-brown solid recovered was dried under vacuum leading to the expected compound **243** (Yield: 62%, 1.34g).

### Step 3. Synthesis of compound (244)

To a solution of compound **243** (1.13g, 4.9mmol) in DMF (25mL) were added Cs₂CO₃ (3.22g, 9.9mmol) followed by PMBCI (1.16g, 7.4mmol) and the mixture was stirred for 18h at r.t.. After filtration, the solvent was removed in vacuo. The residue was solubilized in EtOAc (100mL) and washed with water (50mL) and NH₄Cl sat. (50mL). The organic fraction was dried over MgSO₄, filtered and concentrated in vacuo. The crude obtained was purified by flash chromatography (40g, SiO₂, cyclohexane/EtOAc 100:0→40:60, 10 CV) leading to the expected product **244** (Yield: 78%, 1.35g) as a white solid.

### Step 4. Synthesis of compound (245) (General Procedure N)

Compound **244** (350mg, 1.0mmol), compound **106** (306 mg, 1.2mmol), K₃PO₄ (764mg, 3.6mmol) in anhydrous toluene (10mL) was degassed under Ar at r.t. for 15min. Then tBuXPhos Pd G3 (79mg, 0.1mmol) was added and the mixture was heated to 120°C for 2h. After cooling and concentration under vacuum, the crude obtained was diluted in AcOEt and washed with water. The organic layer was dried over MgSO₄, filtered and concentrated in vacuo. The residue obtained was purified flash chromatography (40g, SiO₂, cyclohexane/EtOAc 100:0→50:50, 10 CV) to afford the expected product **245** (Yield: 77%, 403mg) as a beige foam.

### Step 5. Synthesis of compound (246): 8-(4-amino-3-(methylthio)phenoxy)-2H-pyrido[3,2-b][1,4]oxazin-3(4H)-one hydrochloride

To a solution of compound **245** (200mg, 0.38mmol) in DCM (38mL) was added TFA (2.90mL, 38mmol) and trifluoromethanesulfonic acid (1.35mL, 15mmol). The reaction mixture was stirred at r.t. for 90min, diluted with MeOH (3mL) and the pH was adjusted to 8-9 by addition of sat. aq. NaHCO₃. The aqueous layer was extracted with DCM (2x20mL). The combined organic fractions were dried over MgSO₄, filtered and concentrated under reduced pressure to furnish the expected product **246** (170mg, quantitative yield) as a beige powder.

### Synthesis of 247: 5-((4-aminonaphthalen-1 -yl)oxy)-3,4-dihydro-1 ,8-naphthyridin-2(1H)-one according to General Procedure L

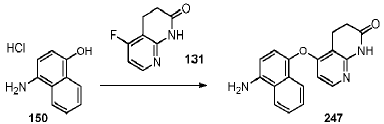

tBuOK (445mg, 3.97mmol), compound **131** (300mg, 1.80 mmol), compound **150** (530mg, 2.70mmol) in dry DMA (15mL). Reaction mixture stirred at 120°C for 2h. Flash chromatography (40g, SiO₂, cyclohexane/EtOAc 100:0→0:100,10 CV and EtOAc/MeOH 100:0→95:5, 5 CV) (80mg of **247,** 68% LC/MS purity).

### Synthesis of 252: according to General Procedure K

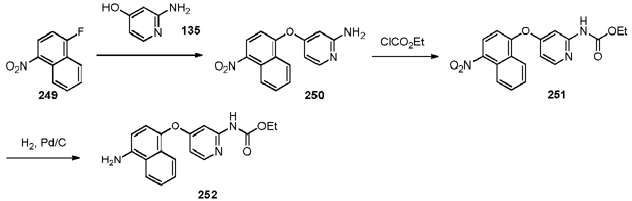

### Step 1. Synthesis of compound (250)

DBU (1.63mL, 10.9mmol), compound **135** (0.87g, 7.9mmol) in dry ACN (0.1M). Compound **249** (1.16g, 6.1 mmol) and reaction mixture stirred at 80°C for 2h. Flash chromatography (80g, SiO₂, cyclohexane/EtOAc 100:0→0:100, 10 CV) (Yield of **250:** 65%, 1.11g).

### Step 2. Synthesis of compound (251)

A stirred solution of compound **250** (562mg, 2.0mmol) in anhydrous THF (10mL) was treated at 0°C with LiHMDS 1.0M solution in THF (3.2mL, 3.2mmol). After 15min at 0°C, a solution of ethyl chloroformate (0.23mL, 2.4mmol) in THF (10mL) was added. The mixture was allowed to warm to r.t. and stirred for 4h. The reaction was quenched with sat. aq. NH₄Cl and extracted with EtOAc. The organic fractions were dried over MgSO₄, filtered and concentrated in vacuo. The solid obtained was purified by flash chromatography (40g, SiO₂, cyclohexane/EtOAc 100:0→40:60, 10 CV) leading to the expected product **251** (Yield: 14%, 100mg) as a yellow solid.

### Step 3. Synthesis of compound (252): ethyl (4-((4-aminonaphthalen-1-yl)oxy)pyridin-2-yl)carbamate

Compound **251** (100mg, 0.28mmol), Pd/C (20mg) in a mixture EtOH/DCM (1:1; 20mL) was stirred under hydrogen (1 atm) (Yield of **252:** 85%, 78mg).

### Synthesis of 256:

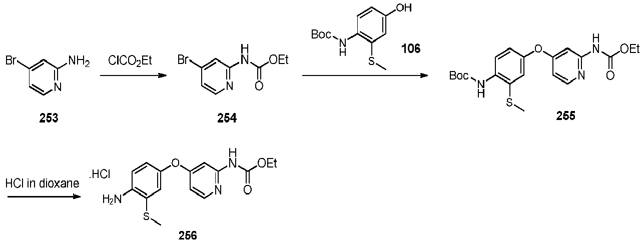

### Step 1. Synthesis of compound (254)

A stirred solution of compound **253** (1.73g, 10mmol) in anhydrous THF (20mL) was treated at 0°C with LiHMDS 1.0M solution in THF (21.0mL, 21.0mmol). After 15min at 0°C, a solution of ethyl chloroformate (1.30g, 12.0mmol) in THF (20mL) was added. The mixture was allowed to warm to r.t. and stirred overnight. The reaction was quenched with sat. aq. NH₄Cl and extracted with EtOAc. The organic fractions were dried over MgSO₄, filtered and concentrated in vacuo. The solid obtained was triturated in Et₂O and filtered off leading to the expected product **254** (Yield: 33%, 819mg) as a brown solid.

### Step 2. Synthesis of compound (255) according to General Procedure O

Compound **254** (245mg, 1.0mmol), compound **106** (306mg, 1.2mmol) in anhydrous toluene (10mL). Then K₃PO₄ (764mg, 3.6mmol), and tBuXPhos Pd G3 (79mg, 0.1mmol) was added. Mixture was stirred and heated (100°C, 18h). Flash chromatography (40g, SiO₂, cyclohexane/EtOAc 100:0→70:30, 10 CV) to obtain the expected product **255** in mixture with unreacted phenol.

### Step 3. Synthesis of compound (256): ethyl (4-(4-amino-3-(methylthio)phenoxy)pyridin-2-yl)carbamate hydrochloride

Compound **255** (100mg, 0.24mmol) was treated with 4N HCl in dioxane (10mL) and stirred at r.t. for 2h. The solvent was removed under vacuum. The beige powder recovered was directly used for next step without any purification.

### Synthesis of 263: according to General Procedure J

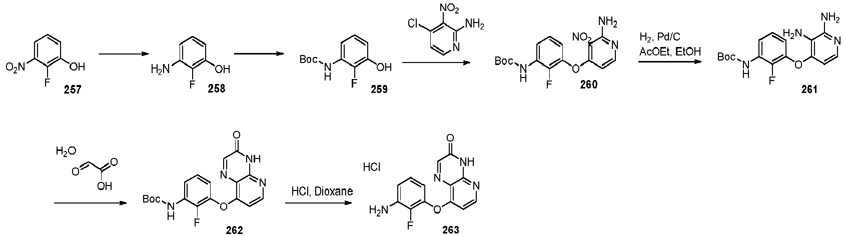

### Step 1. Synthesis of compound (258)

Pd/C (around 300mg) was added to a solution of compound **257** (3.0g, 19.0mmol, 1.0eq) in MeOH (100mL). The reaction mixture was hydrogenated at r.t. over 1 atmosphere of hydrogen for 18h. The reaction mixture was filtrated through a short pad of celite and rinsed with MeOH. The filtrate was evaporated to dryness to afford the expected compound **258** as brown solid (Yield: 95%, 1.89g).

### Step 2. Synthesis of compound (259)

Compound **258** (300mg, 2.36mmol, 1.0eq), Boc₂O (515mg, 2.36mmol, 1.0eq), InCl₃ (6mg, 0.02mmol, 0.01eq). Reaction mixture stirred at 40°C for 4h. Flash chromatography on silica gel using cyclohexane and EtOAc (600mg of **259** with 80% of LC/MS purity).

### Step 3. Synthesis of compound (260)

Compound **259** (800mg, 3.52mmol, 1.0eq), 40mL of DMF, tBuOK (514mg, 4.57mmol, 1.3eq). 4-chloro-2-amino-3-nitropyridine (611mg, 3.52mmol, 1.0eq) added and the mixture heated (60°C, 4h). Flash chromatography on silica gel using cyclohexane and EtOAc (Yield of **260:** 41%, 530mg).

### Step 4. Synthesis of compound (261)

Pd/C (around 100mg), compound **260** (950mg, 2.6mmol, 1.0eq) in EtOH (150mL). Hydrogenation at r.t. over 1 atmosphere of hydrogen for 20h (900mg, quantitative yield of **261**).

### Step 5. Synthesis of compound (262)

Compound **261** (900mg, 2.69mmol, 1.0eq) in MeOH, glyoxylic acid monohydrate (2.5g, 26.9mmol, 10.0eq) in MeOH (50mL). Mixture was stirred at r.t. for 18h (Yield of **262**: 19%, 190mg).

### Step 6. Synthesis of compound (263): 8-(3-amino-2-fluorophenoxy)pyrido[2,3-b]pyrazin-3(4H)-one hydrochloride

Compound **262** (190mg, 0.51mmol, 1.0eq), 4N HCl in dioxane (4.2mL) and reaction mixture stirred at r.t. for 45min (170mg, quantitative yield of **263**).

### Synthesis of 266:

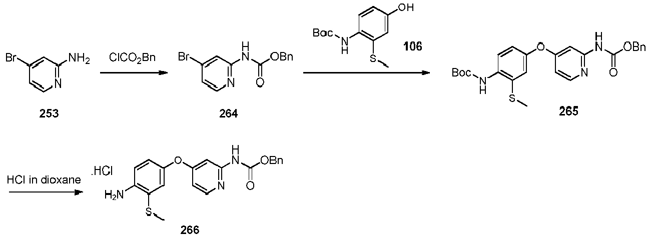

### Step 1. Synthesis of compound (264)

A stirred solution of compound **253** (1.73g, 10mmol) in anhydrous THF (20mL) was treated at 0°C with LiHMDS 1.0M solution in THF (21.0mL, 21.0mmol). After 15min at 0°C, a solution of benzyl chloroformate (1.70ml, 12.0mmol) in THF (20mL) was added. The mixture was allowed to warm to r.t. and stirred overnight. The reaction was quenched with sat. aq. NH₄Cl and extracted with EtOAc. The organic fractions were dried over MgSO₄, filtered and concentrated in vacuo. The solid obtained was triturated in Et₂O and filtered off leading to the expected product **264** (Yield: 64%, 1.41g) as a beige solid.

### Step 2. Synthesis of compound (265) according to General Procedure N

Compound **264** (307mg, 1.0mmol), compound **106** (306mg, 1.2mmol) in anhydrous toluene (10mL). Then K₃PO₄ (764mg, 3.6mmol), and tBuXPhos Pd G3 (79mg, 0.1mmol) was added. Mixture stirred and heated (100°C, 18h). Flash chromatography (40g, SiO₂, cyclohexane/EtOAc 100:0→70:30, 10 CV) the expected product **265** in mixture with unreacted phenol. The batch was used without further purification for next step.

### Step 3. Synthesis of compound (266): benzyl (4-(4-amino-3-(methylthio)phenoxy)pyridin-2-yl)carbamate hydrochloride

Compound **265** (108mg, 0.22mmol) was treated with 4N HCl in dioxane (10mL) and stirred at r.t. for 2h. The solvent was removed under vacuum. The beige powder recovered was directly used for next step without any purification.

### Synthesis of 271:

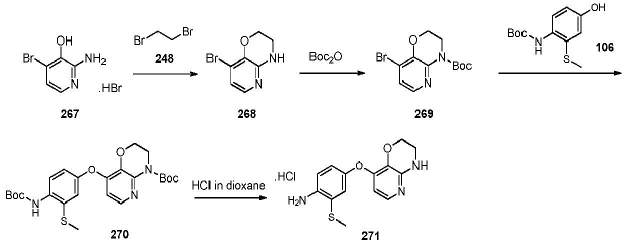

### Step 1. Synthesis of compound (268)

In a sealed tube containing compound **267** (2.50g, 9.3mmol) in ACN (25mL) were added Cs₂CO₃ (9.1g, 27.9mmol) followed by compound **248** (2.6g, 13.9mmol). The mixture was refluxed for 72h. After cooling and filtration of the suspension, the filtrate was concentrated under reduced pressure. The residue was purified by flash chromatography (40g, SiO₂, cyclohexane/EtOAc 100:0→0:100, 10 CV followed by EtOAc/MeOH 100:0→80:20, 5 CV) leading to the expected product **268** (Yield: 13%, 274mg) as a beige solid.

### Step 2. Synthesis of compound (269)

To a solution of compound **268** (330mg, 1.53mmol) in anhydrous THF (5mL) were dropwise added LiHMDS 1.0M solution in THF (1.84mL, 9.9mmol) followed by Boc₂O (502mg, 2.30 mmol). The mixture was stirred at 0°C for 1h. The reaction was quenched with sat. aq. NH₄Cl and extracted with EtOAc. The organic fractions were dried over MgSO₄, filtered and concentrated in vacuo. The residue obtained was purified by flash chromatography (40g, SiO₂, cyclohexane/EtOAc 100:0→50:50, 12 CV) leading to the expected product **269** (Yield: 81%, 395mg) as a yellow solid.

### Step 3. Synthesis of compound (270) according to General Procedure N

Compound **269** (315mg, 1.0mmol), compound **106** (306mg, 1.2mmol) in anhydrous toluene (10mL). Then K₃PO₄ (764mg, 3.6mmol) and tBuXPhos Pd G3 (79mg, 0.1mmol were added. Mixture was stirred and heated (100°C, 8h). Flash chromatography (40g, SiO₂, cyclohexane/EtOAc 100:0→60:40, 10 CV) (Yield of **270:** 32%, 161mg).

### Step 4. Synthesis of compound (271): 4-((3,4-dihydro-2H-pyrido[3,2-b][1,4]oxazin-8-yl)oxy)-2-(methylthio)aniline hydrochloride

Compound **270** (160mg, 0.33mmol), 4N HCl in dioxane (10mL) at r.t. for 24h. The suspension obtained was filtered off, washed with Et₂O and dried under vacuum. The beige powder recovered (100mg) was directly used for next step without any purification

### Synthesis of 273: 2,2,2-trichloroethyl (3-(tert-butyl)isoxazol-5-yl)carbamate according to General Procedure I

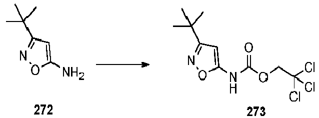

Compound **272** (5.0g, 35.7mmol, 1.0eq), dry THF (0.2M), dry pyridine (1.1eq), 2,2,2-trichloroethylchloroformate (5.4mL, 39.2mmol, 1.1 eq). Reaction mixture stirred at r.t. for 5h (11.5g, quantitative yield of **273**).

### Synthesis of 275: 2,2,2-trichloroethyl (5-(tert-butyl)isoxazol-3-yl)carbamate according to General Procedure I

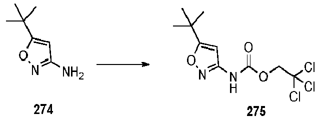

Compound **274** (1.0g, 7.13mmol, 1.0 eq), dry THF (0.2 M), dry pyridine (1.1eq), 2,2,2-trichloroethylchloroformate (1.1mL, 7.8mmol, 1.1 eq). Reaction mixture stirred at r.t. for 24h (2.26g, quantitative yield of **275**).

### Synthesis of 279: according to General Procedure D & I

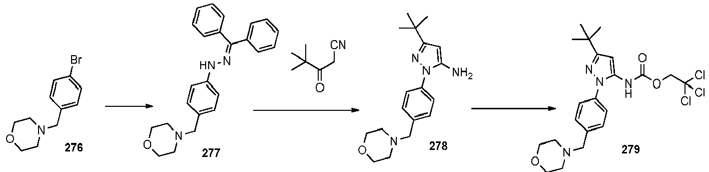

### Step 1. Synthesis of compound (277)

In a sealed tube, compound **276** (2.0g, 7.8mmol), compound **83** (1.53g, 7.8mmol, 1.0eq),tBuONa (1.1g, 11.71mmol, 1.5eq) were put in suspension in 100mL of toluene. The mixture was degassed with Ar for 30min before addition of BINAP (97mg, 0.156mmol, 0.02eq) and palladium acetate (26mg, 0.117mmol, 0.015eq). The mixture was heated at 100°C for 18h. After cooling down, the precipitate was filtered and rinsed with EtOAc and MeOH. The filtrate was adsorbed on silica gel and purified by flash chromatography using cyclohexane and EtOAc to afford the expected compound **277** as yellow foam (Yield: 53%, 1.53g)

### Step 2. Synthesis of compound (278)

Compound **277** (1.2g, 3.28mmol, 1.0eq), pivaloyl acetonitrile (616mg, 4.92mmol, 1.5eq) in 20mL of EtOH, HCl 12N (2.7mL, 32.8mmol, 10.0eq). Mixture stirred at reflux for 20h. NaHCO₃ solid added until pH was around 7. Flash chromatography on silica gel using cyclohexane and EtOAc (Yield of **278:** 78%, 802mg).

### Step 3. Synthesis of compound (279): 2,2,2-trichloroethyl (3-(tert-butyl)-1-(4-(morpholinomethyl)phenyl)-1H-pyrazol-5-yl)carbamate

Compound **278** (1.69g, 5.37mmol, 1.0eq), dry pyridine (1.1eq), 2,2,2-trichloroethylchloroformate (0.81mL, 5.91mmol, 1.1eq). Reaction mixture stirred at r.t. for 24h. Flash chromatography over silica gel using cyclohexane and EtOAc (Yield of **279:** 81%, 2.13g).

### Synthesis of 281:

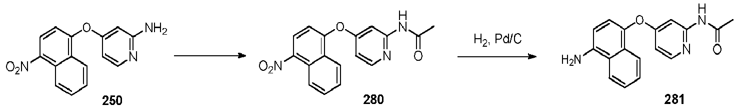

### Step 1. Synthesis of compound (280)

A stirred solution containing compound **250** (250mg, 0.89mmol), propylphosphonic anhydride (1.60ml, 2.67mmol), and DIEA (3.10mL, 20mmol) in DCE (10mL) was heated at 80°C in a sealed tube for 20h. After cooling at r.t., the reaction mixture was concentrated in vacuo. The crude obtained was purified by flash chromatography (40g, SiO₂, cyclohexane/EtOAc 100:0→0:100, 10 CV) leading to the expected product **280** (345mg) as a yellow solid.

### Step 2. Synthesis of compound (281): N-(4-((4-aminonaphthalen-1-yl)oxy)pyridin-2-yl)acetamide

A suspension of compound **280** (345mg, 1.07mmol) and Pd/C (50mg) in a mixture EtOH/DCM (1:1; 100mL) was stirred under hydrogen (1atm). After 18h at r.t. and work-up, the expected product **281** (Yield: 80%, 250mg) was recovered as a dark purple solid.

### Synthesis of 283: according to General Procedure K

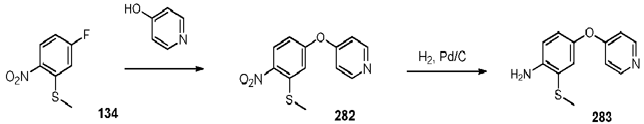

### Step 1. Synthesis of compound (282)

DBU (0.72mL, 4.81 mmol), 4-hydroxypyridine (330mg, 3.47mmol) in dry ACN (0.1 M). **134** (500mg, 2.67mmol). Reaction mixture stirred at 80°C for 2h (Yield of **282:** 92%, 645mg).

### Step 2. Synthesis of compound (283): 2-(methylthio)-4-(pyridin-4-yloxy)aniline

Compound **282** (645mg, 2.46mmol), Pd/C (100mg) in EtOH (50mL) and DCM (50mL) stirred under hydrogen (1atm) (570mg, quantitative yield of 283).

### Synthesis of 285:

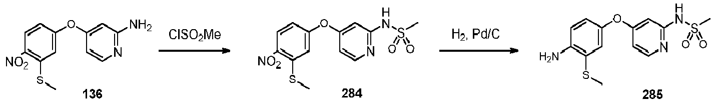

### Step 1. Synthesis of compound (284)

To a stirred solution of compound **136** (400mg, 1.44mmol) in DCM (40mL) were slowly added at 0°C triethylamine (0.60mL, 4.30mmol), and mesylchloride (0.13mL, 1.73mmol). The reaction mixture was stirred at r.t. for 18h and concentrated in vacuo. The crude obtained was purified by flash chromatography (40g, SiO₂, DCM/EtOAc 100:0→50:50, 10 CV) leading to the expected product **284** (90mg) as a yellow solid.

### Step 2. Synthesis of compound (285): N-(4-(4-amino-3-(methylthio)phenoxy)pyridin-2-yl)methanesulfonamide

A suspension of compound **284** (42mg, 0.12mmol) and Pd/C (20mg) in a mixture EtOH/DCM (2:1, 15mL) was stirred under hydrogen (1atm). After 4h at r.t. and work-up, the expected product **285** (41 mg, quantitative) was recovered as a yellow powder.

### Synthesis of 287:

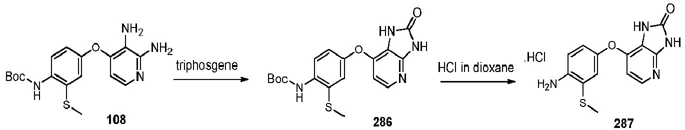

### Step 1. Synthesis of compound (286)

In a sealed tube, a solution of compound **108** (450mg, 1.24mmol) and pyridine (1.0mL, 12.4mmol) in dry THF (11mL) was treated dropwise at 0°C by a solution of triphosgene (368mg, 1.24mmol) in THF (4mL). The reaction mixture was stirred at r.t. for 18h. After evaporation of the solvent, the crude solid obtained was washed successively with water and Et₂O. The brown solid recovered after drying was dissolved in dry THF (15mL). Pyridine (1.0mL, 12.4mmol) was added and the mixture was heated at 70°C for 48h. After cooling at r.t. and evaporation in vacuo, water (30mL) and EtOAc (50mL) were added. The aqueous layer was extracted with EtOAc (2 x 30mL). The combined organic fractions were dried over MgSO₄, filtered and concentrated in vacuo. The crude obtained was purified by flash chromatography (40g, SiO₂, cyclohexane/EtOAc 100:0→0:100,10 CV) leading to the expected product **286** (436mg, 54% LC/MS purity) as a brown solid.

### Step 2. Synthesis of compound (287): 7-(4-amino-3-(methylthio)phenoxy)-1H-imidazo[4,5-b]pyridin-2(3H)-one hydrochloride

Compound **286** (436mg, 1.12mmol) was treated with 4N HCl in dioxane (10mL) at r.t. for 2h. The suspension obtained was filtered off, washed with ether, and dried under vacuum. The brown powder recovered (Yield: 93%, 337mg) was directly used for next step without any purification.

### Synthesis of 296: according to General Procedure J

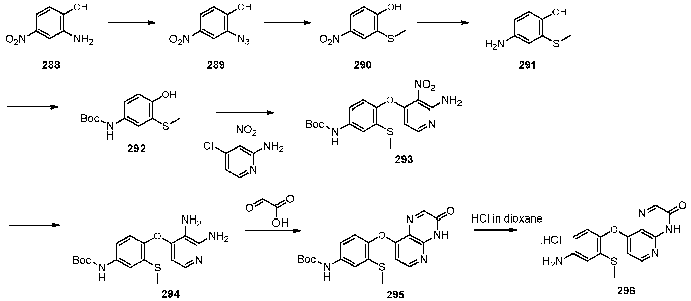

### Step 1. Synthesis of compound (289)

A solution of sodium nitrite (2.24 g, 32.4 mmol) in water (12mL) was added dropwise at 0°C to a solution of 2-amino-4-nitro-phenol **288** (5.0g, 32.4mmol) and tetrafluoroboric acid in water (48% wt., 12mL). The mixture was stirred 1h at r.t.. The precipitate formed was filtered off, washed with water, and triturated in acetone. The white solid recovered was dried leading to the expected product **289** (Yield: 73%, 4.25g).

### Step 2. Synthesis of compound (290)

Copper (730mg, 11.5mmol) was added at 0°C to a suspension of 2-azido-4-nitro-phenol **289** (4.25g, 22.9mmol) in water (100mL), followed by sodium thiomethoxide (3.22g, 45.9mmol) in water (100mL). After 18h at r.t., the reaction mixture is filtered over Celite. The filtrate was acidified by addition of HCl 1N. The precipitate formed was filtered off, washed with water, and dryed under vacuum leading to a black solid (Yield: 77%, 3.25g) corresponding to the expected product **290.**

### Step 3. Synthesis of compound (291)

Sodium hydrosulfite (7.6g, 43.7mmol) was added at 50°C to a solution of 2-methylsulfanyl-4-nitro-phenol 290 (3.25g, 17.5mmol) in NaOH 1N (52mL, 52mmol). The reaction mixture was stirred at 100°C for 4h. The precipitate formed was filtered off to recover a brown solid (Yield: 26%, 700mg) corresponding to the expected product **291.**

### Step 4. Synthesis of compound (292)

InCl₃ (10mg, 0.05mmol), Boc₂O (986mg, 4.5mmol), dry THF (3mL), stirred at 40°C 15min. **291** (700mg, 4.5mmol) added and mixture heated at 40°C for 18h. Flash chromatography (40g, SiO₂, cyciohexane/EtOAc 100:0→0:100, 10 CV) (Yield of **292:** 87%, 1.0g).

### Step 5. Synthesis of compound (293)

tBuOK (569mg, 5.1mmol), added at r.t. to **292** (1.0g, 3.9mmol) in DMF (20mL). After 15min, 4-chloro-3-nitro-pyridin-2-amine (680mg, 3.9mmol) added and reaction mixture heated at 80°C for 3h. Flash chromatography (40g, SiO₂, cyclohexane/EtOAc 100:0→80:20, 10 CV) **293** (Yield of **293:** 85%, 1.3g).

### Step 6. Synthesis of compound (294)

**293** (1.3g, 3.31mmol) and Pd/C (120mg) in EtOH (50mL) stirred under hydrogen (3 bars) for 20h at r.t.. Flash chromatography (40g, SiO₂, cyclohexane/EtOAc 100:0→0:100, 10 CV) (Yield of **294:** 58%, 700mg).

### Step 7. Synthesis of compound (295)

**294** (450mg, 1.24mmol) in MeOH, glyoxylic acid (1.15g, 12.4mmol) in MeOH (10mL). Stirring 18h at r.t.. Flash chromatography (40g, SiO₂, cyclohexane/EtOAc 100:0→0:100, 10 CV) (Yield of **295:** 14%, 70mg).

### Step 8. Synthesis of compound (296): 8-(4-amino-2-(methylthio)phenoxy)pyrido[2,3-b]pyrazin-3(4H)-one hydrochloride

**295** (220mg, 0.73mmol), HCl 4N in dioxane (4mL) at r.t. for 1h30 (Yield of **296:** 73%, 160mg).

### Synthesis of 298: according to General Procedure B

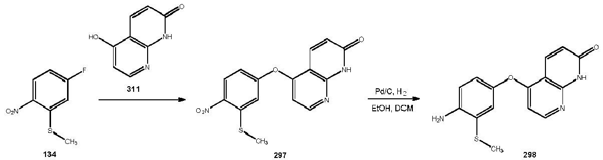

### Step 1. Synthesis of compound (297)

tBuOK (389mg, 3.45mmol, 1.3eq), **311** (430mg, 2.65mmol, 1.0eq) in DMF (10mL). Stirring 30min at r.t., **134** (496mg, 2.65mmol, 1.0eq) added. Solution stirred at 120°C for 72h. Flash chromatography over silica gel using cyclohexane and EtOAc (Yield of **297:** 13%, 113mg).

### Step 2. Synthesis of compound (298): 5-(4-amino-3-(methylthio)phenoxy)-1,8-naphthyridin-2(1H)-one

Pd/C (around 60mg), **297** (113mg, 0.34mmol) in EtOH/DCM (20mL, 3:1). Hydrogenation at 30°C over 1 atmosphere for 20h (101mg, quantitative yield of **298**).

### Synthesis of 303: according to General Procedure D & I

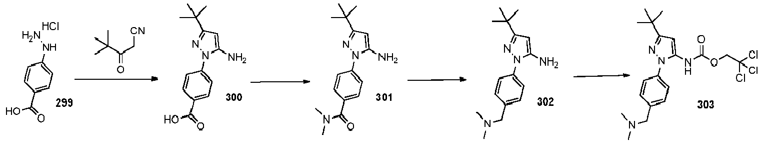

### Step 1. Synthesis of compound (300)

**299** (3.0g, 19.7mmol, 1.0eq), pivaloyl acetonitrile (2.7g, 21.7 mmol, 1.1eq) in EtOH, HCl 12 N (10.0 eq). Mixture stirred at reflux for 18h. NaHCO₃ solid added until pH was around 7 (5.6 g, quantitative yield of **300**).

### Step 2. Synthesis of compound (301)

EDCl.HCl (1.6g, 8.27mmol, 1.3eq) was added to a solution of **300** (1.65g, 6.36mmol, 1.0eq) and dimethyl amine 2M in MeOH (4.8mL, 9.5mmol, 1.5eq) in 20mL of THF. After 3h at r.t., the reaction mixture was concentrated, taken up in DCM and washed twice with brine. The organic phase was dried over MgSO₄, filtered and evaporated to afford the expected compound **301** (Yield: 80%, 1.45g).

### Step 3. Synthesis of compound (302)

1M solution of boron trifluoride diethyl etherate in THF (40.5mL, 40.5mmol, 8.0eq) was slowly added to a solution of **301** (1.45g, 5.1mmol, 1.0eq) in 50mL of dry THF. The reaction mixture was stirred at r.t. for 4h and was carefully quenched with MeOH. The residue was dissolved several times in MeOH and evaporated to be purified by flash chromatography over silica gel using cyclohexane and EtOAc and MeOH to afford expected compound **302** (Yield: 38%, 528mg).

### Step 4. Synthesis of compound (303): 2,2,2-trichloroethyl (3-(tert-butyl)-1-(4-((dimethylamino)methyl)phenyl)-1H-pyrazol-5-yl)carbamate

**302** (528mg, 1.94mmol, 1.0eq), DIEA (6.0 equiv.), ACN (0.1M), 2,2,2-trichloroethylchloroformate (0.29mL, 2.13mmol, 1.1 eq). Reaction mixture stirred at r.t. for 18h (1.03g of **303,** 69% by LC/MS).

### Synthesis of 305:

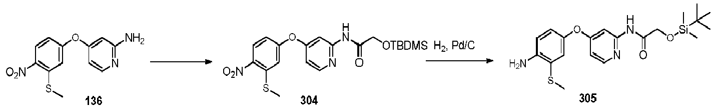

### Step 1. Synthesis of compound (304)

A stirred solution containing compound **136** (890mg, 3.21mmol), 2-[tert-butyl(dimethyl]silyl]oxyacetamide (611mg, 3.1mmol), propylphosphonic anhydride (5.70mL, 9.63mmol), and DIEA (1.70mL, 9.63mmol) in DCE was heated at 80°C in a sealed tube for 18h. After cooling at r.t., NaHCO₃ sat (30mL) was added. The aqueous layer was extracted with DCM (2 x 30mL). The combined organic fractions were dried over MgSO₄, filtered and concentrated in vacuo. The crude obtained was purified by flash chromatography (80g, SiO₂, cyclohexane/EtOAc 100:0→60:40, 10 CV) leading to the expected product **304** (Yield: 24%, 347mg) as a yellow solid.

### Step 2. Synthesis of compound (305): N-(4-(4-amino-3-(methylthio)phenoxy)pyridin-2-yl)-2-((tertbutyldimethylsilyl)oxy)acetamide

A suspension of compound **304** (300mg, 0.94mmol) and Pd/C (70mg) in a mixture EtOH/DCM (4:1, 50mL) was stirred under hydrogen (1atm). After 18h at r.t. and work-up, the expected product (Yield: 61%, 228mg) was recovered as an orange oil.

### Synthesis of 307: 2,2,2-trichloroethyl (3-(tert-butyl)-1-(p-tolyl)-1H-pyrazol-5-yl)carbamate according to General Procedure I

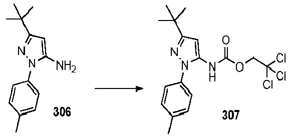

**306** (550mg, 2.4mmol, 1.0eq), dry pyridine (1.1eq), 2,2,2-trichloroethylchloroformate (0.36mL, 2.64mmol, 1.1 eq). Reaction mixture stirred at r.t. for 18h (Yield of **307:** 98%, 950mg).

### Synthesis of 310:

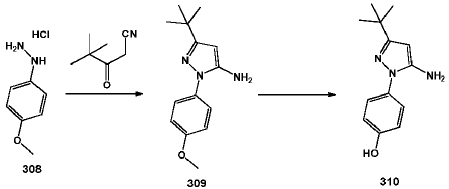

### Step 1. Synthesis of compound (309) according to General Procedure D

**308** (1.5g, 8.59mmol, 1.0eq), pivaloyl acetonitrile (1.1g, 8.59mmol, 1.0eq) were reacted at reflux for 18h. Flash chromatography over silica gel using cyclohexane and EtOAc (2.1g, quantitative yield of **309**).

### Step 2. Synthesis of compound (310): 4-(5-amino-3-tert-butyl-pyrazol-1-yl)phenol

**309** (2.1g, 8.56mmol, 1.0eq) was solubilized in DCM (30mL). AlCl₃ (5.7g, 42.8mmol, 5.0eq) was added and the reaction mixture was heated at reflux for 72h. After cooling down, the mixture was poured in water and DCM and the organic layer was washed with water and brine, dried over MgSO₄ and evaporated. The residue was purified by flash chromatography over silica gel using cyclohexane and EtOAc to afford compound **310** as yellow solid (Yield: 35%, 690mg).

### Synthesis of specific compounds

### • Synthesis of compound (a):

### Step 1. Synthesis of compound 1.5

According to Scheme 2 Step 1: Compound **3** (1.62g, 6.0mmol, 1.05eq) was dissolved in ACN (20mL) and compound **1.1** (1.0g, 5.77mmol, 1eq) was added to the solution. The reaction mixture was stirred at ambient temperature for 12h, then the reaction mixture was evaporated under reduced pressure. The residue was purified by column chromatography silica gel (DCM) (Yield: 87%, 2.2g).

### Step 2. Synthesis of compound 1.6

According to Scheme 2 Step 2: Et₃N.3HF (8.1mL, 50mmol, 10eq) was added to the solution of compound **1.5** (2.2g, 5mmol) in THF (20mL) and the reaction mixture was stirred at ambient temperature for 12h. The solvent was removed under reduced pressure, water was added to the residue, formed precipitate was filtered off, washed with water and dried to give product (Yield: 70%, 1.15g).

### Step 3. Synthesis of compound 1.7

According to Scheme 2 Step 3: Compound **1.6** (1.15g, 3.52mmol, 1eq) was added to the suspension of NaH (60%, 150mg, 3.7mmol, 1.05eq) in DMSO (10mL) and the reaction mixture was stirred at ambient temperature for 0.5h. Then 4-chloro-3-nitropyridin-2-amine (610mg, 3.52 mmol, 1.0eq) was added and the mixture was stirred at 60°C overnight. Then the mixture was cooled down, water (50mL) was added, the product was extracted with EtOAc (3x50mL), the organic layers were dried under Na₂SO₄ and evaporated under reduced pressure. The residue was purified residue was purified by column chromatography on silica gel (DCM→EtOAc) (Yield: 68%, 1.12g).

### Step 4. Synthesis of compound 1.8

According to Scheme 2 Step 4: A mixture of compound **1.7** (1.12g, 2.42mmol, 1eq) and 10% Pd/C (10% by weight, 0.12g) in MeOH (20mL) was stirred at r.t. under flow of H₂ for 12h. Then Pd/C was filtered, the solvent was evaporated under reduced pressure. The residue was used directly to the next step without additional purification (Yield: 95%, 1.0g).

### Step 5. Synthesis of compound 1.9: compound (a)

According to Scheme 2 Step 5: A mixture of compound **1.8** (1.0g, 2.3mmol, 1eq) and ethyl glyoxalate (50% in toluene, 0.7mL, 3.45mmol, 1.5eq) in EtOH (10mL) was stirred at ambient temperature for 12h. The solvents were evaporated under reduced pressure, the residue was purified twice by column chromatography (DCM→DCM/MeOH 98:2→DCM/MeOH/NH₃ 98:2:0.1) to afford **compound (a)** (Yield: 0.5%, 5mg).

### • Synthesis of compound (b): according to the synthesis of compound (a)

### Step 1. Synthesis of compound 1.5

According to Scheme 2 Step 1: Compound **3** (1.46g, 5.47mmol, 1.1eq), ACN (20mL), compound **1.1** (1.0g, 4.97mmol, 1eq) (Yield of **1.5:** 82%, 1.9g).

### Step 2. Synthesis of compound 1.6

According to Scheme 2 Step 2: Et₃N.3HF (6.6mL, 40.5mmol, 10eq), compound **1.5** (1.9g, 4.05mmol), THF (20mL) (Yield of **1.6:** 70%, 1.0g).

### Step 3. Synthesis of compound 1.7

According to Scheme 2 Step 3: Compound **1.6** (1g, 2.82mmol, 1eq), NaH (60%, 130mg, 3.1 mmol, 1.1eq), DMSO (10mL), 4-chloro-3-nitropyridin-2-amine (510mg, 2.96 mmol, 1.05eq) (Yield of **1.7:** 44%, 600mg).

### Step 4. Synthesis of compound 1.8

According to Scheme 2 Step 4: Compound **1.7** (600mg, 1.22mmol, 1eq), 10% Pd/C (10% by weight, 60mg), MeOH (20mL) (560mg, quantitative yield of **1.8**).

### Step 5. Synthesis of compound 1.9: compound (b)

According to Scheme 2 Step 5: Compound **1.8** (560mg, 1.22mmol, 1eq), ethyl glyoxalate (50% in toluene, 0.5mL, 2.44mmol, 2eq), EtOH (10mL) (Yield of **compound (b):** 2.6%, 16mg).

### • Synthesis of compound (c): according to the synthesis of compound (a)

### Step 1. Synthesis of compound 1.5

According to Scheme 2 Step 1:Compound **3** (1.37g, 5.11mmol, 1.1eq), ACN (20mL) and compound **1.1** (1.0g, 4.64mmol, 1eq) (Yield of **1.5:** 67%, 1.5g).

### Step 2. Synthesis of compound 1.6

According to Scheme 2 Step 2: Et₃N.3HF (5.1mL, 31.1mmol, 10eq), compound **1.5** (1.5g, 3.11mmol), THF (20mL) (Yield of **1.6:** 80%, 920mg).

### Step 3. Synthesis of compound 1.7

According to Scheme 2 Step 3: Compound **1.6** (920mg, 2.49mmol, 1eq), NaH (60%, 110mg, 2.74mmol, 1.1eq), DMSO (10mL), 4-chloro-3-nitropyridin-2-amine (450mg, 2.61mmol, 1.05eq) (Yield of **1.7:** 50%, 630mg).

### Step 4. Synthesis of compound 1.8

According to Scheme 2 Step 4: Compound **1.7** (630mg, 1.25mmol, 1eq), 10% Pd/C (10% by weight, 60mg), MeOH (20mL), (590mg, quantitative yield of **1.8**).

### Step 5. Synthesis of compound 1.9: compound (c)

According to Scheme 2 Step 5: Compound **1.8** (590mg, 1.25mmol, 1eq), ethyl glyoxalate (50% in toluene, 0.5mL, 2.50mmol, 2eq), EtOH (10mL) (Yield of **compound (c):** 10%, 64mg).

### • Synthesis of compound (d):

### Step 1. Synthesis of compound 1.2

According to Scheme 1 Step 1: To a mixture of compound **14** (200mg, 0.7mmol) and DIPEA (0.4mL, 2.2 mmol) in DCM (5mL), triphosgene (220mg, 0.7mmol) was added at 0°C and the reaction mixture was stirred at r.t. for 16h. Then reaction mixture was evaporated to dryness, the residue was dissolved in THF and precipitate was removed by filtration and the solution was evaporated to dryness. The corresponding isocyanate **1.2** was used in next synthesis without purification.

### Step 2. Synthesis of compound 1.4: compound (d)

According to Scheme 1 Step 2: To a solution of compound **1.2** (170mg, 0.7mmol) and compound **8** (100mg, 0.36mmol) in DMSO (2mL) was stirred at 60°C for 12h. The reaction mixture was cooled and diluted with water. The precipitate was filtrated, washed with Et₂O (100mL) and dried. The solid after evaporation was subjected to column chromatography on silica gel eluting with DCM/EtOAc (0→100%) to afford the **compound (d)** (Yield: 4%, 7mg).

### • Synthesis of compound (e):

### Step 1. Synthesis of compounds 1.13

According to Scheme 5 Step 1: Compound **18** (1g, 4.4mmol) was dissolved in DCM and (COCl)₂ (830mg, 6.6mmol) was added and reaction mixture was stirring for 12h at 20°C. Then solvent was concentrated in vacuo (Yield: 92%, 1g).

### Step 2. Synthesis of compounds 1.14

According to Scheme 5 Step 2: Compound **1.13** (1g, 4mmol), NaN₃ (400mg, 6mmol) was mixed in Me₂CO/H₂O (40/10mL). After stirring for 2h at 20°C, water (200mL) was added to the reaction mixture, and the resulting mixture was extracted with EtOAc (2×50mL). The combined organic extracts were washed with brine and dried over Na₂SO₄. The solvent was then removed under reduced pressure (Yield: 97%, 1g).

### Step 3. Synthesis of compounds 1.4: compound (e)

According to Scheme 5 Step 3: A solution of compound **1.14** (180mg, 0.7mmol) in DMSO (2mL) was stirring for 2h at 120°C, reaction mixture cooling to r.t.. Compound **8** (100mg, 0.36mmol) was added to a reaction mixture. After stirring for 12h at 100°C, reaction mixture cooling to r.t., water (25mL) was added to the reaction mixture, and the resulting mixture was extracted with Et₂O (50mL). The crude product was filtered and purified with column using THF as eluent to afford **compound (e)** (Yield: 40%, 140mg).

### • Synthesis of compound (f):

### Step 1. Synthesis of compounds 1.11

According to Scheme 4 Step 1: To a solution of compound **22** (100mg, 0.4mmol), pyridine (70mg, 0.8mmol) in THF (5mL) phenyl chloroformate (72mg, 0.45mmol) was added and reaction mixture was stirred at ambient temperature for 2h. The solvent was evaporated and the residue after evaporation was taken up with EtOAc and water. The organic layer was separated and filtered through anhydrous sodium sulfate, evaporated. The residue after evaporation was subjected to column chromatography on silica gel eluting with DCM/EtOAc (0→10%) to afford the compound **1.11** (Yield: 92%, 0.14g).

### Step 2. Synthesis of compounds 1.4: compound (f)

According to Scheme 4 Step 2: To a mixture of compound **1.11** (140mg, 0.4mmol) and NEt₃ (0.06mL, 0.4mmol) in dioxane (2mL) a compound **8** (100mg, 0.36mmol) was added, reaction mixture was stirred at 100°C for 12h. The reaction mixture was cooled and diluted with water. The precipitate was filtrated, washed with ether and dried. The solid was subjected to column chromatography on silica gel eluting with DCM/ethyl acetate (0→100%) to afford the **compound (f)** (Yield: 9%, 17mg).

### • Synthesis of compound (g): according to the synthesis of compound (a)

### Step 1. Synthesis of compound 1.5

According to Scheme 2 Step 1: Compound **3** (2.78g, 10.4mmol, 1.1eq), ACN (20mL), compound **1.1** (2.0g, 9.47mmol, 1eq) (Yield of **1.5:** 77%, 3.5g).

### Step 2. Synthesis of compound 1.6

According to Scheme 2 Step 2: Et₃N.3HF (11.2mL, 72.5mmol, 10eq), compound **1.5** (3.5g, 7.25mmol, 1eq), THF (30mL) (Yield of **1.6:** 72%, 1.9g).

### Step 3. Synthesis of compound 1.7

According to Scheme 2 Step 3: Compound **1.6** (1.9g, 5.21mmol, 1eq), NaH (60%, 230mg, 5.73mmol, 1.1eq), DMSO (15mL), 4-chloro-3-nitropyridin-2-amine (950mg, 5.21mmol, 1.0eq) (Yield of **1.7:** 23%, 600mg).

### Step 4. Synthesis of compound 1.8

According to Scheme 2 Step 4: Compound **1.7** (600mg, 1.19mmol, 1eq), 10% Pd/C (10% by weight, 60mg), MeOH (20mL) (Yield of **1.8:** 95%, 540mg).

### Step 5. Synthesis of compound 1.9: compound (g)

According to Scheme 2 Step 5: Compound **1.8** (540mg, 1.13mmol, 1eq), ethyl glyoxalate (50% in toluene, 0.46mL, 2.26mmol, 2eq), EtOH (10mL) (Yield of **compound (g):** 0.85%, 5mg).

### • Synthesis of compounds (h) and (o):

### Step 1. Synthesis of compound (o): according to the synthesis of compound (d)

According to Scheme 1 Step 1 & 2: Triphosgene (0.246g, 0.8mmol, 1eq), compound **24** (0.15g, 0.83mmol, 1eq), Et₃N (0.32mL, 2.5mmol, 3eq), DCM (50mL) at -20°C. Reaction mixture stirred at r.t. for 12h. Intermediate in 3mL DMSO, compound **8** (160mg, 0.58mmol) added and mixture stirred for 12h at 50°C. Water added and precipitate filtered off and purified by column chromatography silica gel (DCM) (Yield of **compound (o):** 40%, 150mg).

### Step 3. Synthesis of compound (h):

100mg (0.21mmol) of **compound (o)** in MeOH (20mL) were added 5mL of 25% ammonia aqueous solution and stirred 3h at 20°C. Then mixture evaporated off and purified by column chromatography silica gel (DCM) to obtain **compound (h)** (Yield: 33%, 30mg).

### • Synthesis of compound (i): according to the synthesis of compound (d)

### Step 1. Synthesis of compound 1.2

According to Scheme 1 Step 1: Triphosgene (115mg, 0.38mmol, 1eq), compound **1.1** (70mg, 0.38mmol, 1eq), Et₃N (0.13mL, 1.15mmol, 3eq), DCM (10mL), -20°C, then r.t. 12h.

### Step 2. Synthesis of compound 1.4: compound (i)

According to Scheme 1 Step 2: Precipitate **1.2,** 3mL of DMSO, compound **8** (100mg, 0.36mmol) stirred for 12h at 50°C. (Yield of **compound (i):** 10%, 18mg).
¹H-NMR (DMSO-d₆), δ (ppm, *J* (Hz): 1.20 (s 9H, *t*-Bu), 1.35 (1, 6H, *i*-Pr), 4.4 (m, H, *i*-Pr), 6.05 (s, 1H, H*_{pyrazole}*),6.65 (d, *J*=5.6, 1H, H*_{pyridine}*)*,* 7.05 (m, 1H, H*ₐᵣₒₘ*), 7.40 (m, 1H, H*ₐᵣₒₘ*), 8.39 (d, *J*=5.6, 1H, *H_{pyridine}*)*,* 8.72 (s, 1H, NH), 8.80 (s, 1H, NH), 12.93 (s, 1H, NH).

### • Synthesis of compound (j): according to the synthesis of compound (a)

### Step 1. Synthesis of compound 1.5

According to Scheme 2 Step 1: Compound **3** (2.01g, 7.52mmol, 1.15eq), ACN (20mL), compound **1.1** (1.5g, 6.54mmol, 1eq) (Yield of **1.5:** 86%, 2.8g).

### Step 2. Synthesis of compound 1.6

According to Scheme 2 Step 2: Et₃N.3HF (9.18mL, 56.3mmol, 10eq), compound **1.5** (2.8g, 5.63mmol, 1eq), THF (30mL) (Yield of **1.6:** 93%, 2.0g).

### Step 3. Synthesis of compound 1.7

According to Scheme 2 Step 3: Compound **1.6** (2.0g, 5.23mmol, 1eq), NaH (60%, 0.25g, 6.27mmol, 1.2eq), DMSO (10mL), 4-chloro-3-nitropyridin-2-amine (1.04g, 6.01mmol, 1.15eq) (Yield of **1.7:** 22%, 600mg).

### Step 4. Synthesis of compound 1.8

According to Scheme 2 Step 4: Compound **1.7** (500mg, 0.96mmol, 1eq), 10% Pd/C (10% by weight, 50mg), MeOH (10mL) (Yield of **1.8:** 85%, 400mg).

### Step 5. Synthesis of compound 1.9: compound (j)

According to Scheme 2 Step 5: Compound **1.8** (400mg, 0.82mmol, 1eq), ethyl glyoxalate (50% in toluene, 0.33mL, 1.64mmol, 2eq), EtOH (10mL) (Yield of **compound (j):** 2.8%, 12mg).

### • Synthesis of compound (k): according to the synthesis of compound (d)

### Step 1. Synthesis of compound 1.2

According to Scheme 1 Step 1: Triphosgene (115mg, 0.38mmol, 1eq), compound **26** (70mg, 0.38mmol, 1eq), Et₃N (0.13mL, 1.15mmol, 3eq), DCM (10mL), -20°C then r.t. for 12h. Solvent was removed under reduced pressure, THF (50mL) was added to the residue, the formed precipitate was filtered off, solvent was evaporated under reduced pressure

### Step 2. Synthesis of compound 1.4: (compound (k))

According to Scheme 1 Step 2: Precipitate **1.2,** 3mL DMSO, compound **8** (0.1g, 0.36mmol) stirred for 12h at 50°C. Water added and precipitate filtered off purified by column chromatography silica gel (DCM) (Yield of **compound (k):** 2%, 4mg).

### • Synthesis of compound (l): according to the synthesis of compound (d)

### Step 1. Synthesis of compound 1.2

According to Scheme 1 Step 1: Compound **1.1** (200mg, 0.93mmol), DCM (30mL), saturated aq. NaHCO₃ solution (20mL) and diphosgene (0.50mL, 3.7mmol) added at 0°C then warmed to r.t. for 1h. Organic layer separated, dried, and evaporated in vacuo to give a brown oil triturated from hexane (2.0mL) and filtered. Filtrate was concentrated in vacuo (Yield of **1.2:** 40%, 90mg).

### Step 2. Synthesis of compound 1.4: compound (I)

According to Scheme 1 Step 2: Compound **8** (100mg, 0.37mmol), DMF (3mL), compound **1.2** (58mg, 0.37mmol) stirred at 80°C for 12h. Column chromatography on silica gel eluting with DCM/EtOAc (0→100%) (Yield of **compound (l):** 21%, 40mg).

### • Synthesis of compound (m): according to the synthesis of compound (a)

### Step 1. Synthesis of compound 1.5

According to Scheme 2 Step 1: Compound **3** (2.97g, 11.1mmol, 1.15eq), ACN (20mL) and compound **1.1** (2.1g, 9.66mmol, 1eq) (Yield of **1.5:** 72%, 3.4g).

### Step 2. Synthesis of compound 1.6

According to Scheme 2 Step 2: Et₃N.3HF (11.4mL, 70.1mmol, 10eq), compound **1.5** (3.4g, 7mmol, 1eq), THF (30mL) (Yield of **1.6:** 85%, 2.2g).

### Step 3. Synthesis of compound 1.7

According to Scheme 2 Step 3: Compound **1.6** (2.2g, 5.94mmol, 1eq), NaH (60%, 270mg, 6.83mmol, 1.15eq), DMSO (10mL), 4-chloro-3-nitropyridin-2-amine (1.08g, 6.24mmol, 1.05eq) (Yield of **1.7:** 20%, 600mg).

### Step 4. Synthesis of compound 1.8

According to Scheme 2 Step 4: Compound **1.7** (600mg, 1.2mmol, 1eq), 10% Pd/C (10% by weight, 60mg), MeOH (10mL) (Yield of **1.8:** 88%, 500mg).

### Step 5. Synthesis of compound 1.9: compound (m)

According to Scheme 2 Step 5: Compound **1.8** (500mg, 1.05mmol, 1eq), ethyl glyoxalate (50% in toluene, 0.43mL, 2.1mmol, 2eq), EtOH (10mL)) (Yield of **compound (m):** 1.1%, 6mg).

### • Synthesis of compound (n):

According to Scheme 4 Step 2: Mixture of compound **98** (200mg, 0.45mmol) and compound **8** (132 mg, 0.48mmol) in anhydrous DMSO (2mL) was stirred at 100°C for 2h. The reaction mixture was cooled and diluted with water and EtOAc. The precipitate was filtered off and collected. Organic layer separated, dried, combined with precipitate and evaporated. Residue dissolved in DCM/THF 4:1 and subjected to column chromatography (eluent DCM/THF 4:1) to afford **compound (n)** (Yield: 4.4%, 12mg).

### • Synthesis of compound (p): according to the synthesis of compound (d)

According to Scheme 1 Step 2: Compound **34** (130mg, 0.53mmol), compound **8** (60mg, 0.22mmol), anhydrous DMSO (2mL) stirred at 60°C for 12h. The reaction mixture was cooled and diluted with water and EtOAc. The precipitate was filtered off and discarded. Organic layer collected and product started to precipitate. This solid was filtered off washed with EtOAc and dried (Yield of **compound (p):** 10.6%, 12mg).

### • Synthesis of compound (q): according to the synthesis of compound (f)

### Step 1. Synthesis of compound 1.11

According to Scheme 4 Step 1: Compound **1.1** (313mg, 2mmol), 0.3mL of pyridine, 10mL of THF at 0°C, phenyl chloroformate (626mg, 4mmol) in THF. The mixture was stirred for 2h at r.t., treated with water, extracted with EtOAc, dried with MgSO₄, and evaporated on a rotor. The residue was chromatographed, eluent EtOAc/hexane (1:4) (Yield of **1.11:** 65%, 450mg).

### Step 2. Synthesis of compound 1.4: compound (q)

According to Scheme 4 Step 2: Compound **1.11** (176mg, 0.64mmol), compound **8** (174mg, 0.64mmol), NEt₃ (0.64mmol) in 1mL of dioxane stirred for 12h at 90°C. Cooled, poured into water, the precipitate was filtered off, washed with water, dried and further purified by HPLC (Yield of **compound(q):** 5%, 14.5mg).

### • Synthesis of compound (r):

One pot Scheme 1 following synthesis: To a mixture of compound **104** (70mg, 0.64mmol), compound **8** (174mg, 0.64mmol) and NET₃ (0.64mmol) in 1mL of dioxane at 0°C, a solution of triphosgene (70mg, 0.23mmol) in 0.5mL THF was added and stirred for 12h at r.t.. The mixture was poured into water, the precipitate was filtered off, washed with water, dried and further purified by HPLC to afford **compound (r)** (Yield: 6%, 17.5mg).

### • Synthesis of compound (s): according to the synthesis of compound (a)

### Step 1. Synthesis of compound 1.5

According to Scheme 2 Step 1: Compound **11** (4g, 16mmol, 1.15eq), ACN (20mL), compound **53** (3.0g, 13.9mmol, 1eq) (Yield of **1.5:** 43%, 2.8g).

### Step 2. Synthesis of compound 1.6

According to Scheme 2 Step 2: Et₃N.3HF (9.8mL, 60mmol, 10eq), compound **1.5** (2.8g, 6mmol, 1eq), THF (20mL) (Yield of **1.6:** 57%, 1.2g).

### Step 3. Synthesis of compound 1.7

According to Scheme 2 Step 3: Compound **1.6** (1.2g, 3.4mmol, 1eq), NaH (60%, 0.16g, 3.91mmol, 1.15eq), DMSO (10mL), 4-chloro-3-nitropyridin-2-amine (0.62g, 3.57mmol, 1.05eq) (Yield of **1.7:** 36%, 600mg).

### Step 4. Synthesis of compound 1.8

According to Scheme 2 Step 4: Compound **1.7** (600mg, 1.2mmol, 1eq), 10% Pd/C (10% by weight, 0.06g), MeOH (10mL) (Yield of **1.8:** 89%, 500mg).

### Step 5. Synthesis of compound 1.9: compound (s)

According to Scheme 2 Step 5: Compound **1.8** (500mg, 1.09mmol, 1eq), ethyl glyoxalate (50% in toluene, 0.33mL, 1.63mmol, 1.5eq), EtOH (10mL) (Yield of **compound (s):** 3.7%, 20mg).

### • Synthesis of compound (t): according to the synthesis of compound (n)

According to Scheme 4 Step 2: Compound **54** (234mg, 0.7mmol), DMSO (10mL), compound **50** (179mg, 0.7mmol) stirred at 80°C 1h. Solvent evaporated and residue purification by HPLC (Yield of **compound (t):** 8%, 27.8mg).

### • Synthesis of compound (u):

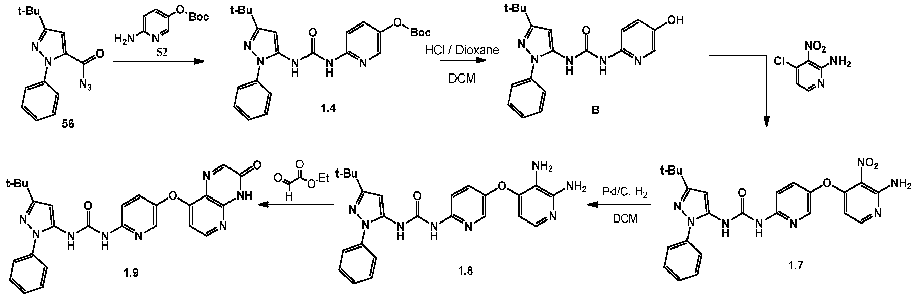

### Step 1. Synthesis of compound 1.4

According to Scheme 5 Step 3: Stirring of compound **56** (1.2g, 4.46mmol) in DMSO (15mL) for 1h at 110°C and compound **52** (670mg, 3.2mmol) added. Stirring for 12h at 100°C reaction mixture was cooled to r.t., water (100mL) added and precipitate filtered, washed with Et₂O (100mL) and dried to afford 1.2g of compound **1.4.**

### Step 2. Synthesis of compound B

1.2g of compound **1.4** was dissolved in 50mL absolute DCM and 1mL of 3M HCl in dioxane was added and stirred at ambient temperature for 60min (control by TLC). The reaction mixture was diluted with ether (50mL) and precipitate was filtered, washed with Et₂O (100mL). Then the precipitate was dissolved in mixture of DCM (50mL) and saturated aqueous NaHCO₃ (10mL). Organic layer was separated, water was extracted with 2x20mL of DCM. Combined organic layers were dried, filtrated and evaporated to dryness to afford 520mg (Yield: 55%) of pure **compound B.**

### Step 3. Synthesis of compound 1.7

According to Scheme 2 Step 3: NaH (60% w/w, 62mg, 1.55mmol), **compound B** (520mg, 1.47mmol), absolute DMF (30mL), 4-Chloro-3-nitropyridin-2-amine (260mg, 1.47mmol). Reaction mixture heated at 80°C for 30min (control by TLC) (Yield of **1.7:** 81%, 580mg).

### Step 4. Synthesis of compound 1.8

According to Scheme 2 Step 4: 10% Pd/C (300mg), compound **1.7** (580mg, 1.19mmol), EtOH/THF (100mL, 2:1). Hydrogenation at 20atm. H₂ at 40°C for 1h (Yield of **1.7:** 89%, 480mg).

### Step 5. Synthesis of compound 1.9: compound (u)

According to Scheme 2 Step 5: Compound **1.8** (480mg, 1.05mmol), 50mL of dry EtOH, ethyl glyoxalate (50% solution in toluene, 1.0mL, 5mmol). Mixture stirred 36h at r.t. under argon atmosphere. The solvent was partially evaporated and formed precipitate was filtered off. The filtrate containing regioisomer of **1.9** was discarded. The precipitate was purified by column chromatography on silica gel with THF as eluent (Yield of **compound (u):** 10%, 50mg).

### • Synthesis of compound (v): according to the synthesis of compound (a)

### Step 1. Synthesis of compound 1.5

According to Scheme 2 Step 1: The residue containing compound **59** in DMSO (25mL), compound **53** (1.112g, 5.15mmol). Column chromatography, using hexane, hexane/Et₂O 3:1 as eluent (Yield of **1.5:** 27.1%, 743mg).

### Step 2. Synthesis of compound 1.6

According to Scheme 2 Step 2: Compound **1.5** (743mg, 1.39mmol), HCl (3M solution in dioxane, 2mL), MeOH (20mL) was stirred at r.t. for 12h. The solvents removed under reduced pressure and the residue washed with DCM and Et₂O (Yield of **1.6:** 99%, 580mg).

### Step 3. Synthesis of compound 1.7

According to Scheme 2 Step 3: NaH (60%, 0.055g, 1.38mmol), compound **1.6** (580mg, 1.38mmol), DMF (10mL), 4-chloro-3-nitropyridin-2-amine (280mg, 1.62mmol). Product extracted with DCM (2x20mL). The residue purified with twice crystallization from DCM (Yield of **1.7:** 74%, 460mg).

### Step 4. Synthesis of compound 1.8

According to Scheme 2 Step 4: Compound **1.7** (460mg, 0.828mmol), Pd/C (10%, 50mg), EtOH (20mL). Column chromatography, using DCM and 2% MeOH in DCM as eluent (Yield of **1.8:** 54%, 235mg).

### Step 5. Synthesis of compound 1.9: compound (v)

According to Scheme 2 Step 5: Compound **1.8** (235mg, 0.447mmol), ethyl glyoxalate (50% in toluene, 137mg, 0.671 mmol), EtOH (10mL). Column chromatography, using DCM and 2% MeOH in DCM as eluent (Yield of **compound (v):** 15.9%, 40mg).

### • Synthesis of compound (w): according to the synthesis of compound (n)

According to Scheme 4 Step 2: Compound **80** (200mg, 0.70mmol), DMSO (2mL), compound **54** (234mg, 0.70mmol) stirred at 100°C for 12h. After cooling mixture quenched with water and EtOAc. Precipitate filtered, washed with water, then EtOAc and dried. Then redissolved in THF and filtered through celite pad. Filtrate evaporated (Yield of **compound (w):** 27.6%, 102 mg).

### • Synthesis of compound (x): according to the synthesis of compound (d)

### Step 1. Synthesis of compound 1.2

According to Scheme 1 Step 1: Compound **1.1** (200mg, 1.0mmol), DIPEA (0.5mL, 3.0mmol), DCM (5mL), triphosgene (290mg, 1.0mmol) added at 0°C and stirred at r.t. for 16h.

### Step 2. Synthesis of compound 1.4: compound (x)

According to Scheme 1 Step 2: Compound **1.2** (125mg, 0.54mmol) and compound **8** (100mg, 0.36mmol), DMSO (2mL) stirred at 60°C for 12h. Column chromatography on silica gel eluting with DCM/EtOAc (0→100%) (Yield of **compound (x):** 14%, 25mg).

### • Synthesis of compound (y):

### Steps 1 & 2. Synthesis of compound 1.14

According to Scheme 5 Steps 1 & 2: A solution of compound **89** (503mg, 2.21mmol) in SOCl₂ (20mL) was stirred at boiling temperature for 2h. The excess of SOCl₂ was removed under reduced pressure, the residue was dissolved in THF (20mL), NaN₃ (287mg, 4.42mmol) was added, the mixture was stirred at r.t. for 1h. Water (50mL) was added, the product was extracted with DCM (2x30mL), the combined organic layers were dried under MgSO₄, the solvents were removed under reduced pressure to provide compound **1.14** (Yield: 82%, 459mg).

### Steps 3. Synthesis of compound 1.4: compound (y)

According to Scheme 5 Step 3: A solution of compound **1.14** (459mg, 1.81mmol) in toluene (10mL) was stirred at boiling temperature for 1.5h. The solvent was removed under reduced pressure, the residue was dissolved in DMSO (10mL), compound **8** (287mg, 4.42mmol) was added, the mixture was stirred at 60°C for 12h. Water (50mL) was added, the product was extracted with EtOAc (2x30mL), the combined organic layers were dried under MgSO₄, the solvent was removed under reduced pressure, the residue was purified twice by crystallization from EtOAc and five times by column chromatography, using DCM and 2% MeOH in DCM as eluent, to provide **compound (y)** (Yield: 0.4%, 4mg).

### • Synthesis of compound (z): according to the synthesis of compound (aj)

According to Scheme 4 Step 2: To a solution of compound **111** (270mg, 0.90mmol, 1eq), compound **105** (300mg, 0.90mmol, 1eq), 4mL of DMSO, DIEA (502µL, 2.08mmol, 3.2eq). Mixture stirred at 70°C for 8 h. After cooling down, water added and the mixture extracted with EtOAc. Orange residue triturated in DCM and MeOH and filtered. Powder purified by preparative HPLC (C8 spheric column, 150x30mm, gradient of ACN (5 to 100%) in water + 0.1%HCOOH) to afford a white solid (Yield of **compound (z):** 2%, 9.6mg).

### • Synthesis of compound (ab): according to the synthesis of compound (d)

According to Scheme 1 Step 2: Compound **69** (197mg, 0.81mmol), compound **54** (272mg, 0.81mmol), DMSO (10mL) was at 60°C 12h. Column chromatography on silica gel eluting with DCM/EtOAc (0→100%) (Yield of **compound (ab):** 2.5%, 10mg). MS m/z (ES) [M+H]+: 485.50

### • Synthesis of compound (ac):

### Step 1. Synthesis of compound 1.10

According to Scheme 3 Step 1: Under Argon, triphosgene (143mg, 0.483 mmol) was added portions to a solution of compound **71** (98mg, 0.483mmol) and Et₃N (0.08mL, 0.58mmol) in DCM (5mL) at -20°C, the reaction mass was stirred at r.t. for 1 h. Then the solvent was removed under reduced pressure, THF (5mL) was added to the residue, the formed precipitate was filtered. The crude residue was used in the next step without further purification.

### Step 2. Synthesis of compound 1.4: compound (ac)

According to Scheme 3 Step 2: Compound **53** (108mg, 0.483mmol) was added to the mother liquor. The reaction mass was stirred at r.t. for 12h, the solvent was removed under reduced pressure, the residue was purified by column chromatography, using hexane and hexane/Et₂O 3:1 as eluent. The obtained impure product (73mg) was purified with crystallization from H₂O/ACN 1:1 to provide the pure **compound (ac)** (Yield: 22.3%, 48mg).

### • Synthesis of compound (ad): according to the synthesis of compound (y)

According to Scheme 5 Step 3: Compound **56** (458mg, 1.7mmol) in toluene (10mL) stirred at 115°C for 1h, then the solvent removed. Residue dissolved in DMSO (10mL), and aniline **72** (170mg, 0.833mmol) added to this solution. Reaction mass stirred at 60°C for 12h. Mixture poured in water (50mL) and extraction with EtOAc (2x20mL). Organic layers dried under Na₂SO₄. Residue purified twice by column chromatography, using DCM and 2% MeOH in DCM as eluent. The obtained impure product was purified by crystallization from EtOH (Yield of **compound (ad):** 6.7%, 25mg).

### • Synthesis of compound (ae): according to the synthesis of compound (u)

According to Scheme 5 Step 3: Compound **56** (150mg, 0.55mmol) in DMSO (2mL) stirred for 2h at 120°C, then reaction mixture cooled to r.t.. Compound **75** (110mg, 0.4mmol) added to a reaction mixture. After stirring for 12h at 100°C, reaction mixture cooling to r.t., water (25mL) added and the resulting mixture was diluted with Et₂O (50mL). Crude product filtered and purified by crystallization from ACN (Yield of **compound (ae):** 52 %, 110mg).

### • Synthesis of compound (af): according to the synthesis of compound (ac)

### Step1. Synthesis of compound 1.10

According to Scheme 3 Step 1: Triphosgene (134mg, 0.452mmol), compound **81** (100mg, 0.452mmol) and Et₃N (0.075mL, 0.542mmol), DCM (5mL).

### Step 2. Synthesis of compound 1.4: compound (af)

According to Scheme 3 Step 2: Compound **53** (97mg, 0.452 mmol) added to the mother liquor. Reaction mass stirred at r.t. for 12h. The solvent removed and the residue purified twice by column chromatography, using hexane and hexane/Et₂O 3:1. The obtained impure product (120mg) was purified by crystallization from H₂O/ACN 1:1 (Yield of **compound (af):** 27.7%, 58mg).

### • Synthesis of compound (ag): according to the synthesis of compound (aj)

According to Scheme 4 Step 2: Compound **98** (580mg, 1.31mmol, 1eq), compound **103** (380mg, 1.31mmol, 1eq) in 6mL of DMSO, DIEA (0.5mL, 2.89mmol, 2.2eq). Mixture stirred at 70°C for 8h. Water added and extraction with EtOAc. Residue was purified by flash chromatography on silica gel using cyclohexane/EtOAc then EtOAc/MeOH to a afford a white powder (Yield of **compound (ag):** 16%, 125mg).

### Synthesis of compound (ah): according to the synthesis of compound (aj)

According to Scheme 4 Step 2: Compound **111** (194mg, 0.65mmol, 1.0eq), DIEA (6eq) in DMSO(0.1M), compound **157** (261mg, 0.65mmol, 1.0eq). Reaction mixture stirred at 70°C for 20h then 100°C during 6h. Residue purified by semi-preparative HPLC to afford a beige powder (Yield of **compound (ah):** 23%, 43mg).

### • Synthesis of compound (ai): according to the synthesis of compound (aj)

According to Scheme 4 Step 2: Compound **103** (120mg, 0.41 mmol), DIEA (0.40mL, 2.48mmol) in DMSO (0.1M), compound **158** (242mg, 0.62mmol). Reaction mixture stirred at 70°C for 24h to afford, after flash chromatography on silica gel (12g, SiO₂, DCM/EtOAc 100:0 → 0:100, 10 CV), a red foam (Yield of **compound (ai):** 18%, 40mg).

### • Synthesis of compound (aj):

### Step1. Synthesis of compound 1.11

According to Scheme 4 Step 1: At 0°C, to a solution of compound **309** (500mg, 2.04mmol, 1.0eq) in dry THF (0.2M) was added dropwise dry pyridine (1.1eq) followed by 2,2,2-trichloroethylchloroformate (0.31mL, 2.24mmol, 1.1eq). The reaction mixture was stirred at r.t. for 18h. Water was added, and the reaction mixture was extracted with EtOAc. The organic layers were combined, dried over MgSO₄, filtered and evaporated. The compound **1.11** was obtained as brown gum (900 mg, quantitative yield) and was used without purification in the next step.

### Step2. Synthesis of compound 1.4: compound (aj)

According to Scheme 4 Step 2: In a sealed tube containing compound **111** (210mg, 0.50mmol, 1.0eq), DIEA (6eq) in DMSO (0.1M) was added compound **1.11** (210mg, 0.50mmol, 1.0eq). The reaction mixture was stirred at 70°C for 18h. The residue was purified by 2 flash chromatography on silica gel (cyclohexane/EtOAc then EtOAc/MeOH) and a semi-preparative HPLC to afford expected **compound (aj)** as a white powder (Yield: 30%, 30mg).

### • Synthesis of compound (ak): according to the synthesis of compound (aj)

According to Scheme 4 Step 2: Compound **117** (250mg, 0.87mmol, 1.0eq), DIEA (1.5mL, 8.7mmol, 10.0eq) in DMSO (0.1M), compound **158** (305mg, 0.87mmol, 1.0eq). Mixture stirred at 70°C for 18h. A rose orange solid obtained after 2 flash chromatography (cyclohexane/EtOAc then EtOAc/MeOH) and semi-preparative HPLC (elution: water/ACN) (Yield of **compound (ak):** 8%, 39mg).

### • Synthesis of compound (al): according to the synthesis of compound (aj)

According to Scheme 4 Step 2: Compound **123** (209mg, 0.78mmol, 1.0eq), DIEA (1.3mL, 7.8mmol, 10.0eq) in DMSO (0.1M), compound **158** (305mg, 0.78mmol, 1.0eq). Mixture stirred at 70°C for 18h. A white solid obtained after 2 flash chromatography (cyclohexane/EtOAc then EtOAc/MeOH) and semi-preparative HPLC (elution: water/ACN) (Yield of **compound (al):** 26%, 102mg).

### • Synthesis of compound (am):

According to Scheme 4 Step 2: In a sealed containing tube compound **130** (180mg, 0.51mmol, 1.0eq) in ACN (0.1M) was added compound **158** (200mg, 0.51mmol, 1.0eq). The mixture was stirred at 70°C for 18h to afford after extraction, flash chromatography and semi-preparative HPLC (elution: water/ACN), the title compound as a white solid (Yield: 14%, 41mg).

### • Synthesis of compound (an): according to the synthesis of compound (am)

According to Scheme 4 Step 2: Compound **132** (102mg, 0.34mmol), DIEA (0.35mL, 2.03mmol) in ACN (0.1M), compound **158** (159mg, 0.40mmol). Reaction mixture stirred at 70°C for 18h to furnish, after filtration and purification by semi-preparative HPLC (elution: water/ACN) a pink powder (Yield of **compound (an):** 25%, 47mg).
¹H NMR (300 MHz, DMSO-d₆) δ 10.48 (s, 1H), 8.96 (s, 1H), 8.34 (s, 1H), 7.95 (d, J = 5.7 Hz, 1H), 7.70 (d, J = 8.8 Hz, 1H), 7.52-7.49 (m, 4H), 7.42-7.36 (m, 1H), 7.09 (d, J = 2.6 Hz, 1H), 6.91 (dd, J = 8.8, 2.7 Hz, 1H), 6.34 (s, 1H), 6.29 (d, J = 5.8 Hz, 1H), 2.90 (t, J = 7.3 Hz, 2H), 2.51 (t, J = 7.3 Hz, 2H), 2.40 (s, 3H), 1.26 (s, 9H).

### • Synthesis of compound (ao): according to the synthesis of compound (aj)

### Step1. Synthesis of compound 1.11

According to Scheme 4 Step 1: Compound **1.1** (500mg, 2.0mmol, 1.0eq) ) in dry THF (0.2M), dry pyridine (1.1 eq), 2,2,2-trichloroethylchloroformate (0.31mL, 2.2mmol, 1.1eq). Reaction mixture stirred at r.t. for 18h. Water added, and extraction with EtOAc. Organic layers combined, dried over MgSO₄, filtered and evaporated. Compound **1.11** obtained as an orange powder (900mg, quantitative yield).

### Step2. Synthesis of compound 1.4: compound (ao)

According to Scheme 4 Step 2: Compound **111** (175mg, 0.58mmol, 1.0eq) DIEA (6eq) in DMSO (0.1M), compound **1.11** (247mg, 0.58mmol, 1.0eq). Reaction mixture stirred at 70°C for 18h to afford, after 2 flash chromatography on silica gel (cyclohexane/EtOAc then EtOAc/MeOH) and a semi-preparative HPLC, a white powder (Yield of **compound (ao):** 8%, 26 mg).

### • Synthesis of compound (ap): according to the synthesis of compound (aj)

According to Scheme 4 Step2: Compound **140** (110mg, 0.38mmol), DIEA (0.40mL, 2.28mmol) in DMSO (0.1M), compound 158 (178mg, 0.45mmol). Reaction mixture stirred at 70°C for 24h to afford, after flash chromatography on silica gel (12g, SiO₂, cyclohexane/EtOAc 100:0→0:100, 10 CV) and a semi-preparative HPLC, a white powder (Yield of **compound (ap):** 20%, 42 mg).
¹H NMR (300 MHz, DMSO-d₆) δ 8.95 (s, 1H), 8.63 (qd, J = 4.7 Hz, 1H), 8.36 (d, J = 2.7 Hz, 1H), 8.33 (s, 1H), 7.99 (d, J = 8.7 Hz, 1H), 7.69 (d, J = 8.8 Hz, 1H), 7.52-7.50 (m, 4H), 7.46-7.37 (m, 2H), 7.12 (d, J = 2.7 Hz, 1H), 6.94 (dd, J = 8.8, 2.7 Hz, 1H), 6.34 (s, 1H), 2.78 (d, J = 4.8 Hz, 3H), 2.40 (s, 3H), 1.26 (s, 9H).

### • Synthesis of compound (aq): according to the synthesis of compound (aj)

According to Scheme 4 Step 2: Compound **142** (64mg, 0.22mmol), DIEA (0.23 mL, 1.32mmol) in DMSO (0.1M), compound **158** (86mg, 0.22mmol). Reaction mixture stirred at 70°C for 6h to afford, after flash chromatography on silica gel (12g, SiO₂, cyclohexane/EtOAc 100:0→0:100, 10 CV) and a semi-preparative HPLC, a white powder (Yield of **compound (aq):** 3%, 9mg).
¹H NMR (300 MHz, DMSO-d₆) δ 10.52 (s, 1H), 9.01 (s, 1H), 8.37 (s, 1H), 8.17 (d, *J* = 5.7 Hz, 1H), 7.75 (d, *J* = 8.8 Hz, 1H), 6.67 (d, *J* = 2.2 Hz, 1H), 7.55-7.53 (m, 4H), 7.44-7.39 (m, 1H), 7.13 (d, *J* = 2.7 Hz, 1H), 6.96 (dd, *J* = 8.8, 2.7 Hz, 1H), 6.65 (dd, *J* = 5.7, 2.3 Hz, 1H), 6.37 (s, 1H), 2.43 (s, 3H), 2.04 (s, 3H), 1.28 (s, 9H).

### • Synthesis of compound (ar): according to the synthesis of compound (aj)

### Step1. Synthesis of compound 1.11

According to Scheme 4 Step 1: Compound **1.1** (250mg, 1.16mmol, 1.0eq) in dry THF (0.2 M), dry pyridine (1.1eq), 2,2,2-trichloroethylchloroformate (0.18mL, 1.3mmol, 1.1eq). Reaction mixture stirred at r.t. for 18h. Water added, and extraction with EtOAc. Organic layers combined, dried over MgSO₄, filtered and evaporated. The compound **1.11** obtained as a brown gum (500mg).

### Step2. Synthesis of compound 1.4: compound (ar)

According to Scheme 4 Step 2: Compound **111** (150mg, 0.5mmol, 1.0eq), DIEA (6eq) in DMSO (0.1M) compound **1.11** (294mg, 0.75mmol, 1.5eq). Reaction mixture was stirred at 70°C for 18h to afford, after 2 flash chromatography on silica gel (cyclohexane/EtOAc then EtOAc/MeOH) and a semi-preparative HPLC, a brown powder (Yield of **compound (ar):** 3%, 7mg).

### • Synthesis of compound (as): according to the synthesis of compound (aj)

### Step1. Synthesis of compound 1.11

According to Scheme 4 Step 1: Compound **1.1** (500mg, 2.00mmol, 1.0eq) in dry THF (0.2M), dry pyridine (1.1eq), 2,2,2-trichloroethylchloroformate (0.31mL, 2.2mmol, 1.1eq). Reaction mixture stirred at r.t. for 18h. Water added, and extraction with EtOAc. Organic layers combined, dried over MgSO₄, filtered and evaporated. Compound **1.11** was obtained as brown gum (927mg, quantitative yield).

### Step2. Synthesis of compound 1.4: compound (as)

According to Scheme 4 Step 2: Compound **111** (150mg, 0.5mmol, 1.0eq), DIEA (6eq) in DMSO (0.1M), compound **1.11** (425mg, 1.0mmol, 2.0eq). Reaction mixture stirred at 70°C for 48h to afford, after 2 flash chromatography on silica gel (cyclohexane/EtOAc then EtOAc/MeOH) and a semi-preparative HPLC, a brown powder (Yield of **compound (as):** 4%, 11mg).

### • Synthesis of compound (at): according to the synthesis of compound (aj)

### Step1. Synthesis of compound 1.11

According to Scheme 4 Step 1: Compound **1.1** (500mg, 2.00mmol, 1.0eq) in dry THF (0.2M), dry pyridine (1.1eq), 2,2,2-trichloroethylchloroformate (0.31mL, 2.2mmol, 1.1eq). Reaction mixture was stirred at r.t. for 18h. Water added, and extraction with EtOAc. Organic layers combined, dried over MgSO₄, filtered and evaporated. Compound **1.11** obtained as brown gum (933mg, quantitative yield).

### Step2. Synthesis of compound 1.4: compound (at)

According to Scheme 4 Step 2: Compound **111** (150mg, 0.5mmol, 1.0eq), DIEA (6eq) in DMSO (0.1M), compound **1.11** (425mg, 1.0mmol, 2.0eq). Reaction mixture was stirred at 70°C for 48h to afford, after 2 flash chromatography on silica gel (cyclohexane/EtOAc then EtOAc/MeOH) and a semi-preparative HPLC, a brown powder (Yield of **compound (at):** 10%, 28mg).

### • Synthesis of compound (au): according to the synthesis of compound (aj)

According to Scheme 4 Step 2: Compound **144** (222mg, 0.63mmol), DIEA (1.10mL, 6.3mmol) in DMSO (0.1M), compound **158** (370mg, 0.95mmol). Reaction mixture stirred at 70°C for 6h to afford, after flash chromatography on silica gel (12g, SiO₂, cyclohexane/EtOAc 100:0→0:100, 10 CV) and a semi-preparative HPLC, a yellow powder (Yield of **compound (au):** 25%, 88mg).
¹H NMR (300 MHz, DMSO-d₆) δ 8.98 (s, 1H), 8.45 (d, J = 5.6 Hz, 1H), 8.37 (s, 1H), 8.26 (s, 1H), 7.75 (d, J = 8.8 Hz, 1H), 7.53-7.50 (m, 4H), 7.42-7.36 (m, 1H), 7.18 (d, J = 2.7 Hz, 1H), 7.01 (dd, J = 8.8, 2.7 Hz, 1H), 6.67 (d, J = 5.6 Hz, 1H), 6.35 (s, 1H), 3.64 (s, 3H), 2.42 (s, 3H), 1.26 (s, 9H).

### • Synthesis of compound (av): according to the synthesis of compound (aj)

### Step1. Synthesis of compound 1.11

According to Scheme 4 Step 1: Compound **1.1** (500mg, 2.04mmol, 1.0eq) in dry THF (0.2M), dry pyridine (1.1eq), 2,2,2-trichloroethylchloroformate (0.31mL, 2.24mmol, 1.1eq). Reaction mixture stirred at r.t. for 18h. Water added, and extraction with EtOAc. Organic layers combined, dried over MgSO₄, filtered and evaporated. Compound **1.11** obtained as a white solid (1.2g, quantitative yield).

### Step2. Synthesis of compound 1.4: compound (av)

According to Scheme 4 Step 2: Compound **111** (150mg, 0.5mmol, 1.0eq), DIEA (6eq) in DMSO (0.1M), compound **1.11** (563mg, 1.34mmol, 2.0eq). Reaction mixture stirred at 70°C for 48h to afford, after 2 flash chromatography on silica gel (cyclohexane/EtOAc then EtOAc/MeOH) and a semi-preparative HPLC, a white powder (Yield of **compound (av):** 13%, 34mg).

### • Synthesis of compound (aw): according to the synthesis of compound (aj)

### Step1. Synthesis of compound 1.11

According to Scheme 4 Step 1: Compound **1.1** (500mg, 2.04mmol, 1.0eq) in dry THF (0.2M), dry pyridine (1.1eq), 2,2,2-trichloroethylchloroformate (0.31mL, 2.24mmol, 1.1eq). Reaction mixture stirred at r.t. for 18h. Water added, and extraction with EtOAc. Organic layers combined, dried over MgSO₄, filtered and evaporated. Compound **1.11** obtained as white solid (1.2 g, quantitative yield).

### Step2. Synthesis of compound 1.4: compound (aw)

According to Scheme 4 Step 2: Compound **111** (150mg, 0.5mmol, 1.0eq), DIEA (6eq) in DMSO (0.1M), compound **1.11** (281mg, 0.67mmol, 1.5eq). Reaction mixture stirred at 70°C for 48h to afford, after 2 flash chromatography on silica gel (cyclohexane/EtOAc then EtOAc/MeOH) and a semi-preparative HPLC, a white powder (Yield of **compound (aw):** 25%, 63mg).

### • Synthesis of compound (ax): according to the synthesis of compound (d)

### Step1. Synthesis of compound 1.2

According to Scheme 1 Step1: Triphosgene (1.1eq), compound **1.1** (118mg, 0.55mmol, 1.1eq) in dry THF. Then NEt₃ (2.3eq) added and mixture heated at 80°C in a sealed tube for 2h. After cooling down, the solvent evaporated, and the residue dissolved in 10mL mixture of toluene and ACN (1/1).

### Step2. Synthesis of compound 1.4: compound (ax)

According to Scheme 1 Step 2: Addition of compound **111** (168mg, 0.50mmol, 1.0eq) to the above mixture. Mixture heated at 120°C for 3h. Purification by semi-preparative HPLC (Yield of **compound (ax):** 7%, 20mg).

### • Synthesis of compound (ay): according to the synthesis of compound (d)

### Step1. Synthesis of compound 1.2

According to Scheme 1 Step 1: Triphosgene (1.1eq), compound **1.1** (97mg, 0.55mmol, 1.1eq) in dry THF. Then NEt₃ (2.3eq) added and mixture heated at 80°C in a sealed tube for 2h. After cooling down, the solvent evaporated, and the residue dissolved in 10mL mixture of toluene and ACN (1/1).

### Step2. Synthesis of compound 1.4: compound (ay)

According to Scheme 1 Step 2: Addition of compound **111** (168mg, 0.50mmol, 1.0eq) to the above mixture. Mixture heated at 120°C for 3h. Purification by semi-preparative HPLC (Yield of **compound (ay):** 9%, 29mg).

### • Synthesis of compound (az): according to the synthesis of compound (d)

### Step1. Synthesis of compound 1.2

According to Scheme 1 Step 1: Compound **306** (126mg, 0.55mmol) in THF (5mL), triphosgene (163mg, 0.55mmol) in THF (2mL) at r.t.. Then NEt₃ (2.3eq) added and mixture heated at 80°C in a sealed tube for 2h. After cooling down, the solvent evaporated, and the residue dissolved in 10mL mixture of toluene and ACN (1/1).

### Step2. Synthesis of compound 1.4: compound (az)

According to Scheme 1 Step 2: Addition of compound **149** (161mg, 0.50mmol) to the above mixture. Mixture heated at 120°C for 3h. Purification by semi-preparative HPLC (Yield of **compound (az):** 8%, 23mg).
¹H NMR (300 MHz, DMSO-d*₆*) δ 12.95 (s, 1H), 8.98 (s, 1H), 8.44 (s, 1H), 8.37 (d, *J=* 5.6 Hz, 1H), 8.16 (d, *J =* 2.0 Hz, 1H), 7.84 (d, *J* = 8.9 Hz, 1H), 7.55 (d, *J =* 2.8 Hz, 1H), 7.51-7.30 (m, 5H), 6.67 (d, *J =* 5.6 Hz, 1H), 6.32 (s, 1H), 2.36 (s, 3H), 1.25 (s, 9H).

### • Synthesis of compound (ba): according to the synthesis of compound (aj)

According to Scheme 4 Step 2: Compound **155** (140mg, 0.41mmol), DIEA (0.7mL, 4.1mmol) in DMSO (0.1M), compound **157** (273mg, 0.82mmol). Reaction mixture stirred at 70°C for 24h to afford after flash chromatography on silica gel (12g, SiO₂, cyclohexane/EtOAc 100:0→0:100, 10 CV) and a semi-preparative HPLC a white powder (Yield of **compound (ba):** 5%, 18mg).
¹H NMR (300 MHz, DMSO-d₆) δ 12.93 (s, 1H), 10.72 (s, 1H), 9.30 (s, 1H), 8.25 (d, J = 5.7 Hz, 1H), 8.24 (s, 1H), 8.17 (d, J = 8.6 Hz, 1H), 8.06 (d, J = 8.3 Hz, 1H), 7.86 (d, J = 7.9 Hz, 1H), 7.72-7.55 (m, 2H), 7.40 (d, J = 8.4 Hz, 1H), 7.09 (s, 1H), 6.40 (d, J = 5.6 Hz, 1H), 1.31 (s, 9H).

### • Synthesis of compound (bb): according to the synthesis of compound (aj)

According to Scheme 4 Step 2: Compound **155** (140mg, 0.41mmol), DIEA (0.7ml, 4.1mmol) in DMSO (0.1M), compound **98** (373mg, 0.84mmol). Reaction mixture stirred at 70°C for 24h to afford after flash chromatography on silica gel (12g, SiO₂, cyclohexane/EtOAc 100:0→80:20, 10 CV) and a semi-preparative HPLC a white powder (Yield of **compound (bb):** 21%, 54mg).
¹H NMR (300 MHz, DMSO-d*₆*) δ 12.92 (s, 1H), 9.12 (s, 1H), 8.97 (s, 1H), 8.95 (d, *J=* 5.9 Hz, 1H), 8.46 (d, *J =* 5.7 Hz, 1H), 8.24-8.19 (m, 3H), 8.15 (d, *J =* 8.6 Hz, 1H), 8.03-8.00 (m, 2H), 7.90 (d, *J =* 8.3 Hz, 1H), 7.80 (d, *J =* 8.3 Hz, 1H), 7.63-7.54 (m, 3H), 7.35 (d, *J =* 8.3 Hz, 1H), 6.48 (s, 1H), 6.35 (d, *J =* 5.7 Hz, 1H), 1.31 (s, 9H).

### • Synthesis of compound (bc): according to the synthesis of compound (aj)

According to Scheme 4 Step 2: Compound **164** (200mg, 0.7mmol, 1.0eq), DIEA (1.2mL, 7.0mmol, 10.0eq) in DMSO (0.1M), compound **158** (547mg, 1.4mmol, 2.0eq). Reaction mixture stirred at 70°C for 48h. A pale brown solid was obtained after 2 flash chromatography (cyclohexane/EtOAc then EtOAc/MeOH) and semi-preparative HPLC (Yield of **compound (bc):** 6%, 23mg).

### • Synthesis of compound (bd): according to the synthesis of compound (ac)

### Step1. Synthesis of compound 1.10

According to Scheme 3 Step 1: Triphosgene (0.130g, 0.44mmol), compound **111** (150mg, 0.44mmol, 1.0eq), Et₃N (0.075mL, 0.542mmol), DCM (5mL).

### Step 2. Synthesis of compound 1.4: compound (bd)

According to Scheme 3 Step 2: Then a solution of compound **166** (91mg, 0.4mmol, 0.9eq) in CH₂Cl₂ (5mL) was added dropwise at 0°C. Reaction mixture stirred at r.t. for 20h. Residue purified by semi-preparative HPLC to afford, after basic extraction, a white powder (Yield of **compound (bd):** 16%, 40mg).

### • Synthesis of compound (be):

NaBH₄ (15mg, 0.41mmol) was added to a solution of **compound (an)** (45mg, 0.08mmol) in dry THF (2mL). The mixture was cooled at 0°C under argon before addition of BF₃.Et₂O 47% (0.15mL, 0.58mmol). The reaction mixture was stirred at r.t. for 3h. Then, aq. sat. NH₄Cl (20mL) and EtOAc (30mL) were added. The aqueous layer was extracted with EtOAc (2x20mL). The combined organic fractions were dried over MgSO₄, filtered and concentrated in vacuo. The crude obtained was purified by semi-preparative HPLC leading to **compound (be)** (Yield: 52%, 22mg) as a white solid.
¹H NMR (300 MHz, DMSO-d*₆*) δ 8.92 (s, 1H), 8.30 (s, 1H), 7.63 (d, *J =* 5.6 Hz, 1H), 7.62 (d, *J =* 8.8 Hz, 1H), 7.52-7.50 (m, 4H), 7.53-7.49 (m, 1H), 6.99 (d, *J* = 2.7 Hz, 1H), 6.80 (dd, J = 8.8, 2.7 Hz, 1H), 6.49 (s, 1H), 6.33 (s, 1H), 5.81 (d, *J* = 5.7 Hz, 1H), 3.22 (br s, 2H), 2.62 (t, *J =* 6.1 Hz, 2H), 2.39 (s, 3H), 1.79-1.71 (m, 2H), 1.25 (s, 9H).

### • Synthesis of compound (bf): according to the synthesis of compound (aj)

According to Scheme 4 Step 2: Compound **167** (115mg, 0.40mmol), DIEA (0.40mL, 2.35mmol), DMSO (0.1M), compound **98** (340mg, 0.78mmol). Reaction mixture stirred at 70°C for 18h to afford after flash chromatography on silica gel (12g, SiO₂, cyclohexane/EtOAc 100:0→80:20, 5 CV) and a semi-preparative HPLC a white powder (Yield of **compound (bf):** 20%, 48mg).
¹H NMR (300 MHz, DMSO-d₆) δ 9.14 (s, 1H), 9.04 (s, 1H), 8.94 (dd, J = 4.2, 1.6 Hz, 1H), 8.75 (qd, J = 4.9 Hz, 1H), 8.51-8.48 (m, 2H), 8.22-8.17 (m, 2H), 8.04-7.98 (m, 2H), 7.93 (d, J = 8.3 Hz, 1H), 7.79-7.76 (m, 1H), 7.63-7.53 (m, 3H), 7.37 (d, J = 8.3 Hz, 1H), 7.29 (d, J = 2.6 Hz, 1H), 7.17 (dd, J = 5.6, 2.6 Hz, 1H), 6.49 (s, 1H), 2.72 (d, J = 4.8 Hz, 3H), 1.31 (s, 9H).

### • Synthesis of compound (bg): according to the synthesis of compound (ac)

### Step1. Synthesis of compound 1.10

According to Scheme 3 Step 1: Triphosgene (0.130g, 0.44mmol), compound **111** (150mg, 0.44mmol, 1.0eq), Et₃N (0.075mL, 0.542mmol), DCM (5mL).

### Step 2. Synthesis of compound 1.4: compound (bg)

According to Scheme 3 Step 2: Then a solution of compound **169** (101mg, 0.48mmol, 1.1eq) in DCM (5mL) was added dropwise at 0°C. Reaction mixture stirred at r.t. for 20h. Residue purified by semi-preparative HPLC to afford, after basic extraction, an off-white powder (Yield of **compound (bg):** 19%, 45mg).

### • Synthesis of compound (bh): according to the synthesis of compound (ac)

### Step1. Synthesis of compound 1.10

According to Scheme 3 Step 1: Triphosgene (0.130g, 0.44mmol), compound **111** (150mg, 0.44mmol, 1.0eq), Et₃N (0.075mL, 0.542mmol), DCM (5mL).

### Step 2. Synthesis of compound 1.4: compound (bh)

According to Scheme 3 Step 2: Then a solution of compound **171** (128mg, 0.48mmol, 1.1eq) in DCM (5mL) was added dropwise at 0°C. Reaction mixture was stirred at r.t. for 20h. The residue purified by semi-preparative HPLC to afford, after basic extraction, a yellow powder (Yield of **compound (bh):** 30%, 77mg).

### • Synthesis of compound (bi): according to the synthesis of compound (d)

According to Scheme 1 Step 2: Compound **173** (192mg, 0.50mmol) in ACN (5mL) and toluene (5mL), was added compound **54** (184mg, 0.55 mmol). Reaction mixture stirred at 80°C for 18h. Purification by semi-preparative HPLC leading to a brown solid (Yield of **compound (bi):** 6%, 19mg).
¹H NMR (300 MHz, DMSO-d₆) δ 9.79 (s, 1H), 8.99 (s, 1H), 8.37 (s, 1H), 8.09 (d, J = 5.7 Hz, 1H), 7.73 (d, J = 8.8 Hz, 1H), 7.53-7.51 (m, 4H), 7.42-7.37 (m, 1H), 7.35 (d, J = 2.2 Hz, 1H), 7.12 (d, J = 2.7 Hz, 1H), 6.96 (dd, J = 8.8, 2.7 Hz, 1H), 6.57 (dd, J = 5.7, 2.3 Hz, 1H), 6.35 (s, 1H), 2.41 (s, 3H), 1.40 (s, 9H), 1.26 (s, 9H).

### • Synthesis of compound (bj):

**Compound (bi)** (94mg, 0.16mmol) was treated with 4N HCl in dioxane (5mL) at r.t. for 4h. The solvent was removed under vacuum leading to the expected **compound (bj)** after lyophilization (84mg, quantitative yield) as a yellow foam.
¹H NMR (300 MHz, DMSO-d₆) δ 13.09 (br s, 1H), 9.08 (s, 1H), 8.44 (s, 1H), 7.92 (d, J = 7.2 Hz, 1H), 7.79-7.75 (m, 2H), 7.53-7.48 (m, 4H), 7.43-7.36 (m, 1H), 7.20 (d, J = 2.7 Hz, 1H), 7.06 (dd, J = 8.8, 2.7 Hz, 1H), 6.64 (dd, J = 7.2, 2.5 Hz, 1H), 6.34 (s, 1H), 6.09 (d, J = 2.5 Hz, 1H), 2.43 (s, 3H), 1.26 (s, 9H).

### • Synthesis of compound (bk): according to the synthesis of compound (aj)

According to Scheme 4 Step 2: Compound **178** (333mg, 1.0mmol), DIEA (1.74mL, 10.0mmol), DMSO (0.1M), compound **158** (781mg, 2.0mmol). Reaction mixture stirred at 70°C for 18h to afford after flash chromatography on silica gel (40g, SiO₂, cyclohexane/EtOAc 100:0→80:20, 10 CV) and a semi-preparative HPLC a white powder (Yield of **compound (bk):** 3%, 125mg).
¹H NMR (300 MHz, DMSO-d₆) δ 12.90 (s, 1H), 8.64 (s, 1H), 8.35-8.30 (m, 2H), 8.16 (s, 1H), 7.58 (d, J = 8.8 Hz, 1H), 7.55-7.48 (m, 4H), 7.41-7.36 (m, 1H), 7.11 (d, J = 2.8 Hz, 1H), 6.99 (dd, J = 8.7, 2.8 Hz, 1H), 6.50 (d, J = 5.7 Hz, 1H), 6.33 (s, 1H), 3.10-3.01 (m, 1H), 1.25 (s, 9H), 1.12 (d, J = 6.8 Hz, 6H).

### • Synthesis of compound (bl): according to the synthesis of compound (am)

According to Scheme 4 Step 2: Compound **158** (475mg, 1.21mmol, 1.5eq), 4-(4-pyridyloxy)aniline (150mg, 0.81mmol, 1.0eq), DIEA (1.74mL, 10.0mmol) in acetonitrile (0.1M). Reaction mixture stirred at 70°C for 3h. Mixture was directly absorbed on silica gel to be purified by flash chromatography using cyclohexane and EtOAc to afford a white foam after lyophilization (Yield of **compound (bl):** 45%, 156mg).

### • Synthesis of compound (bm): according to the synthesis of compound (ac)

### Step1. Synthesis of compound 1.10

According to Scheme 3 Step 1: Triphosgene (0.148g, 0.50mmol), compound **111** (169mg, 0.50mmol, 1.0eq), Et₃N (0.085mL, 0.616mmol), DCM (5mL).

### Step 2. Synthesis of compound 1.4: compound (bm))

According to Scheme 3 Step 2: Then a solution of compound **27** (147mg, 0.55mmol, 1.1eq) in DCM (5mL) was added dropwise at 0°C. Reaction mixture was stirred at r.t. for 18h. The residue was purified by semi-preparative HPLC. Fractions collected and evaporated, and the residue triturated in DCM and filtered to afford a yellow solid (Yield of **compound (bm):** 4%, 12mg).

### • Synthesis of compound (bn): according to the synthesis of compound (ac)

### Step1. Synthesis of compound 1.10

According to Scheme 3 Step 1: Triphosgene (0.130g, 0.44mmol), compound **111** (150mg, 0.44mmol, 1.0eq), Et₃N (0.075mL, 0.542mmol), DCM(5mL).

### Step 2. Synthesis of compound 1.4: compound (bn))

According to Scheme 3 Step 2: Then a solution of compound **180** (107mg, 0.48mmol, 1.1eq) in DCM (5mL) was added dropwise at 0°C. Reaction mixture stirred at r.t. for 18h. The residue purified by semi-preparative HPLC. Fractions collected and evaporated, and the residue triturated in DCM and filtered to afford a yellow solid (Yield of **compound (bn):** 10%, 23mg).

### • Synthesis of compound (bo): according to the synthesis of compound (aj)

According to Scheme 4 Step 2: Compound **186** (150mg, 0.46mmol, 1.0eq), DIEA (0.48mL, 2.75mmol, 6.0eq), DMSO (0.1M), compound **158** (179mg, 0.46mmol, 1.0eq). Reaction mixture stirred at 70°C for 20h. A white solid was obtained after 2 flash chromatography (cyclohexane/EtOAc then EtOAc/MeOH) and semi-preparative HPLC (Yield of **compound (bo):** 14%, 34mg).

### • Synthesis of compound (bp):

To a solution of **compound (bj)** (84mg, 0.1 mmol) and Boc-Sat-OH (39mg, 0.21mmol) in dry DMF (5mL) were added DIEA (0.05mL, 0.27mmol) followed by HATU (79mg, 0.21mmol). The reaction mixture was stirred at r.t. for 48h. Then sat. aq. NaHCO₃ (40mL) and EtOAc (40mL) were added. The aqueous layer was extracted with EtOAc (2 x 20mL). The combined organic fractions were dried over MgSO₄, filtered and concentrated in vacuo. The crude obtained was purified by flash chromatography (12g, SiO₂, cyclohexane/EtOAc 100:0→0:100, 15 CV) to furnish the **compound (bp)** (Yield: 23%, 25mg) as a yellow oil.
¹H NMR (300 MHz, DMSO-d₆) δ 10.57 (d, J = 6.7 Hz, 1H), 9.00 (s, 1H), 8.36 (s, 1H), 8.17 (d, J = 5.7 Hz, 1H), 7.73 (d, J = 8.8 Hz, 1H), 7.62 (s, 1H), 7.53-7.51 (m, 4H), 7.42-7.37 (m, 1H), 7.12 (d, J = 2.5 Hz, 1H), 6.95 (dd, J = 8.8, 2.7 Hz, 1H), 6.66 (s, 1H), 6.35 (s, 1H), 3.96 (d, J = 10.2 Hz, 2H), 2.81 and 2.78 (2s, 3H), 2.40 (s, 3H), 1.37 and 1.27 (2s, 18H).

### • Synthesis of compound (bq):

**Compound (bp)** (18mg, 27µmol) was treated with 4N HCl in dioxane (5mL) and the reaction mixture was stirred at r.t. for 2h. The solvent was removed under vacuum leading to the expected **compound (bq)** after lyophilization (Yield: 10mg, 58%) as a brown gum.
¹H NMR (300 MHz, DMSO-d₆) δ 11.15 (s, 1H), 9.10 (s, 1H), 8.91 (s, 1H), 8.44 (s, 1H), 8.22 (d, J = 5.8 Hz, 1H), 7.70 (d, J = 8.8 Hz, 1H), 7.55-7.48 (m, 5H), 7.42-7.39 (m, 1H), 7.14 (d, J = 2.7 Hz, 1H), 6.97 (dd, J = 8.8, 2.6 Hz, 1H), 6.79 (dd, J = 5.8, 2.4 Hz, 1H), 6.33 (s, 1H), 3.90 (br s, 2H), 2.55 and 2.53 (2s, 3H), 2.41 (s, 3H), 1.26 (s, 9H).

### • Synthesis of compound (br): according to the synthesis of compound (aj)

According to Scheme 4 Step 2: Compound **103** (120mg, 0.41mmol), DIEA (0.40mL, 2.48mmol), DMSO (0.1M), compound **158** (242mg, 0.62mmol). Reaction mixture stirred at 70°C for 18h to afford after flash chromatography on silica gel (12g, SiO₂, cyclohexane/EtOAc 100:0→0:100, 10 CV) a red foam (Yield of **compound (br):** 18%, 40mg).
¹H NMR (300 MHz, DMSO-d*₆*) δ 9.01 (s, 1H), 8.76 (qd, *J =* 4.9 Hz, 1H), 8.49 (d, *J =* 5.6 Hz, 1H), 8.38 (s, 1H), 7.76 (d, *J =* 8.8 Hz, 1H), 7.53-7.51 (m, 4H), 7.42-7.37 (m, 2H), 7.17 (d, *J =* 2.7 Hz, 1H), 7.13 (dd, J = 5.6, 2.6 Hz, 1H), 7.00 (dd, *J* = 8.8, 2.7 Hz, 1H), 6.35 (s, 1H), 2.77 (d, *J* = 4.8 Hz, 3H), 2.41 (s, 3H), 1.26 (s, 9H).

### • Synthesis of compound (bs): according to the synthesis of compound (aj)

According to Scheme 4 Step 2: Compound **188** (150mg, 0.47mmol), DIEA (0.80mL, 4.68mmol), DMSO (0.1M), compound **158** (365mg, 0.94mmol). Reaction mixturestirred at 70°C for 24h to afford after flash chromatography on silica gel (40g, SiO₂, cyclohexane/EtOAc 100:0→90:10, CV) a beige powder (Yield of **compound (bs):** 23%, 57mg).
¹H NMR (300 MHz, DMSO-d*₆*) δ 9.17 (d, *J=* 1.7 Hz, 1H), 9.05 (d, *J*= 1.7 Hz, 1H), 9.01 (s, 1H), 8.93 (d, *J* = 5.2 Hz, 1H), 8.41 (s, 1H), 7.78 (d, *J* = 8.8 Hz, 1H), 7.534-7.52 (m, 4H), 7.43-7.37 (m, 1H), 7.27 (d, *J =* 2.6 Hz, 1H), 7.09 (dd, *J* = 8.8, 2.6 Hz, 1H), 6.97 (d, *J =* 5.2 Hz, 1H), 6.36 (s, 1H), 2.42 (s, 3H), 1.26 (s, 9H).

### • Synthesis of compound (bt): according to the synthesis of compound (aj)

According to Scheme 4 Step 2: Compound **195** (150mg, 0.46mmol, 1.0eq), DIEA (0.48mL, 2.75mmol, 6.0eq), DMSO (0.1M), compound **158** (179mg, 0.46mmol, 1.0eq). Reaction mixture stirred at 70°C for 20h. A white solid was obtained after 2 flash chromatography (cyclohexane/EtOAc then EtOAc/MeOH) and semi-preparative HPLC (Yield of **compound (bt):** 12%, 28mg).

### • Synthesis of compound (bu): according to the synthesis of compound (aj)

According to Scheme 4 Step 2: Compound **199** (160mg, 0.53mmol), DIEA (0.93ml, 5.3mmol), DMSO (0.1M), compound **158** (418mg, 1.1mmol). Reaction mixture stirred at 70°C for 18h to afford after flash chromatography on silica gel (40g, SiO₂, cyclohexane/EtOAc 100:0→90:10, 10 CV) a yellow solid (Yield of **compound (bu):** 23%, 66mg).
¹H NMR (300 MHz, DMSO-d*₆*) δ 12.51 (s, 1H), 8.87 (s, 1H), 8.26 (s, 1H), 8.10 (s, 1H), 7.56-7.41 (m, 6H), 7.40-7.35 (m, 1H), 7.06 (d, *J =* 7.4 Hz, 1H), 6.99 (d, *J =* 2.8 Hz, 1H), 6.78-6.70 (m, 2H), 6.32 (s, 1H), 2.37 (s, 3H), 1.25 (s, 9H).

### • Synthesis of compound (bv): according to the synthesis of compound (aj)

According to Scheme 4 Step 2: Compound **203** (81mg, 0.24mmol), DIEA (0.25mL, 1.44mmol), DMSO (0.1M), compound **158** (94mg, 0.24mmol). Reaction mixture stirred at 80°C for 18h to afford after flash chromatography on silica gel (12g, SiO₂, cyclohexane/EtOAc 100:0→90:10, 5 CV) a yellow solid (Yield of **compound (bv):** 31%, 40mg).
¹H NMR (300 MHz, DMSO-d₆) δ 13.28 (s, 1H), 8.99 (s, 1H), 8.52 (d, J = 3.4 Hz, 1H), 8.38 (s, 1H), 8.23 (s, 1H), 7.71 (d, J = 8.8 Hz, 1H), 7.53 (d, J = 4.3 Hz, 4H), 7.44-7.38 (m, 1H), 7.22 (d, J = 2.6 Hz, 1H), 7.06 (dd, J = 8.8, 2.6 Hz, 1H), 6.37 (s, 1H), 2.42 (s, 3H), 1.28 (s, 9H).

### • Synthesis of compound (bw): according to the synthesis of compound (ac)

### Step1. Synthesis of compound 1.10

According to Scheme 3 Step 1: Triphosgene (0.130g, 0.44mmol), compound **111** (150mg, 0.44mmol, 1.0eq), Et₃N (0.075mL, 0.542mmol), DCM (5mL).

### Step 2. Synthesis of compound 1.4: compound (bw)

According to Scheme 3 Step 2: Then a solution of 5-tert-butyl-2-(3-fluorophenyl)pyrazol-3-amine hydrochloride (132mg, 0.48mmol, 1.1eq) and NEt₃ (0.93mL, 0.7mmol, 1.5eq) in toluene (3mL) was added dropwise at 0°C. Mixture stirred in a sealed tube at 80°C for 20h, then at 100°C for 20h. After cooling down, the solvent was evaporated and the residue was purified by semi-preparative HPLC to afford a white powder (Yield of **compound (bw):** 8%, 21mg).

### • Synthesis of compound (bx): according to the synthesis of compound (ac)

### Step1. Synthesis of compound 1.10

According to Scheme 3 Step 1: Triphosgene (0.130g, 0.44mmol), compound **111** (150mg, 0.44mmol, 1.0eq), Et₃N (0.075mL, 0.542mmol), DCM (5mL).

### Step 2. Synthesis of compound 1.4: compound (bx)

According to Scheme 3 Step 2: Then a solution of compound **272** (69mg, 0.49 mmol, 1.1eq) in toluene (3mL) added dropwise at 0°C. Mixture stirred in a sealed tube at 80°C for 20h. Then solid filtrated, rinsed with EtOAc and MeOH and purified by semi-preparative HPLC to afford a white solid (Yield of **compound (bx):** 9%, 19mg).

### • Synthesis of compound (by): according to the synthesis of compound (aj)

According to Scheme 4 Step 2: Compound **132** (95mg, 0.31mmol), DIEA (0.30mL, 1.89mmol), DMSO (0.1M), compound **307** (191mg, 0.47mmol). Reaction mixture stirred at 70°C for 24h to afford after filtration and semi-preparative HPLC a white solid (Yield of **compound (by):** 21%, 37mg).
¹H NMR (300 MHz, DMSO-d₆) δ 10.49 (s, 1H), 8.91 (s, 1H), 8.35 (s, 1H), 7.95 (d, J = 5.7 Hz, 1H), 7.70 (d, J = 8.8 Hz, 1H), 7.38 (d, J = 8.3 Hz, 2H), 7.31 (d, J = 8.5 Hz, 2H), 7.09 (d, J = 2.6 Hz, 1H), 6.91 (dd, J = 8.8, 2.6 Hz, 1H), 6.32 (s, 1H), 6.29 (d, J = 5.8 Hz, 1H), 2.90 (t, J = 7.4 Hz, 2H), 2.51 (t, J = 7.4 Hz, 2H), 2.40 (s, 3H), 2.35 (s, 3H), 1.25 (s, 9H).

### • Synthesis of compound (bz): according to the synthesis of compound (aj)

### Step1. Synthesis of compound 1.11

According to Scheme 4 Step 1: Amine **1.1** (500mg, 2.0mmol, 1.0eq), dry THF (0.2M), dry pyridine (1.1eq), 2,2,2-trichloroethylchloroformate (0.31mL, 2.2mmol, 1.1eq). Reaction mixture stirred at r.t. for 18h. Water added, and the reaction mixture extracted with EtOAc. Organic layers were combined, dried over MgSO₄, filtered and evaporated. Compound **1.11** was obtained as a brown gum (0.927g, quantitative yield).

### Step2. Synthesis of compound 1.4: compound (bz)

According to Scheme 4 Step 2: Compound **132** (120mg, 0.40mmol), DIEA (0.40mL, 2.40mmol), DMSO (0.1M), compound **1.11** (245mg, 0.60mmol). Reaction mixture stirred at 70°C for 18h to furnish after filtration a white solid (Yield of **compound (bz):** 67%, 155mg).
¹H NMR (300 MHz, DMSO-d₆) δ 10.49 (s, 1H), 8.99 (s, 1H), 8.33 (s, 1H), 7.95 (d, J = 5.8 Hz, 1H), 7.65 (d, J = 8.8 Hz, 1H), 7.60 (s, 1H), 7.54-7.52 (m, 2H), 7.48-7.42 (m, 1H), 7.09 (d, J = 2.7 Hz, 1H), 6.88 (dd, J = 8.8, 2.6 Hz, 1H), 6.35 (s, 1H), 6.29 (d, J = 5.7 Hz, 1H), 2.90 (t, J = 8.0 Hz, 2H), 2.51 (t, J = 8.0 Hz, 2H), 2.41 (s, 3H), 1.26 (s, 9H).

### • Synthesis of compound (ca): according to the synthesis of compound (ac)

### Step1. Synthesis of compound 1.10

According to Scheme 3 Step 1: Triphosgene (0.130g, 0.44mmol), compound **111** (150mg, 0.44mmol, 1.0eq), Et₃N (0.075mL, 0.542mmol), DCM (5mL).

### Step 2. Synthesis of compound 1.4: compound (ca)

According to Scheme 3 Step 2: Compound **156** (77mg, 0.49mmol, 1.1eq) in toluene (3mL) was added dropwise at 0°C. The mixture was stirred in a sealed tube at 80°C for 20h. The solid was filtrated, rinsed with EtOAc and MeOH and purified by semi-preparative HPLC to afford a beige solid (Yield of **compound (ca):** 28%, 61mg).

### • Synthesis of compound (cb): according to the synthesis of compound (aj)

According to Scheme 4 Step 2: Compound **132** (95mg, 0.31mmol), DIEA (0.30mL, 1.90mmol) in DMSO (0.1 M), compound **157** (160mg, 0.47mmol). Reaction mixture stirred at 70°C for 24h to furnish after filtration a beige powder (Yield of **compound (cb):** 76%, 115mg).
¹H NMR (300 MHz, DMSO-d₆) δ 10.91 (br s, 1H), 10.49 (s, 1H), 8.58 (br s, 1H), 7.96 (d, J = 5.8 Hz, 1H), 7.84 (d, J = 8.8 Hz, 1H), 7.14 (d, J = 2.7 Hz, 1H), 7.05 (s, 1H), 6.96 (dd, J = 8.8, 2.7 Hz, 1H), 6.32 (d, J = 5.8 Hz, 1H), 2.91 (t, J = 7.4 Hz, 2H), 2.51 (t, J = 8.1 Hz, 2H), 2.44 (s, 3H), 1.29 (s, 9H).

### • Synthesis of compound (cc): according to the synthesis of compound (aj)

According to Scheme 4 Step 2: Compound **111** (200mg, 0.59mmol, 1.0eq) DIEA (6eq), DMSO (0.1M), compound 206 (490mg, 1.18mmol, 2.0eq). Reaction mixture stirred at 70°C for 18h The residue was evaporated and purified by semi-preparative HPLC followed by trituration in MeOH to afford an orange solid (Yield of **compound** (cc): 10%, 33mg).

### • Synthesis of compound (cd): according to the synthesis of compound (aj)

According to Scheme 4 Step 2: Compound **111** (150mg, 0.45mmol, 1.0eq), DIEA (6eq), DMSO (0.1M), compound **209** (373mg, 0.89mmol, 2.0eq). Reaction mixture stirred at 70°C for 18h. Concentration and purification by 2 flash chromatography using cyclohexane and EtOAc and then EtOAc and MeOH. A semi-preparative HPLC was the performed to obtain a beige powder (Yield of **compound (cd):** 7%, 18mg).

### • Synthesis of compound (ce): according to the synthesis of compound (aj)

According to Scheme 4 Step 2: Compound **111** (265mg, 0.79mmol, 1.0eq), DIEA (6eq), DMSO (0.1M), compound **211** (612mg, 1.18mmol, 1.5eq). Reaction mixture stirred at 70°C for 18h. Concentration and purification by flash chromatography over silica gel using cyclohexane and EtOAc and then EtOAc and MeOH. The compound obtained was purified by semi-preparative HPLC to afford, after lyophilization, a yellow foam (Yield of **compound (ce):** 3%, 13mg).

### • Synthesis of compound (cf): according to the synthesis of compound (aj)

According to Scheme 4 Step 2: Compound **111** (262mg, 0.78mmol, 1.0eq), DIEA (6eq), DMSO (0.1M), compound **213** (606mg, 1.16mmol, 1.5eq). Reaction mixture stirred at 70°C for 18h. Concentration and purification by flash chromatography over silica gel using cyclohexane and EtOAc and then EtOAc and MeOH. The compound obtained was purified by semi-preparative HPLC to afford, after lyophilization, a yellow solid (Yield of **compound (cf):** 2%, 11mg).

### • Synthesis of compound (ch):

A mixture of 2-dimethylaminoethylchloride hydrochloride (98mg, 0.68mmol) and NEt₃ (0.14mL, 1.02mmol) in dry DMF (2.5mL) was added to a solution of **compound (bi)** (100mg, 0.17mmol) and NaH (41mg, 1.02mmol) in dry DMF (2.5mL). The reaction mixture was stirred at r.t. for 20h. Then water (20mL) and EtOAc (40mL) were added. The aqueous layer was extracted with EtOAc (2x20mL). The combined organic fractions were dried over MgSO₄, filtered and concentrated in vacuo. The crude obtained was purified by flash chromatography (12g, SiO₂, cyclohexane/EtOAc 100:0→0:100, 10 CV and EtOAc/MeOH 100:0→85:15, 5 CV) to furnish **compound (ch)** (Yield: 39%, 44mg) as a white solid.
¹H NMR (300 MHz, DMSO-d₆) δ 8.99 (s, 1H), 8.38 (s, 1H), 8.25 (d, J = 5.7 Hz, 1H), 7.75 (d, J = 8.8 Hz, 1H), 7.54-7.52 (m, 4H), 7.44-7.39 (m, 1H), 7.12 (dd, J = 8.4, 2.7 Hz, 2H), 6.97 (dd, J = 8.8, 2.7 Hz, 1H), 6.73 (dd, J = 5.7, 2.3 Hz, 1H), 6.36 (s, 1H), 3.92 (t, J = 6.9 Hz, 2H), 2.43 (s, 3H), 2.41-2.39 (m, 2H), 2.13 (s, 6H), 1.38 (s, 9H), 1.28 (s, 9H).

### • Synthesis of compound (ci): according to the synthesis of compound (aj)

According to Scheme 4 Step 2: Compound **132** (214mg, 0.71mmol), DIEA (0.37mL, 2.14mmol), DMSO (0.1M), compound **158** (278mg, 0.71mmol). Reaction mixture stirred at 80°C for 18h to furnish, after filtration and semi-preparative HPLC a white solid (Yield of **compound (ci):** 18%, 68mg).
¹H NMR (300 MHz, DMSO-d₆) δ 10.22 (s, 1H), 8.87 (s, 1H), 8.26 (s, 1H), 7.56 (d, J = 8.8 Hz, 1H), 7.53-7.51 (m, 4H), 7.43-7.38 (m, 1H), 7.13 (t, J = 8.1 Hz, 1H), 6.93 (d, J = 2.7 Hz, 1H), 6.70-6.67 (m, 2H), 6.49 (d, J = 7.5 Hz, 1H), 6.34 (s, 1H), 2.81 (t, J = 7.1 Hz, 2H), 2.43 (t, J = 7.1 Hz, 2H), 2.38 (s, 3H), 1.27 (s, 9H).

### • Synthesis of compound (cj): according to the synthesis of compound (ac)

### Step1. Synthesis of compound 1.10

According to Scheme 3 Step 1: Triphosgene (0.130g, 0.44mmol), compound **111** (150mg, 0.44mmol, 1.0eq), Et₃N (0.075mL, 0.542mmol), DCM (5mL).

### Step 2. Synthesis of compound 1.4: compound (cj)

According to Scheme 3 Step 2: Then a solution 5-tert-1 ,3,4-oxadiazol-2-amine (69mg, 0.49mmol, 1.1eq) in toluene (3mL) was added dropwise at 0°C. The mixture was stirred in a sealed tube at 100°C for 2 days. The solid was filtrated, rinsed with diethyl ether and purified by flash chromatography on silica gel using cyclohexane and EtOAc and then EtOAc and MeOH to afford a white solid (Yield of **compound (cj):** 10%, 23mg).

### • Synthesis of compound (ck):

**Compound (ch)** (20mg, 32µmol) was treated with 4N HCl in dioxane (3mL) and the reaction mixture was stirred at r.t. for 3h. Then the solvent was removed under vacuum leading to the expected **compound (ck)** after lyophilization (Yield: 88%, 1 mg) as a beige foam.
¹H NMR (300 MHz, DMSO-d₆) δ 10.17 (br s, 1H), 9.10 (s, 1H), 8.47 (s, 1H), 7.97 (d, J = 7.1 Hz, 1H), 7.78 (d, J = 8.8 Hz, 1H), 7.54-7.53 (m, 4H), 7.43-7.39 (m, 1H), 7.19 (d, J = 8.4, 2.6 Hz, 1H), 7.04 (d, J = 8.8 Hz, 1H), 6.66 (br s, 1H), 6.35 (s, 1H), 6.23 (br s, 1H), 3.78-3.73 (m, 2H), 3.29-3.24 (m, 2H), 2.80 (s, 6H), 2.45 (s, 3H), 1.28 (s, 9H).

### • Synthesis of compound (cl): according to the synthesis of compound (aj)

According to Scheme 4 Step 2: Compound **111** (150mg, 0.45mmol, 1.0eq), DIEA (6eq), DMSO (0.1M), compound **216** (391mg, 0.89mmol, 2.0eq). Reaction mixture stirred at 70°C for 18h. The residue was evaporated and purified by semi-preparative HPLC. The fractions were evaporated and triturated twice in MeOH to afford a beige powder (Yield of **compound (cl):** 8%, 21mg).

### • Synthesis of compound (cm): according to the synthesis of compound (aj)

According to Scheme 4 Step 2: Compound **111** (150mg, 0.45mmol, 1.0eq), DIEA (6eq), DMSO (0.1M), compound **219** (395mg, 0.89mmol, 2.0eq). Reaction mixture stirred at 70°C for 18h. The reaction mixture evaporated and triturated in hot MeOH. Solid obtained filtered and purified by semi-preparative HPLC. The fractions were evaporated and triturated twice in MeOH to a beige powder (Yield of **compound (cm):** 5%, 14mg).

### • Synthesis of compound (cn): according to the synthesis of compound (aj)

According to Scheme 4 Step 2: Compound **222** (80mg, 0.29mmol), DIEA (0.30mL, 1.77mmol) in DMSO (0.1M), compound **158** (172mg, 0.44mmol). Reaction mixture stirred at 70°C for 18h to furnish after flash chromatography (12g, SiO₂, cyclohexane/EtOAc 100:0→0:100,10 CV) and semi-preparative HPLC a white solid (Yield of **compound (cn):** 6%, 8mg).
¹H NMR (300 MHz, DMSO-d₆) δ 13.21 (s, 1H), 8.93 (s, 1H), 8.32 (s, 1H), 7.85 (s, 1H), 7.52-7.50 (m, 4H), 7.42-7.36 (m, 1H), 7.25 (d, J = 4.5 Hz, 2H), 7.10 (d, J = 2.7 Hz, 1H), 6.87 (dd, J = 8.8, 2.7 Hz, 1H), 6.46 (t, J = 4.1 Hz, 1H), 6.34 (s, 1H), 2.38 (s, 3H), 1.25 (s, 9H).

### • Synthesis of compound (co): according to the synthesis of compound (aj)

According to Scheme 4 Step 2: Compound **224** (200mg, 0.57mmol), DIEA (0.60mL, 3.41mmol), DMSO (0.1M), compound **158** (333mg, 0.85mmol). Reaction mixture stirred at 70°C for 24h to furnish after flash chromatography (12g, SiO₂, cyclohexane/EtOAc 100:0→70:30, 10 CV) and semi-preparative HPLC a beige foam (Yield of **compound (co):** 5%, 25mg).
¹H NMR (300 MHz, DMSO-d*₆*) δ 10.63 (s, 1H), 9.02 (s, 1H), 8.60 (d, *J =* 5.6 Hz, 1H), 8.40 (s, 1H), 7.86 (d, *J =* 8.7 Hz, 1H), 7.86 (d, *J =* 8.8 Hz, 1H), 7.53-7.51 (m, 4H), 7.40-7.01 (m, 6H), 6.36 (s, 1H), 2.43 (s, 3H), 1.26 (s, 9H).

### • Synthesis of compound (cp): according to the synthesis of compound (aj)

According to Scheme 4 Step 2: Compound **231** (150mg, 0.46mmol, 1.0eq), DIEA (0.48mL, 2.75mmol, 6.0eq), DMSO (0.1M), compound **158** (179mg, 0.46mmol, 1.0eq). Reaction mixture stirred at 80°C for 20h. A beige solid was obtained after 2 flash chromatography (cyclohexane/EtOAc then EtOAc/MeOH) and semi-preparative HPLC (Yield of **compound (cp):** 33%, 76mg).

### • Synthesis of compound (cq): according to the synthesis of compound (aj)

According to Scheme 4 Step 2: Compound **238** (300mg, 0.97mmol, 1.0eq), DIEA (6.0eq), DMSO (0.1 M), compound **158** (759mg, 1.94mmol, 2.0eq). Reaction mixture stirred at 70°C for 24h. Concentration and purification by flash chromatography over silica gel using cyclohexane and EtOAc first and then EtOAc and MeOH. The fractions were collected, evaporated and purified again by semi-preparative HPLC to afford, after basic extraction and lyophilization a white solid (Yield of **compound (cq):** 12%, 60mg).

### • Synthesis of compound (cr): according to the synthesis of compound (aj)

According to Scheme 4 Step 2: Compound **111** (489mg, 1.45mmol, 1.0eq), DIEA (6eq), DMSO (0.1M), compound **240** (1.07g, 2.17mmol, 1.5eq). Reaction mixture stirred at 70°C for 3 days. Concentration and purification by flash chromatography over silica gel using cyclohexane and EtOAc, then EtOAc and MeOH. and then DCM and ammoniac 7N in MeOH. The residue obtained was purified again by semi-preparative HPLC to afford a yellow solid (Yield of **compound (cr):** 4%, 45mg).

### • Synthesis of compound (cs): according to the synthesis of compound (am)

According to Scheme 4 Step 2: (4-aminophenyl)-(4-pyridyl)methanone (150mg, 0.76mmol, 1.0eq) in acetonitrile (2mL), compound **158** (591mg, 1.52mmol, 2.0eq). Reaction mixture stirred at 70°C for 72h. Mixture directly absorbed on silica gel to be purified by flash chromatography using cyclohexane and EtOAc followed by a semi-preparative HPLC (proceeded on only 100mg of compound). A white foam obtained after extraction with a saturated solution of NaHCO₃ and EtOAc followed by lyophilization (Yield of **compound (cs):** 20%, 67mg).

### • Synthesis of compound (ct): according to the synthesis of compound (aj)

According to Scheme 4 Step 2: Compound **246** (116mg, 0.38mmol), DIEA (0.40mL, 2.29mmol), DMSO (0.1M), compound **158** (224mg, 0.57mmol). Reaction mixture was stirred at 70°C for 3 days to furnish after filtration and semi-preparative HPLC a white powder (Yield of **compound (ct):** 30%, 63mg).
¹H NMR (300 MHz, DMSO-d₆) δ 11.30 (s, 1H), 8.93 (s, 1H), 8.32 (s, 1H), 7.77 (d, *J =* 5.6 Hz, 1H), 7.65 (d, *J* = 8.8 Hz, 1H), 7.52-7.50 (m, 4H), 7.42-7.37 (m, 1H), 7.06 (d, *J =* 2.7 Hz, 1H), 6.88 (dd, *J =* 8.8, 2.7 Hz, 1H), 6.48 (d, *J =* 5.6 Hz, 1H), 6.33 (s, 1H), 4.63 (s, 2H), 2.40 (s, 3H), 1.25 (s, 9H).

### • Synthesis of compound (cu): according to the synthesis of compound (aj)

According to Scheme 4 Step 2: Compound **247** (120mg, 0.20mmol), DIEA (0.30mL, 1.90mmol), DMSO (0.1M), compound **157** (100mg, 0.30mmol). Reaction mixture stirred at 70°C for 18h to furnish after flash chromatography (12g, SiO₂, cyclohexane/EtOAc 100:0→0:100, 10 CV and EtOAc/MeOH 100:0→80:20, 5 CV) and semi-preparative HPLC a purple solid (Yield of **compound (cu):** 36%, 35mg).
¹H NMR (300 MHz, DMSO-d₆) δ 10.70 (br s, 1H), 10.52 (s, 1H), 9.27 (s, 1H), 8.14 (d, J = 8.5 Hz, 1H), 7.92-7.86 (m, 3H), 7.69 (t, J = 7.0 Hz, 1H), 7.59 (t, J = 7.3 Hz, 1H), 7.27 (d, J = 8.4 Hz, 1H), 7.08 (s, 1H), 6.10 (d, J = 5.8 Hz, 1H), 3.07 (t, J = 7.4 Hz, 2H), 2.62 (t, J = 7.5 Hz, 2H), 1.31 (s, 9H).

### • Synthesis of compound (cv): according to the synthesis of compound (aj)

According to Scheme 4 Step 2: Compound **256** (76mg, 0.24mmol), DIEA (0.25mL, 1.43mmol), DMSO (0.1M), compound **158** (140mg, 0.36 mmol). Reaction mixture stirred at 70°C for 24h to furnish after filtration and semi-preparative HPLC a white solid (Yield of **compound (cv):** 8%, 11mg).
¹H NMR (300 MHz, DMSO-d*₆*) δ 10.16 (s, 1H), 9.00 (s, 1H), 8.37 (s, 1H), 8.11 (d, *J =* 5.7 Hz, 1H), 7.73 (d, *J* = 8.8 Hz, 1H), 7.53-7.51 (m, 4H), 7.42-7.37 (m, 2H), 7.12 (d, *J =* 2.7 Hz, 1H), 6.96 (dd, *J =* 8.8, 2.7 Hz, 1H), 6.60 (dd, *J =* 5.7, 2.3 Hz, 1H), 6.35 (s, 1H), 4.07 (qd, *J =* 7.0 Hz, 2H), 2.41 (s, 3H), 1.26 (s, 9H), 1.17 (t, *J =* 7.1 Hz, 3H).

### • Synthesis of compound (cw): according to the synthesis of compound (aj)

According to Scheme 4 Step 2: Compound **263** (170mg, 0.55mmol, 1.0eq), DIEA (6eq), DMSO (0.1M), compound **158** (324mg, 0.83mmol, 1.5eq). Reaction mixture stirred at 70°C for 24 h. The expected compound obtained after 2 flash chromatography over silica gel using cyclohexane and EtOAc first and then EtOAc and MeOH. Fractions were collected, evaporated and purified again in semi-preparative HPLC to afford, after lyophilization, an orange foam (Yield of **compound (cw):** 9%, 37mg).

### • Synthesis of compound (cx): according to the synthesis of compound (aj)

According to Scheme 4 Step 2: Compound **266** (74mg, 0.22mmol), DIEA (0.23mL, 1.35mmol), DMSO (0.1M), compound **158** (131mg, 0.34mmol). Reaction mixture stirred at 70°C for 24h to furnish, after flash chromatography (12g, SiO₂, cyclohexane/EtOAc 100:0→40:60, 10 CV) and semi-preparative HPLC a beige solid (Yield of **compound (cx):** 4%, 5mg).
¹H NMR (300 MHz, DMSO-d₆) δ 10.34 (s, 1H), 9.01 (s, 1H), 8.38 (s, 1H), 8.12 (d, J = 5.7 Hz, 1H), 7.73 (d, J = 8.8 Hz, 1H), 7.53-7.51 (m, 4H), 7.42-7.30 (m, 7H), 7.12 (d, J = 2.6 Hz, 1H), 6.96 (dd, J = 8.8, 2.6 Hz, 1H), 6.62 (dd, J = 5.7, 2.2 Hz, 1H), 5.11 (s, 2H), 2.41 (s, 3H), 1.26 (s, 9H).

### • Synthesis of compound (cy): according to the synthesis of compound (aj)

According to Scheme 4 Step 2: Compound **271** (90mg, 0.33mmol), DIEA (0.35mL, 1.98mmol) in DMSO (0.1M), compound **158** (190mg, 0.50mmol). Reaction mixture stirred at 70°C for 18h to furnish, after flash chromatography (12g, SiO₂, cyclohexane/EtOAc 100:0→0:100, 10 CV followed by EtOAc/MeOH 100:0→80:20, 5CV) and semi-preparative HPLC a beige foam (Yield of **compound (cy):** 12%, 21mg).
¹H NMR (300 MHz, DMSO-d₆) δ 8.88 (s, 1H), 8.27 (s, 1H), 7.56 (d, J = 8.8 Hz, 1H), 7.52-7.50 (m, 4H), 7.46 (d, J = 5.6 Hz, 1H), 7.41 - 7.37 (m, 1H), 6.95 (d, J = 2.7 Hz, 1H), 6.80 (s, 1H), 6.75 (dd, J = 8.8, 2.7 Hz, 1H), 6.33 (s, 1H), 6.06 (d, J = 5.6 Hz, 1H), 4.07 (t, J = 3.9 Hz, 2H), 3.38 (t, J = 3.9 Hz, 2H), 2.38 (s, 3H), 1.25 (s, 9H).

### • Synthesis of compound (cz): according to the synthesis of compound (aj)

According to Scheme 4 Step 2: Compound **132** (40mg, 0.13mmol), DIEA (0.15ml, 0.80mmol), DMSO (0.1M), compound **273** (82mg, 0.20mmol). Reaction mixture stirred at 70°C for 18h to furnish, after filtration and semi-preparative HPLC a beige foam (Yield of **compound** (cz): 29%, 18mg).
¹H NMR (300 MHz, DMSO-d*₆*) δ 10.68 (br s, 1H), 10.51 (s, 1H), 8.27 (s, 1H), 7.96 (d, *J =* 5.8 Hz, 1H), 7.79 (d, *J* = 8.8 Hz, 1H), 7.15 (d, *J* = 2.4 Hz, 1H), 6.95 (dd, *J* = 8.8, 2.5 Hz, 1H), 6.31 (s, 1H), 6.01 (s, 1H), 2.90 (t, *J =* 7.7 Hz, 2H), 2.51 (t, *J =* 7.7 Hz, 2H), 2.43 (s, 3H), 1.23 (s, 9H).

### • Synthesis of compound (da): according to the synthesis of compound (aj)

According to Scheme 4 Step 2: Compound **252** (78mg, 0.24mmol), DIEA (0.21mL, 1.21mmol), DMSO (0.1M), compound **158** (141 mg, 0.36mmol). Reaction mixture stirred at 70°C for 5h to furnish, after filtration and semi-preparative HPLC a white solid (Yield of **compound (da):** 24%, 33mg).
¹H NMR (300 MHz, DMSO-d₆) δ 10.14 (s, 1H), 9.12 (s, 1H), 8.83 (s, 1H), 8.10 (d, J = 5.7 Hz, 1H), 8.05 (d, J = 8.4 Hz, 1H), 7.93 (d, J = 8.4 Hz, 1H), 7.81 (d, J = 7.6 Hz, 1H), 7.65-7.52 (m, 6H), 7.45-7.40 (m, 1H), 7.35-7.30 (m, 2H), 6.62 (dd, J = 5.7, 2.3 Hz, 1H), 6.41 (s, 1H), 4.01 (qd, J = 7.1 Hz, 2H), 1.27 (s, 9H), 1.13 (t, J = 7.1 Hz, 3H).

### • Synthesis of compound (db): according to the synthesis of compound (aj)

According to Scheme 4 Step 2: Compound **252** (70mg, 0.22mmol), DIEA (0.19mL, 1.08mmol), DMSO (0.1M), compound **273** (102mg, 0.32mmol). Reaction mixture stirred at 70°C for 6h to furnish, after filtration and semi-preparative HPLC a white solid (Yield of **compound (db):** 28%, 30mg).
¹H NMR (300 MHz, DMSO-d₆) δ 10.41 (s, 1H), 10.15 (s, 1H), 9.00 (s, 1H), 8.11 (d, J = 5.9 Hz, 2H), 7.97 (d, J = 8.3 Hz, 1H), 7.85 (d, J = 7.7 Hz, 1H), 7.69 (t, J = 7.0 Hz, 1H), 7.58 (t, J = 7.2 Hz, 1H), 7.37 (s, 1H), 7.34 (d, J = 8.6 Hz, 1H), 6.64 (dd, J = 5.7, 2.3 Hz, 1H), 6.07 (s, 1H), 4.01 (qd, J = 7.1 Hz, 2H), 1.24 (s, 9H), 1.13 (t, J = 7.1 Hz, 3H).

### • Synthesis of compound (dc): according to the synthesis of compound (aj)

According to Scheme 4 Step 2: Compound **252** (70mg, 0.22mmol), DIEA (0.19ml, 1.08mmol), DMSO (0.1M), compound **157** (108mg, 0.32mmol). Reaction mixture stirred at 70°C for 6h to furnish, after filtration and semi-preparative HPLC a white solid (Yield of **compound (dc):** 16%, 18mg).
¹H NMR (300 MHz, DMSO-d₆) δ 10.68 (s, 1H), 10.15 (s, 1H), 9.27 (br s, 1H), 8.16-8.10 (m, 2H), 8.05 (d, J = 8.3 Hz, 1H), 7.85 (d, J = 8.0 Hz, 1H), 7.69 (t, J = 6.9 Hz, 1H), 7.59 (t, J = 7.3 Hz, 1H), 7.36 (s, 1H), 7.34 (d, J = 6.8 Hz, 1H), 7.09 (s, 1H), 6.64 (dd, J = 5.7, 2.3 Hz, 1H), 4.01 (qd, J = 7.1 Hz, 2H), 1.31 (s, 9H), 1.13 (t, J = 7.1 Hz, 3H).

### • Synthesis of compound (dd): according to the synthesis of compound (aj)

According to Scheme 4 Step 2: Compound **111** (1.33g, 3.95mmol, 1.0eq), DIEA (6eq), DMSO (0.1M), compound **279** (2.13g, 4.35mmol, 1.1eq). Reaction mixture stirred at 70°C for 24h. Filtrate purified by flash chromatography over silica gel using DCM and EtOAc first, and then EtOAc and MeOH. A second flash chromatography over silica gel to afford an orange powder (Yield of **compound (dd):** 11%, 280mg).

### • Synthesis of compound (de): according to the synthesis of compound (aj)

According to Scheme 4 Step 2: Compound **256** (80mg, 0.25mmol), DIEA (0.22mL, 1.25mmol), DMSO (0.1M), compound **273** (119mg, 0.38mmol). Reaction mixture stirred at 70°C for 24h to furnish, after filtration and semi-preparative HPLC a white solid (Yield of **compound (de):** 27%, 33mg).
¹H NMR (300 MHz, DMSO-d₆) δ 10.69 (s, 1H), 10.18 (s, 1H), 8.28 (s, 1H), 8.12 (d, J = 5.7 Hz, 1H), 7.82 (d, J = 8.8 Hz, 1H), 7.38 (d, J = 2.2 Hz, 1H), 7.18 (d, J = 2.7 Hz, 1H), 7.02 (dd, J = 8.8, 2.7 Hz, 1H), 6.62 (dd, J = 5.7, 2.3 Hz, 1H), 6.02 (s, 1H), 4.06 (qd, J = 7.1 Hz, 2H), 2.44 (s, 3H), 1.23 (s, 9H), 1.17 (t, J = 7.1 Hz, 3H).

### • Synthesis of compound (df): according to the synthesis of compound (aj)

According to Scheme 4 Step 2: Compound **256** (80mg, 0.25mmol), DIEA (0.22mL, 1.25mmol), DMSO (0.1M), compound **157** (125mg, 0.38mmol). Reaction mixture stirred at 70°C for 24h to furnish, after flash chromatography (12g, SiO₂, cyclohexane/EtOAc 100:0→40:60, 10 CV) and semi-preparative HPLC a white foam (Yield of **compound (df):** 12%, 15mg).
¹H NMR (300 MHz, DMSO-d₆) δ 10.93 (s, 1H), 10.17 (s, 1H), 8.60 (br s, 1H), 8.11 (d, J = 5.7 Hz, 1H), 7.86 (d, J = 8.8 Hz, 1H), 7.39 (d, J = 2.3 Hz, 1H), 7.18 (d, J = 2.7 Hz, 1H), 7.05 (s, 1H), 7.01 (dd, J = 8.8, 2.7 Hz, 1H), 6.62 (dd, J = 5.7, 2.3 Hz, 1H), 4.07 (qd, J = 7.1 Hz, 2H), 2.44 (s, 3H), 1.30 (s, 9H), 1.17 (t, J = 7.1 Hz, 3H).

### • Synthesis of compound (dg): according to the synthesis of compound (aj)

According to Scheme 4 Step 2: Compound **247** (80mg, 0.25mmol), DIEA (0.30mL, 1.57mmol), DMSO (0.1M), compound **273** (125mg, 0.38mmol). Reaction mixture stirred at 70°C for 18h to furnish, after flash chromatography (12g, SiO₂, cyclohexane/EtOAc 100:0→0:100, 10 CV and EtOAc/MeOH 100:0 → 80:20, 5 CV) and semi-preparative HPLC a purple powder (Yield of **compound (dg):** 10%, 12mg).
¹H NMR (300 MHz, DMSO-d*₆*) δ 10.54 (s, 1H), 10.45 (br s, 1H), 9.02 (s, 1H), 8.11 (d, *J = 8.5* Hz, 1H), 7.94-7.87 (m, 3H), 7.69 (t, *J =* 7.2 Hz, 1H), 7.59 (t, *J =* 7.6 Hz, 1H), 7.27 (d, *J =* 8.3 Hz, 1H), 6.11 (d, *J =* 5.7 Hz, 1H), 6.05 (s, 1H), 3.07 (t, *J =* 7.5 Hz, 2H), 2.62 (t, *J =* 7.5 Hz, 2H), 1.24 (s, 9H).

### • Synthesis of compound (dh): according to the synthesis of compound (aj)

According to Scheme 4 Step 2: Compound **111** (150mg, 0.44mmol, 1.0eq), DIEA (6eq), DMSO (0.1M), compound **275** (211mg, 0.67mmol, 1 .5eq). Reaction mixture stirred at 70°C for 24h. The precipitate filtered, and the filtrate purified by flash chromatography over silica gel first using cyclohexane and EtOAc and then using EtOAc and MeOH. The compound obtained purified again by semi-preparative HPLC to afford a salmon powder (Yield of **compound (dh):** 2%, 4mg).

### • Synthesis of compound (di): according to the synthesis of compound (aj)

According to Scheme 4 Step 2: Compound **256** (80mg, 0.25mmol), DIEA (0.22mL, 1.25mmol), DMSO (0.1M) compound **275** (119mg, 0.38mmol). Reaction mixture stirred at 70°C for 24h to furnish, after flash chromatography (12g, SiO₂, cyclohexane/EtOAc 100:0→20:80, 10 CV) and semi-preparative HPLC a white solid (Yield of **compound (di):** 6%, 8mg).
¹H NMR (300 MHz, DMSO-d₆) δ 10.16 (s, 1H), 10.11 (s, 1H), 8.43 (s, 1H), 8.12 (d, J = 5.7 Hz, 1H), 7.84 (d, J = 8.8 Hz, 1H), 7.39 (d, J = 2.1 Hz, 1H), 7.16 (d, J = 2.5 Hz, 1H), 6.99 (dd, J = 8.8, 2.4 Hz, 1H), 6.62 (dd, J = 5.6, 2.2 Hz, 1H), 6.42 (s, 1H), 4.06 (qd, J = 7.1 Hz, 2H), 2.44 (s, 3H), 1.27 (s, 9H), 1.17 (t, J = 7.1 Hz, 3H).

### • Synthesis of compound (dj): according to the synthesis of compound (aj)

According to Scheme 4 Step 2: Compound **281** (115mg, 0.39mmol), DIEA (0.70mL, 3.90mmol), DMSO (0.1M), compound **158** (306mg, 0.78mmol). Reaction mixture stirred at 70°C for 24h to furnish, after flash chromatography (12g, SiO₂, cyclohexane/EtOAc 100:0 → 0:100, 10 CV and EtOAc/MeOH 100:0 → 80:20, 5 CV) and semi-preparative HPLC a white solid (Yield of **compound (dj):** 12%, 25mg).
¹H NMR (300 MHz, DMSO-d₆) δ 10.53 (s, 1H), 9.14 (s, 1H), 8.79 (s, 1H), 8.15 (d, J = 5.7 Hz, 1H), 8.07 (d, J = 8.5 Hz, 1H), 7.93 (d, J = 8.4 Hz, 1H), 7.65 (d, J = 2.4 Hz, 1H), 7.63-7.52 (m, 7H), 7.46-7.40 (m, 1H), 7.31 (d, J = 8.3 Hz, 1H), 6.63 (dd, J = 5.7, 2.4 Hz, 1H), 6.42 (s, 1H), 1.99 (s, 3H), 1.28 (s, 9H).

### • Synthesis of compound (dk): according to the synthesis of compound aj)

According to Scheme 4 Step 2: Compound **283** (150mg, 0.65mmol), DIEA (6eq.), DMSO (0.1M), compound **158** (504mg, 1.30mmol). Reaction mixture stirred at 70°C for 24h to furnish, after flash chromatography (12g, SiO₂, cyclohexane/EtOAc 100:0→0:100, 10 CV then EtOAc/CH₃OH 100:0 → 80:20, 10 CV) and semi-preparative HPLC a white solid (Yield of **compound (dk):** 14%, 44mg).
¹H NMR (300 MHz, DMSO-d₆) δ 9.10 (s, 1H), 8.48 (s, 1H), 7.98 (d, J = 7.8 Hz, 1H), 7.85 (d, J = 8.8 Hz, 2H), 7.53-7.50 (m, 4H), 7.43-7.38 (m, 2H), 7.31 (dd, J = 8.8, 2.6 Hz, 1H), 6.37 (s, 1H), 6.21 (d, J = 7.7 Hz, 2H), 2.49 (s, 3H), 1.27 (s, 9H).

### • Synthesis of compound (dl): according to the synthesis of compound (aj)

According to Scheme 4 Step 2: Compound **285** (59mg, 0.18mmol), DIEA (0.20mL, 0.73mmol), DMSO (0.1M), compound **273** (410mg, 1.30mmol). Reaction mixture stirred at 70°C for 36h to furnish after semi-preparative HPLC a beige solid (Yield of **compound (dl):** 5%, 4.8mg).
¹H NMR (300 MHz, DMSO-d₆) δ 10.87 (s, 1H), 8.45 (s, 1H), 8.02 (d, J = 6.3 Hz, 1H), 7.81 (d, J = 8.8 Hz, 1H), 7.19 (d, J = 2.6 Hz, 1H), 7.02 (dd, J = 8.8, 2.6 Hz, 1H), 6.54 (dd, J = 8.4, 2.1 Hz, 1H), 6.46 (d, J = 2.2 Hz, 1H), 6.01 (s, 1H), 3.10 (s, 3H), 2.44 (s, 3H), 1.23 (s, 9H).

### • Synthesis of compound (dm): according to the synthesis of compound (aj)

According to Scheme 4 Step 2: Compound **287** (150mg, 0.52mmol), DIEA (0.90mL, 5.2mmol), DMSO (0.1M), compound **158** (407mg, 1.04mmol). Reaction mixture stirred at 70°C for 24h to furnish, after filtration and semi-preparative HPLC a brown solid (Yield of **compound (dm):** 6%, 17mg).
¹H NMR (300 MHz, DMSO-d₆) δ 11.38 (s, 1H), 11.20 (s, 1H), 8.99 (s, 1H), 8.37 (s, 1H), 7.75 (d, J = 5.9 Hz, 1H), 7.68 (d, J = 8.8 Hz, 1H), 7.53-7.51 (m, 4H), 7.44-7.37 (m, 1H), 7.12 (d, J = 2.7 Hz, 1H), 6.94 (dd, J = 8.8, 2.7 Hz, 1H), 6.38 (d, J = 5.9 Hz, 1H), 6.34 (s, 1H), 2.41 (s, 3H), 1.26 (s, 9H).

### • Synthesis of compound (dn): according to the synthesis of compound (aj)

According to Scheme 4 Step 2: Compound **296** (160mg, 0.53mmol), DIEA (0.91mL, 5.3mmol),DMSO (0.1M), compound 158 (313mg, 0.80mmol). Reaction mixture stirred at 70°C for 20h to furnish, after flash chromatography (40g, SiO₂, cyclohexane/EtOAc 100:0→0:100, 10 CV) an orange solid (Yield of **compound (dn):** 30%, 86mg).
¹H NMR (300 MHz, DMSO-d₆) δ 12.89 (s, 1H), 9.25 (s, 1H), 8.47 (s, 1H), 8.27 (d, J = 5.7 Hz, 1H), 8.18 (s, 1H), 7.53-7.49 (m, 5H), 7.43-7.38 (m, 1H), 7.26 (dd, J = 8.7, 2.3 Hz, 1H), 7.11 (d, J = 8.7 Hz, 1H), 6.37 (s, 1H), 6.34 (d, J = 5.7 Hz, 1H), 2.36 (s, 3H), 1.27 (s, 9H).

### • Synthesis of compound (do): according to the synthesis of compound (aj)

According to Scheme 4 Step 2: Compound **298** (244mg, 0.81mmol, 1.0eq), DIEA (468µL, 3.26mmol, 4.0eq), DMSO (0.1M), compound **158** (318mg, 0.81mmol, 1.0eq). Reaction mixture stirred at 80°C for 18h. After cooling down, precipitate filtered and purified by semi-preparative HPLC to afford after lyophilization a grey solid (Yield of **compound (do):** 8%, 34mg).

### • Synthesis of compound (dp): according to the synthesis of compound (aj)

According to Scheme 4 Step 2: Compound **111** (266mg, 0.79mmol, 1.0eq), DIEA (6eq), DMSO (0.1M), compound **303** (708mg estimated, 1.58mmol, 2.0eq). Reaction mixture stirred at 70°C for 18h. Concentration and purification by flash chromatography over silica gel using DCM and MeOH. Fractions evaporated and purified again twice by semi-preparative HPLC. Fractions collected, extracted with a saturated solution of NaHCO₃ and EtOAc and evaporated to afford, after lyophilization, a yellow solid (Yield of **compound (dp):** 2%, 14mg).

### • Synthesis of compound (dq):

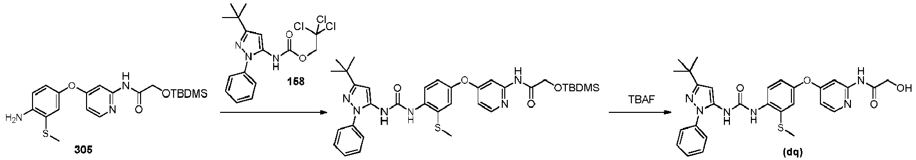

### Step1. Synthesis of compound 1.4

According to Scheme 4 Step 2: In a sealed tube containing compound **305** (228mg, 0.54mmol), DIEA (0.95ml, 5.43mmol) in DMSO (0.1M) was added compound **158** (425mg, 1.09mmol). The reaction mixture was stirred at 70°C for 18h to furnish, after flash chromatography (40g, SiO₂, cyclohexane/EtOAc 100:0→60:40, 10 CV) the compound **1.4** in mixture with side products (230mg) as an orange foam.

### Step2. Synthesis of compound (dq)

A solution of compound **1.4** (230mg, 0.35mmol) and TBAF (1M in THF, 0.21mL, 0.21mmol) in dry THF (5mL) was stirred at r.t. for 4h. Then water (10mL) and EtOAc (20mL) were added. The aqueous layer was extracted with EtOAc (2x20mL). The combined organic fractions were dried over MgSO₄, filtered and concentrated in vacuo. The crude obtained was purified by flash chromatography (12g, SiO₂, cyclohexane/EtOAc 100:0 → 0:100, 10 CV then EtOAc/MeOH 100:0→90:10, 5 CV) and semi-preparative HPLC, to furnish **compound (dq)** (Yield: 17%, 32mg) as a white solid.
¹H NMR (300 MHz, DMSO-d₆) δ 9.74 (s, 1H), 9.00 (s, 1H), 8.37 (s, 1H), 8.17 (d, J = 5.7 Hz, 1H), 7.73 (d, J = 8.8 Hz, 1H), 6.67 (d, J = 2.2 Hz, 1H), 7.53-7.49 (m, 4H), 7.42-7.36 (m, 1H), 7.14 (d, J = 2.7 Hz, 1H), 6.97 (dd, J = 8.8, 2.7 Hz, 1H), 6.69 (dd, J = 5.7, 2.3 Hz, 1H), 6.35 (s, 1H), 5.66 (t, J = 6.0 Hz, 1H), 3.98 (d, J = 6.0 Hz, 2H), 2.41 (s, 3H), 1.26 (s, 9H). ESIMS m/z [M+H]⁺ 547.33.

**Table 2: MS m/z (ES) [M+H]+ of Compounds**

| | | | | | |
|---|---|---|---|---|---|
| Compound | MS m/z (ES) [M+H]+ | (x) | 498.50 | (aw) | 572.33 |
| (a) | 472.10 | (y) | 498.40 | (ax) | 539.17 |
| (b) | 500.20 | (z) | 484.40 | (av) | 504.17 |
| (c) | 514.30 | (ab) | 485.50 | (az) | 578.42 |
| (d) | 566.30 | (ac) | 445.50 | (ba) | 487.42 |
| (e) | 498.30 | (ad) | 446.20 | (bb) | 597.33 |
| (^{f}) | 528.30 | (ae) | 503.30 | (bc) | 526.42 |
| (9) | 514.30 | (af) | 463.40 | (bd) | 557.33 |
| (h) | 438.40 | (ag) | 582.40 | (be) | 529.42 |
| (i) | 480.50 | (ah) | 483.42 | (bf) | 586.42 |
| (j) | 528.40 | (ai) | 531.42 | (bg) | 537.42 |
| (k) | 551.30 | (ai) | 572.42 | (bh) | 593.42 |
| (l) | 515.30 | (ak) | 530.42 | (bi) | 561.42 |
| (m) | 516.40 | (al) | 510.42 | (bi) | 489.42 |
| (n) | 565.40 | (am) | 556.33 | (bk) | 538.42 |
| (o) | 480.40 | (an) | 543.25 | (bl) | 428.33 |
| (p) | 515.30 | (ao) | 576.33 | (bm) | 593.33 |
| (q) | 455.50 | (ap) | 531.50 | (bn) | 548.33 |
| (r) | 456.40 | (aq) | 531.50 | (bo) | 532.33 |
| (s) | 496.20 | (ar) | 543.33 | (bp) | 660.42 |
| (t) | 497.50 | (as) | 576.33 | (bq) | 560.42 |
| (u) | 497.30 | (at) | 576.33 | (br) | 531.42 |
| (v) | 564.40 | (au) | 556.42 | (bs) | 526.33 |
| (w) | 528.60 | (av) | 572.33 | (bt) | 532.50 |
| (bu) | 541.42 | (cl) | 590.42 | (dc) | 506.25 |
| (bv) | 543.42 | (cm) | 594.42 | (dd) | 641.50 |
| (bw) | 560.25 | (cn) | 513.40 | (de) | 486.33 |
| (bx) | 467.25 | (co) | 593.42 | (df) | 502.33 |
| (by) | 557.42 | (cp) | 580.50 | (dg) | 472.33 |
| (bz) | 577.33 | (cq) | 514.40 | (dh) | 467.33 |
| (ca) | 483.33 | (cr) | 643.42 | (di) | 486.33 |
| (cb) | 484.33 | (cs) | 440.50 | (dj) | 535.42 |
| (cc) | 567.42 | (ct) | 545.50 | (dk) | 474.33 |
| (cd) | 570.50 | (cu) | 488.33 | (dl) | 492.42 |
| (ce) | 669.33 | (cv) | 561.42 | (dm) | 530.33 |
| (cf) | 671.50 | (cw) | 514.42 | (dn) | 542.42 |
| (cg) | 490.25 | (cx) | 561.42 | (do) | 541.30 |
| (ch) | 660.42 | (cy) | 531.50 | (dp) | 599.42 |
| (ci) | 542.33 | (cz) | 468.33 | (dq) | 547.33 |
| (cj) | 468.33 | (da) | 565.42 | | |
| (ck) | 560.42 | (db) | 490.42 | | |

### Example 2: In vitro testing

### Example 2.1: Myotube assay workflow

DMD human induced pluripotent stem cells (hereafter "DMD hiPSC"), generated from the NCRM-1 hiPSC WT cell line by gene editing, or WT human induced pluripotent stem cells (hereafter "WT hiPSC"), being NCRM-1 hiPSC wt cell line, have been differentiated according to Chal et al. 2015, 2016, to produce muscle progenitors (hereafter "hMP"). The hMP population contains satellite-like cells (i.e Pax7-positive cells) and satellite cells' progenies (i.e. Myogenin-positive cells).

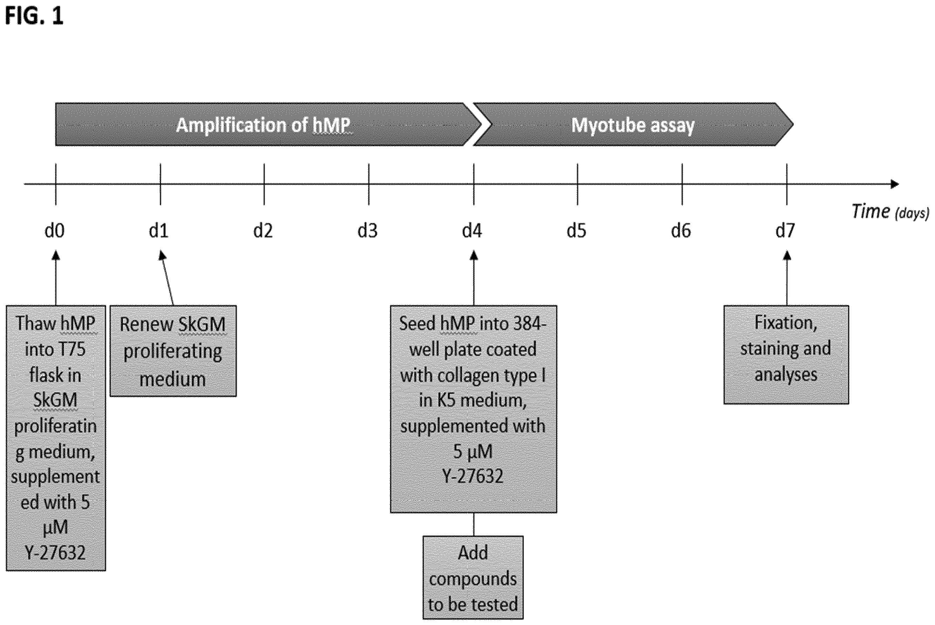

Figure 1 provides an example of a Myotube assay workflow. At day 0, hMP are thawed and 3.1 million cells are plated into a T75-flask coated with hESC-qualified Matrigel^{™} (Corning) in SkGM proliferating medium (PromoCells), supplemented with 5 µM Y-27632 (Tocris Bioscience). On day 2, SkGM proliferating medium is refreshed. On day 4, hMP are harvested and transferred into a 384-well plate coated with collagen type I at a density of 3500 cells/well in a DMEM-based medium, containing 5% of knockout serum replacement (Gibco), 1% PenStrep (Gibco), 1% MEM NEAA (Gibco) and 0.1 mM 2-mercaptoethanol (Gibco) (hereafter "K5 medium") and 5 µM Y-27632. Compounds to be tested are then added in the medium and incubated for 3 days at 37°C. On day 7, cells are fixed and immunostained. For example, myotubes (alpha-actinin staining) and satellite-like (Pax7 staining) cells can be analyzed for total myotube area per well or Pax7-positive cell quantification.

### Example 2.2: Dose response assay of compound (i)

DMD hMP were put in the Myotube assay according to the workflow described in Example 2.1. In particular, compound (i) was added at concentrations ranging from 1.5 nM to 30 µM, starting from day 4. hMP were cultivated for three more days in presence of compound (i), then fixed and analyzed for quantification of total myotube area per well. Duplicates of each condition were averaged.

As shown in figure 2, treatment of DMD hMP in the myotube assay at 10 different increasing concentrations of compound (i) resulted in dose-dependent increase of myotube surface.. This result indicates that compound (i) has a myogenic activity.

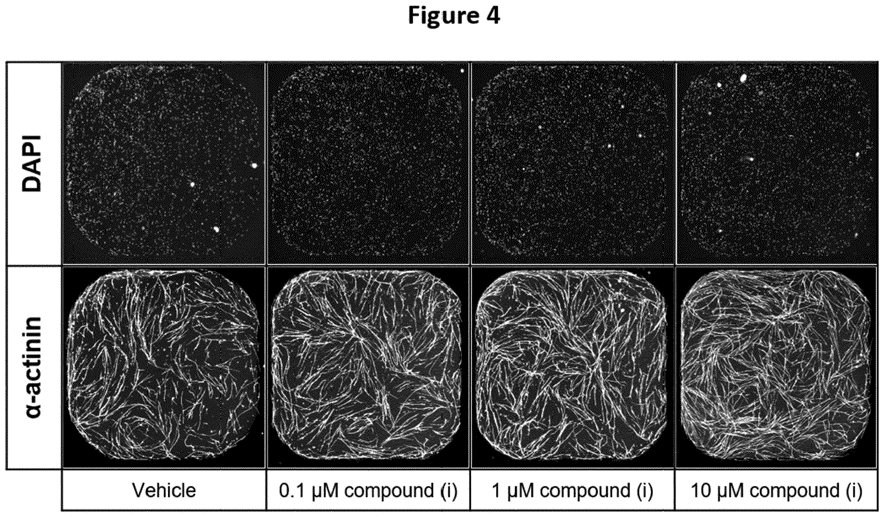

Figure 4 provides representative images of the dose response experiment shown in figure 2.

### Example 2.3: Identification of compounds that promote myogenic differentiation and maintain or increase satellite-like cells

This example describes multiple compounds identified as promoting muscle progenitor differentiation, with the ability to preserve the satellite-like cell pool.

Using the myotube assay described in Example 2.1, all the compounds listed in Table 3, have been shown to be active in the myotube assay.

Table 3 shows myogenic activities (EC₅₀) and efficacies (MA= normalized Myotube Area) of compounds of the present invention. Qualitative scale: ++++: EC₅₀ <500 nM; +++: 500 nM≤ EC₅₀ < 1000 nM; ++: 1000 nM ≤ EC₅₀ < 5000 nM; +: EC₅₀≥5000 nM; +++: MA ≥ 75%; ++: 50% ≤ MA <75%; +: 25% ≤ MA < 50%.

**Table 3**

| Compound | EC₅₀ | MA | Compound | EC₅₀ | MA | Compound | EC₅₀ | MA |
|---|---|---|---|---|---|---|---|---|
| (a) | +++ | + | (ba) | ++++ | +++ | (da) | ++++ | +++ |
| (b) | + | + | (bb) | ++++ | +++ | (db) | ++++ | +++ |
| (c) | + | ++ | (be) | ++ | +++ | (dc) | ++++ | +++ |
| (d) | ++ | ++ | (bd) | ++++ | +++ | (dd) | ++++ | +++ |
| (e) | + | + | (be) | +++ | +++ | (de) | ++++ | +++ |
| (f) | + | ++ | (bf) | ++ | +++ | (df) | ++++ | +++ |
| (g) | +++ | +++ | (bq) | + | +++ | (dg) | ++++ | +++ |
| (h) | + | + | (bh) | + | +++ | (dh) | ++ | +++ |
| (i) | ++ | ++ | (bi) | ++++ | +++ | (di) | ++++ | +++ |
| (j) | ++ | ++ | (bj) | + | +++ | (dj) | ++++ | +++ |
| (k) | ++ | ++ | (bk) | + | +++ | (dk) | + | + |
| (l) | +++ | ++ | (bl) | +++ | ++ | (dl) | ++ | +++ |
| (m) | + | ++ | (bm) | ++++ | +++ | (dm) | ++ | +++ |
| (n) | ++++ | +++ | (bn) | + | ++ | (dn) | +++ | +++ |
| (o) | + | + | (bo) | ++++ | +++ | (do) | ++++ | +++ |
| (p) | +++ | + | (bp) | ++++ | +++ | (dp) | ++++ | +++ |
| (q) | ++ | ++ | (bq) | ++++ | +++ | (dq) | +++ | +++ |
| (r) | + | +++ | (br) | + | +++ | | | |
| (s) | +++ | ++ | (bs) | + | +++ | | | |
| (t) | ++++ | + | (bt) | +++ | +++ | | | |
| (u) | ++ | + | (bu) | + | + | | | |
| (v) | ++++ | +++ | (bv) | + | +++ | | | |
| (w) | ++ | ++ | (bw) | ++++ | +++ | | | |
| (x) | + | + | (bx) | +++ | +++ | | | |
| (y) | + | + | (by) | ++++ | +++ | | | |
| (z) | + | +++ | (bz) | ++++ | +++ | | | |
| (ab) | +++ | ++ | (ca) | + | +++ | | | |
| (ac) | ++ | ++ | (cb) | ++++ | +++ | | | |
| (ad) | +++ | + | (cc) | ++++ | +++ | | | |
| (ae) | + | ++ | (cd) | ++++ | +++ | | | |
| (af) | ++ | + | (ce) | ++++ | +++ | | | |
| (ag) | ++ | +++ | (cf) | +++ | +++ | | | |
| (ah) | + | +++ | (cg) | + | +++ | | | |
| (ai) | +++ | ++ | (ch) | + | ++ | | | |
| (ai) | +++ | +++ | (ci) | + | + | | | |
| (ak) | + | +++ | (cj) | + | +++ | | | |
| (al) | + | +++ | (ck) | +++ | +++ | | | |
| (am) | ++++ | +++ | (cl) | ++++ | +++ | | | |
| (an) | ++++ | +++ | (cm) | +++ | +++ | | | |
| (ao) | + | +++ | (cn) | ++ | ++ | | | |
| (ap) | +++ | + | (co) | + | + | | | |
| (aq) | +++ | +++ | (cp) | +++ | ++ | | | |
| (ar) | + | +++ | (cq) | +++ | +++ | | | |
| (as) | ++++ | +++ | (cr) | ++++ | +++ | | | |
| (at) | + | +++ | (cs) | + | + | | | |
| (au) | + | +++ | (ct) | ++++ | +++ | | | |
| (av) | + | +++ | (cu) | ++++ | +++ | | | |
| (aw) | +++ | +++ | (cv) | ++++ | +++ | | | |
| (ax) | + | ++ | (cw) | + | +++ | | | |
| (av) | ++ | +++ | (cx) | +++ | +++ | | | |
| (az) | +++ | +++ | (cy) | +++ | +++ | | | |
| | | | (cz) | +++ | +++ | | | |

### Example 2.4: Compounds activity on satellite cell (PAX7+) subpopulation in vitro

DMD hMP were put in the Myotube assay according to the workflow described in Example 2.1. In particular, compound (i) was added at concentrations ranging from 1.5 nM to 30 µM, starting from day 4. hMP were cultivated for three more days in presence of compound (i), then fixed and analyzed for percentage of Pax7-positive cells and nuclei number. Duplicates of each condition were averaged.

As shown in figure 3, treatment of DMD hMP in the myotube assay at 10 different increasing concentrations of compound (i) resulted in dose-dependent increase percentage of Pax7-positive cells. This result indicates that compound (i) at least maintains the pool of satellite-like cells.

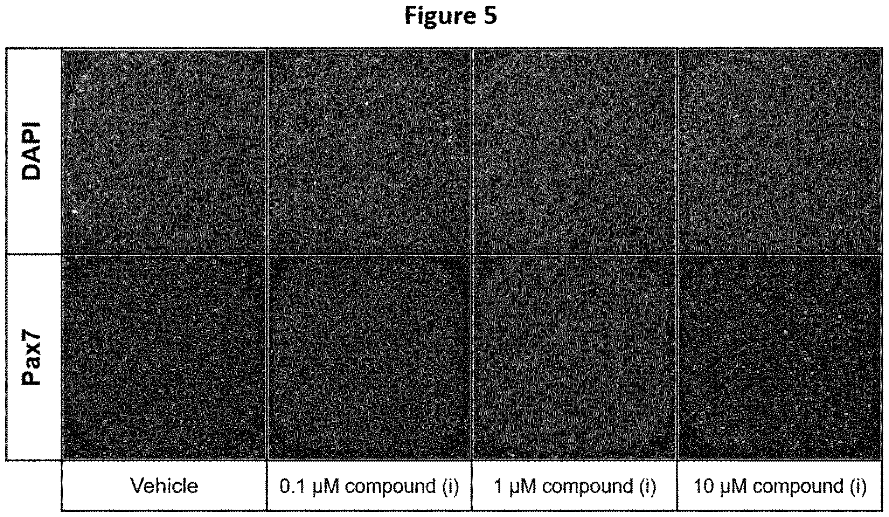

Figure 5 provides representative images of the dose response experiment shown in figure 3.

This example describes multiple compounds identified as promoting muscle progenitor differentiation, with the ability to preserve the satellite-like cell pool.

Using the assay for measuring Pax7-positive cells described in Example 2.1, almost all the compounds listed in Table 4, have been shown to be capable to maintain or increase the pool of Pax7-positive cells in the assay.

The results are presented in Table 4. (-) : Depletes the pool of cells, while positively affects the myotubes; (+) : Maintains or increases the pool of cells, with at least 1 of the highest concentration known to be cytotoxic; and (++) Maintains or increases the pool of cells, without any toxicity.

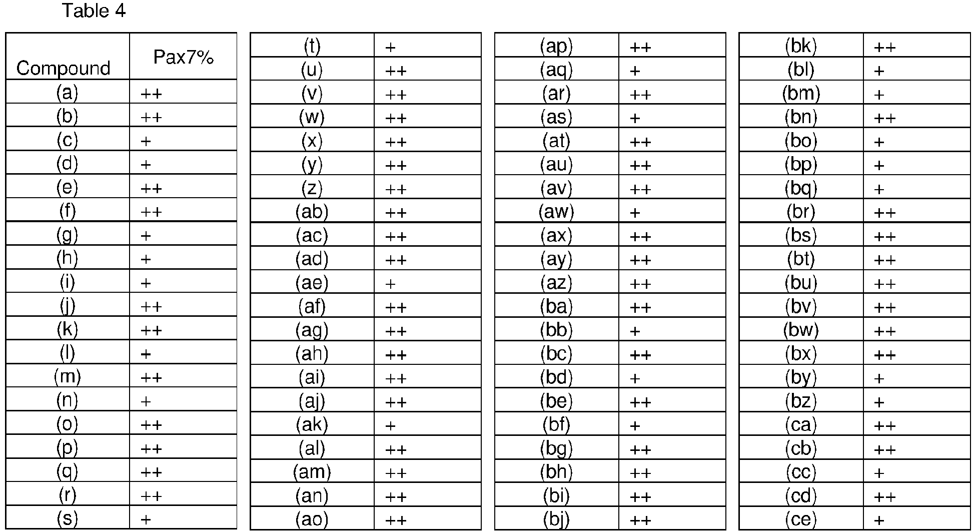

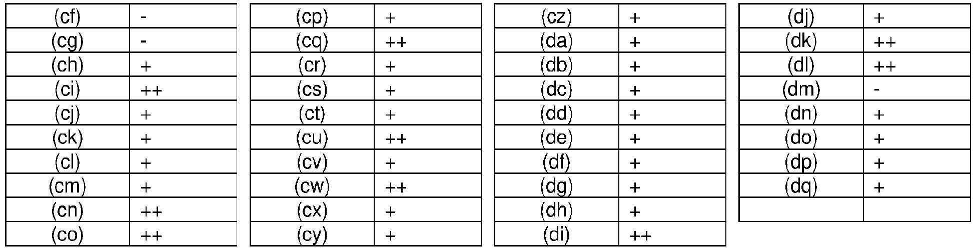

### Example 2.5: Comparative examples

Other compounds have been tested in the same assays. The results are presented in Table 5.

**Table 5**

| Cpd name | EC₅₀ | MA | Pax7 |
|---|---|---|---|
| Rebastinib (Fig 18 of WO2019/084499) | ++ | ++++ | + |
| CCT196969 (Fig 18 of WO2019/084499) | +++ | ++ | + |
| CEP-32496 (Fig 18 of WO2019/084499) | NA | NA | n.a. |
| P38a MAPK-IN-1 (Fig 18 of WO2019/084499) | + | + | ++ |
| BIRB-796 (Fig 18 of WO2019/084499) | + | +++ | ++ |
| Sorafenib (Fig 18 of WO2019/084499) | + | + | ++ |
| Cabozantinib (Fig 18 of WO2019/084499) | NA | NA | n.a. |
| Foretinib (Fig 18 of WO2019/084499) | NA | NA | n.a. |

| | | | |
|---|---|---|---|
| NA: non active | | | |

### Example 2.6: Combination with prednisolone or givinostat

Dose response assay of combination treatments with Givinostat or Prednisolone:
Since Prednisolone is the standard of care in DMD patients in Europe, and Givinostat is currently in phase 3 clinical trial, it has been proposed a combination of Prednisolone or Givinostat treatments with 3 new chemical entities described in this patent: *az*, *bw* and *q*.

DMD hMPs were put in the Myotube assay according to the workflow described in example 2.1. In particular, Prednisolone (Sigma) or Givinostat (Exclusive Chemistry) were added in all conditions at a fixed concentration equal to 0.3 µM in combination with compounds *az* or *bw* or *q* tested in DRC at 8 concentrations ranging from 4.5 nM to 10 µM, starting from day 4. Also, as control, DRCs with the same range of concentrations were done with Prednisolone alone, Givinostat (figure 6) alone or compounds *az or bw or q* alone (figures 7 to 9). The hMPs were cultivated for three more days in presence of treatments (combination and standalone), then fixed and analyzed for quantification of total myotube area/well, percentage of Pax7-positive cells and nuclei number. Duplicates of each condition were averaged.

Figure 6 provides results from the dose response experiment of Prednisolone or Givinostat alone. Both compounds show a beneficial effect on myotube surface with a potency (EC50) equal or below 170 nM accompanied with a dramatic decrease of satellite cell population (Pax7 positive cells). At 0.3 µM, the dose selected for combination with compounds *az* or *bw* or *q,* the drop of satellite cells content was about 50 % for both conditions.

As shown in figures 7 to 9, all combination treatments allow to improve overall myotube surface values compared to the compound alone or Prednisolone or Givinostat alone. All combination also neutralized or decreased the negative effect of Prednisolone alone or Givinostat alone on satellite cells shown in figure 6.

In particular in figure 7, the combination of compound *az* with both Prednisolone or Givinostat increased the total myotube surface and improved by two fold the potency. The satellite cell population is at least maintained with the combination of *az* with Givinostat or slightly increased (by 1.5 fold) with Prednisolone.

In particular in figure 8, the combination of compound bwwith both Prednisolone or Givinostat increased the total myotube surface. The potency is improved when combined with Givinostat, while it is slightly decreased with Prednisolone. However, the decrease of satellite cell pool which is only seen at high concentration of treatment with compound *bw* alone is slightly diminished when given with any of the other two reference compounds.

In particular in figure 9, the combination of compound *q* with Prednisolone or Givinostat show similar results to those of figure 7. Indeed, the myotube surface is strongly increased in parallel with the improvement of potency by 1.4 fold with Givinostat and by 5 fold with Prednisolone. This effect is strengthened by the maintenance of the satellite cells in all conditions compared to Givinostat or Prednisolone alone as shown in figure 6.

Altogether, these results indicate that combination treatments allow the enhancement of myogenic activity while allowing to at least lower the depletion or maintain or increase the pool of satellite cells, compared to Givinostat, Prednisolone, or compounds *az, bw,* and *q* alone.

## Claims

1. A compound for use in treating, ameliorating, delaying, curing and/or preventing a disease or condition associated with muscle cells and/or satellite cells, or a pharmaceutically acceptable salt thereof in combination with a corticosteroid or a histone deacetylase (HDAC) inhibitor, wherein said compound is represented by structure (I), 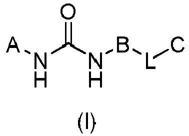 wherein
L is -O-
and
1)
A is a ring system selected from the group consisting of 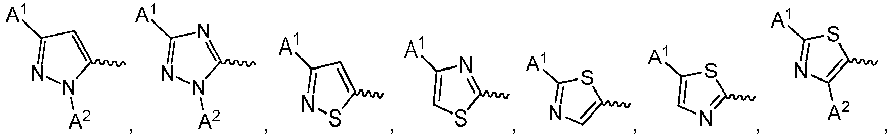 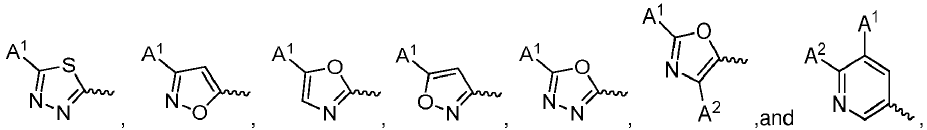 wherein A¹ is selected from the group consisting of C₁-C₄ alkyl, C₁-C₄ cycloalkyl, 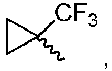 C3-C7 heterocycloalkyl, 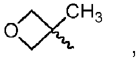 phenyl, benzyl and 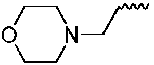 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 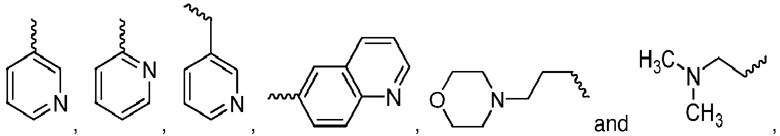 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 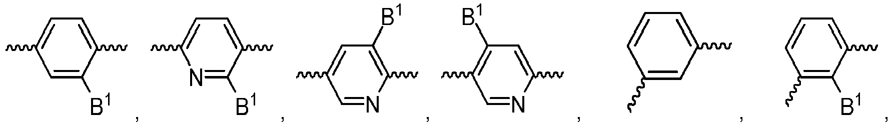 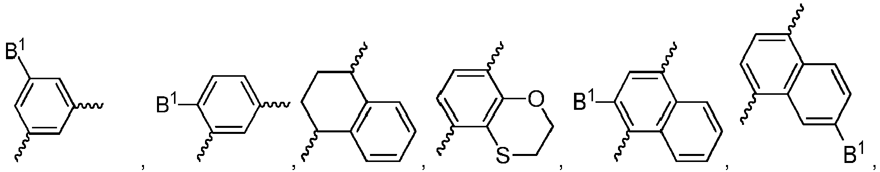 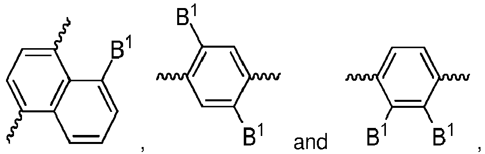 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
C is selected from the group consisting of 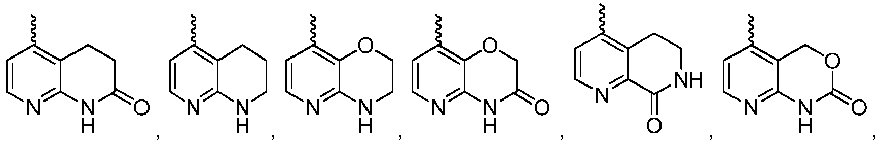 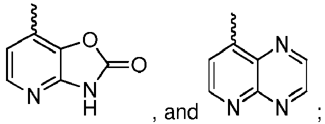
or
2)
A is a ring system selected from the group consisting of 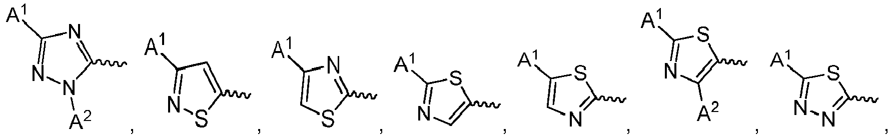 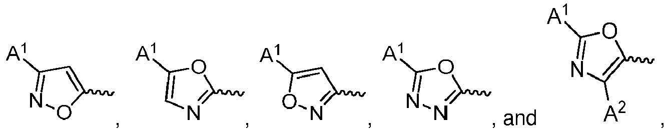 wherein A¹ is selected from the group consisting of C₁-C₄ alkyl, C₁-C₄ cycloalkyl, 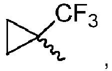 C3-C7 heterocycloalkyl, 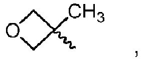 phenyl, benzyl and 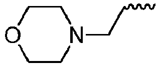 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 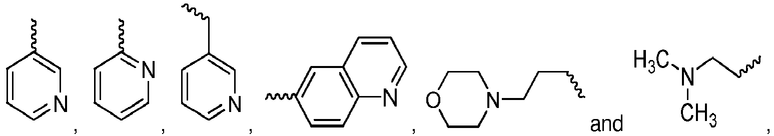 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 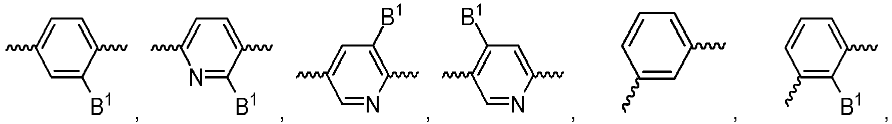 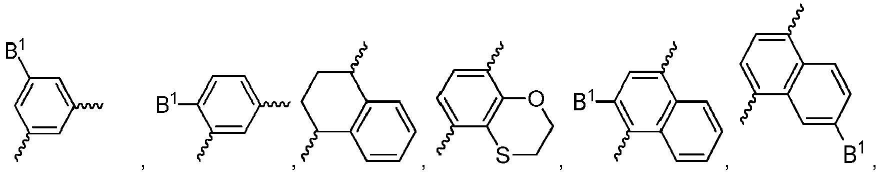 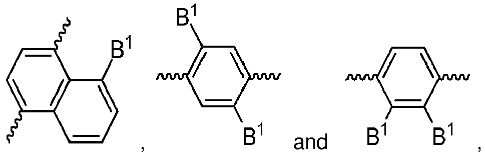 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
C is selected from the group consisting of 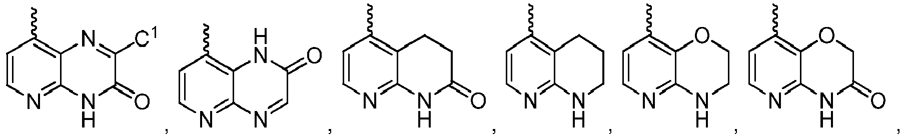 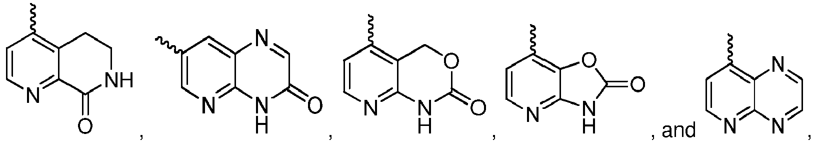 wherein C¹ is selected from the group consisting of -H, -CH₃, -OCH₃, -CN or 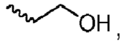 or C is selected from the group consisting of 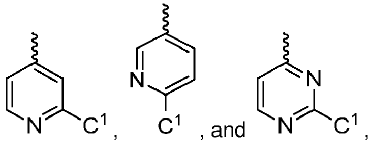 wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR''' or COOR''', wherein R', R" and R''' are independently a C₁-C₆ alkyl;
or
3)
A is a ring system selected from the group consisting of 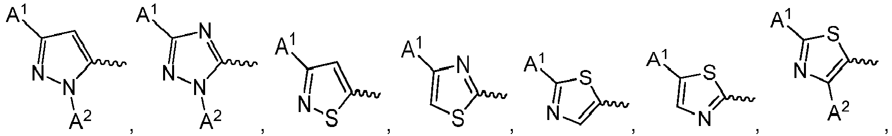 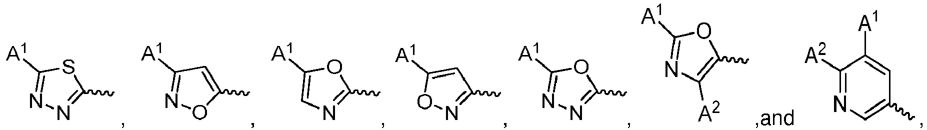 wherein A¹ is selected from the group consisting of C₁-C₄ alkyl, C₁-C₄ cycloalkyl, 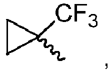 C3-C7 heterocycloalkyl, 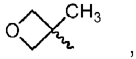 phenyl, benzyl and 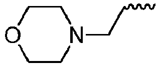 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 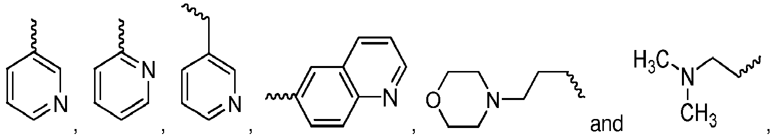 wherein A¹ and A² are optionally fluorinated, and A² is optionally substituted with 1, 2 or 3 substituents selected from the group consisting of C₁-C₄ alkyl, -CI, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 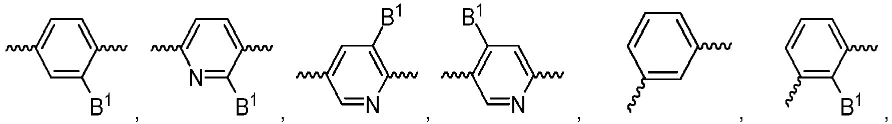 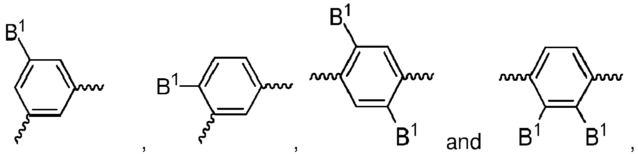 wherein B¹ is selected from the group consisting of -CF₃, -OCF₃, -OCH₃, and SCH₂CH₃, or B has two B¹ groups selected independently from the group consisting of -F, -Cl, -SCH₃, -SCH₂CH₃, -CH₃, isopropyl, -CF₃, -OCH₃, -OCF₃,
C is selected from the group consisting of 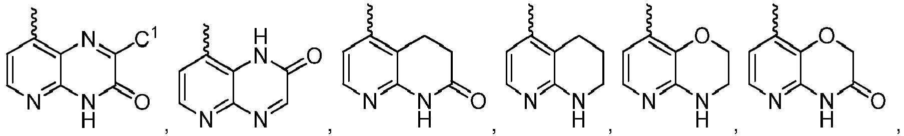 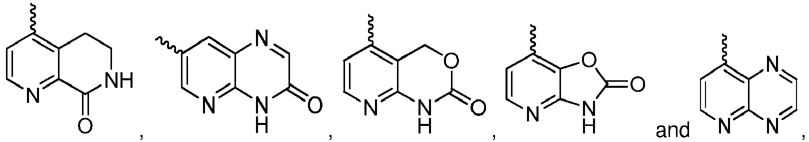 wherein C¹ is selected from the group consisting of -H, -CH₃, -OCH₃, -CN or 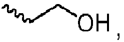 or C is selected from the group consisting of 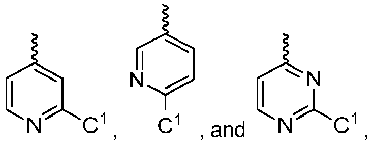 with C¹ being selected from the group consisting of -H, -NH₂, 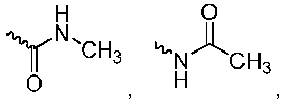 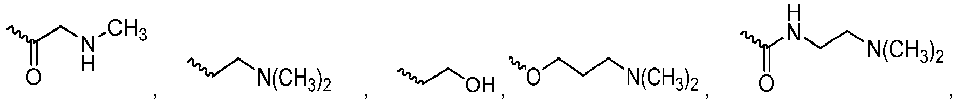 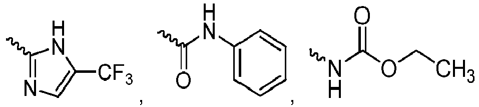 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH₃; preferably from the group consisting of 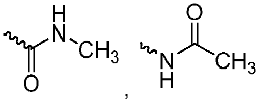 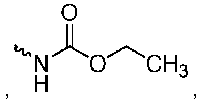 -NH-C(O)OtBu, -NH- C(O)N(CH₃)-C(O)OtBu, -NH-C(O)-CH₂-N(CH₃)-C(O)OtBu, - NH-C(O)-CH₂-NHCH₃, -NH-C(O)-Obenzyl, and -NH-C(O)-CH₂-OH;
or
4)
A is 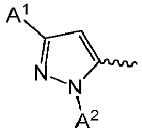 wherein A¹ is selected from the group consisting of C₁-C₄ alkyl, C₁-C₄ cycloalkyl, 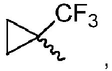 C3-C7 heterocycloalkyl, 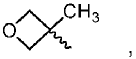 phenyl, benzyl and 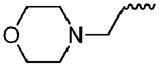 wherein A¹ is optionally fluorinated, and A² is selected from the group consisting of a phenyl substituted with 1, 2 or 3 substituents selected from the group consisting of -Cl, -F, -CN, C₁-C₃ alkyloxy optionally substituted by C₃-C₇ heterocycloalkyl or NRR, with R being a C₁-C₃ alkyl, and C₁-C₄ alkyl substituted by C₃-C₇ heterocycloalkyl or NRR, with R being a C₁-C₃ alkyl; a phenyl or pyridinyl substituted by 2 or 3 C₁-C₃ alkyls; and a pyridinyl substituted with 1, 2 or 3 substituents selected from the group consisting of -Cl, -F, -CN, C₁-C₁₀ alkyloxy substituted by C₃-C₇ heterocycloalkyl or NRR, with R being a C₁-C₃ alkyl, and C₁-C₄ alkyl substituted by C₃-C₇ heterocycloalkyl or NRR, with R being a C₁-C₃ alkyl;
B is a ring system selected from the group consisting of 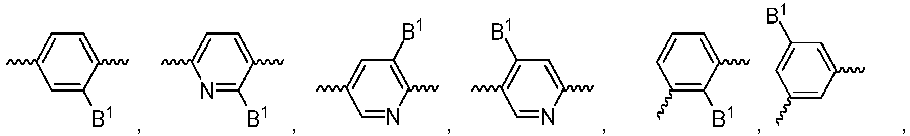 and 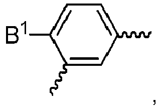 wherein B¹ is -SCH₃,
C is selected from the group consisting of 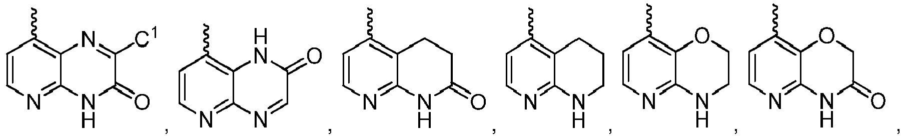 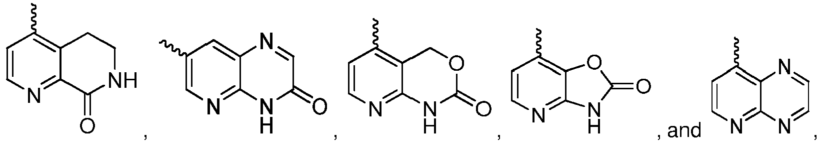 wherein C¹ is selected from the group consisting of -H, -CH₃, -OCH₃, -CN or 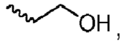
or
5)
A is 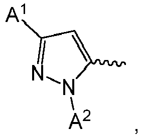 and A¹ is selected from the group consisting of C₁-C₄ alkyl, C₃-C₆ cycloalkyl, 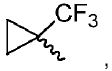 C3-C7 heterocycloalkyl, 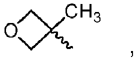 phenyl, benzyl and 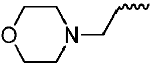 wherein A¹ is optionally fluorinated, A² is 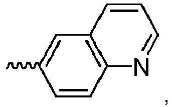
B is a ring system selected from the group consisting of 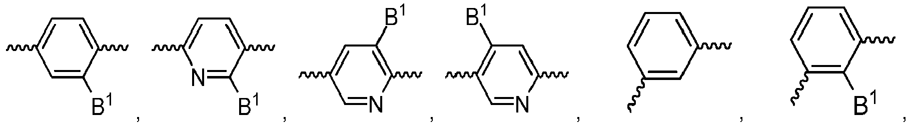 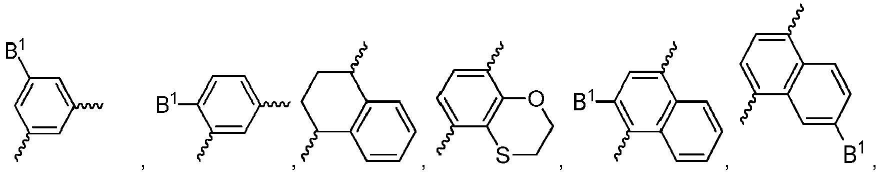 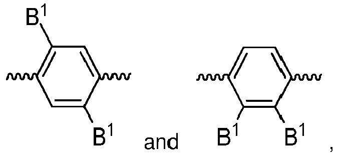 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
C is selected from the group consisting of 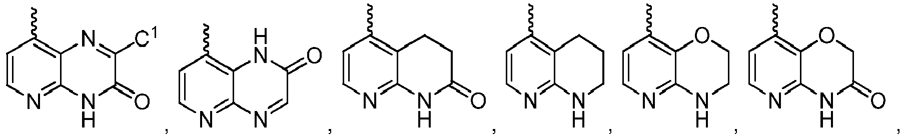 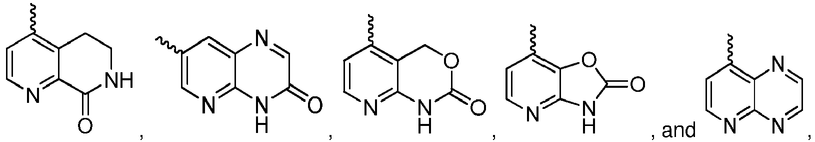 wherein C¹ is selected from the group consisting of -H, -CH₃, -OCH₃, -CN or 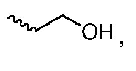
or
6)
A is a ring system selected from the group consisting of 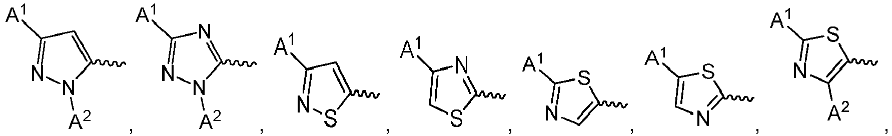 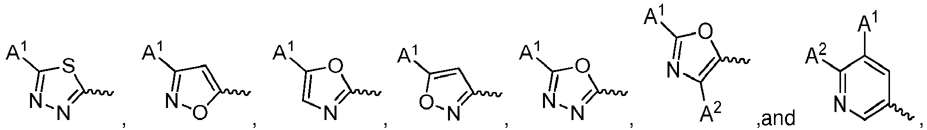 wherein A¹ is selected from the group consisting of C₁-C₄ alkyl, C₁-C₄ cycloalkyl, 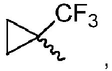 C3-C7 heterocycloalkyl, 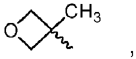 phenyl, benzyl and 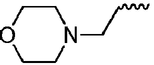 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 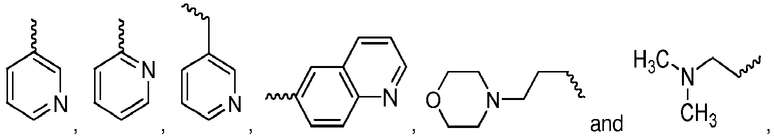 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 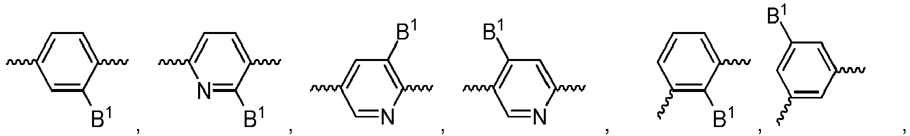 and 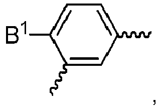 wherein B¹ is -SCH₃,
C is selected from the group consisting of 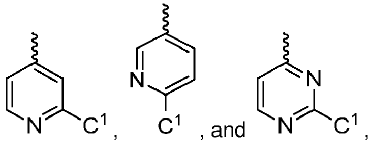 wherein C¹ is selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₂-C₁₀ alkenyl, C₃-C₁₀ cycloalkyl, C₄-C₂₀ alkcycloalkyl, C₆-C₁₄ aryl, C₃-C₁₃ heteroaryl, C₇-C₂₄ alkaryl, C₃-C₇ heterocycloalkyl and C₄-C₁₇ alkheterocycloalkyl, wherein each hydrogen in each of these groups may be independently replaced by a group selected from halogen, hydroxy, -OR', -COR', -COOR', and -NR'R", each hydrogen in each of R' and R" may be independently replaced by -COR''' or COOR''', wherein R', R" and R''' are independently a C₁-C₆ alkyl;
or
7)
A is a ring system selected from the group consisting of 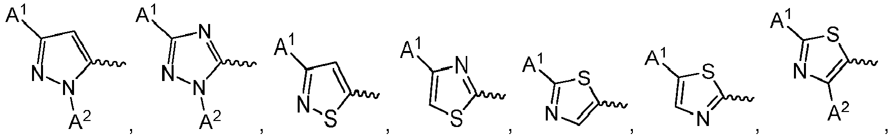 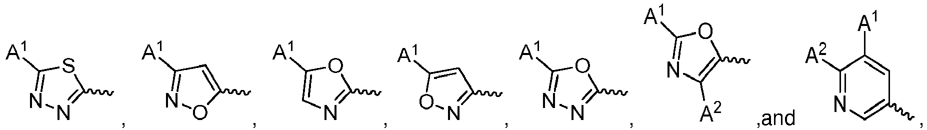 wherein A¹ is selected from the group consisting of C₁-C₄ alkyl, C₁-C₄ cycloalkyl, 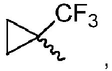 C3-C7 heterocycloalkyl, 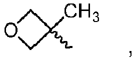 phenyl, benzyl and 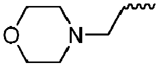 wherein A¹ is optionally fluorinated, and A² is selected from the group consisting of -H, C₁-C₄ alkyl, C₃-C₆ cycloalkyl, C₃-C₇ heterocycloalkyl, halogen, benzyl, phenyl, tolyl, 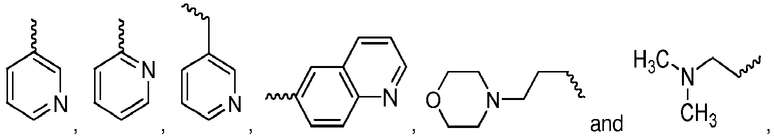 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 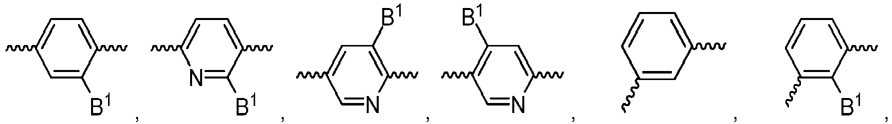 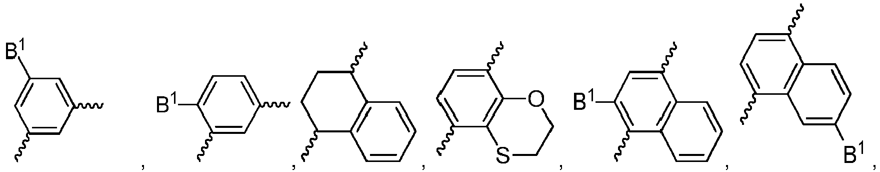 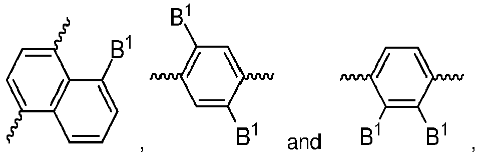 wherein B¹ is selected from the group consisting of -H, -F, -Cl, -SCH₃, -SCH₂CH₃, -CH₃, isopropyl, -CF₃, -OCH₃, -OCF₃ and wherein two B¹ can be linked to form a fused bicyclic ring system containing 0 to 3 heteroatoms; and wherein B¹ is not H when B is a phenyl;
C is selected from the group consisting of 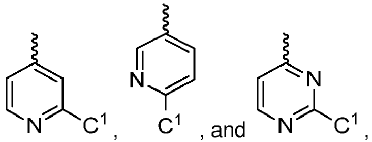 with C¹ being selected from the group consisting of -H, -NH₂, 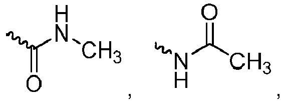 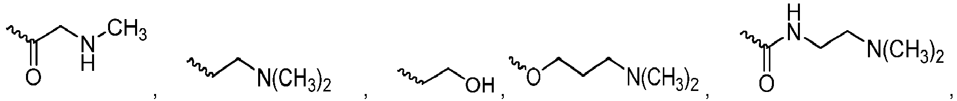 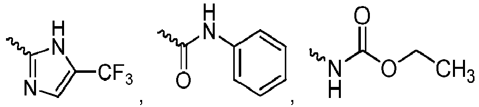 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH₃; preferably from the group consisting of 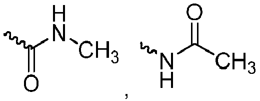 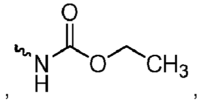 -NH-C(O)OtBu, -NH- C(O)N(CH₃)-C(O)OtBu, -NH-C(O)-CH₂-N(CH₃)-C(O)OtBu,-NH-C(O)-CH₂-NHCH₃, -NH-C(O)-Obenzyl, and -NH-C(O)-CH₂-OH;

2. A pharmaceutical composition comprising a compound as defined in claim 1 and a corticosteroid or a HDAC inhibitor.

3. The compound for use according to claim 1 or the pharmaceutical composition according to claim 2 for use in treating, ameliorating, delaying, curing and/or preventing a disease or condition associated with muscle cells and/or satellite cells.

4. The compound for use according to claim 1, or the pharmaceutical composition for use according to claim 3, wherein said disease or condition is selected from the group consisting of Duchenne muscular dystrophy, Becker muscular dystrophy, cachexia and sarcopenia.

5. The compound for use according to claim 1, or the pharmaceutical composition for use according to claim 3, wherein said disease or condition is selected from the group consisting of Duchenne muscular dystrophy, Becker muscular dystrophy and sarcopenia.

6. The compound for use according to any one of claims 1 and 3-5, the pharmaceutical composition according to claim 2 or the pharmaceutical composition for use according to any one of claims 3-5, wherein the corticosteroid is selected from the group consisting of prednisone, prednisolone, methylprednisolone, oxandrolone, dexamethasone, deflazacort and valmorolone preferably selected from the group consisting of prednisolone, deflazacort and valmorolone.

7. The compound for use according to any one of claims 1 and 3-5, the pharmaceutical composition according to claim 2 or the pharmaceutical composition for use according to any one of claims 3-5, wherein the HDAC inhibitor is selected from the group consisting of Abexinostat (PCI- 24781), Apicidin (OS 12040), AR-42, Belinostat (PXD101), BG45, BML-210, BML-281, BMN290, BRD0302, BRD2283, BRD3227, BRD3308, BRD3349, BRD3386, BRD3493, BRD4161, BRD4884, BRD6688, BRD8951, BRD9757, BRD9757, CBHA, Chromopeptide A, Citarinostat (ACY-214), CM-414, compound 25, CRA-026440, Crebinostat, CUDC-101, CUDC-907, Curcumin, Dacinostat (LAQ824), Depudecin, Domatinostat (4SC-202), Droxinostat, Entinostat (MS0275), EVX001688, FR901228, FRM-0334, Givinostat, HDACi-4b, HDACi-109, HPOB, 12, KD5170, LB-205, M344, Martinostat, Merck60 (BRD6929), Mocetinostat (MGCD0103), OBP-801, Oxamflatin, Panobinostat (LBH589), PCI-34051, PCI-48000, Pracinostat (SB939), Pyroxamide, Quisinostat (JNJ-26481585), Resminostat, RG2833 (RGFP109), RGFP963, RGFP966, RGFP968, Rocilinostat (ACY-1215), Romidepsin (FK228), Scriptaid, sodium phenylbutyrate, Splitomicin, T247, Tacedinaline (CI994), Trapoxin, Trichostatin A (TSA), Tucidinostat (chidamide), Valproic acid, vorinostat (SAHA), W2, MC1742, MC2625, A8B4, A14B3, A12B4, A14B4, A7B4, or any combination thereof, preferably is givinostat.

8. The compound for use according to any one of claims 1 and 3-7, the pharmaceutical composition according to any one of claims 2 and 6-7 or the pharmaceutical composition for use according to any one of claims 3-7, wherein
L is -O-
A is a ring selected from the group consisting of 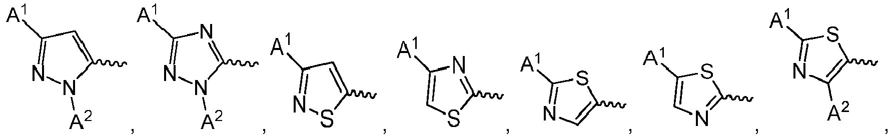 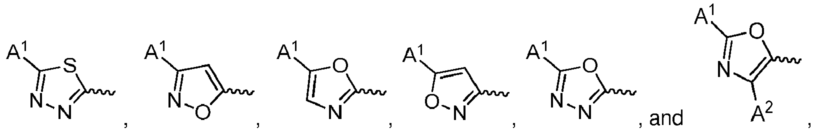
A¹ is tert-butyl,
A² is a phenyl or 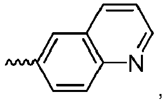 optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 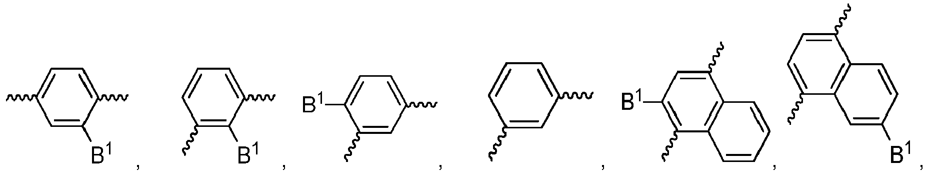 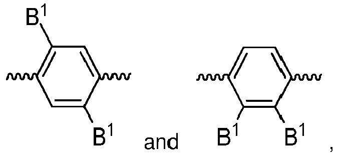 wherein, when B is a phenyl, B¹ is selected from the group consisting of -F, -SCH₃, -SCH₂CH₃, -CF₃, -OCH₃, and -OCF₃ and, when B is a naphtyl, B¹ is -H, and
C is selected from the group consisting of

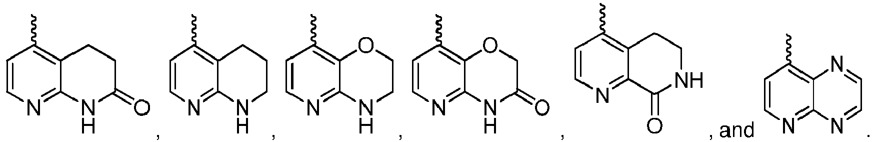

9. The compound for use according to any one of claims 1 and 3-7, the pharmaceutical composition according to any one of claims 2 and 6-7 or the pharmaceutical composition for use according to any one of claims 3-7, wherein
L is -O-
A is a ring selected from the group consisting of 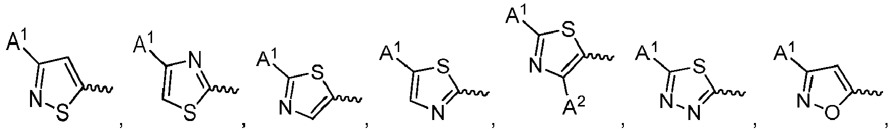 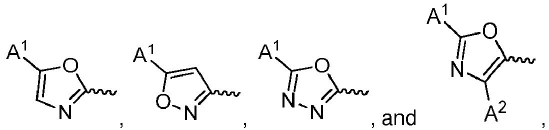 A¹ is selected from the group consisting of methyl, -CF₃, isopropyl, cyclopropyl, isobutyl, sec-butyl, tert-butyl, 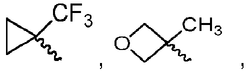 A² is -H,
B is a ring system selected from the group consisting of 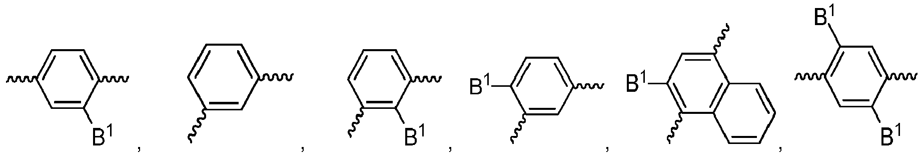 and 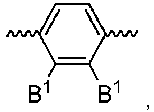 wherein, when B is a phenyl, B¹ is selected from the group consisting of -SCH₃, -SCH₂CH₃, -CF₃, -OCH₃, and -OCF₃ and, when B is a naphtyl, B¹ is -H, and
C is selected from the group consisting of 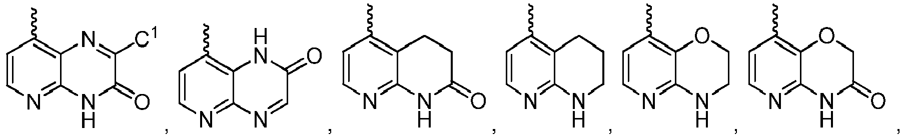 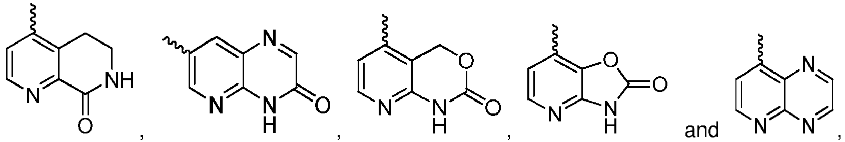 wherein C¹ is selected from the group consisting of -H, -CH₃, -OCH₃, -CN or 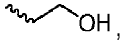 or
C is selected from the group consisting of 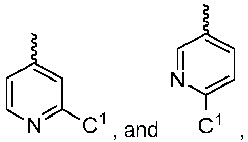 with C¹ being selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₆-C₁₄ aryl, C₃-C₁₃ heteroaryl, and C₇-C₂₄ alkaryl, wherein each hydrogen in each of these groups may be independently replaced by a group selected from halogen, hydroxy, -OR', and -NR'R", each hydrogen in each of R' and R" may be independently replaced by -COR''' or COOR''', wherein R', R" and R''' are independently a C₁-C₆ alkyl.

10. The compound for use according to any one of claims 1 and 3-7, the pharmaceutical composition according to any one of claims 2 and 6-7 or the pharmaceutical composition for use according to any one of claims 3-7, wherein
L is -O-
A is a ring selected from the group consisting of 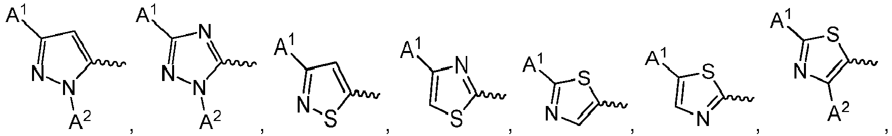 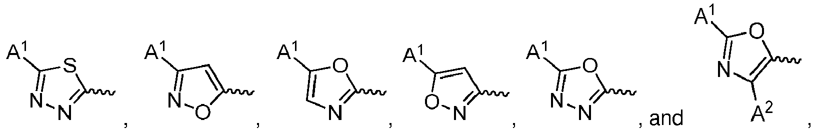 A¹ is tert-butyl,
A² is a phenyl or 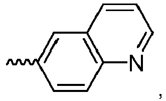 optionally substituted with 1, 2 or 3 substituents selected from the group consisting of C₁-C₄ alkyl, -Cl, -F, -CN, -OR^{ABC}, and -SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 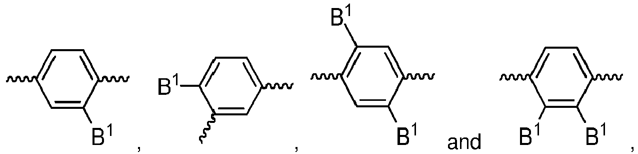 wherein B¹ is selected from the group consisting of -CF₃, -OCF₃, -OCH₃, and SCH₂CH₃, or B has two B¹ groups selected independently from the group consisting of -F, -CI, -SCH₃, -SCH₂CH₃, -CH₃, isopropyl, - CF₃, -OCH₃, -OCF₃,
C is selected from the group consisting of 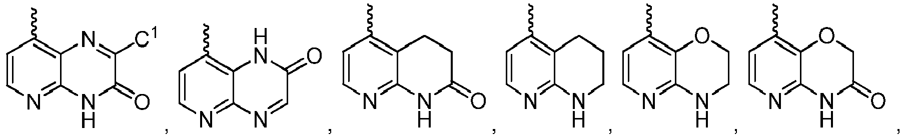 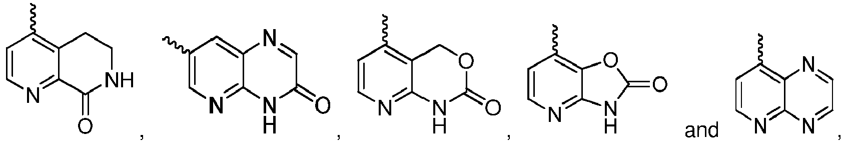 wherein C¹ is selected from the group consisting of -H, -CH₃, -OCH₃, -CN or 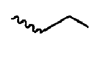 or
C is selected from the group consisting of 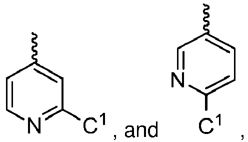 with C¹ being selected from the group consisting of -H, -NH₂, 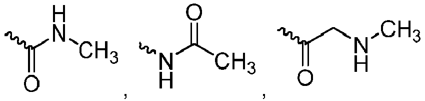 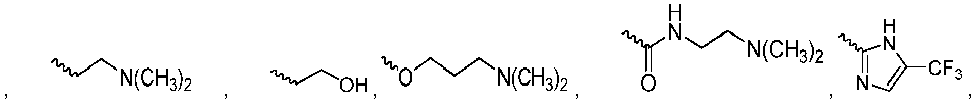 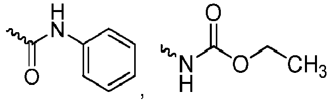 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH₃; preferably from the group consisting of 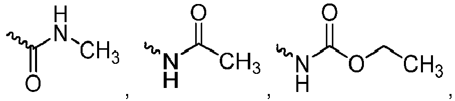 -NH-C(O)OtBu, -NH- C(O)N(CH₃)-C(O)OtBu, -NH-C(O)-CH₂-N(CH₃)-C(O)OtBu, -NH-C(O)-CH₂-NHCH₃, -NH-C(O)-Obenzyl, and -NH-C(O)-CH₂-OH.

11. The compound for use according to any one of claims 1 and 3-7, the pharmaceutical composition according to any one of claims 2 and 6-7 or the pharmaceutical composition for use according to any one of claims 3-7, wherein
A is 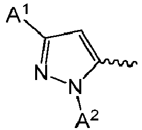 A¹ is selected from the group consisting of methyl, -CF₃, isopropyl, cyclopropyl, isobutyl, sec-butyl, tert-butyl, 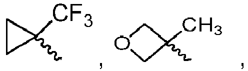 and A² is selected from the group consisting of a phenyl substituted with 1, 2 or 3 substituents selected from the group consisting of methoxy, -CI, -F, -CN, -O-(CH₂)₂-piperidinyl, -O-(CH₂)₂-morpholinyl, -O-(CH₂)₃-N(CH₃)₂, and -CH₂-morpholinyl; a phenyl or pyridinyl substituted with 2 or 3 methyls, and a pyridinyl substituted with 1, 2 or 3 substituents selected from the group consisting of methyl, -CI, -F, -CN, -O-(CH₂)₂-piperidinyl, -O-(CH₂)₂-morpholinyl, -O-(CH₂)₃-N(CH₃)₂, and -CH₂-morpholinyl;
B is a ring system selected from the group consisting of 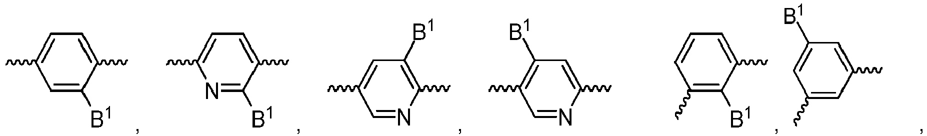 and 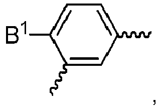 wherein B¹ is -SCH₃,
C is selected from the group consisting of 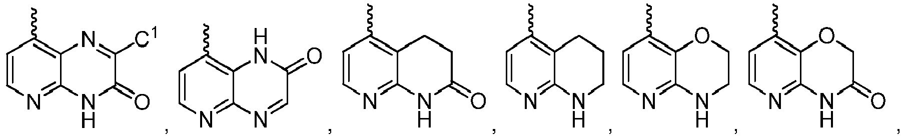 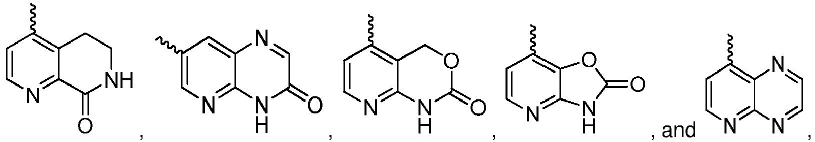 wherein C¹ is selected from the group consisting of -H, -CH₃, -OCH₃, -CN or

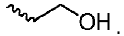

12. The compound for use according to any one of claims 1 and 3-7, the pharmaceutical composition according to any one of claims 2 and 6-7 or the pharmaceutical composition for use according to any one of claims 3-7, wherein
L is -O-
A is 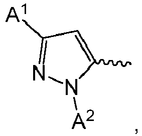 A¹ is selected from the group consisting of methyl, -CF₃, isopropyl, cyclopropyl, isobutyl, sec-butyl, tert-butyl, 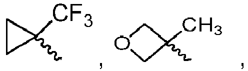 A² is 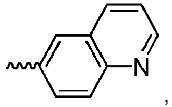 B is a ring system selected from the group consisting of 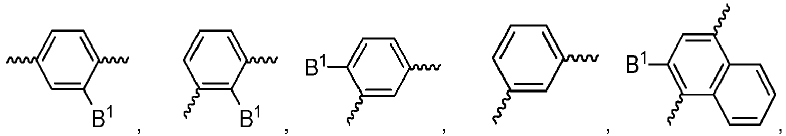 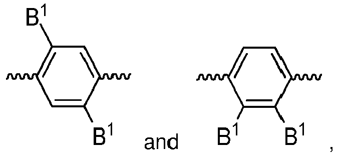 wherein, when B is a phenyl, B¹ is selected from the group consisting of -F, -Cl, -SCH₃, -SCH₂CH₃, -CH₃,-CF₃, -OCH₃, and -OCF₃ and, when B is a naphtyl, B¹ is -H, and
C is selected from the group consisting of 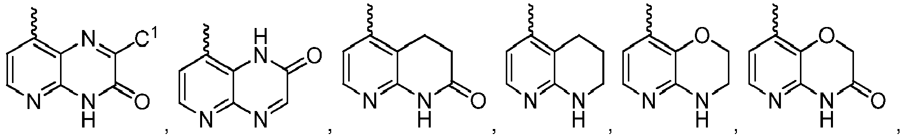 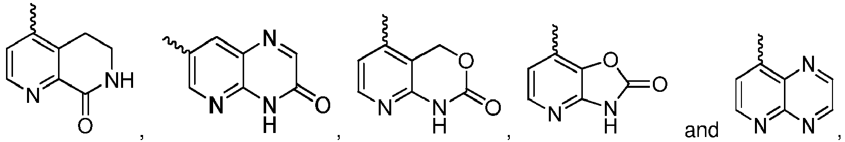 wherein C¹ is selected from the group consisting of -H, -CH₃, -OCH₃, -CN or

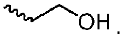

13. The compound for use according to any one of claims 1 and 3-7, the pharmaceutical composition according to any one of claims 2 and 6-7 or the pharmaceutical composition for use according to any one of claims 3-7, wherein
L is -O-
A is a ring selected from the group consisting of 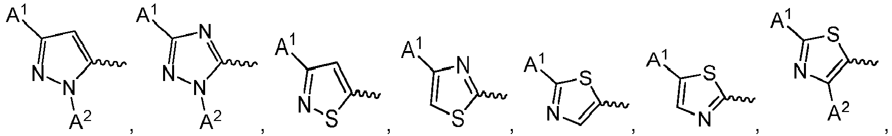 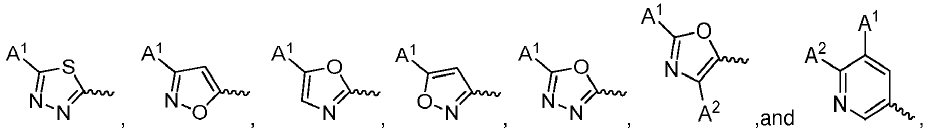 A¹ is selected from the group consisting of methyl, -CF₃, isopropyl, cyclopropyl, isobutyl, sec-butyl, tert-butyl, 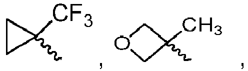 and A² is selected from the group consisting of H, methyl, iso-propyl, benzyl, phenyl, chlorine, 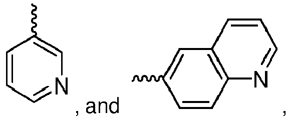 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and-SO₂R^{ABC}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 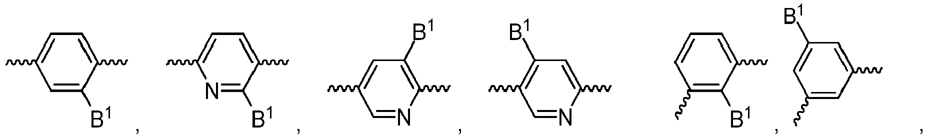 and 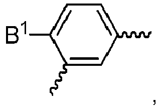 wherein B¹ is -SCH₃,
C is selected from the group consisting of 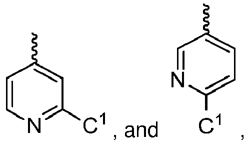 with C¹ being selected from the group consisting of -H, -NH₂, -NR¹, -C(O)-NH-R¹, -NH-C(O)-R¹, -NH-S(O)₂-R¹, -NH-C(O)-OR¹, -C(O)-R¹, -R¹, -OR¹, -SO₂R¹, with R¹ being selected from the group consisting of C₁-C₁₀ alkyl, C₆-C₁₄ aryl, C₃-C₁₃ heteroaryl, and C₇-C₂₄ alkaryl, wherein each hydrogen in each of these groups may be independently replaced by a group selected from a halogen, a hydroxy, -OR', and -NR'R", each hydrogen in each of R' and R" may be independently replaced by -COR''' or COOR''', wherein R', R" and R''' are independently a C₁-C₆ alkyl.

14. The compound for use according to any one of claims 1 and 3-7, the pharmaceutical composition according to any one of claims 2 and 6-7 or the pharmaceutical composition for use according to any one of claims 3-7, wherein
A is a ring system selected from the group consisting of 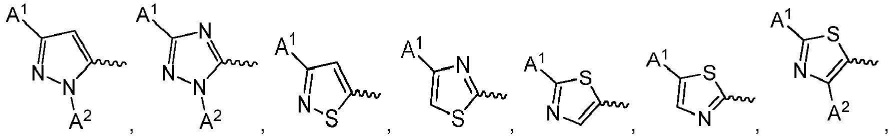 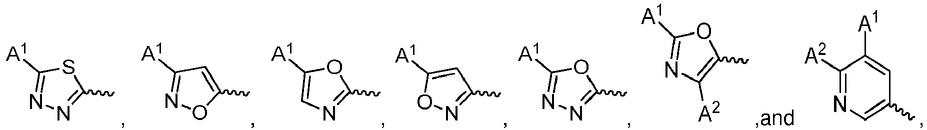 A¹ is selected from the group consisting of methyl, -CF₃, isopropyl, cyclopropyl, isobutyl, sec-butyl, tert-butyl, 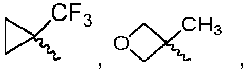 and A² is selected from the group consisting of H, methyl, iso-propyl, benzyl, phenyl, chlorine, 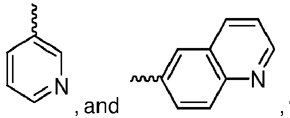 wherein A¹ and A² are optionally fluorinated, and A² is optionally substituted with 1, 2 or 3 substituents selected from the group consisting of C₁-C₄ alkyl, -Cl, -F, -CN, -OR^{ABC}, and-SO₂R^{abc}, wherein R^{ABC} is C₁-C₁₀ alkyl, and the substituent being optionally substituted by C₃-C₇ heterocycloalkyl or NRR, with R being a C₁-C₃ alkyl,
B is a ring system selected from the group consisting of 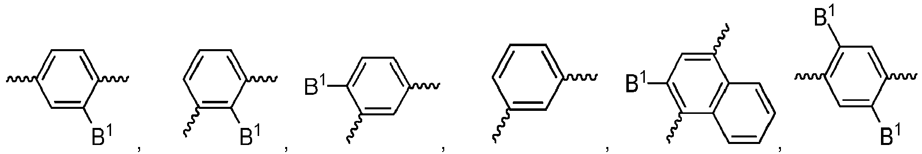 and 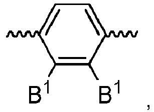 wherein, when B is a phenyl, B¹ is selected from the group consisting of -F, -Cl, -SCH₃, -SCH₂CH₃, -CH₃,-CF₃, -OCH₃, and -OCF₃ and, when B is a naphtyl, B¹ is -H, and
C is selected from the group consisting of 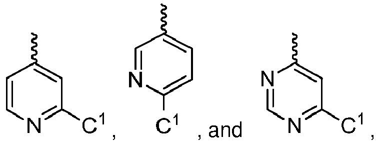 with C¹ being selected from the group consisting of -H, -NH₂, 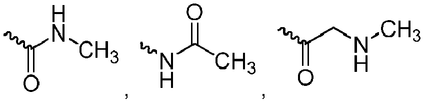 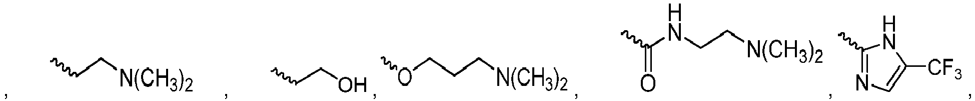 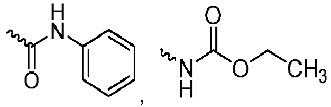 -NH-C(O)OtBu, -NH-C(O)N(CH3)-C(O)OtBu, -NH-C(O)-CH₂-N(CH₃)-C(O)OtBu, -NH-C(O)-CH₂-NHCH₃, -NH-C(O)-Obenzyl, -NH-C(O)-CH₂-OH, -NH-(CH₂)₂-N(CH₃)₂, and -NH-S(O)₂-CH₃; preferably from the group consisting of 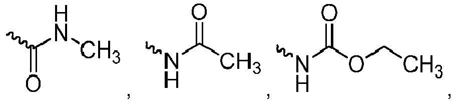 -NH-C(O)OtBu, -NH- C(O)N(CH₃)-C(O)OtBu, -NH-C(O)-CH₂-N(CH₃)-C(O)OtBu, -NH-C(O)-CH₂-NHCH₃, -NH-C(O)-Obenzyl, and -NH-C(O)-CH₂-OH.

15. The compound for use according to any one of claims 1 and 3-7, the pharmaceutical composition according to any one of claims 2 and 6-7 or the pharmaceutical composition for use according to any one of claims 3-7, wherein the compound is a compound selected in the compounds disclosed in Table 1 or a pharmaceutically acceptable salt thereof.
